# FASCICULE DE BREVET EUROPEEN

(11) **EP 2 411 009 B1**
(45) Date de publication et mention de la délivrance du brevet: **20.03.2013**
(21) Numéro de dépôt: 10715983.2
(22) Date de dépôt: 25.03.2010
(51) Int. Cl.: A61K 31/517, A61P 25/00, A61P 25/16, A61P 25/02, A61P 25/14, A61P 25/28, A61P 25/30, A61P 25/08, A61P 25/18, A61P 25/22, A61P 25/24

(54) **Applications thérapeutiques de dérivés de quinazolinedione**
Therapeutische Anwendungen von Chinazolindion-Derivaten
Therapeutic uses of quinazolinedione derivatives

(30) Priorité: 27.03.2009 FR 0901460
(43) Date de publication de la demande: 01.02.2012
(73) Titulaire: SANOFI, 75008 Paris (FR)
(72) Inventeur: MARCINIAK, Gilbert, F-75013 Paris (FR); NAVE, Jean-François, F-75013 Paris (FR); VIVIANI, Fabrice, F-75013 Paris (FR)
(74) Mandataire: Veinante, Aude
(86) Numéro de dépôt international: PCT/FR2010/050549
(87) Numéro de publication internationale: WO 2010/109148

(56) Documents cités:
- EP-A- 1 348 701
- WO-A-2006/003148
- WO-A-2008/119057
- WO-A-2009/077680
- PÉREZ-TORRES S ET AL: "Alterations on phosphodiesterase type 7 and 8 isozyme mRNA expression in Alzheimer's disease brains examined by in situ hybridization." EXPERIMENTAL NEUROLOGY AUG 2003, vol. 182, no. 2, août 2003 (2003-08), pages 322-334, XP002552388 ISSN: 0014-4886
- LOWE J A ET AL: "Structure-Activity Relationship of Quinazolinedione Inhibitors of Calcium-Independant Phosphodiesterase" JOURNAL OF MEDICINAL CHEMISTRY, AMERICAN CHEMICAL SOCIETY, WASHINGTON, US, vol. 34, no. 2, 1 janvier 1991 (1991-01-01), pages 624-628, XP002483510 ISSN: 0022-2623

## Description

L'invention a pour objet l'utilisation de dérivés de quinazolinedione comme médicaments ou pour fabriquer un médicament destiné(s) au traitement et/ou à la prévention de désordre(s) lié(s) au système nerveux central (abrégé SNC) et/ou lié(s) au système nerveux périphérique (abrégé SNP). L'invention concerne plus particulièrement l'utilisation de dérivés de quinazolinedione comme médicaments ou pour fabriquer un médicament destiné(s) au traitement et/ou à la prévention de désordre(s) psychiatrique(s) et/ou destiné(s) au traitement et/ou à la prévention de désordre(s) neurologique(s).

Le système nerveux central ou SNC comprend l'encéphale, la moelle épinière et les nerfs crâniens.

Le système nerveux périphérique ou SNP est la partie du système nerveux formée de ganglions et de nerfs qui fait circuler l'information entre les organes et le système nerveux central ou SNC et qui réalise les commandes motrices de ce dernier. Il comprend le système nerveux somatique et le système nerveux autonome.

L'invention concerne des dérivés de quinazolinedione, inhibiteurs de la phosphodiestérase 7 (PDE7). Certains de ces dérivés inhibent également la phosphodiestérase 8 (PDE8).

Les phosphodiestérases (PDEs) sont des enzymes intracellulaires responsables de l'hydrolyse des messagers secondaires AMPc (adénosine-3',5'-monophosphate cyclique) et GMPc (guanosine-3',5'-monophosphate cyclique) en nucléotides 5'-monophosphates inactifs. L'AMPc et le GMPc jouent un rôle essentiel dans les voies de signalisation cellulaire et interviennent dans de nombreux processus physiologiques.

L'inhibition des phosphodiestérases se traduit par une augmentation des concentrations intracellulaires d'AMPc et de GMPc, produisant l'activation spécifique de voies de phosphorylation impliquées dans des réponses fonctionnelles variées. L'augmentation des concentrations intracellulaires en AMPc ou en GMPc à l'aide d'inhibiteurs sélectifs de phosphodiestérases apparait comme une approche prometteuse pour le traitement de diverses maladies (Bender et Beavo, Pharmacol Rev (2006) 58, 488-520). Les inhibiteurs de phosphodiestérases présentent donc un intérêt comme agents thérapeutiques et comme outils pharmacologiques.

A ce jour, onze familles de phosphodiestérases ont été identifiées. Elles se distinguent par leur structure primaire, leur spécificité de substrat et leur sensibilité vis-à-vis de divers effecteurs et inhibiteurs spécifiques de PDEs. Chaque famille est constituée d'un ou plusieurs gènes qui sont exprimés dans différents tissus sous forme de variants d'épissage (Bender et Beavo, Pharmacol Rev (2006) 58, 488-520; Lugnier, Pharmacol Therapeut (2006) 109, 366-398).

Les PDE4, 7, et 8 hydrolysent spécifiquement l'AMPc et les PDE5, 6 et 9 le GMPc.

La famille PDE7 est représentée par les isoformes PDE7A et PDE7B, provenant de deux gènes distincts.

La PDE7A humaine (Michaeli et al, J Biol Chem (1993) 268, 12925-12932; Han et al, J Biol Chem (1997) 272, 16152-16157 ; Wang et al, Biochem Biophys Res Commun (2000) 276, 1271-1277) et la PDE7B humaine (Sasaki et al, Biochem Biophys Res Commun (2000), 271, 575-583 ; Gardner et al, Biochem Biophys Res Commun (2000) 272, 186-192) hydrolysent sélectivement l'AMPc avec des constantes de Michaelis (Km) de 0.1 à 0.2 µM et de 0.13 à 0.2 µM, respectivement. La partie catalytique de la PDE7B présente environ 67% d'homologie avec celle de la PDE7A.

Trois variants d'épissage sont connus pour la PDE7A. Les PDE7A1 et PDE7A3 sont exprimées principalement dans les cellules du système immunitaire et des poumons alors que la PDE7A2 est surtout exprimée dans les muscles du squelette, le coeur et les reins. Pour la PDE7B, trois variants ont également été récemment identifiés (Giembycz et Smith, Drugs Future (2006) 31, 207-229).

Les profils de distribution tissulaire de PDE7A et PDE7B sont très différents, suggérant que ces deux isoformes ont des fonctions distinctes du point de vue physiologique. Alors que la PDE7A est abondamment exprimée dans les cellules hématopoïétiques, les poumons, le placenta, les cellules de Leydig, la rate, les tubes collecteurs des reins et les surrénales, une forte expression de PDE7B est détectée dans le pancréas, le coeur, la thyroïde et les muscles du squelette (Giembycz et Smith, Drugs Future (2006) 31, 207-229). Une co-expression des RNA messagers (RNAm) de la PDE7A et de la PDE7B est cependant observée dans certains tissus. C'est le cas dans les ostéoblastes (Ahlstrom et al, Cell Mol Biol Lett (2005) 10, 305-319) et dans certaines régions du cerveau : plusieurs zones du cortex, le gyrus denté, la plupart des composants du système olfactif, le striatum, de nombreux noyaux du thalamus et les cellules pyramidales de l'hippocampe (Miro et al, Synapse (2001) 40, 201-214; Reyes-Irisarri et al, Neuroscience (2005) 132, 1173-1185). Par contre, dans certaines zones du cerveau, seulement une des deux isoformes est exprimée. Ainsi, seuls les RNAm de PDE7A sont présents dans de nombreux noyaux du tronc cérébral. De même, les RNAm de PDE7B sont présents en fortes concentrations dans le noyau accumbens et le noyau dorsal moteur du nerf vague alors que les RNAm de PDE7A n'y sont pas détectés (Miro et al, Synapse (2001) 40, 201-214; Reyes-Irisarri et al, Neuroscience (2005) 132, 1173-1185).

Le document WO2008/119057 décrit une méthode de traitement d'anomalies de mouvement associées à une pathologie de désordre neurologique de mouvement, telle que la maladie de Parkinson, la méthode de traitement comprenant l'administration à un patient d'une quantité d'un agent inhibiteur de PDE7, efficace pour inhiber l'activité enzymatique de PDE7.

Le document J. Med. Chem., 1991, 34, 624-628 divulgue l'utilisation de quinazoline 2,4- dione pour traiter la dépression. Le document WO 2006/003148 divulgue l'utilisation de quinazoline 2,4-dione pour traiter des maladies neurodégénératives.

La présente invention a en particulier pour objet des applications thérapeutiques de dérivés de quinazolinedione, pouvant s'avérer de puissants inhibiteurs de PDE7, ou de PDE7 et de PDE8 selon les dérivés.

L'invention concerne l'utilisation d'un composé répondant à la formule générale (I), suivante: 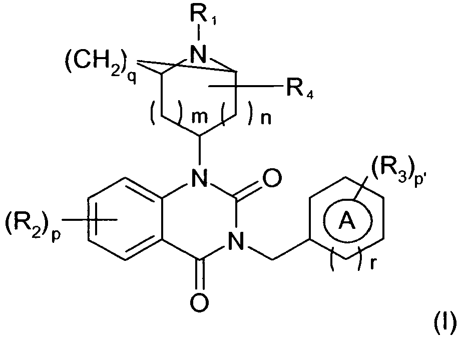 dans laquelle
- A représente un groupe aryle ou un groupe hétéroaryle;
- R₁ représente :
   ■ un atome d'hydrogène,
   ■ -C(O)R dans lequel R est un atome d'hydrogène, un groupe (C₁-C₆) alkoxy, un groupe aryle, un groupe (C₃-C₆) cycloalkyle, ou un groupe (C₁-C₆) alkyle, ledit alkyle étant éventuellement substitué par :
      . un ou plusieurs groupe(s) hydroxy,
      . un groupe benzyloxy,
      . un groupe (C₁-C₆) alkoxy, éventuellement substitué par un aryle, ou
      . un groupe (C₃-C₆) cycloalkyle,
   ■ un groupe (C₁-C₆) alkyle éventuellement substitué;
- R₂ représente :
   ■ un atome d'hydrogène,
   ■ un atome d'halogène,
   ■ un groupe cyano,
   ■ un groupe nitro,
   ■ un groupe (C₁-C₆) alkyle éventuellement substitué par un -NH₂, ou bien par un groupe -NHC(O)Rb, avec Rb tel que défini plus bas,
   ■ un groupe -ORa dans lequel Ra représente :
      . un atome d'hydrogène,
      . un groupe (C₁-C₆) alkyle éventuellement substitué par un ou plusieurs atome(s) d'halogène, par un ou plusieurs groupe(s) hydroxy, par un groupe aryle et/ou par un ou plusieurs groupe(s) cyano,
      . un groupe (C₂-C₆) alcynyle,
      . un groupe aryle ;
- R₃ représente :
   ■ un atome d'hydrogène,
   ■ un atome d'halogène,
   ■ un groupe hydroxy,
   ■ un groupe cyano,
   ■ un groupe -SCF₃,
   ■ un groupe nitro,
   ■ un groupe oxo,
   ■ un groupe -S(O)₀₋₂-alkyle, un groupe -S(O)₀₋₂-hétérocycloalkyle, un groupe - O- SO₂-aryle éventuellement substitué par un ou plusieurs atome(s) d'halogène ;
   ■ un groupe -alkyl-amino-alkyle ou -cycloalkyl-amino-alkyle, chacun éventuellement substitué sur l'alkyle terminal,
   ■ un groupe sulfonamide éventuellement substitué,
   ■ un groupe aryle ou un groupe hétéroaryle, ledit groupe étant monocyclique ou polycyclique et étant de plus éventuellement substitué par un groupe (C₁-C₆) alkyle, par un ou plusieurs atome(s) d'halogène, ou par un groupe (C₁-C₆) alkoxy,
   ■ un groupe hétérocycloalkyle éventuellement substitué par un groupe (C₁-C₆) alkyle,
   ■ un groupe (C₁-C₆) alkyle éventuellement substitué par :
      - un ou plusieurs atome(s) d'halogène,
      - un groupe aryle pouvant être substitué par un ou plusieurs atome(s) d'halogène, ou par un ou plusieurs groupe(s) hydroxy,
      - un groupe hétéroaryle,
      - un ou plusieurs groupe(s) hydroxy pouvant être substitué(s) par un groupe aryle lui-même éventuellement substitué par un ou plusieurs atome(s) d'halogène, ou
      - un groupe hétérocycloalkyle éventuellement sunstitué par un groupe CO(O)Ra, ou par un groupe (C₁-C₆) alkyle, Ra étant tel que défini précédemment ;
   ■ un groupe -C(O)NRbRc, avec Rb et Rc étant tels que définis plus bas,
   ■ un groupe -C(O)ORc, ou un groupe -O-C(O)ORc, avec Rc étant tel que défini plus bas,
   ■ un groupe (C₁-C₆) alkoxy, éventuellement substitué par
      - un groupe amino-alkyle,
      - un groupe amino-cycloalkyle,
      - un groupe cycloalkyle,
      - un groupe hétérocycloalkyle
      - un groupe hétéroaryle monocyclique ou polycyclique,
      - un ou plusieurs groupement(s) hydroxy,
      - un ou plusieurs atome(s) d'halogène,
      - un groupe (C₁-C₆) alkoxy,
      - un groupe -C(O)ORc, avec Rc étant tel que défini plus bas,
      - un groupe -C(O)NRbRc, avec Rb et Rc étant tels que déinfis plus bas,
      - un groupe oxo, et/ou
      - un groupe aryle, lui-même éventuellement substitué par un ou plusieurs atome(s) d'halogène, un groupe cyano, un groupe (C₁-C₆) alkoxy, un groupe -O- halogénoalkyle et/ou par un groupe halogénoalkyle,
   ■ un groupe -O-cycloalkyle, un groupe -O-aryle, ou un groupe -O-hétérocycloalkyle, chacun éventuellement substitué par
      - un groupe aryle, lui-même éventuellement substitué par un ou plusieurs atome(s) d'halogène, ou par un groupe (C₁-C₆) alkyle,
      - un groupe oxo,
      - un ou plusieurs atome(s) d'halogène, et/ou
      - un groupe (C₁-C₆) alkyle, lui-même pouvant être substitué par un groupe aryle et/ou un groupe oxo,
   ■ un groupe -NH-CO-NH-aryle, un groupe -NH-CO-NH-hétéroaryle, ou un groupe -NH-CO-NH-(C₁-C₆) alkyle, chacun étant éventuellement substitué par un ou plusieurs atome(s) d'halogène, par un groupe cyano, par un groupe nitro, par un ou plusieurs groupe(s) hydroxy, ou par un groupe (C₁-C₆) alkoxy,
   ■ un groupe -N-(C₁-C₆) alkyle, le groupe (C₁-C₆) alkyle pouvant être substitué par
      - un ou plusieurs groupe(s) oxo, et/ou
      - un ou plusieurs groupe(s) aryle éventuellement substitué(s) par un ou plusieurs atome(s) d'halogène et/ou par un groupe SO₂,
   ■ un groupe -NH-CO-aryle, un groupe -NH-CO-hétéroaryle, chacun étant éventuellement substitué par un ou plusieurs atome(s) d'halogène ;
   ou bien R₃ forme avec A un groupe hétéroaryle polycyclique éventuellement substitué par un groupe (C₁-C₆) alkoxy, un groupe (C₁-C₆) alkyle éventuellement substitué par un groupe aryle pouvant lui-même être substitué par un ou plusieurs atome(s) d'halogène ;
- R₄ représente un atome d'hydrogène, un groupe oxo, ou un groupe (C₁-C₆) alkyle ;
- Rb représente :
   . un atome d'hydrogène,
   . un groupe (C₁-C₆) alkyle éventuellement substitué par un ou plusieurs atome(s) d'halogène, par un ou plusieurs groupe(s) hydroxy, cyano, amino, hétérocycloalkyle, (C₁-C₆) alkoxy, ou par un groupe aryle éventuellement substitué par un ou plusieurs atome(s) d'halogène,
   . un groupe (C₃-C₆) cycloalkyle,
   . un groupe (C₂-C₆) alcynyle,
   . un groupe (C₁-C₆) alkoxy,
   . un groupe aryle éventuellement substitué par un ou plusieurs atome(s) d'halogène;
- Rc représente un atome d'hydrogène, ou un groupe (C₁-C₆) alkyle éventuellement substitué par un ou plusieurs atome(s) d'halogène ;
   ou alors Rb et Rc forment ensemble avec l'atome d'azote auquel ils sont attachés un groupe hétéroaryle polycyclique, ou un groupe hétérocycloalkyle ;
- m et n représentent indépendamment l'un de l'autre la valeur 0, 1 ou 2, étant entendu que m+n ≤ 3 ;
- p et p' représentent indépendamment l'un de l'autre la valeur 1, 2 ou 3, étant entendu que lorsque p est supérieur ou égal à 2, alors les groupes R₂ sont sur des atomes de carbone distincts et peuvent être différents les uns des autres, et lorsque p' est supérieur ou égal à 2 alors les groupes R₃ sont sur des atomes de carbone distincts et peuvent être différents les uns des autres;
- q représente la valeur 0 ou 2, étant entendu que lorsque q = 0, alors le groupe hétérocyclique azoté rattaché à l'azote situé en position 1 du noyau 2,4-dioxo-1,2,3,4 tétrahydroquinazoline n'est plus ponté et est du type : 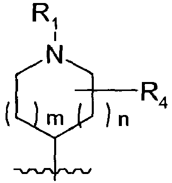 avec R1, R4, m et n tels que définis précédemment,
   - r représente la valeur 0 ou 1,
   comme médicament ou pour la préparation d'un médicament destiné(s) au traitement et/ou à la prévention des désordres liés au système nerveux central et/ou liés au système nerveux périphérique.

Selon un mode de réalisation, lesdits désordres sont choisis parmi les désordres psychiatriques et les désordres neurologiques.

Les composés de formule générale (I) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

De par leur structure, les composés de formule générale (I) peuvent également exister sous forme d'isomères de type rotamère ou d'atropoisomère.

Les composés de formule (I) peuvent également exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou la séparation des composés de formule générale (I) font également partie de l'invention.

Les composés de formule générale (I) peuvent également se trouver sous forme cristalline, amorphe ou huileuse, ces formes faisant partie de l'invention.

Les composés de formule générale (I) peuvent, en outre, se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Selon la présente invention, les N-oxydes des composés comportant une amine font également partie de l'invention.

Les composés de formule (I), conformes à l'invention, comprennent également ceux dans lesquels un ou plusieurs atomes d'hydrogène, de carbone ou d'halogène, notamment de chlore ou de fluor ont été remplacés par leurs isotopes radioactifs, par exemple le tritium pour remplacer l'hydrogène ou le carbone 14 pour remplacer le carbone 12. De tels composés marqués sont utiles dans des travaux de recherche, de métabolisme ou de pharmacocinétique, dans des essais biologiques et pharmacologiques en tant qu'outils.

Dans le cadre de l'invention, on définit ce qui suit :
- dans (C₁-C₆), les indices numériques déterminent le nombre possible d'atomes de carbone présents dans une chaîne ou un cycle. Ainsi, à titre d'exemple, C₁-C₆ représente une chaîne carbonée pouvant avoir de 1 à 6 atomes de carbone. De même, à titre d'exemple, (C₁-C₅) représente une chaîne carbonée qui peut avoir de 1 à 5 atomes de carbone, ou encore (C₃-C₆) peut représenter un cycle carboné saturé pouvant avoir de 3 à 6 atomes de carbone ;
- alkoxy: un groupe -O-alkyle à chaîne aliphatique saturée, linéaire ou ramifiée ;
- alcynyle : un groupe aliphatique mono- ou poly-insaturé, linéaire ou ramifié, comprenant par exemple une ou deux insaturations acétyléniques. Par exemple, un groupe (C₂-C₆) alcynyle peut représenter un ethynyle, propynyle ...;
- alkyle : un groupe aliphatique saturé, linéaire ou ramifié; par exemple, un groupe (C₁-C₆) alkyle représente une chaîne carbonée de 1 à 6 atomes de carbone, linéaire ou ramifiée, notamment un méthyle, éthyle, propyle, isopropyle, butyle, isobutyle, secbutyle, tertbutyle, pentyle ;
- aminoalkyle : un groupe -NH(C₁-C₆) alkyle, ou encore -N((C₁-C₆) alkyle)₂ ;
- aryle : un système aromatique monocyclique éventuellement substitué comprenant de 5 à 14 chaînons par cycle, de préférence de 5 à 10 chaînons par cycle. A titre d'exemples de groupes aryles monocycliques, on peut citer le phényle, ou le naphtyle ; le groupe aryle peut être substitué par un groupe pouvant être un ou plusieurs atome(s) d'halogène, un groupe hydroxy, un groupe cyano, un groupe trifluorométhylthio, un groupe nitro, un groupe alkyle, un groupe alkoxy, un groupe alkylthio, un groupe méthylsulfonyle, un groupe alkyl-amino-alkyle ou alkyl-amino-cycloalkyle éventuellement substitué, un groupe alkyl-amino-alkoxy ou cycloalkyl-amino-alkoxy ou un groupe sulfonamide, par exemple ;
- aryle polycyclique : un système aromatique polycyclique éventuellement substitué comprenant de 5 à 14 chaînons par cycle, de préférence de 5 à 10 chaînons par cycle, et comprenant de 2 à 10 cycles, dont au moins un des cycles est aromatique. A titre d'exemple de groupe aryles polycycliques, on peut citer l'acéanthrylène, l'anthracène, l'azulène, le coronène, le rubicène, le naphtalène ; le groupe aryle polycyclique peut être substitué par un groupe pouvant être un ou plusieurs atome(s) d'halogène, un groupe hydroxy, un groupe cyano, un groupe trifluorométhylthio, un groupe nitro, un groupe alkyle, un groupe alkoxy, un groupe alkylthio, un groupe méthylsulfonyle, un groupe alkyl-amino-alkyle ou alkyl-amino-cycloalkyle éventuellement substitué, un groupe alkyl-amino-alkoxy ou cycloalkyl-amino-alkoxy ou un groupe sulfonamide, par exemple ;
- un cycle ponté : une structure bicyclique, comprenant selon l'invention un atome d'azote, dans laquelle au moins 2 atomes de carbone sont reliés par une liaison simple ou une chaîne carbonée pouvant comporter 2 atomes de carbone. A titre d'exemple, le cycle précité est du type : 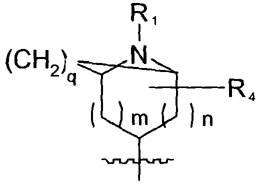 avec q = 1 ou 2 et avec les autres groupes et indices tels que définis plus haut ;
- cycloalkyle : un groupe aliphatique cyclique saturé comprenant de 3 à 8 atomes de carbone. A titre d'exemple, on peut citer les groupes cyclopropyle, méthylcyclopropyle, cyclobutyle, cyclopentyle, cyclohexyle ;
- halogène : un fluor, un chlore, un brome ou un iode ;
- halogénoalkyle : (C₁-C₆) alkyle substitué par un à trois atome(s) d'halogène;
- hétéroaryle : un système aromatique monocyclique comprenant de 5 à 14 chaînons, de préférence de 5 à 10 chaînons et comprenant un à plusieurs hétéroatomes tels que les atomes d'azote, d'oxygène ou de soufre. Les atomes d'azote peuvent être sous forme de N-oxydes. Par exemple, un hétérocycle monocyclique peut être un pyrane, une pyrazine, un pyrazole, une pyridazine, une pyridine, une pyrimidine, un pyrrole, un isothiazole, un isoxazole, un furane, un imidazole, une morpholine, un thiophène, une pipérazine, une diazétidine, une dihydropyrrolidine, une pipéridine, ou une azépine, etc ; un hétérocycle bicyclique peut être une isoquinoléine, une ptéridine, un chromane, etc ; un hétérocycle tricyclique peut être une phénanthroline, un xanthène, etc
- hétéroaryle polycyclique : un système aromatique polycyclique éventuellement substitué comprenant de 5 à 14 chaînons par cycle, de préférence de 5 à 10 chaînons par cycle, et comprenant de 2 à 10 cycles, comprenant de plus un à plusieurs hétéroatomes tels que les atomes d'azote, d'oxygène ou de soufre sur au moins l'un des cycles, et dont au moins des cycles est aromatique. A titre d'exemple de groupe hétéroaryles polycycliques, on peut citer l'indole, le benzofurane, le benzimidazole, le benzothiophène, le benzotriazole, le benzothiazole, la benzoxazole, la quinoline, l'isoquinoline, l'indazole, la quinazoline, la phthalazine, la quinoxaline, la naphtyridine, le 2,3-dihydro-1H-indole, le 2,3-dihydro-benzofurane, le 2,3-dihydro-indène, la tétrahydroquinoline, la tétrahydroisoquinoline, la tétrahydroisoquinazoline ;
- un hétérocycloalkyle : un cycle saturé, éventuellement substitué, comprenant de 3 à 8 atomes et comprenant un à plusieurs hétéroatomes tels que les atomes d'azote, d'oxygène ou de soufre sur au moins l'un des cycles, ou plusieurs hétéroatomes identiques ou différents entre eux. Par exemple, un hétérocycloalkyle peut être une pyrrolidine, une morpholine, une pipérazine, une diazétidine, une dihydropyrrolidine, une pipéridine, une pipéradine, une azépane, une imidazolidine, thiomorpholine, tétrahydropyrane, tétrahydrothiopyrane, pipérazine, diazépane, etc ;
- hydroxy : un groupe -OH ;
- nitro : un groupe -NO₂ ;
- oxo : un groupe -C(O)- ;
- sulfonamide : groupe répondant à la formule SO₂-N-alkyl ou SO₂-N-cycloalkyl, alkyl et cycloalkyl étant tels que définis ci-dessus ;
- trifluorométhylthio est défini par la formule -S-CF₃,

Il est de plus entendu que dans la présente description, lorsqu'un atome ou un groupe est substitué ou éventuellement substitué par un ou plusieurs groupe(s) ou atome(s) définis, les substituants peuvent être identiques ou différents entre eux, et être portés le cas échéant par le même atome ou des atomes différents.

Parmi les composés objet de l'invention, on peut citer un groupe de composés de formule (I) dans laquelle A représente un groupe aryle, en particulier un groupe phényle ou hétéroaryle, en particulier un groupe pyridyle, et tous les autres substituants et indices sont tels que définis dans la formule générale (I) définie plus haut.

Parmi les composés conformes à l'invention, on peut citer un groupe de composés de formule (I) dans laquelle q = 0, m et n représente chacun 1, et tous les autres substituants et indices sont tels que définis dans la formule générale (I) définie plus haut.

Parmi les composés conformes à l'invention, on peut citer un groupe de composés de formule (I), dans laquelle R₂ représente un groupe (C₁-C₆) alkyle, en particulier un méthyle, substitué par un groupe -NHC(O)Rb dans lequel Rb, les autres substituants et indices sont tels que définis pour le composé de formule générale (I) définie plus haut.

Parmi les composés conformes à l'invention, on peut citer un groupe de composés de formule générale (I), dans laquelle R₂ représente un groupe -ORa, le groupe Ra et tous les autres substituants et indices étant tels que définis dans la formule générale (I) définie plus haut.

Parmi les composés conformes à l'invention, on peut citer un groupe de composés de formule générale (I), dans laquelle R₂ est un atome d'halogène, ou un cyano, ou un hydrogène, ou un hydroxyle, ou un (C₁-C₆) alkyle éventuellement substitué par un -NH₂, ou bien par un groupe -NHC(O)Rb, Rb et les autres substituants et indices étant tels que définis dans la formule générale (I) définie plus haut.

Parmi les composés conformes à l'invention, on peut citer un groupe de composés de formule générale (I), dans laquelle A est un phényle, R₁ est un groupe - C(O)R dans lequel R représente un atome d'hydrogène, q est égal à 0, n et m valent chacun 1, et R₂ représente -ORa, Ra et les autres substituants et indices étant tels que définis dans la formule générale (I) définie plus haut.

Parmi les composés conformes à l'invention, on peut citer un groupe de composés de formule générale (I), dans laquelle A est un phényle, R₁ est un groupe - C(O)R dans lequel R représente un atome d'hydrogène, q est égal à 0, n et m valent chacun 1, p est égal à 2, l'un des R₂ est -ORa et l'autre des R₂ est un atome d'halogène, Ra et les autres substituants et indices étant tels que définis dans la formule générale (I) définie plus haut.

Parmi les composés conformes à l'invention, on peut citer un groupe de composés de formule générale (I), dans laquelle A est un phényle, R₁ est un groupe - C(O)R dans lequel R représente un atome d'hydrogène, q est égal à 0, n et m valent chacun 1, p=1 et R₂ est un méthyle substitué par un groupe -NH-CO-Rb, Rb et les autres substituants et indices étant tels que définis dans la formule générale (I) définie plus haut.

Avantageusement, dans les composés de formule (I), le groupe R₂ est en position 6 du noyau 2,4-dioxo-1,2,3,4 tétrahydroquinazoline. Les composés de formule (I) peuvent également avoir un groupe R2 en position 7 du noyau 2,4-dioxo-1,2,3,4 tétrahydroquinazoline. Les groupes R2 en posiiton 6 et 7 peuvnet être identiques ou différents.

Avantageusement, dans les composés de formule (I), p est égal à 1 ou 2.

Les composés de formule générale (I) peuvent être à l'état de base, d'hydrate, de solvat, d'isomères ou de leurs mélanges.

Dans un mode particulier, lorsque p' = 2, alors les deux groupes R₃ sont en position 3 et 4 du noyau A et peuvent être différents l'un de l'autre.

Les combinaisons des groupes de composés conformes à l'invention précités font également partie de l'invention.

A titre d'exemple de composés préférés conformes à l'invention, on peut citer les composés suivants :
n°1 : 2-{[3-(3,4-diméthoxybenzyl)-1-(1-formylpipéridin-4-yl)-2,4-dioxo-1,2,3,4-tétrahydroquinazolin-6-yl]oxy}propanenitrile
n°2 : 1-(1-acétylpipéridin-4-yl)-3-(3,4-diméthoxybenzyl)-6-hydroxyquinazoline-2,4(1H,3H)-dione
n°3 : {[1-(1-acétylpipéridin-4-yl)-3-(3,4-diméthoxybenzyl)-2,4-dioxo-1,2,3,4-tétrahydroquinazolin-6-yl]oxy}acétonitrile
n°4 : 2-{[1-(1-acétylpipéridin-4-yl)-3-(3,4-diméthoxybenzyl)-2,4-dioxo-1,2,3,4-tétrahydroquinazolin-6-yl]oxy}propanenitrile
n°6 : {[3-(3,4-diméthoxybenzyl)1-(1-formylpipéridin-4-yl)-2,4-dioxo-1,2,3,4-tétrahydroquinazolin-6-yl]oxy}acétonitrile
n°11 : 4-[3-(3,4-diméthoxybenzyl)6-[2-fluoro-1-(fluorométhyl)éthoxy]-2,4-dioxo-3,4-dihydroquinazolin-1(2H)-yl]pipéridine-1-carbaldéhyde
n°12 :1-(1-acétylpipéridin-4-yl)-3-(3,4-diméthoxybenzyl)-6-[2-fluoro-1-(fluorométhyl)éthoxy]quinazoline-2,4(1H,3H)-dione
n°13 : 4-[3-(3,4-diméthoxybenzyl)-2,4-dioxo-6-(2,2,2-trifluoroéthoxy)3,4-dihydroquinazolin-1 (2H)-yl]pipéridine-1-carbaldéhyde
n°14 : 1-(1-acétylpipéridin-4-yl)-6-(2,2-difluoroéthoxy)-3-(3,4-diméthoxybenzyl)quinazoline-2,4(1H,3H)dione
n°16 :4-[6-(2,2-difluoroéthoxy)3-(3,4-diméthoxybenzyl)2,4-dioxo-3,4-dihydroquinazolin-1(2H)-yl]pipéridine-1-carbaldéhyde
n°20 : N-{[3-(3,4-diméthoxybenzyl)-1-(1-formylpipéridin-4-yl)-2,4-dioxo-1,2,3,4-tétrahydroquinazolin-6-yl]méthyl}acétamide
n°22 : chlorhydrate de 1-(1-acétylpipéridin-4-yl)-6-(aminométhyl)3-(3,4-diméthoxybenzyl)quinazoline-2,4(1H,3H)-dione
n°23 : N-{[3-(3,4-diméthoxybenzyl)-1-(1-formylpipéridin-4-yl)-2,4-dioxo-1,2,3,4-tétrahydroquinazolin-6-yl]méthyl}formamide
n°24 : N-{[1-(1-acétylpipéridin-4-yl)-3-(3,4-diméthoxybenzyl)-2,4-dioxo-1,2,3,4-tétrahydroquinazolin-6-yl]méthyl}formamide
n°25 : N-{[1-(1-acétylpipéridin-4-yl)-3-(3,4-diméthoxybenzyl)-2,4-dioxo-1,2,3,4-tétrahydroquinazolin-6-yl]méthyl}acétamide
n°32 : 4-[6-(2,2-difluoroéthoxy)-2,4-dioxo-3,4-dihydroquinazolin-1(2H)-yl]pipéridine-1-carbaldéhyde
n°33 : 4-[3-(3,4-dichlorobenzyl)-6-(2,2-difluoroéthoxy)-2,4-dioxo-3,4-dihydroquinazolin-1(2H)-yl]pipéridine-1-carbaldéhyde
n°34 : 4-[3-(4-chlorobenzyl)-6-(2,2-difluoroéthoxy)-2,4-dioxo-3,4-dihydroquinazolin-1(2H)-yl]pipéridine-1-carbaldéhyde
n°35 : 4-{[6-(2,2-difluoroéthoxy)-1-(1-formylpipéridin-4-yl)-2,4-dioxo-1,4-dihydroquinazolin-3(2H)-yl]méthyl)benzoate de méthyle
n°36 : acide 4-{[6-(2,2-difluoroéthoxy)-1-(1-formylpipéridin-4-yl)-2,4-dioxo-1,4-dihydroquinazolin-3(2H)-yl]méthyl}benzoïque
n°37 : 4-{[6-(2,2-difluoroéthoxy)-1-(1-formylpipéridin-4-yl)-2,4-dioxo-1,4-dihydroquinazolin-3(2H)-yl]méthyl}-N-(2-méthoxyéthyl)benzamide
n°38 : 4-[3-(3,4-diméthoxybenzyl)-6-méthyl-2,4-dioxo-3,4-dihydroquinazolin-1(2H)-yl]pipéridine-1-carbaldéhyde
n°39 : 4-[6-(2,2-difluoroéthoxy)-3-(3-hydroxy-4-méthoxybenzyl)2,4-dioxo-3,4-dihydroquinazolin-1(2H)-yl]pipéridine-1-carbaldéhyde
n°40 : 4-[6-(2,2-difluoroéthoxy)3-[3-(2-hydroxyéthoxy)-4-méthoxybenzyl]-2,4-dioxo-3,4-dihydroquinazolin-1(2H)-yl]pipéridine-1-carbaldéhyde
n°41 : 4-[6-(2,2-difluoroéthoxy)-3-(3-éthoxy-4-méthoxybenzyl)-2,4-dioxo-3,4-dihydroquinazolin-1(2H)-yl]pipéridine-1-carbaldéhyde
n°42 : 4-[6-(2,2-difluoroéthoxy)3-[4-méthoxy-3-(2-méthoxyéthoxy)benzyl]-2,4-dioxo-3,4-dihydroquinazolin-1(2H)-yl]pipéridine-1-carbaldéhyde
n°43 : 4-[6-(2,2-difluoroéthoxy)-3-(3,4-diméthoxybenzyl)-2,4-dioxo-3,4-dihydroquinazolin-1 (2H)-yl]azépane-1-carbaldéhyde
n°47 : 4-[6-(2,2-difluoroéthoxy)-3-[3-(3-hydroxypropoxy)-4-méthoxybenzyl]-2,4-dioxo-3,4-dihydroquinazolin-1(2H)-yl]pipéridine-1-carbaldéhyde
n°48 : 4-[5-chloro-3-(3,4-diméthoxybenzyl)-2,4-dioxo-3,4-dihydroquinazolin-1(2H)-yl]pipéridine-1-carbaldéhyde
n°49 : 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde
n°50 : 2-(5-{[6-(2,2-difluoroéthoxy)-1-(1-formylpipéridin-4-yl)-2,4-dioxo-1,4-dihydroquinazolin-3(2H)-yl]méthyl}-2-méthoxyphénoxy)acétamide
n°51 : 4-[6-(2,2-difluoroéthoxy)-3-(3,4-diméthoxybenzyl)-2,4-dioxo-3,4-dihydroquinazolin-1(2H)-yl]-3-méthylpipéridine-1-carbaldéhyde
n°52 : 3-[6-(2,2-difluoroéthoxy)-3-(3,4-diméthoxybenzyl)-2,4-dioxo-3,4-dihydroquinazolin-1(2H)-yl]-8-azabicyclo[3.2.1]octane-8-carbaldéhyde
n°56 : 4-{3-[4-(cyclopentyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°57 : 4-[3-(3-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°58 : 4-[3-(4-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°59 : 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n °72 : 4-[3-(3,4-diméthoxybenzyl)-6-(2-hydroxyéthoxy)2,4-dioxo-3,4-dihydroquinazolin-1(2H)-yl]pipéridine-1-carbaldéhyde
n°74 : 4-[3-(3,4-dichlorobenzyl)-6-[2-fluoro-1-(fluorométhyl)éthoxy]-2,4-dioxo-3,4-dihydroquinazolin-1 (2H)-yl]pipéridine-1-carbaldéhyde
n°76 : 4-{3-[(6-chloropyridin-3-yl)méthyl]-6-[2-fluoro-1-(fluorométhyl)éthoxy]-2,4-dioxo-3,4-dihydroquinazolin-1(2H)-yl}pipéridine-1-carbaldéhyde
n°78 : 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°79 : 4-[3-(3,4-diméthoxybenzyl)-6-(2-fluoroéthoxy)-2,4-dioxo-3,4-dihydroquinazolin-1(2H)-yl]pipéridine-1-carbaldéhyde
n°89: 2-[5-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]acétamide
n°90 : 4-{6-[2-fluoro-1-(fluorométhyl)éthoxy]-3-(3-hydroxy-4-méthoxybenzyl)-2,4-dioxo-3,4-dihydroquinazolin-1(2H)-yl}pipéridine-1-carbaldéhyde
n°91: 4-[3-(3,4-diméthoxybenzyl)-6-éthoxy-2,4-dioxo-3,4-dihydroquinazolin-1(2H)-yl]pipéridine-1-carbaldéhyde
n°97 : 4-[5,7-dichloro-3-(3,4-diméthoxybenzyl)-2,4-dioxo-3,4-dihydroquinazolin-1(2H)-yl]pipéridine-1-carbaldéhyde
n°102: 4-[7-chloro-3-(3,4-diméthoxybenzyl)-2,4-dioxo-3,4-dihydroquinazolin-1(2H)-yl]pipéridine-1-carbaldéhyde
n°108 : 4-{6-[2-fluoro-1-(fluorométhyl)éthoxy]-3-(3-fluoro-4-méthoxybenzyl)-2,4-dioxo-3,4-dihydroquinazolin-1(2H)-yl}pipéridine-1-carbaldéhyde
n°111 : 4-[6-(difluorométhoxy)-3-(3,4-diméthoxybenzyl)-2,4-dioxo-3,4-dihydroquinazolin-1(2H)-yl]pipéridine-1-carbaldéhyde
n°112 : 4-[3-(3,4-diméthoxybenzyl)-6-(1-méthyléthoxy)-2,4-dioxo-3,4-dihydroquinazolin-1(2H)-yl]pipéridine-1-carbaldéhyde
n°114 : 4-{6-[2-fluoro-1-(fluorométhyl)éthoxy]-3-[4-méthoxy-3-(1-méthyléthoxy)benzyl]-2,4-dioxo-3,4-dihydroquinazolin-1(2H)-yl}pipéridine-1-carbaldéhyde
n°116 : 4-{6-[2-fluoro-1-(fluorométhyl)éthoxy]-3-(3-méthoxybenzyl)-2,4-dioxo-3,4-dihydroquinazolin-1(2H)-yl}pipéridine-1-carbaldéhyde
n°117 : 4-{3-[3,5-bis(trifluorométhyl)benzyl]-6-[2-fluoro-1-(fluorométhyl)éthoxy]-2,4-dioxo-3,4-dihydroquinazolin-1(2H)-yl}pipéridine-1-carbaldéhyde
n°118 : 4-[3-(3-éthoxybenzyl)-6-[2-fluoro-1-(fluorométhyl)éthoxy]-2,4-dioxo-3,4-dihydroquinazolin-1 (2H)-yl]pipéridine-1-carbaldéhyde
n°124 : 4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°130 : 4-[3-(3,4-diéthoxybenzyl)-6-[2-fluoro-1-(fluorométhyl)éthoxy]-2,4-dioxo-3,4-dihydroquinazolin-1 (2H)-yl]pipéridine-1-carbaldéhyde
n°131 : 4-[3-(4-éthoxy-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°133 : 4-{6-[2-fluoro-1-(fluorométhyl)éthoxy]-3-(4-méthoxy-3-méthylbenzyl)-2,4-dioxo-3,4-dihydroquinazolin-1(2H)-yl}pipéridine-1-carbaldéhyde
n°134 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
n°135 : 4-{6-[2-fluoro-1-(fluorométhyl)éthoxy]-2,4-dioxo-3-(3-phénoxybenzyl) -3,4-dihydroquinazolin-1(2H)-yl}pipéridine-1-carbaldéhyde
n ° 143 : 4-{3-[4-(benzyloxy)3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°145 : 4-{6-[2-fluoro-1-(fluorométhyl)éthoxy]-3-(3-méthoxy-4-nitrobenzyl)-2,4-dioxo-3,4-dihydroquinazolin-1(2H)-yl}pipéridine-1-carbaldéhyde
n°155 : 4-[3-(4-éthoxybenzyl)-6-[2-fluoro-1-(fluorométhyl)éthoxy]-2,4-dioxo-3,4-dihydroquinazolin-1(2H)-yl]pipéridine-1-carbaldéhyde
n°158 : 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°160 : 4-{6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°165 : 4-[3-(biphényl-4-ylméthyl)-6-[2-fluoro-1-(fluorométhyl)éthoxy]-2,4-dioxo-3,4-dihydroquinazolin-1(2H)-yl]pipéridine-1-carbaldéhyde
n°166 : 4-{6-[2-fluoro-1-(fluorométhyl)éthoxy]-3-[4-(méthylsulfanyl)benzyl]-2,4-dioxo-3,4-dihydroquinazolin-1(2H)-yl}pipéridine-1-carbaldéhyde
n°167 : 4-{6-[2-fluoro-1-(fluorométhyl)éthoxy]-2,4-dioxo-3-(4-pyridin-3-ylbenzyl)-3,4-dihydroquinazolin-1(2H)-yl}pipéridine-1-carbaldéhyde
n° 170 : 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde
n°175 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide
n° 178 : 4-{6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°183 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylacétamide
n ° 184 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N,N-diméthylacétamide
n°185 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide
n°186 : 4-{3-[4-(cyclopentyloxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
n°188 : 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°189 : 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde
n°190 : 4-{3-[4-(cyclopentyloxy)-3-hydroxybenzyl]-6-[2-fluoro-1-(fluorométhyl)éthoxy]-2,4-dioxo-3,4-dihydroquinazolin-1(2H)-yl}pipéridine-1-carbaldéhyde
n°193 : 4-{3-[4-(difluorométhoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°194 : 4-{3-[4-(difluorométhoxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
n°200 : 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde
n°201 : 4-{6-[2-fluoro-1-(fluorométhyl)éthoxy]-2,4-dioxo-3-(4-pyridin-4-ylbenzyl}-3,4-dihydroquinazolin-1(2H)-yl}pipéridine-1-carbaldéhyde
n°203 : 4-{6-[2-fluoro-1-(fluorométhyl)éthoxy]-3-[(1-méthyl-1H-indol-6-yl)méthyl]-2,4-dioxo-3,4-dihydroquinazolin-1(2H)-yl}pipéridine-1-carbaldéhyde
n°206 : 4-{3-[4-(cyclopropylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°207 : 2-[4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]-N-méthylacétamide
n°212 : 4-{6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°213 : 4-{6-[2-fluoro-1-(fluorométhyl)éthoxy]-2,4-dioxo-3-(4-pyridin-2-ylbenzy)-3,4-dihydroquinazolin-1(2H)-yl}pipéridine-1-carbaldéhyde
n°215 : 4-{6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°216 : 4-{6-[2-fluoro-1-(fluorométhyl)éthoxy]-2,4-dioxo-3-(quinoléin-7-ylméthyl)-3,4-dihydroquinazolin-1(2H)-yl}pipéridine-1-carbaldéhyde
n°218 : 4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde
n°223 : 4-{3-[4-(1H-benzimidazol-1-yl)benzyl]-6-[2-fluoro-1-(fluorométhyl)éthoxy]-2,4-dioxo-3,4-dihydroquinazolin-1(2H)-yl}pipéridine-1-carbaldéhyde
n°224 : 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n °226 : 4-{6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl}pipéridine-1-carbaldéhyde
n°228 : 4-[3-{4-[(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°230 : 4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde
n°232 : 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°233 : 4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°234 : 4-[3-{4-[(1-acétylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°239 : 4-[3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°240 : 4-[3-{4-[(4-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°242 : 4-[3-{4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°243 : 4-{6-[2-fluoro-1-(fluorométhyl)éthoxy]-2,4-dioxo-3-(3-thiophén-3-ylbenzyl)-3,4-dihydroquinazolin-1(2H)-yl}pipéridine-1-carbaldéhyde
n°245 : 4-[3-(4-éthoxy-3-méthoxybenzyl)-6-(2-hydroxyéthoxy)-2,4-dioxo-3,4-dihydroquinazolin-1 (2H)-yl]pipéridine-1-carbaldéhyde
n°246 : 4-[3-{4-[2-(2,3-dihydro-1H-indol-1-yl)-2-oxoéthoxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°250 : 4-[3-{4-[(3,4-dichlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°251 : 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
n°254 : 4-{3-[3-éthoxy-4-(thiophén-2-ylméthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°258 : 4-[3-(3,4-diméthoxybenzyl)-6-[2-fluoro-1-(hydroxyméthyl)éthoxy]-2,4-dioxo-3,4-dihydroquinazolin-1(2H)-yl]pipéridine-1-carbaldéhyde
n°263 : acide (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
n°264 : 4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
n°270 : 4-{6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
n°275 : 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°276 : 4-{6-[2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde
n°278 : 4-{6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°279 : 4-{6-[2-fluoro-1-(fluorométhyl)éthoxy]-2,4-dioxo-3-[(2-thiophén-2-ylpyrimidin-5-yl)méthyl]-3,4-dihydroquinazolin-1(2H)-yl}pipéridine-1-carbaldéhyde
n°280 : 4-{6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°282 : 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde
n°283 : acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétique
n°285 : 4-{6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde
n°286 : 4-{6-[2-fluoro-1-(fluorométhyl)éthoxy]-2,4-dioxo-3-[(6-phénylpyridin-3-yl)méthyl]-3,4-dihydroquinazolin-1(2H)-yl}pipéridine-1-carbaldéhyde
n°287 : 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)-yl}pipéridine-1-carbaldéhyde
n°289 : 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°292 : 4-{6-[2-fluoro-1-(fluorométhyl)éthoxy]-3-[(1-méthyl-5-phényl-1H-pyrazol-3-yl)méthyl]-2,4-dioxo-3,4-dihydroquinazolin-1(2H)-yl}pipéridine-1-carbaldéhyde
n°294 : 4-({6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°295 : (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide
n°297 : 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°298 : 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde
n°299 : 4-{6-[2-fluoro-1-(fluorométhyl)éthoxy]-3-[3-méthoxy-4-(pipéridin-1-ylméthyl)benzyl]-2,4-dioxo-3,4-dihydroquinazolin-1(2H)-yl}pipéridine-1-carbaldéhyde
n°300 :4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°301 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide
n°302 : acide (2S)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
n°305 : 4-{6-[2-fluoro-1-(fluorométhyl)éthoxy]-3-(3-méthoxy-4-{[(3R)-2-oxo-1-phénylpyrrolidin-3-yl]oxy}benzyl)-2,4-dioxo-3,4-dihydroquinazolin-1(2H)-yl}pipéridine-1-carbaldéhyde
n°306 : 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°307 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°308 : 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde
n°309 : 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde n°310 : 4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n°311 : 4-{7-fluoro-6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°312 : 4-[3-(4-éthoxy-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°315 : 4-{7-fluoro-6-[2-fluoro-1-(fluorométhyl)éthoxy]-3-{[6-(3-méthoxyphényl)pyridin-3-yl]méthyl}2,4-dioxo-3,4-dihydroquinazolin-1(2H)-yl}pipéridine-1-carbaldéhyde
n°316 : 4-{7-fluoro-6-[2-fluoro-1-(fluorométhyl)éthoxy]-3-{[6-(2-fluorophényl)pyridin-3-yl]méthyl}-2,4-dioxo-3,4-dihydroquinazolin-1(2H)-yl}pipéridine-1-carbaldéhyde
n°317 : 4-{7-fluoro-6-[2-fluoro-1-(fluorométhyl)éthoxy]-3-{[6-(4-fluorophényl)pyridin-3-yl]méthyl}-2,4-dioxo-3,4-dihydroquinazolin-1(2H)-yl}pipéridine-1-carbaldéhyde
n°318 : 4-{7-fluoro-6-[2-fluoro-1-(fluorométhyl)éthoxy]-3-{[6-(4-méthoxyphényl)pyridin-3-yl]méthyl}-2,4-dioxo-3,4-dihydroquinazolin-1(2H)-yl}pipéridine-1-carbaldéhyde
n°319 : 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°320 : 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°321 : 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°322 : 4-{7-fluoro-6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°323 : 4-{7-fluoro-6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
n°324 : 4-{7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
n°325 : 4-[3-{4-[2-(2,3-dihydro-1H-indol-1-yl)-2-oxoéthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°326 : 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
n°327 : 4-[3-{4-[(3-chlorobenzyl)oxy]-3-méthoxybenzyl]-7-fluoro-6-[2-fluoro-1-(fluorométhyl)éthoxy]-2,4-dioxo-3,4-dihydroquinazolin-1(2H)-yl]pipéridine-1-carbaldéhyde
n°328 : 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde
n°329 : 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°330 : 4-{7-fluoro-6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°331 :4-{7-fluoro-6-[2-fluoro-1-(fluorométhyl)éthoxy]-3-{[6-(3-fluorophényl)pyridin-3-yl]méthyl}-2,4-dioxo-3,4-dihydroquinazolin-1(2H)-yl}pipéridine-1-carbaldéhyde
n°332 : 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile
n°333 : 4-[3-(3,4-diéthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°334 : 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°335 : 4-{7-fluoro-6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°336 : 4-{7-fluoro-6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°337 : 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°338 : 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°339 : 4-{3-[4-(1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°340 : 5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxybenzonitrile
n°341 : 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile
n°342 : 4-[3-(4-bromobenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°343 : 4-[3-{4-[(3,4-dichlorobenzyl)oxy]-3-(2-méthoxyéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°344 : 4-{3-[4-(benzyloxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°345 : 4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°349 : 4-[3-{4-[(3,4-dichlorobenzyl)oxy]-3-(2-fluoroéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°350 : 4-[3-{4-[(2-chloro-4-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°351 : 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°352 : 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°353 : 4-[3-{4-[(2,6-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°354 : 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°355 : 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°357 : 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile
n°358 : 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°360 : 4-{7-fluoro-6-[2-fluoro-1-(fluorométhyl)éthoxy]-2,4-dioxo-3-[4-(2-phényléthyl)benzyl]-3,4-dihydroquinazolin-1(2H)-yl}pipéridine-1-carbaldéhyde
n°362 : 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde n°369 : 4-[3-{4-[(4-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°371 : 4-[3-{3-chloro-4-[(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°373 : 4-[3-{3-chloro-4-[(2,4-dichlorobenzyl)oxy]-5-éthoxybenzyl}-7-fluoro-6-(2-fluoro-1-(fluorométhyl)éthoxy]-2,4-dioxo-3,4-dihydroquinazolin-1(2H)-yl]pipéridine-1-carbaldéhyde
n°375 : 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°376 : 4-[3-{4-[(3,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°377 : 4-[3-(4-{[4-chloro-3-(trifluorométhyl)benzyl]oxy}-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde n°379 : 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°380 : 4-[3-{4-[(3,5-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°381 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°382 : 4-[3-{4-[(3-chloro-5-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°383 : 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde
n°384 : 4-[3-{4-[(2,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°385 : 4-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
n°386 : 3-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
n°387 : 4-[3-{4-[(4-chloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°388 : 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°389 : 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1(2H)-yl}pipéridine-1-carbaldéhyde
n°390 : 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°391 : 4-[3-{4-[(3,4-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°392 : 4-{3-[4-(5,6-dichloro-1H-benzimidazol-1-yl)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°393 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle
n°394 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle
n°403 : 3,4-dichloro-N-[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényl]benzamide

Les composés conformes à l'invention peuvent être préparés par les méthodes illustrées dans les schémas 1 à 4 qui suivent.

Dans ce qui suit, on entend par groupe partant, un groupe pouvant être facilement substitué, avec départ d'une paire électronique, par rupture d'une liaison hétérolytique. Ce groupe peut ainsi être remplacé facilement par un autre groupe, lors d'une réaction de substitution par exemple. De tels groupes partants sont, par exemple, les halogènes, ou un groupe hydroxy activé tel qu'un mésylate, tosylate, triflate, etc. Des exemples de groupes partants ainsi que des références pour leur préparation sont donnés dans « Advanced Organic Chemistry », J. March, 3rd Edition, Wiley Interscience, p 310-316.

On entend par groupe protecteur PG, un groupe permettant d'empêcher la réactivité d'une fonction ou position, lors d'une réaction chimique pouvant l'affecter, et qui restitue la molécule après clivage selon des méthodes connues de l'homme du métier.

Par groupe protecteur temporaire des amines ou alcools, on entend les groupes protecteurs tels que ceux décrits dans Protective Groups in Organic Synthesis, Greene T.W. et Wuts P.G.M., Ed. Willey Intersciences 1999 et dans Protecting Groups, Kocienski P.J., 1994, Georg Thieme Verlag.

On peut citer par exemple des groupements protecteurs temporaires des amines : benzyles, carbamates, (tels que *tert*-butyloxycarbonyle clivable en milieu acide, benzyloxycarbonyle clivable par hydrogénolyse), des groupements protecteurs temporaires d'acides carboxyliques : esters d'alkyle (tels que méthyle ou éthyle, *tert*butyle hydrolysables en milieu basique ou acide) et benzyliques hydrogénolysables, des groupements protecteurs temporaires d'alcools ou de phénols tels que les éthers de tétrahydropyranyle, métyloxyméthyle ou méthyléthoxyméthyle, *tert*-butyle et benzyle, des groupements protecteurs temporaires de dérivés carbonylés tels que les acétals linéaires ou cycliques comme par exemple 1,3-dioxane-2-yle ou 1,3-dioxolan-2-yle ; et se référer aux méthodes générales bien connues décrites dans Protective Groups, cité ci-dessus.

Selon les cas, l'homme de l'art sera à même de choisir les groupes protecteurs appropriés. Les composés de formule (I) peuvent comporter des groupes précurseurs d'autres fonctions qui sont générées ultérieurement en une ou plusieurs autres étapes.

Dans les schémas généraux de synthèse qui suivent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les énantiomères purs des composés conformes à l'invention peuvent être obtenus à partir de précurseurs énantiomèriquement purs ou bien par chromatographie sur des phases chirales ou bien, lorsque les composés comportent des fonctions acides ou des amines par cristallisations sélectives de sels diastéréoisomèriques obtenus par réaction des composés (I) avec, respectivement, des amines ou des acides chiraux.

Les composés de formule générale (I) peuvent être obtenus selon les Schémas 1 à 4 qui suivent. Par souci de clarté, on a choisi le groupe R4 comme étant un hydrogène, p et p' représentent 1 et 2 respectivement et on a fixé les groupes R₂ et R₃ tels qu'indiqués dans les schémas. Cependant, il s'entend que R₄ peut être tel que défini dans la formule générale de (I), que R₂ et R₃ peuvent avoir les positions indiquées dans la formule générale (I), et que p et p' peuvent être tels que définis dans la formule générale de (I).

Les voies de synthèse décrites ci-après servent uniquement à titre illustratif et ne sont en aucun cas limitatives. L'homme du métier pourra appliquer sans difficultés l'enseignement ci-dessous aux composés de formule (I) pour lesquels R, R₁, R₂, R₃, R₄, Ra, Rb, Rc, m, n, p, p' et q sont tels que définis dans la formule générale (I).

Selon le schéma 1, le composé de formule (IV) est obtenu par une réaction d'amination réductrice en faisant réagir un composé de formule (II) dans lequel R' représente un groupe (C₁-C₆) alkyle, et R₂ est tel que défini pour le composé de formule (I), avec un composé de formule (III) en milieu acide et en présence d'un agent réducteur tel que le tri-acétoxy-borohydrure de sodium. Le groupe GP du composé de formule (III) est un groupe protecteur de la fonction amine, qui peut avantageusement être du tert-butyloxycarbonyle (boc). Le composé de formulé (IV) ainsi formé est ensuite acylé selon les méthodes bien connues de l'homme du métier, avec un chloroformate d'alkyle ou d'aryle pour donner le composé de formule (V) dans lequel R" représente un groupe (C₁-C₆) alkyle ou un groupe aryle substitué. Une réaction d'hydrolyse en milieu basique permet d'obtenir les composés de formule (VI) qui par une réaction de couplage avec un composé de formule (VII), dans lequel R₃ est tel que défini pour le composé de formule (I), conduit aux composés de formule (VIII). Une réaction de cyclisation intra-moléculaire en milieu basique permet d'obtenir les dérivés de quinazolinedione de formule (IX). Le groupe GP protecteur de la fonction amine est ensuite clivé, en milieu acide par exemple lorsque GP est un boc, pour donner les composés de formule (la) qui par une réaction d'acylation donne les composés de formule (Ib).

Les composés de formule (la) sont des composés de formule (I) et peuvent servir d'intermédiaire à d'autres composés de formule (I), tels que les composés de formule (Ib).

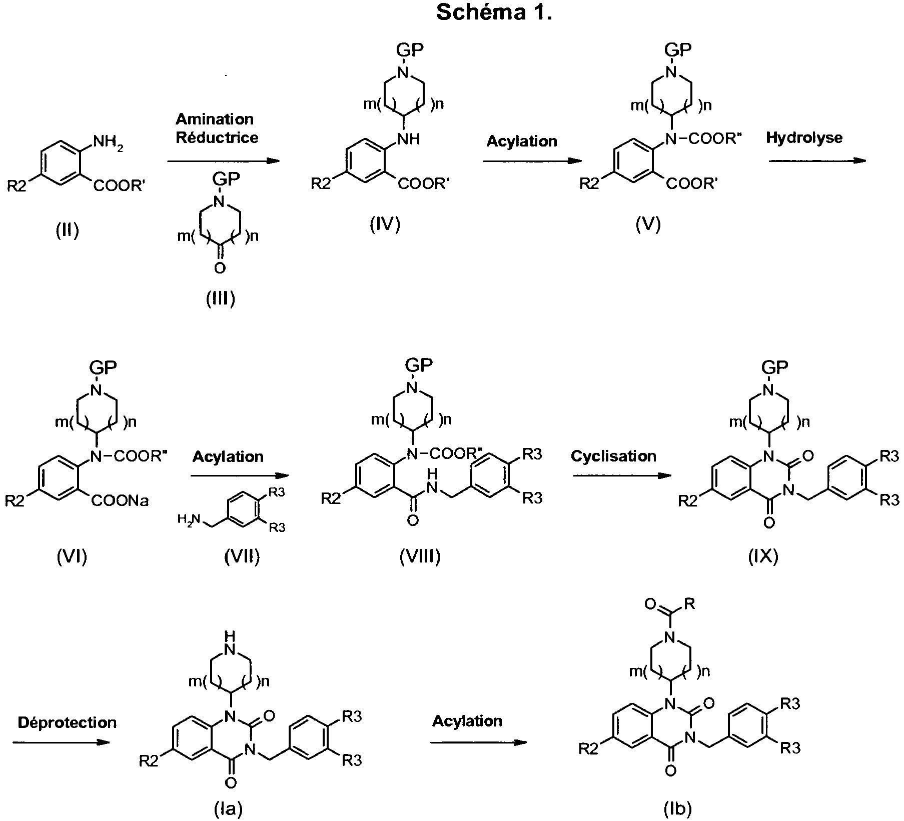

Les composés de formule (I) pour lesquels R₂ représente -ORa, Ra étant tel que défini pour le composé de formule (I), répondent à la formule (Id). Ils peuvent être obtenus à partir des composés de formule (X) selon le schéma 2 suivant. Les composés de formule (Ic), obtenus par une réaction d'hydrogénolyse des composés de formule (X), sont soumis par exemple à une réaction d'alkylation avec un agent alkylant de type Ra-X dans lequel Ra est tel que défini pour le composé de formule (I) et X représente un groupement partant (tel qu'un atome d'halogène par exemple) en présence d'une base telle que du carbonate de césium (Cs₂CO₃), ou encore à une réaction de Mitsunobu (Synthesis 1981, 1) avec un alcool de type Ra-OH, Ra étant tel que défini pour le composé de formule (I), pour donner des composés de formule (Id).

Les composés de formule (X) ainsi que les composés de formule (Ic) sont des composés de formule (I) et peuvent servir d'intermédiaire à d'autres composés de formule (I), tels que les composés de formule (Id).

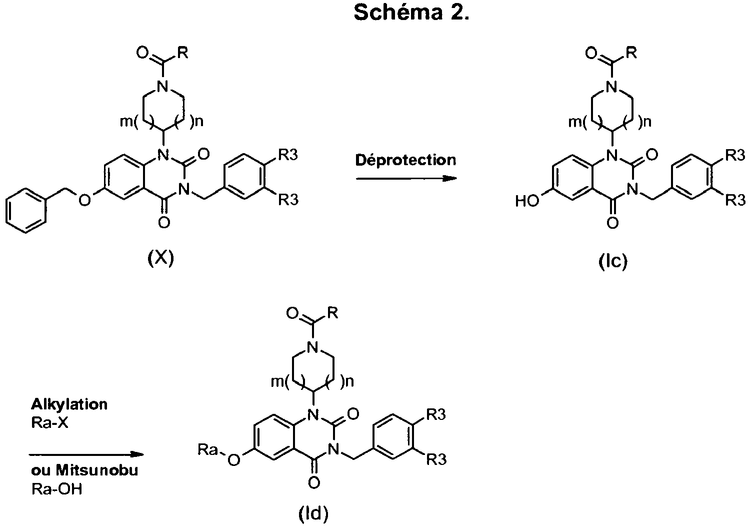

Alternativement les composés de formule (Id) peuvent être obtenus en suivant le procédé décrit dans le schéma 3.

Les composés de formule (XII) sont obtenus par une réaction de substitution nucléophile aromatique impliquant un composé de formule (XI) dans lequel R' est tel que défini précédemment et un alcool de type Ra-OH, dans lequel Ra est tel que défini pour le composé de formule (I), en présence d'une base. La réduction du groupement nitro des composés de formule (XII) conduit aux dérivés anilino correspondants (XIII). Une réaction d'amination réductrice avec un composé de formule (III), dans lequel GP est un groupe protecteur de fonctions amines, tel par exemple que le boc, conduit aux composés de formule (XIV). L'obtention des composés de formule (XV) se fait par réaction d'un composé de formule (XIV) avec de l'isocyanate de potassium (KNCO) en milieu acide. Une réaction de cyclisation intra-moléculaire en milieu basique permet d'obtenir les composés de formule (XVI). Le groupement protecteur GP est clivé par des méthodes bien connues de l'homme de l'art pour donner les composés de formule (XVII). Une réaction d'acylation conduit aux composés de formule (XVIII). Finalement, on obtient les composés de formule (Id) soit par réaction d'alkylation avec un dérivé de type (XIX) dans lequel X représente un groupement partant tel qu'un atome d'halogène en présence d'une base comme par exemple le carbonate de césium, soit encore par une réaction de Mitsunobu avec un alcool benzylique de type (XX). Dans les composés (XIX) et (XX), R₃ est tel que défini précédemment.

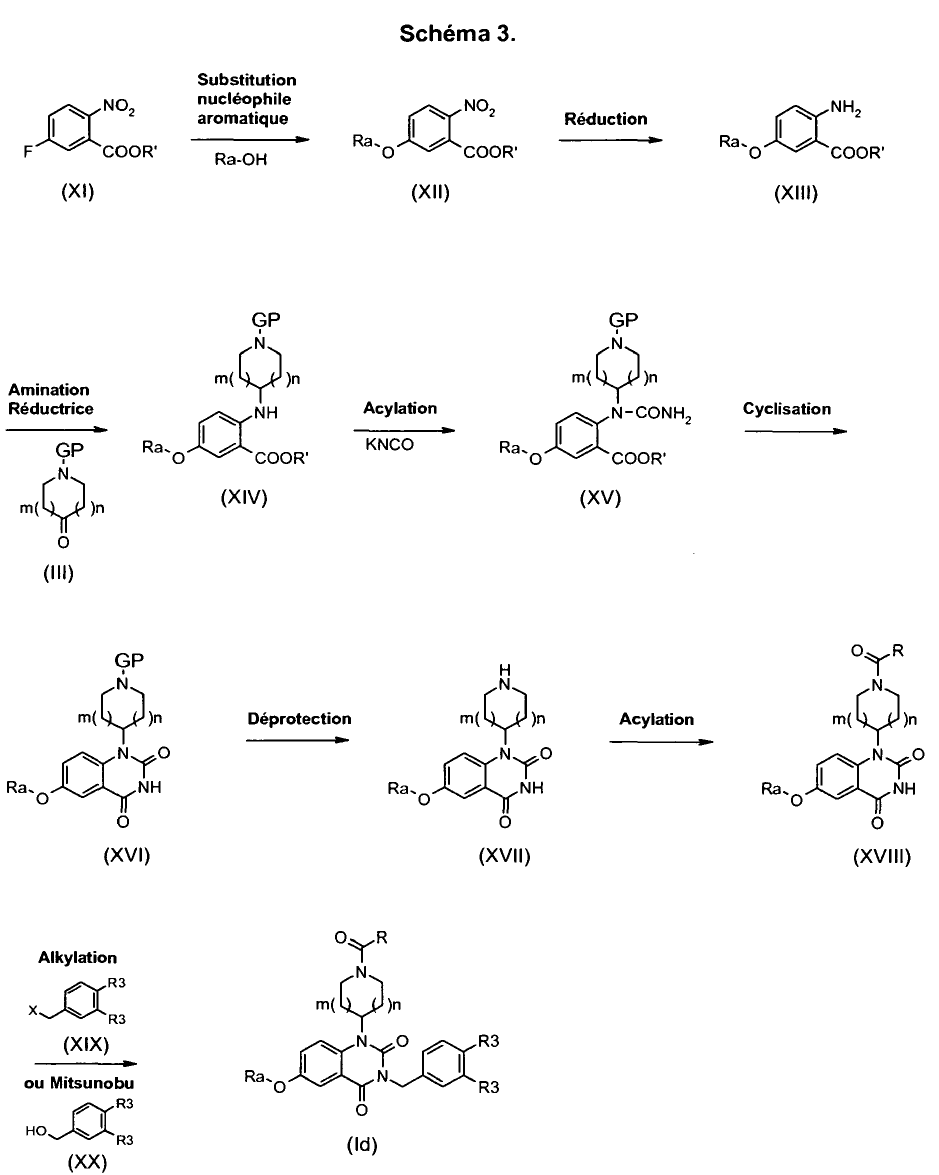

Les composés de formule (le) et (If) dans lesquels R₂ représente plus particulièrement un groupe de type -CH₂-NHC(O)Rb, Rb étant défini comme dans le composé de formule (I) peuvent être préparés selon le Schéma 4 suivant.

Il est entendu que dans le schéma 3, le groupement R2 illustré est de type -O-Ra et est en position 6 de la structure quinazoline-dione (voir par exemple composé (XVIII)), mais qu'il est également possible d'avoir un deuxième groupement R2 tel que déini dans la formule générale de (I) en position 7 du même groupement quinazoline-dione.

La réduction du groupement nitro des composés de type (XXI), dans lequel R' et GP' sont tels que définis précédemment, le groupe GP' étant avantageusement du boc, conduit aux dérivés anilino correspondants (XXII), qui par une réaction d'amination réductrice en faisant réagir en milieu acide et aventageusement en présence d'un agent réducteur tel que le tri-acétoxy-borohydrure de sodium, avec un composé de formule (III) dans lequel GP représente un groupement protecteur d'amine benzyloxycarbonyl, donnent des composés de formule (XXIII). Une réaction d'acylation avec un chloroformate d'alkyle ou d'aryle dans lequel R" représente un groupe (C₁-C₆) alkyle ou un groupe aryle substitué conduit aux composés de formule (XXIV). Les analogues de quinazolinedione de formule (XXV) peuvent être obtenus par une réaction d'hydrolyse en milieu basique puis par une réaction de couplage avec un composé de formule (VII) dans lequel R₃ est tel que défini pour le composé de formule (I), suivie par une réaction de cyclisation intra-moléculaire en milieu basique. Le groupe GP' (préférablement un boc) est ensuite clivé en milieu acide pour conduire aux composés de formule (XXVI), qui par acylation donnent des composés de formule (XXVII), dans lesquels Rb est tel que défini pour le composé de formule (I). Le groupe protecteur GP de (XXVII) est clivé par une réaction d'hydrogénolyse pour donner les composés de formule (le). Finalement les composés de formule (If) sont obtenus par une réaction d'acylation des composés de formule (le).

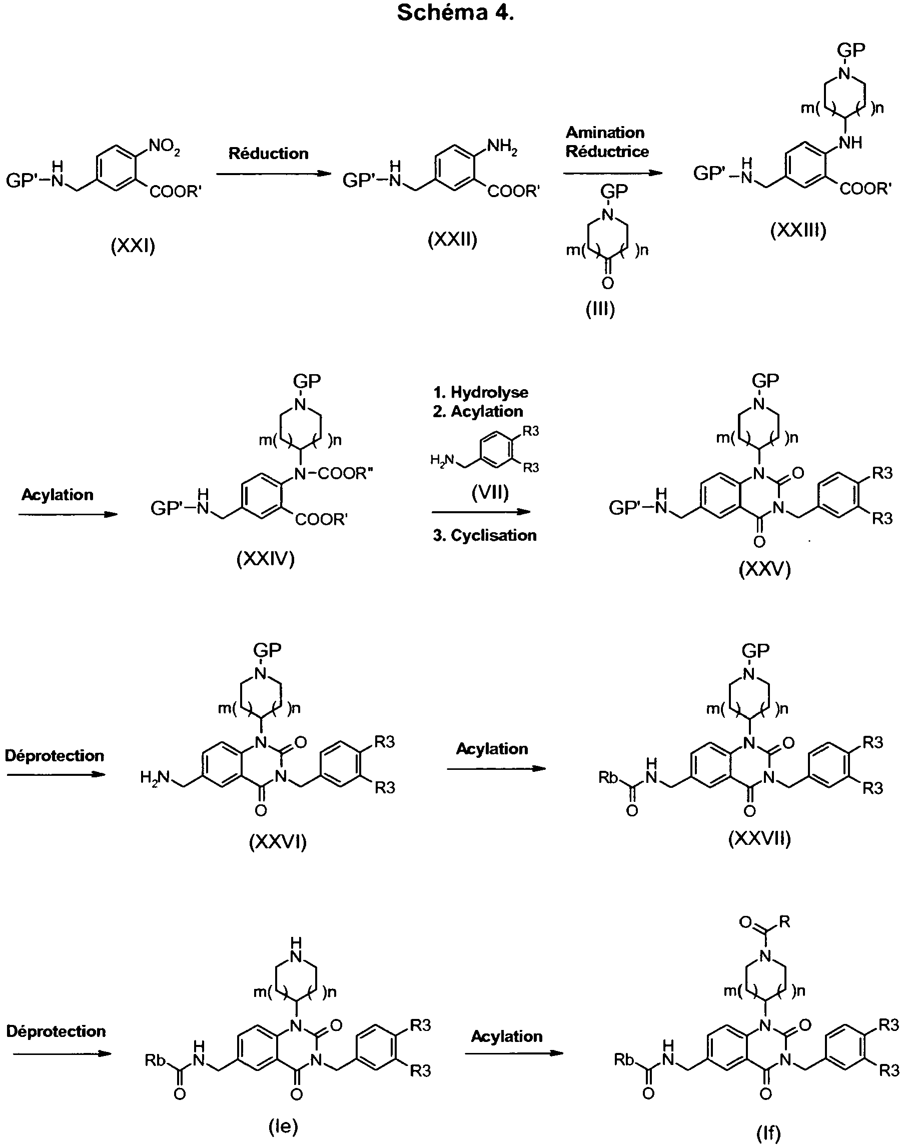

Il va de soi que l'homme du métier sera à même de choisir, à la lumière de ses connaissances et de la littérature; d'autres groupements protecteurs appropriés permettant l'introduction de tous les groupements décrits dans la formule générale (I).

Lorsque le composé de formule (I) comporte un cycle ponté, il peut être indifféremment obtenu par l'une des voies de synthèse décrites ci-dessus.

Les modes opératoires et exemples suivants décrivent la préparation de certains composés conformes à l'invention. Ces modes opératoires et exemples ne font qu'illustrer la présente invention.

Dans les modes opératoires et exemples ci-dessous :
- Les spectres de masse sont réalisés sur un spectromètre quadripolaire de type Platform LCZ (WATERS) ou de type ZQ 4000 (WATERS) en mode d'ionisation par électrospray positif ;
- Les spectres de RMN (résonnance magnétique nucléaire) sont réalisés sur un spectromètre à transformée de Fourier (BRUKER), à la température de 300°K (protons échangeables non enregistrés) ;
- s = singulet,
- d = doublet,
- m = multiplet,
- br = signal large (broad signal)
- t = triplet,
- q = quadruplet
- DMSO-d₆ = diméthylsulfoxyde deutéré
- CDCl₃ = chloroforme deutéré ;

Les mélanges de solvants sont quantifiés en rapports volumétriques ;

Les spectres RMN et spectres de masse confirment les structures des composés obtenus selon les exemples ci-dessous.

Dans les exemples qui suivent, on utilise les abréviations suivantes :
ACN : acétonitrile
AcOEt : acétate d'éthyle
AcOH : acide acétique
DBU : 1,8-diazabicyclo[5.4.0]undec-7-ène
DCM : dichlorométhane
DCE : 1,2-dichloroéthane
DIAD : diisopropyl azodicarboxylate
DIEA : di-isopropylamine
DMF : N,N-diméthylformamide
EtOH : éthanol
HBTU : O-(benzotriazol-1-yl)-N,N,N',N', tetraméthyluronium hexafluorophosphate
IBCF : isobutylchloroformate
MeOH : méthanol
NaBH(OAc)₃ : tri-acétoxy-borohydrure de sodium
TA : température ambiante
min : minute
THF : tétrahydrofurane
NEt₃ = triéthylamine
TFA : acide trifluoroacétique

### EXEMPLES

Les exemples suivants décrivent la préparation de certains composés conformes à l'invention. Ces exemples ne font qu'illustrer la présente invention. Les numéros des composés exemplifiés renvoient à ceux donnés dans le tableau ci-après, qui illustre les structures chimiques et les propriétés physiques de quelques composés conformes à l'invention.

### EXEMPLE 1 : composé n°6

### Préparation du {[3-(3,4-diméthoxybenzyl)-1-(1-formylpipéridin-4-yl)-2,4-dioxo-1,2,3,4-tétrahydroquinazolin-6-yl]oxy}acétonitrile

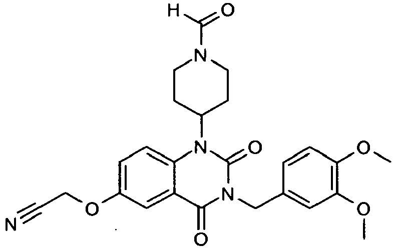

### Etape 1.1 :

### 4-{[4-(Benzyloxy)-2-(méthoxycarbonyl)phényl]amino}pipéridine-1-carboxylate de 1,1-diméthyléthyle

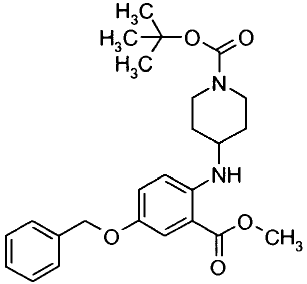

On irradie sous champ de micro-ondes (Biotage Initiator Sixty) pendant 20 min à 110°C, un mélange de 2 g de 2-amino-5-(benzyloxy)benzoate de méthyle, 3,1g de 4-oxopipéridine-1-carboxylate de 1,1-diméthyléthyle et 3,29 g de NaBH(OAc)₃ dans 10 ml de AcOH. On répète la même réaction avec 2 autres lots de 2 g de 2-amino-5-(benzyloxy)benzoate de méthyle. On rassemble les 3 milieux réactionnels. On reprend dans l'AcOEt. On lave la phase organique à l'eau, par une solution saturée de NH₄Cl, par une solution saturé de NaHCO₃, sèche sur Na₂SO₄, filtre et évapore le solvant sous pression réduite. On chromatographie le résidu sur gel de silice en éluant par un mélange AcOEt/Heptane (5/95, v/v) jusqu'à (30/70, v/v) pour donner 10,2 g du produit attendu.

### Etape 1.2 :

### 4-{[4-(Benzyloxy)-2-(méthoxycarbonyl)phényl][(2-méthylpropoxy)carbonyl]amino}pipéridine-1-carboxylate de 1,1-diméthyléthyle

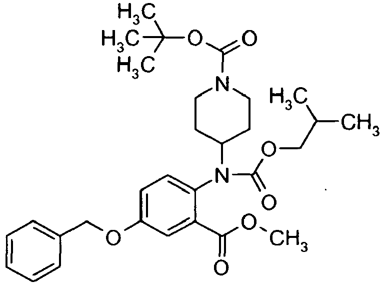

On irradie sous champ de micro-ondes pendant 30 min à 80°C, un mélange de 2 g de 4-{[4-(Benzyloxy)-2-(méthoxycarbonyl)phényl]amino}pipéridine-1-carboxylate de 1,1-diméthyléthyle obtenu à l'étape 1.1, de 0,87 ml de DIEA, de 1,78 ml d'IBCF, 1g de NaOH dans 10 ml de DCE. On répète la même réaction avec 4 autres lots de 2 g de 4-{[4-(Benzyloxy)-2-(méthoxycarbonyl)phényl]amino}pipéridine-1-carboxylate de 1,1-diméthyléthyle. On rassemble les 5 milieux réactionnels. On reprend dans l'AcOEt, filtre et évapore le filtrat sous pression réduite. On chromatographie le résidu sur gel de silice en éluant par un mélange AcOEt/Heptane (10/90, v/v) jusqu'à (50/50, v/v) pour donner 9,3 g du produit attendu.

### Etape 1.3 :

### Sel de sodium de l'acide 5-(benzyloxy)-2-({1-[(1,1-diméthyléthoxy)carbonyl] pipéridin-4-yl}[(2-méthylpropoxy)carbonyl]amino)benzoïque

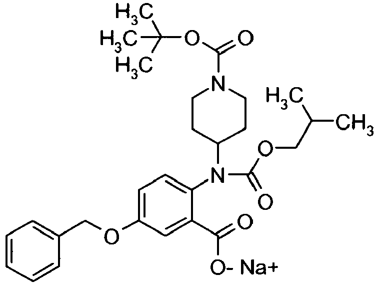

On chauffe 3h00 à 100°C un mélange de 9,3 g de 4-{[4-(Benzyloxy)-2-(méthoxycarbonyl)phényl][(2-méthylpropoxy)carbonyl]amino}pipéridine-1-carboxylate de 1,1-diméthyléthyle obtenu à l'étape 1.2, de 34,4 ml de NaOH 2N dans 57ml de MeOH. On évapore la solution sous pression réduite et on ajoute du DCM. On sèche sur Na₂SO₄, filtre et évapore le solvant sous pression réduite pour donner 8,7 g du produit attendu.

### Etape 1.4 :

### 4-[{4-(Benzyloxy)-2-[(3,4-diméthoxybenzyl)carbamoyl]phényl} (isobutoxycarbonyl)amino]pipéridine-1-carboxylate de 1,1-diméthyléthyle

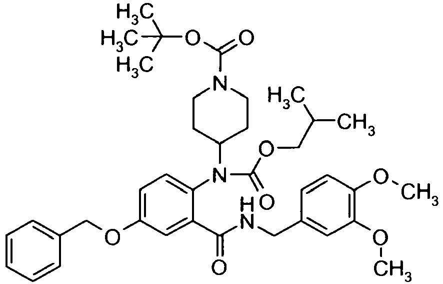

On agite 15 min à TA un mélange de 6g de sel de sodium de l'acide 5-(benzyloxy)-2-({1-[(1,1-diméthyléthoxy)carbonyl]pipéridin-4-yl}[(2-méthylpropoxy)carbonyl] amino)benzoïque obtenu à l'étape 1.3, de 4,42 g de DIEA dans 250 ml de DMF. On ajoute de 6,48 g d'HBTU et on laisse agiter pendant 30 min. On ajoute 2,48 g de vératrylamine et on agite le mélange réactionnel pendant 48h00. On évapore sous pression réduite, reprend le résidu dans de l'AcOEt, lave avec une solution saturée de NH₄Cl, par une solution saturé de NaHCO₃, sèche sur Na₂SO₄, filtre et évapore le solvant sous pression réduite. On chromatographie le résidu sur gel de silice en éluant par un mélange AcOEt/Heptane (20/80, v/v) jusqu'à (60/40, v/v) pour donner 7,5 g du produit attendu.

### Etape 1.5 :

### 4-[6-(Benzyloxy)-3-(3,4-diméthoxybenzyl)-2,4-dioxo-3,4-dihydroquinazolin-1(2H)-yl]pipéridine-1-carboxylate de 1,1-diméthyléthyle

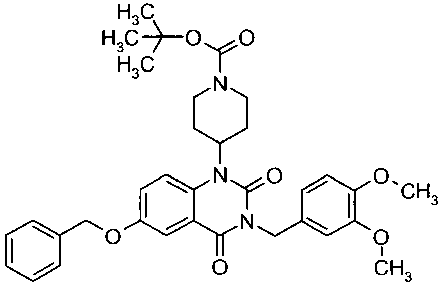

On irradie sous champ de micro-ondes pendant 30 min à 110°C, un mélange de 2.5g de 4-[{4-(benzyloxy)-2-[(3,4-diméthoxybenzyl)carbamoyl]phényl}(isobutoxycarbonyl) amino]pipéridine-1-carboxylate de 1,1-diméthyléthyle obtenu à l'étape 1.4, 7,4 g de NaOH dans 18,5 ml de DCE. On répète la même réaction avec 2 autres lots de 2,5 g de 4-[{4-(benzyloxy)-2-[(3,4-diméthoxybenzyl)carbamoyl]phényl} (isobutoxycarbonyl)amino]pipéridine-1-carboxylate de 1,1-diméthyléthyl. On rassemble les 3 milieux réactionnels. On reprend dans du DCM, lave à l'eau, sèche sur Na₂SO₄, filtre et évapore le solvant sous pression réduite pour donner 6,6 g du produit attendu.

### Etape 1.6 :

### 6-(Benzyloxy)-3-(3,4-diméthoxybenzyl)-1-pipéridin-4-ylquinazoline-2,4(1H,3H)-dione

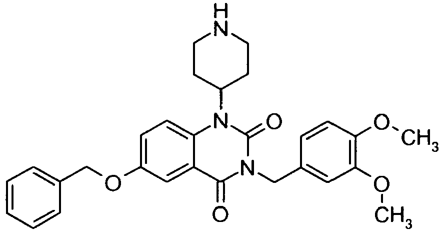

On agite pendant 2h00 à TA un mélange de 3,5 g de 4-[6-(benzyloxy)-3-(3,4-diméthoxybenzyl)-2,4-dioxo-3,4-dihydroquinazolin-1(2H)-yl]pipéridine-1-carboxylate de 1,1-diméthyléthyle obtenu à l'étape 1.5 et 25 ml de TFA dans 50 ml de DCM. On neutralise avec du K₂CO₃. On filtre, évapore le filtrat sous pression réduite. On reprend dans du DCM, lave avec une solution saturée de NaHCO₃, puis par une solution de NaOH à 8%. On sèche sur Na₂SO₄, filtre et évapore le solvant sous pression réduite pour donner 2,67 g du produit attendu.

### Etape 1.7 :

### 4-[6-(Benzyloxy)-3-(3,4-diméthoxybenzyl)-2,4-dioxo-3,4-dihydroquinazolin-1(2H)-yl]pipéridine-1-carbaldéhyde

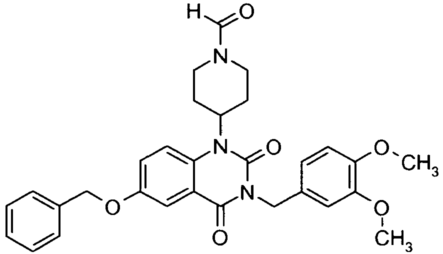

On irradie sous champ de micro-ondes pendant 1h00 à 140°C un mélange de 0,6 g de 6-(benzyloxy)-3-(3,4-diméthoxybenzyl)-1-pipéridin-4-ylquinazoline-2,4(1H,3H)-dione obtenu à l'étape 1.7, de 0,113 g de formiate d'ammonium dans 5 ml d'ACN. On filtre, évapore le filtrat sous pression réduite pour donner 0,62 g du produit attendu.

### Etape 1.8 : composé No.5 :

### 4-[3-(3,4-Diméthoxybenzyl)-6-hydroxy-2,4-dioxo-3,4-dihydroquinazolin-1(2H)-yl]pipéridine-1-carbaldéhyde

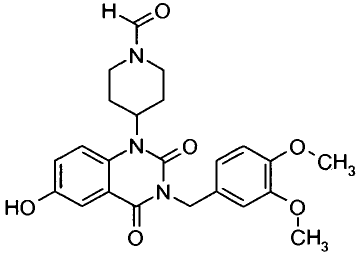

On irradie sous champ de micro-ondes pendant 2h00 à 80°C un mélange de 0,618 g de 4-[6-(benzyloxy)-3-(3,4-diméthoxybenzyl)-2,4-dioxo-3,4-dihydroquinazolin-1(2H)-yl]pipéridine-1-carbaldéhyde obtenu à l'étape 1.7, de 0,44 g de formiate d'ammonium et de 0,124 g de Pd/C (10%) dans 10ml d'EtOH préalablement purgé avec de l'azote. On filtre, évapore le filtrat sous pression réduite pour donner 0,513 g du produit attendu.

### Etape 1.9 : composé No.6

### {[3-(3,4-Diméthoxybenzyl)-1-(1-formylpipéridin-4-yl)-2,4-dioxo-1,2,3,4-tétrahydroquinazolin-6-yl]oxy}acétonitrile

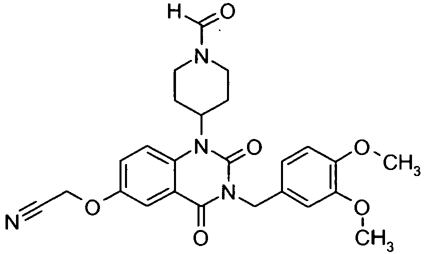

On agite à TA pendant 15 min 0,17 g de 4-[3-(3,4-diméthoxybenzyl)-6-hydroxy-2,4-dioxo-3,4-dihydroquinazolin-1(2H)-yl]pipéridine-1-carbaldéhyde obtenu à l'étape 1.8, de 0,25 g de Cs₂CO₃ dans 3ml de DMF. On ajoute 0,056 g de bromoacétonitrile et on irradie ensuite le mélange réactionnel sous champ de micro-ondes pendant 15 min à 100°C. On filtre, évapore sous pression réduite. On chromatographie le résidu sur gel de silice en éluant par un mélange MeOH/DCM (1/99, v/v) jusqu'à (4/96, v/v) pour donner 0,112 g du produit attendu.

### EXEMPLE 2 : Composé No.3

### Préparation du {[1-(1-acétylpipéridin-4-yl)-3-(3,4-diméthoxybenzyl)-2,4-dioxo-1,2,3,4-tétrahydroquinazolin-6-yl]oxy}acétonitrile

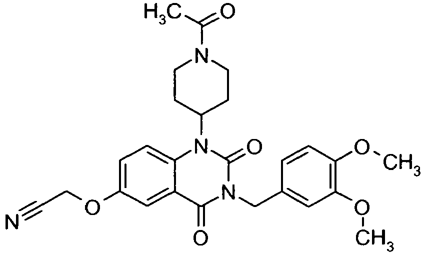

### Etape 2.1 :

### 1-(1-Acétylpipéridin-4-yl)-6-(benzyloxy)-3-(3,4-diméthoxybenzyl)quinazo)ine-2,4(1H,3H)-dione

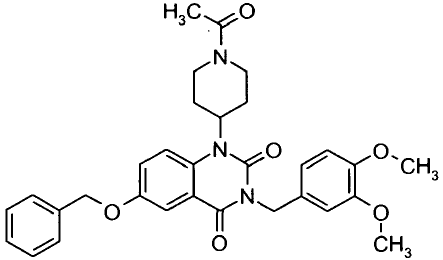

On ajoute 0,14 g de chlorure d'acétyle à un mélange de 0,6 g de 6-(benzyloxy)-3-(3,4-diméthoxybenzyl)-1-pipéridin-4-ylquinazoline-2,4(1H,3H)-dione obtenu selon l'étape 1.6, de 0,24 g de NEt₃ dans 10 ml de DCM refroidit à 0°C. On agite à TA pendant toute la nuit. On lave 2 fois avec une solution saturée de NH₄Cl, filtre, puis évapore le filtrat sous pression réduite pour donner 0,64 g du produit attendu.

### Etape 2.2 : Composé No.2

### 1-(1-Acétylpipéridin-4-yl)-3-(3,4-diméthoxybenzyl)-6-hydroxyquinazoline-2,4(1H,3H)-dione

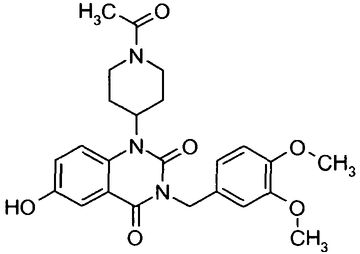

On irradie sous champ de micro-ondes pendant 2h00 à 80°C un mélange de 0,64 g de 1-(1-Acétylpipéridin-4-yl)-6-(benzyloxy)-3-(3,4-diméthoxybenzyl)quinazoline-2,4(1*H*,3*H*)-dione obtenu à l'étape 2.1, de 0,44 g de formiate d'ammonium et de 0,125 g de Pd/C (10%) dans 10 ml d'EtOH préalablement purgé avec de l'azote. On filtre, évapore le filtrat sous pression réduite pour donner 0,48 g du produit attendu.

### Etape 2.3 : Composé No.3

### {[1-(1-Acétylpipéridin-4-yl)-3-(3,4-diméthoxybenzyl)-2,4-dioxo-1,2,3,4-tétrahydroquinazolin-6-yl]oxy}acétonitrile

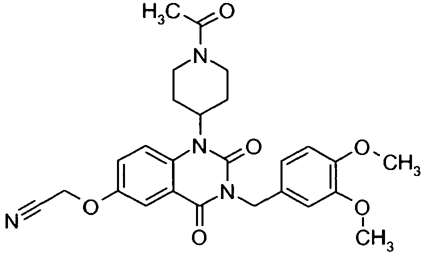

On agite à TA pendant 15min 0,12 g de 1-(1-acétylpipéridin-4-yl)-3-(3,4-diméthoxybenzyl)-6-hydroxyquinazoline-2,4(1H,3H)-dione obtenu à l'étape 2.2, de 0,172 g de Cs₂CO₃ dans 3ml de DMF. On ajoute 0,038 g de bromoacétonitrile et on irradie ensuite le mélange réactionnel sous champ de micro-ondes pendant 15 min à 100°C. On filtre, évapore le filtrat sous pression réduite. On chromatographie le résidu sur gel de silice en éluant par un mélange MeOH/DCM (1/99, v/v) jusqu'à (4/96, v/v) pour donner 0,094 g du produit attendu.

### EXEMPLE 3 : Composé n°34

### Synthèse du 4-[3-(4-chlorobenzyl)-6-(2,2-difluoroéthoxy)-2,4-dioxo-3,4-dihydroquinazolin-1(2H)-yl]pipéridine-1-carbaldéhyde

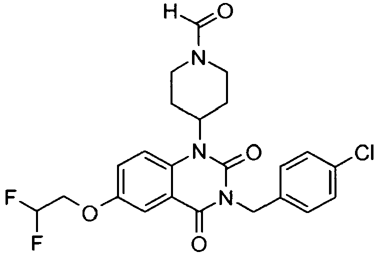

### Etape 3.1 :

### 5-(2,2-Difluoroéthoxy)-2-nitrobenzoate de méthyle

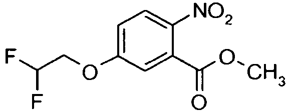

On ajoute 8,53 g de 2,2-difluoroéthanol à une solution de 17,31 g de 5-fluoro-2-nitrobenzoate de méthyle, de 9,64 g de NEt₃, de 32,71 g de 2,8,9-triisobutyl-2,5,8,9-tetraaza-1-phosphabicyclo[3.3.3]undecane dans 250 ml de THF anhydre. On agite 30 min à TA. On évapore le solvant sous pression réduite. On ajoute de l'eau et on extrait à l'AcOEt. On lave avec une solution aqueuse d'HCl 1N, à l'eau puis avec une solution saturée de NaCl. On sèche sur MgSO_{4,} filtre, évapore le solvant sous pression réduite pour donner 21 g du produit attendu.

### Etape 3.2 :

### 2-Amino-5-(2,2-difluoroéthoxy)benzoate de méthyle

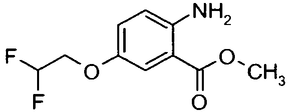

On agite sous atmosphère d'hydrogène pendant 24h00 à TA 21 g de 5-(2,2-difluoroéthoxy)-2-nitrobenzoate de méthyle obtenu à l'étape 3.1 et de 1 g de Pd/C (10%) dans un mélange de 300 ml d'AcOEt, de 50 ml d'EtOH et de 5 ml d'AcOH.

On filtre, évapore sous pression réduite pour donner 18,6 g du produit attendu.

### Etape 3.3 :

### 4-{[4-(2,2-Difluoroéthoxy)-2-(méthoxycarbonyl)phényl]amino}pipéridine-1-carboxylate de 1,1-diméthyléthyle

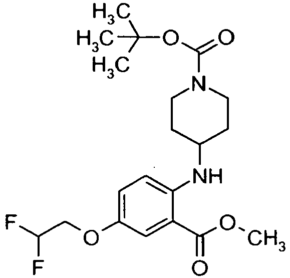

On chauffe à 90°C pendant 10 min un mélange de 4 g de 2-amino-5-(2,2-difluoroéthoxy)benzoate de méthyle, de 6,88 g de 4-oxopipéridine-1-carboxylate de 1,1-diméthyléthyle obtenu à l'étape 3.2 dans 15 ml d'AcOH. On laisse refroidir à TA et on ajoute 7,3 g de NaBH(OAc)₃. On laisse agiter 12h00 à TA. On extrait à l'AcOEt, on lave avec une solution saturée de K₂CO₃, puis à l'eau. On sèche sur MgSO₄, filtre, évapore sous pression réduite pour donner 6,63 g du produit attendu.

### Etape 3.4 :

### 4-{Carbamoyl[4-(2,2-difluoroéthoxy)-2-(méthoxycarbonyl)phényl] amino}pipéridine-1-carboxylate de 1,1-diméthyléthyle

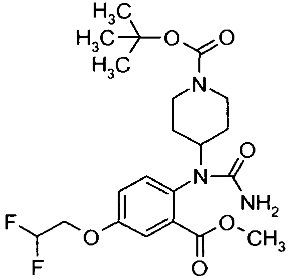

On ajoute 1,95 g d'isocyanate de potassium en solution dans 4 ml d'eau à une solution de 6,63 g de 4-{[4-(2,2-difluoroéthoxy)-2-(méthoxycarbonyl)phényl] amino}pipéridine-1-carboxylate de 1,1-diméthyléthyle obtenu à l'étape 3.3 dans 40 ml d'AcOH. On agite 12h00 à TA. On extrait à l'AcOEt, on lave avec une solution saturée de K₂CO₃, puis à l'eau. On sèche sur MgSO₄, filtre, évapore sous pression réduite pour donner 6,95 g du produit attendu.

### Etape 3.5 :

### 4-[6-(2,2-Difluoroéthoxy)-2,4-dioxo-3,4-dihydroquinazolin-1(2H)-yl]pipéridine-1-carboxylate de 1,1-diméthyléthyle

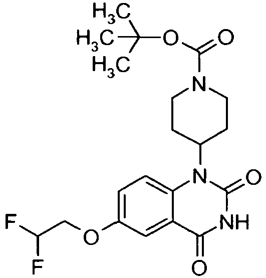

On irradie sous champ de micro-ondes pendant 30 min à 130°C, 2.5g de 4-{carbamoyl[4-(2,2-difluoroéthoxy)-2-(méthoxycarbonyl)phényl]amino}pipéridine-1-carboxylate de 1,1-diméthyléthyle obtenu à l'étape 3.4 en solution dans un mélange de 10 ml de dioxane et de 5ml d'une solution aqueuse de NaOH 1 N. On extrait à l'AcOEt, on neutralise avec une solution aqueuse d'HCl 1N, lave à l'eau, sèche sur MgSO₄, filtre, évapore sous pression réduite. Le résidu obtenu est trituré dans un mélange AcOEt/pentane pour donner le produit attendu. La même réaction est reproduite avec 2 autres lots de 2,5 g de 4-{carbamoyl[4-(2,2-difluoroéthoxy)-2-(méthoxycarbonyl)phényl]amino} pipéridine-1-carboxylate de 1,1-diméthyléthyle obtenu à l'étape 3.4 pour donner au total 5,63 g du produit attendu.

### Etape 3.6 :

### 6-(2,2-Difluoroéthoxy)-1-pipéridin-4-ylquinazoline-2,4(1H,3H)-dione

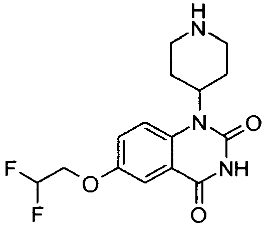

On agite 2h00 à TA une solution de 5,63 g de 4-[6-(2,2-difluoroéthoxy)-2,4-dioxo-3,4-dihydroquinazolin-1(2*H*)-yl]pipéridine-1-carboxylate de 1,1-diméthyléthyle obtenu à l'étape 3.5 dans 70 ml d'acide formique. On évapore le solvant sous pression réduite pour donner 6,13 g du produit attendu sous forme de sel d'acide formique.

### Etape 3.7 :

### 4-[6-(2,2-Difluoroéthoxy)-2,4-dioxo-3,4-dihydroquinazolin-1(2H)-yl]pipéridine-1-carbaldéhyde

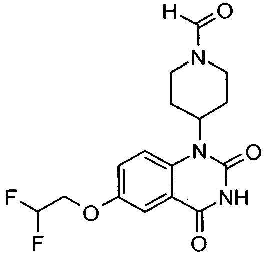

On irradie sous champ de micro-ondes pendant 1 h00 à 140°C, un mélange de 6,13 g de 6-(2,2-difluoroéthoxy)-1-pipéridin-4-ylquinazoline-2,4(1*H*,3*H*)-dione obtenu à l'étape 3.6, de 3,12 g de formiate d'ammonium dans 28 ml d'ACN et 28ml de dioxane. On verse le mélange réactionnel dans de l'eau. On filtre, lave le précipité à l'eau puis à l'éther pour donner 4,47 g du produit attendu.

### Etape 3.8 : Composé n°34

### 4-[3-(4-Chlorobenzyl)-6-(2,2-difluoroéthoxy)-2,4-dioxo-3,4-dihydroquinazolin-1(2H)-yl]pipéridine-1-carbaldéhyde

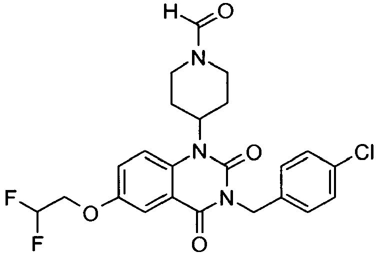

On agite 1h00 à TA un mélange de 0,15 g de 4-[6-(2,2-difluoroéthoxy)-2,4-dioxo-3,4-dihydroquinazolin-1(2*H*)-yl]pipéridine-1-carbaldéhyde obtenu à l'étape 3.7, 0,096 g de 1-(bromométhyl)-4-chlorobenzène et de 0,3 g de Cs₂CO₃ dans 3 ml de DMF. On ajoute de l'AcOEt, lave à l'eau puis avec une solution saturée de NaCl. On sèche sur MgSO₄, filtre, évapore sous pression réduite. On chromatographie le résidu sur gel de silice en éluant à l'AcOEt pour donner 0,116 g du produit attendu.

### Exemple 4 : Composé n°49

### Synthèse du 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde

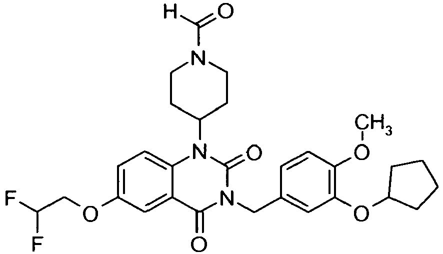

On ajoute 0,172 g de DIAD à une solution de 0,15 g de 4-[6-(2,2-difluoroéthoxy)-2,4-dioxo-3,4-dihydroquinazolin-1(2*H*)-yl]pipéridine-1-carbaldéhyde obtenu à l'étape 3.7, de 0,142 g de [4-(cyclopentyloxy)-3-méthoxyphényl]méthanol et de 0,223 g de PPh₃ dans 3 ml de THF anhydre. On agite 12h00 à TA puis 1h00 à 60°C. On évapore sous pression réduite et on purifie le résidu sur gel de silice en éluant à l'AcOEt pour donner 0,083 g du produit attendu.

### EXEMPLE 5 : Composé n°20

### Synthèse du N-{[3-(3,4-diméthoxybenzyl)-1-(1-formylpipéridin-4-yl)-2,4-dioxo-1,2,3,4-tétrahydroquinazolin-6-yl]méthyl}acétamide

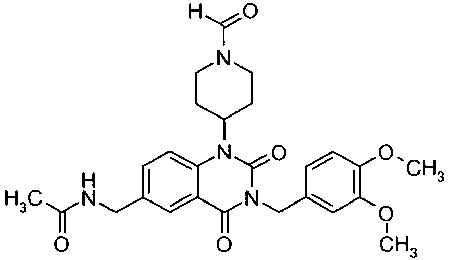

### Etape 5.1 :

### 2-Amino-5-({[(1,1-diméthyléthoxy)carbonyl]amino}méthyl)benzoate de méthyle

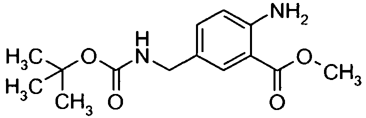

On irradie sous champ de micro-ondes pendant 5 min à 120°C un mélange de 0,273 g de 5-{[(tert-butoxycarbonyl)amino]méthyl}-2-nitrobenzoate de méthyle, 0,166 g de formiate d'ammonium et de 0,094 g de Pd/C (10%) dans 10 ml d'EtOH préalablement purgé avec de l'azote. On filtre, évapore le filtrat sous pression réduite. On chromatographie le résidu sur gel de silice en éluant par un mélange AcOEt/Heptane (5/95, v/v) jusqu'à (30/70, v/v) pour donner 0,2 g du produit attendu.

### Etape 5.2 :

### 4-{[4-({[(1,1-Diméthyléthoxy)carbonyl]amino}méthyl)-2-(méthoxycarbonyl)phényl]amino}pipéridine-1-carboxylate de benzyle

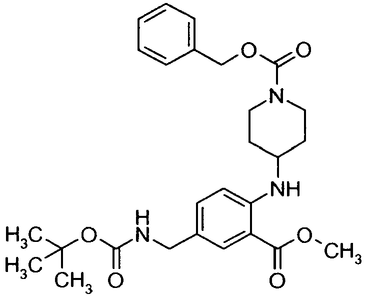

On ajoute goutte à goutte à TA une solution de 1,66 g de 4-oxopipéridine-1-carboxylate de benzyle et de 1g de 2-amino-5-({[(1,1-diméthyléthoxy) carbonyl]amino}méthyl)benzoate de méthyle obtenu à l'étape 5.1 dans 20 ml de DCM à une suspension de 2,04 g de NaBH(OAc)₃ dans un mélange de 20 ml de DCM et de 0,41 ml d'AcOH. On agite 15h00 à TA puis on rajoute 2,04 g de NaBH(OAc)₃. Après 6h00 d'agitation, on ajoute 1,66 g de 4-oxopipéridine-1-carboxylate de benzyle et on agite 48h00 à TA. On ajoute une solution saturée de NaHCO₃, on extrait au DCM. La phase organique est lavée avec une solution saturée de NaHCO₃, 2 fois avec une solution saturée de NH₄Cl. On sèche sur Na₂SO₄, filtre, évapore le filtrat sous pression réduite. On chromatographie le résidu sur gel de silice en éluant par un mélange AcOEt/Heptane (5/95, v/v) jusqu'à (40/60, v/v) pour donner 1,6 g du produit attendu.

### Etape 5.3 :

### 4-{[4-({[(1,1-Diméthyléthoxy)carbonyl]amino}méthyl)-2-(méthoxycarbonyl)phényl](éthoxycarbonyl)amino}pipéridine-1-carboxylate de benzyle

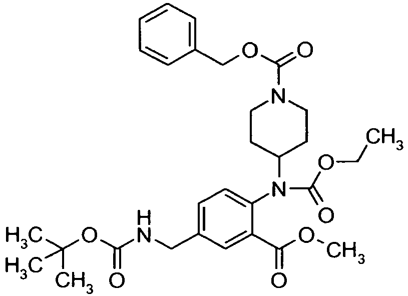

On ajoute 2,08 g de DIEA puis 1,745 g de chloroformate d'éthyle à une solution de 1,6 g de 4-{[4-({[(1,1-Diméthyléthoxy)carbonyl]amino}méthyl-2-(méthoxycarbonyl) phényl]amino}pipéridine-1-carboxylate de benzyle obtenu à l'étape 5.2 dans 11 ml de DCM. On agite à TA pendant 4 jours. On évapore sous pression réduite. On reprend le résidu dans 10 ml de pyridine (10 ml) et on ajoute 0,7 g de chloroformate d'éthyle. On agite 4h00 à TA. On évapore sous pression réduite. On chromatographie le résidu sur gel de silice en éluant par un mélange AcOEt/Heptane (10/90, v/v) jusqu'à (30/70, v/v) pour donner 0,875 g du produit attendu.

### Etape 5.4 :

### 4-[3-(3,4-Diméthoxybenzyl)-6-({[(1,1-diméthyléthoxy)carbonyl]amino}méthyl)-2,4-dioxo-3,4-dihydroquinazolin-1(2H)-yl]pipéridine-1-carboxylate de benzyle

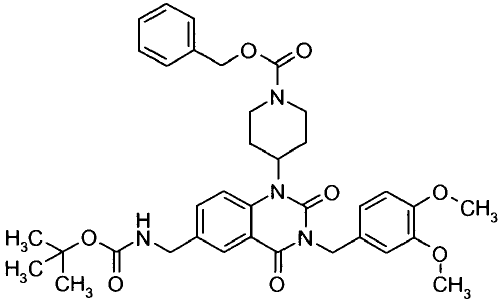

On agite 15h00 à TA un mélange de 0,165 g de 4-{[4-({[(1,1-Diméthyléthoxy) carbonyl]amino}méthyl)-2-(méthoxycarbonyl)phényl](éthoxycarbonyl) amino}pipéridine-1-carboxylate de benzyle obtenu à l'étape 5.3, de 0,028 g de LiOH dans 5 ml de THF/H20 (70/30). On irradie ensuite sous champ de micro-ondes pendant 1h00 à 100°C. On filtre et on évapore le filtrat sous pression réduite. On reprend le résidu dans 5 ml de DMF. On ajoute 0,108 g de DIEA et on agite 10min à TA. On ajoute 0,159 g de HBTU et on agite 30 min à TA. On ajoute ensuite 0,061 g de vératrylamine et on agite 1 h00 à TA. On ajoute 0,5 ml de DBU et on agite 48h00 à TA. On évapore sous pression réduite, reprend le résidu dans de l'AcOEt. On lave 3 fois avec une solution saturée de NH₄Cl et 2 fois à l'eau. On sèche sur Na₂SO₄, filtre, évapore le filtrat sous pression réduite. On chromatographie le résidu sur gel de silice en éluant par un mélange AcOEt/DCM (10/90, v/v) jusqu'à (20/80, v/v) pour donner 0,104 g du produit attendu.

### Etape 5.5 :

### 4-[6-(Aminométhyl)-3-(3,4-diméthoxybenzyl)-2,4-dioxo-3,4-dihydroquinazolin-1(2H)-yl]pipéridine-1-carboxylate de benzyle

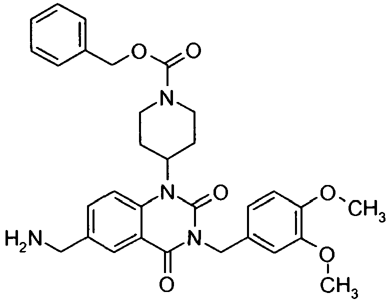

On agite 2h00 à TA une solution de 0,102 g de 4-[3-(3,4-Diméthoxybenzyl)-6-({[(1,1-diméthyléthoxy)carbonyl]amino}méthyl)-2,4-dioxo-3,4-dihydroquinazolin-1(2*H*)-yl]pipéridine-1-carboxylate de benzyle obtenu à l'étape 5.4 et de 0,5 ml de TFA dans 9,5 ml de DCM. On ajoute une solution saturée de NaHCO₃. On sèche la phase organique sur Na₂S0₄, filtre, évapore le filtrat sous pression réduite pour donner 0,09 g du produit attendu.

### Etape 5.6 :

### 4-{6-[(Acétylamino)méthyl]-3-(3,4-diméthoxybenzyl)-2,4-dioxo-3,4-dihydroquinazolin-1(2H)-yl}pipéridine-1-carboxylate de benzyle

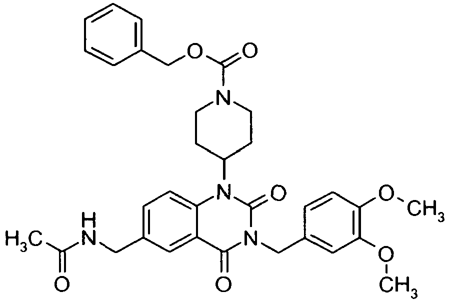

On ajoute 0,049 g d'anhydride acétique à une solution de 0,09 g de 4-[6-(aminométhyl)-3-(3,4-diméthoxybenzyl)-2,4-dioxo-3,4-dihydroquinazolin-1(2*H*)-yl]pipéridine-1-carboxylate de benzyle obtenu à l'étape 5.5, de 0,09 ml de NEt₃ dans 3 ml de DCM et on agite 1h00 à TA. On ajoute du DCM et on lave avec une solution saturée de NH₄Cl, puis par une solution HCl 1 N, NaOH 2N puis à l'eau. On sèche la phase organique sur Na₂SO₄, filtre, évapore le filtrat sous pression réduite pour donner 0,104 g du produit attendu.

### Etape 5.7 :

### N-{[3-(3,4-Diméthoxybenzyl)-2,4-dioxo-1-pipéridin-4-yl-1,2,3,4-tétrahydroquinazolin-6-yl]méthyl}acétamide

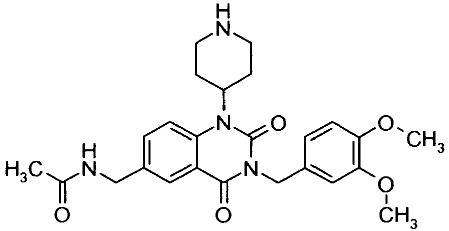

On irradie sous champ de micro-ondes pendant 30 min à 80°C un mélange de 0,1 g de 4-{6-[(acétylamino)méthyl]-3-(3,4-diméthoxybenzyl)-2,4-dioxo-3,4-dihydroquinazolin-1(2*H*)-yl}pipéridine-1-carboxylate de benzyle obtenu à l'étape 5.6, de 0,016 g de formiate d'ammonium et 0,018 g de Pd/C (10%) dans 2 ml d'EtOH préalablement purgé avec de l'azote. On filtre, évapore le filtrat sous pression réduite pour donner 0,077 g du produit attendu.

### Etape 5.8 : Composé n°20

### N-{[3-(3,4-Diméthoxybenzyl)-1-(1-formylpipéridin-4-yl)-2,4-dioxo-1,2,3,4-tétrahydroquinazolin-6-yl]méthyl}acétamide

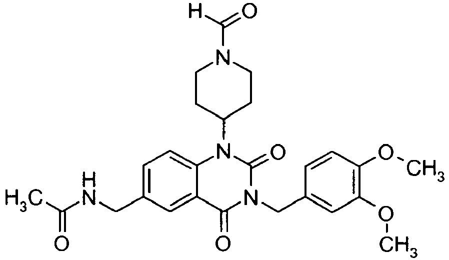

On irradie sous champ de micro-ondes pendant 1h00 à 140°C un mélange de 0,070 g de *N*-{[3-(3,4-diméthoxybenzyl)-2,4-dioxo-1-pipéridin-4-yl-1,2,3,4-tétrahydroquinazolin-6-yl]méthyl}acétamide obtenu à l'étape 5.7, de 0,028 g de formiate d'ammonium dans 2 ml d'ACN. On filtre, évapore le filtrat sous pression réduite. On chromatographie le résidu sur gel de silice en éluant par un mélange MeOH/DCM (0.5/99.5, v/v) jusqu'à (7/93, v/v) pour donner 0,035 g du produit attendu.

Le tableau suivant illustre les structures chimiques et les propriétés physiques de composés répondant à la formule générale (I) selon l'invention, ainsi que de certains de leurs intermédiaires (notamment les composés 32, 55, 120 et 257)

| ***Composé N°*** | ***STRUCTURE*** | ***NOMENCLATURE*** | ***RMN ou MASSE*** |
|---|---|---|---|
| 1 | 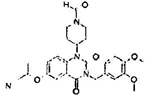 | 2-{[3-(3,4-diméthoxybenzyl)-1-(1-formylpipéridin-4-yl)-2,4-dioxo-1,2,3,4-tétrahydroquinazolin-6-yl]oxy}propanenitrile | ¹H NMR (500 MHz, DMSO-*d₆*) δ ppm 1.71(d, 3H) 1.79(brdd, 2H) 2.41 (dq, 1H) 2.53(dq, 1H) 2.80(td, 1H) 3.25(t, 1H) 3.70(s, 3H) 3.71(s, 3H) 3.80(d, 1H) 4.31(d, 1H) 4.77(brs, 1H) 5.03(s, 2H) 5.60(q, 1H) 6.83(dd, 1H) 6.85 (d, 1H) 6.98(s, 1H) 7.51(dd, 1H) 7.74(d, 1H) 7.84(d, 1H) 8.03(s, 1H) |
| 2 | 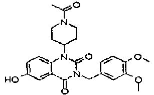 | 1-(1-acétylpipéridin-4-yl)-3-(3,4-diméthoxybenzyl)-6-hydroxyquinazoline-2,4(1H,3H)-dione | ¹H NMR (500 MHz, DMSO-*d*₆) δ ppm 1.69(d, 1H) 1.74(d, 1H) 2.05(s, 3H) 2.42(dq, 1H) 2.57(dq, 1H) 2.70(t, 1H) 3.24(t, 1H) 3.70(br s, 3H) 3.71 (s, 3H) 3.92(d, 1H) 4.51(d, 1H) 4.69(brs, 1H) 5.02(s, 2H) 6.81 (dd, 1H) 6.86(d, 1H) 6.97(d, 1H) 7.21)dd, 1H) 7.44(d, 1H) 7.65(d, 1H) 9.88(brs, 1H) |
| 3 | 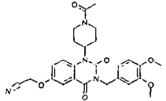 | {[1-(1-acétylpipéridin-4-yl)-3-(3,4-diméthoxybenzyl)-2,4-dioxo-1,2,3,4-tétrahydroquinazolin-6-yl]oxy}acétonitrile | ¹H NMR (500 MHz, DMSO-*d*₆) δ ppm 1.71(d, 1H) 1.76(d, 1H) 2.04(s, 3H) 2.41 (dq, 1H) 2.56(dq, 1H) 2.70(t, 1H) 3.24(t, 1H) 3.70(s, 3H) 3.71(s, 3H) 3.92(d, 1H) 4.51(d, 1H) 4.73(brs, 1H) 5.04(s, 2H) 5.29(s, 2H) 6.82(dd, 1H) 6.85(d, 1H) 6.98(d, 1H) 7.50(dd, 1H) 7.70(d, 1H) 7.83(d, 1H) |
| 4 | 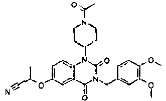 | 2-{[1-(1-acétylpipéridin-4-yl)-3-(3,4-diméthoxybenzyl)-2,4-dioxo-1,2,3,4-tétrahydroquinazolin-6-yl]oxy}propanenitrile | ¹H NMR (500 MHz, DMSO-*d*₆) δ ppm 1.71(d, 3H) 1.72(d, 2H) 1.77(d, 1H) 2.04(s, 3H) 2.41(qd, 1H) 2.56(dq, 1H) 2.70(d, 1H) 3.24(t, 1H) 3.70(s, 3H) 3.71(s, 3H) 3.92(d, 1H) 4.51(d, 1H) 4.73(brs, 1H) 5.03(s, 2H) 5.60(q, 1H) 6.83(d, 1H) 6.85(m, 1H) 6.98(d, 1H) 7.51 (dd, 1H) 7.74(d, 1H) 7.83(d, 1H) |
| 5 | 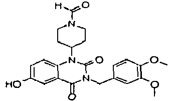 | 4-[3-(3,4-diméthoxybenzyl)-6-hydroxy-2,4-dioxo-3,4-dihydroquinazolin-1(2H)-yl]pipéridine-1-carbaldéhyde | ¹H NMR (500 MHz, DMSO-*d*₆) δ ppm 1.75(d, 1H) 1.78(d, 1H) 2.41(qd, 1H) 2.53(qd, 1H) 2.79(td, 1H) 3.25(td, 1H) 3.70(s, 3H) 3.71 (s, 3H) 3.80(d, 1H) 4.31(d, 1H)4.72 (brs, 1H) 5.02(s, 2H) 6.82(dd, 1H) 6.86(d, 1H) 6.97(d, 1H) 7.21(dd, 1H) 7.44(d, 1H) 7.66(d, 1H) |
| | | | 8.03(s, 1H) 9.85(brs, 1H) |
| 6 | 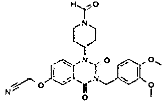 | {[3-(3,4-diméthoxybenzyl)-1-(1-formylpipéridin-4-yl)-2,4-dioxo-1,2,3,4-tétrahydroquinazolin-6-yl]oxy}acétonitrile | ¹H NMR (500 MHz, DMSO-*d*₆) δ ppm 1.78(d, 1H) 1.81(d, 1H) 2.42(qd, 1H) 2.54(dt, 1H) 2.81(td, 4H) 3.26(dt, 1H) 3.71(s, 3H) 3.72(s, 3H) 3.81(d, 1H) 4.32(d, 1H) 4.77(brs, 1H) 5.05(s, 2H) 5.30(s, 2H) 6.84(dd, 1H) 6.87(d, 1H) 6.99(d, 1H) 7.51(dd, 1H) 7.71(d, 1H) 7.85(d, 1H) 8.04(s, 1H) |
| 7 | 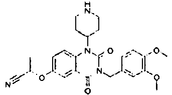 | 2-{[3-(3,4-diméthoxybenzyl)-2,4-dioxo-1-pipéridin-4-yl-1,2,3,4-tétrahydroquinazolin-6-yl]oxy}propanenitrile | ¹H NMR (500 MHz, DMSO-*d*₆) δ ppm1.69(d, 3H) 1.75(d, 2H) 2.65(td, 2H) 2.87(t, 2H) 3.21(d, 2H) 3.69(s, 3H) 3.70(s, 3H) 4.68(brs, 1H) 5.04(s, 2H) 5.59(q, 1H) 6.84(m, 2H) 6.98(d, 1H) 7.50(dd, 1H) 7.73(d, 1H) 7.80(d, 1H) |
| 8 | 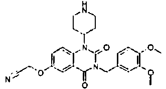 | {[3-(3,4-diméthoxybenzyl)-2,4-dioxo-1-pipéridin-4-yl-1,2,3,4-tétrahydroquinazolin-6-yl]oxy}acétonitrile | ¹H NMR (600 MHz, DMSO-*d*₆) δ ppm 1.77(d, 2H) 2.67(dt, 2H) 2.89(t, 2H) 3.23(d, 2H) 3.69(s, 3H) 3.70(s, 3H) 4.69(brs, 1H) 5.05(s, 2H) 5.28(s, 2H) 6.84(m, 2H) 6.99(s, 1H) 7.49(dd, 1H) 7.70(d, 1H) 7.80(d, 1H) |
| 9 | 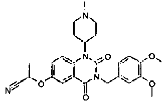 | 2-{[3-(3,4-diméthoxybenzyl)-1-(1-méthylpipéridin-4-yl)-2,4-dioxo-1,2,3,4-tétrahydroquinazolin-6-yl]oxy}propanenitrile | ¹H NMR (500 MHz, DMSO-*d*₆) δ ppm 1.59(d, 2H) 1.66(d, 3H) 2.04(t, 2H) 2.16(s, 3H) 2.62(dt, 2H) 2.82(d, 2H) 3.66(s, 3H) 3.67(s, 3H) 4.43(brs, 1H) 5.01(s, 2H) 5.54(q, 1H) 6.79(m, 1H) 6.82(d, 1H) 6.95(d, 1H) 7.45(dd, 1H) 7.68(d, 1H) 7.70(d, 1H) |
| 10 | 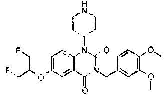 | 3-(3,4-diméthoxybenzyl)-6-[2-fluoro-1-(fluorométhyl)éthoxy]-1-pipéridin-4-ylquinazoline-2,4(1H,3H)-dione | ¹H NMR (500 MHz, DMSO-*d*₆) δ ppm 1.85(d, 2H) 2.78(td, 2H) 3.04(t, 2H) 3.33(d, 2H) 3.71(s, 6H) 4.61-4.82(m, 4H) 5.04(brt, 1H ) 5.05(s, 2H) 6.86(m, 2H) 6.99(s, 1H) 7.49(dd, 1H) 7.68(d, 1H) 7.76(d, 1H) 8.05(brs, 1H) |
| 11 | 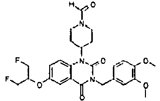 | 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | ¹H NMR (500 MHz, DMSO-*d*₆) δ ppm 1.76(d, 1H) 1.80(d, 1H) 2.41(qd, 1H) 2.53(qd, 1H) 2.80(td, 1H) 3.25(dt, 1H) 3.70(s, 6H) 3.80(d, 1H) 4.31(d, 1H) 4.61-4.83(m, 5H) 5.02(m, 1H) 5.03(s, 2H) 6.83(dd, 1H) 6.85(d, 1H) 6.97(d, 1H) 7.47(dd, 1H) 7.67(d, 1H) 7.77(d, 1H) 8.03(s, 1H) |
| 12 | 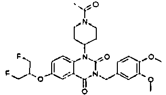 | 1-(1-acétylpipéridin-4-yl)-3-(3,4-diméthoxybenzyl)-6-[2-fluoro-1-(fluorométhyl)éthoxy]quinazolin e-2,4(1H,3H)-dione | ¹H NMR (500 MHz, DMSO-*d*₆) δ ppm 1.70(d, 1H) 1.76(d, 1H) 2.04(s, 3H) 2.41(dq, 1H) 2.56(dq, 1H) 2.70(t, 1H) 3.25(t, 1H) 3.70(s, 3H) 3.71(s, 3H) 3.92(d, 1H) 4.51(d, 1H) 4.61-4.83(m, 5H) 5.02(m, 1H) 5.03(s, 2H) 6.82(dd, 1H) 6.85(m, 1H) 6.97(d, 1H) 7.47(dd, 1H) 7.67(d, 1H) 7.77(d, 1H) |
| 13 | 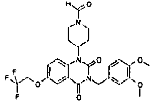 | 4-[3-(3,4-diméthoxybenzyl)-2,4-dioxo-6-(2,2,2-trifluoroéthoxy)-3,4-dihydroquinazolin-1(2H)-yl]pipéridine-1-carbaldéhyde | ¹H NMR (600 MHz, DMSO-*d*₆) δ ppm 1.75(d, 1H) 1.79(d, 1H) 2.40(dq, 1H)) 2.52(dq, 1H) 2.79(td, 1H) 3.25(td, 1H) 3.69(s, 6H) 3.79(d, 1H) 4.30(d, 1H) 4.75(brs, 1H) 4.88(q, 2H) 5.02(s, 2H) 6.82(d, 1H) 6.85(d, 1H) 6.97(s, 1H) 7.49(dd, 1H) 7.66(d, 1H) 7.79(d, 1H) 8.02(s, 1H) |
| 14 | 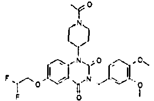 | 1-(1-acétylpipéridin-4-yl)-6-(2,2-difluoroéthoxy)-3-(3,4-diméthoxybenzyl)quinazoline-2,4(1H,3H)-dione | ¹H NMR (500 MHz, DMSO-*d*₆) δ ppm 1.70(d, 1H) 1.74(d, 1H) 2.04(s, 3H) 2.41(qd, 1H) 2.56(qd, 1H) 2.70(t, 1H) 3.24(t, 1H) 3.70(s, 3H) 3.70(s, 3H) 3.91(d, 1H) 4.43(td, 2H) 4.50(d, 1H) 4.73(brs,1H) 5.03 (s, 2H) 6.41(tt, 1H) 6.82(dd, 1H) 6.85(d, 1H) 6.97(d, 1H) 7.46(dd, 1H) 7.60(d, 1H) 7.79(d, 1H) |
| 15 | 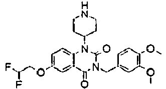 | 6-(2,2-difluoroéthoxy)-3-(3,4-diméthoxybenzyl)-1-pipéridin-4-ylquinazoline-2,4(1H,3H)-dione | ¹H NMR (500 MHz, DMSO-*d*₆) δ ppm 1.83(d, 2H) 2.75(td, 2H) 3.00(t, 2H) 3.30(d, 2H) 3.71(s, 6H) 4.44(td, 2H) 4.75(b. s, 1H) 5.06(s, 2H) 6.42(tt, 1H) 6.83-6.89(m, 2H) 7.00(s, 1H) 7.48(dd, 1H) 7.62(d, 1H) 7.78(d, 1H) |
| 16 | 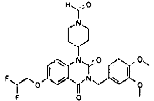 | 4-[6-(2,2-difluoroéthoxy)-3-(3,4-diméthoxybenzyl)-2,4-dioxo-3,4-dihydroquinazolin-1(2H)-yl]pipéridine-1-carbaldéhyde | ¹H NMR (500 MHz, DMSO-*d*₆) δ ppm 1.76(d, 1H) 1.80(d, 1H) 2.41(qd, 1H) 2.53(qd, 1H) 2.81(td, 1H) 3.26(td, 1H) 3.71(s, 6H) 3.80(d, 1H) 4.31 (d, 1H) 4.44(td, 2H) 4.77(brs, 1H) 5.04(s, 2H) 6.42(tt, 1H) 6.83(m, 1H) 6.86(d, 1H) 6.86(d, 1H) 6.98(d, 1H) 7.47(dd, 1H) 7.61(d, 1H) 7.80(d, 1H) 8.03(s, 1H) |
| 17 | 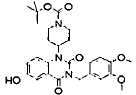 | 4-[3-(3,4-diméthoxybenzyl)-6-hydroxy-2,4-dioxo-3,4-dihydroquinazolin-1(2H)-yl]pipéridine-1-carboxylate de 1,1-diméthyléthyle | ¹H NMR (500 MHz, DMSO-*d*₆) δ ppm 1.43(s, 9H) 1.67(d, 2H) 2.48(m, 2H) 2.92(brs, 2H) 3.70(s, 3 H) 3.71(s, 3H) 4.05(br s, 2H) 4.61(brs, 1H) 5.02(s, 2H) 6.80(dd, 1H) 6.86(d, 1H) 6.97(d, 1H) 7.20(dd, 1H) 7.43(d, 1H) 7.62(d, 1H) 9.95(brs, 1H) |
| 18 | 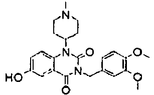 | 3-(3,4-diméthoxybenzyl)-6-hydroxy-1-(1-méthylpipéridin-4-yl)quinazoline-2,4(1H,3H)-dione | ¹H NMR (500 MHz, DMSO-*d*₆) δ ppm 1.60(d, 2H) 2.06(td, 2H) 2.19(s, 3H) 2.65(qd, 2H) 2.85(d, 2H) 3.70(s, 3 H) 3.71(s, 3H) 4.43(br.s, 1H) 5.02(s, 2H) 6.81(m, 1H) 6.85(d, 1H) 6.98(d, 1H) 7.19(dd, 1H) 7.41(d, 1H) 7.55(d, 1H) 9.82(s, 1H) |
| 19 | 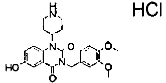 | chlorhydrate de 3-(3,4-diméthoxybenzyl)-6-hydroxy-1-pipéridin-4-ylquinazoline-2,4(1H,3H)-dione | ¹H NMR (500 MHz, DMSO-*d*₆) δ ppm 1.86(d, 2H) 2.83(m, 2H) 3.12(t, 2H) 3.37(m, 2H) 3.70(s, 6H) 4.76(brs, 1H) 5.03(s, 2H) 6.83(dd, 1H) 6.85(d, 1H) 6.98(d, 1H) 7.21(dd, 1H) 7.44(d, 1H) 7.70(d, 1H) 8.70(brs, 2H) 9.91 (s, 1H) |
| 20 | 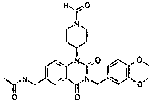 | N-{[3-(3,4-diméthoxybenzyl)-1-(1-formylpipéridin-4-yl)-2,4-dioxo-1,2,3,4-tétrahydroquinazolin-6-yl]méthyl}acétamide | ¹H NMR (500 MHz, DMSO-*d*₆) δ pm1.75(d, 1H) 1.78(d, 1H) 1.86(s, 3H) 2.40(qd, 1H) 2.53(qd, 1H) 2.79(t, 1H) 3.25(t, 1H) 3.69(s, 3H) 3.70(s, 3H) 3.80(d, 1H) 4.29(d, 2H) 4.31(d., 1H) 4.77(brs, 1H) 5.02(s, 2H) 6.80(dd, 1H) 6.85(d, 1H) 6.96(d, 1H) 7.65(dd, 1H) 7.76(d, 1H) 7.96(d, 1H) 8.02(s, 1H) 8.46(t, 1H) |
| 21 | 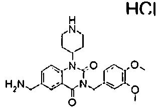 | chlorhydrate de 6-(aminométhyl)-3-(3,4-diméthoxybenzyl)-1-pipéridin-4-ylquinazoline-2,4(1H,3H)-dione | ¹H NMR (500 MHz, DMSO-*d*₆) δ ppm 1.89(d, 2H) 2.83-2.92(m, 2H) 3.16(brs, 2H) 3.38-3.42(m, 2H) 3.71(s, 6H) 4.13(brs, 2H) 4.82-4.97(m, 1H) 5.07(s, 2H) 6.83-6.88(m, 2H) 7.00(s, 1H) 7.91-7.99(m, 2H) 8.24(s, 1H) 8.42(br s, 3H) 9.16(brs, 1H) |
| 22 | 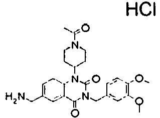 | chlorhydrate de 1-(1-acétylpipéridin-4-yl)-6-(aminométhyl)-3-(3,4-diméthoxybenzyl)quinazoline-2,4(1H,3H)-dione | ¹H NMR (500 MHz, DMSO-*d*₆) δ ppm 1.67-1.79(m, 2H) 2.05(s, 3H) 2.36-2.47(m, 1H) 2.51-2.61(m, 1H) 2.73(t, 1H) 3.27(t, 1H) 3.71 (s, 6H) 3.93(d, 1H) 4.11-4.15(m, 2H) 4.52(d, 1H) 4.78(brs, 1H) 5.05(s, 2H) 6.82(d, 1H) 6.86(d, 1H) 6.98(s, 1H) 7.87(s, 2H) 8.24(s, 1H) 8.32(brs, 3H) |
| 23 | 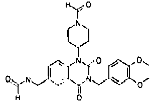 | N-{[3-(3,4-diméthoxybenzyl)-1-(1-formylpipéridin-4-yl)-2,4-dioxo-1,2,3,4-tétrahydroquinazolin-6-yl]méthyl}formamide | ¹H NMR (500 MHz, DMSO-*d*₆) δ ppm 1.73-1.83(m, 2H) 2.36-2.45(m., 1H) 2.48-2.57(m, 1H) 2.77-2.84(m, 1H) 3.23-3.29(m, 1H) 3.70(s, 1H) 3.71(s, 3H) 3.81(d, 1H) 4.31(d, 1H) 4.37(d, 2H) 4.77(brs, 1H) 5.04(s, 2H) 6.82(dd, 1H) 6.86(d, 1H) 6.98(d, 1H) 7.67(dd, 1H) 7.78(d, 1H) 7.99(d, 1H) 8.03(s, 1H) 8.16(s, 1H) 8.62(t, 1H) |
| 24 | 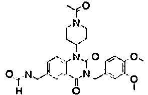 | N-{[1-(1-acétylpipéridin-4-yl)-3-(3,4-diméthoxybenzyl)-2,4-dioxo-1,2,3,4-tétrahydroquinazolin-6- | ¹H NMR (500 MHz, DMSO-*d*₆) δ ppm 1.67-1.78(m, 2H) 2.05(s, 3H) 2.35-2.48(m, 1H) 2.53-2.62(m, 1H) 2.72(t, 1H) 3.25(t, 1H) 3.70(s, 3H) 3.71(s, 3H) 3.93(d, 1H) 4.37(d, 2H) 4.52(d, 1H) |
| | | yl]méthyl}formamide | 4.75(brs, 1H) 5.04(s, 2H) 6.82(dd, 1H) 6.86(d, 1H) 6.98(d, 1H) 7.67(dd,1 H) 7.78(d,1 H) 7.99(d, 1H) 8.16(s, 1H) 8.63(t, 1H) |
| 25 | 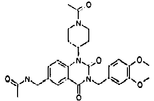 | N-{[1-(1-acétylpipéridin-4-yl)-3-(3,4-diméthoxybenzyl)-2,4-dioxo-1,2,3,4-tétrahydroquinazolin-6-yl]methyl}acétamide | ¹H NMR (500 MHz, DMSO-*d*₆) δ ppm 1.63-1.81(m, 2H) 1.87(s, 3H) 2.05(s, 3H) 2.32-2.42(m, 1H) 2.54-2.63(m, 1H) 2.71(dt, 1H) 3.25(dt, 1H) 3.70(s, 3H) 3.71(s, 3H) 3.93(d, 1H) 4.30(d, 2H) 4.52(d, 1H) 4.76(brs, 1H) 5.04(s, 2H) 6.81(dd 1H) 6.86(d, 1H) 6.97(d, 1H) 7.65(dd, 1H) 7.76(d, 1H) 7.97(d, 1H) 8.47(t, 1H) |
| 26 | 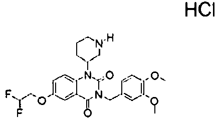 | chlorhydrate de 6-(2,2-difluoroéthoxy)-3-(3,4-diméthoxybenzyl)-1-pipéridin-3-ylquinazoline-2,4(1H,3H)-dione | ¹H NMR (600 MHz, DMSO-*d*₆) δ ppm 1.86-1.97(m, 3H) 2.54-2.62(m, 1H) 2.85-2.92(m, 1H) 3.31-3.35(m, 1H) 3.37-3.42(m, 1H) 3.71(s, 3H) 3.72(s, 3H) 3.73-3.78(m,1H) 3.76(t, 1H) 4.45(td, 2H) 4.87(br s, 1H) 5.05(s, 2H) 6.41(tt, 1H) 6.83-6.88(m, 2H) 6.99(s, 1H) 7.52(dd, 1H) 7.63(d, 1H) 7.72(d, 1H) 8.95(brs, 1H) |
| 27 | 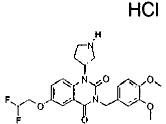 | chlorhydrate de 6-(2,2-difluoroéthoxy)-3-(3,4-diméthoxybenzyl)-1-pyrrolidin-3-ylquinazoline-2,4(1H,3H)-dione | ¹H NMR (500 MHz, DMSO-*d*₆) δ ppm 2.25-2.32(m, 1H) 2.42-2.48(m, 1H) 3.11-3.19(m, 1H) 3.38-3.45(m, 1H) 3.58-3.64(m, 1H) 3.65-3.72(m, 1H) 3.73(s, 3H) 3.71(s, 3H) 4.45(td, 2H) 5.05-5.12(m, 2H) 5.47-5.54(m, 1H) 6.42(tt, 1H) 6.82-6.88(m, 2H) 7.01(s, 1H) 7.55(dd, 1H) 7.62(d, 1H) 7.71(d, 1H) 8.87(brs, 1H) |
| 28 | 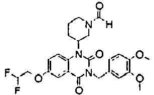 | 3-[6-(2,2-difluoroéthoxy)-3-(3,4-diméthoxybenzyl)-2,4-dioxo-3,4-dihydroquinazolin-1(2H)-yl]pipéridine-1-carbaldéhyde | ¹H NMR (600 MHz, DMSO-*d*₆) mélange de deux isomères δ ppm 1.52-1.62(m, 0.5H) 1.64-1.73(m, 0.5H) 1.75-1.84(m, 1H) 1.84-1.90(m, 1H) 2.54-2.67(m, 1.5H) 3.05(t, 0.5H) 3.59(t, 0.5H) 3.70(s, 3.5H) 3.70(s, 3H) 3.77(dd, 0.5H) 4.06(t, 0.5H) 4.17-4.27(m, 1H) 4.31(brs, 0.5H) 4.42(td, 2H) 4.44(brs, 0.5H) 5.04(s, 2H) 6.38(tt, 1H) 6.80-6.84(m, 1H) 6.84-6.88(m, 1H) 6.98(brs, 1H) 7.43(td, 1H) 7.60(brs, 1H) 7.62(d, 0.5H) 7.79(d, 0.5H) 7.99(s, 0.5H) 8.09(s, 0.5H) |
| 29 | 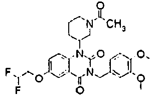 | 1-(1-acétylpipéridin-3-yl)-6-(2,2-difluoroéthoxy)-3-(3,4-diméthoxybenzyl)quinazoline-2,4(1H,3H)-dione | ¹H NMR (500 MHz, DMSO-*d*₆) mélange de deux isomères δ ppm 1.55-1.65(m, 0.5H) 1.67-1.89(m, 2.5H) 1.99(s, 1.5H) 2.06(s, 1.5H) 2.52-2.65(m, 1H) 3.06(t, 0.5H) 3.30-3.37(m, 0.5H) 3.52(t, 0.5H) 3.70(s, 3H) 3.71(s, 3H) 3.52(t, 0.5H) 3.84(d, 0.5H) 3.91(d, 0.5H) 4.09(t, 0.5H) 4.28(brs, 0.5H) 4.39-4.48(m, 3H) 4.49(brs, 0.5H) 5.05(d, 2H) 6.41(tt, 1H) 6.81-6.89(m, 2H) 6.99(d, 1H) 7.44-7.48(m, 1H) 7.59-7.62(m, 1H) 7.64(d, 0.5H) 7.82(d, 0.5H) |
| 30 | 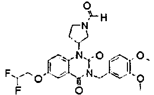 | 3-[6-(2,2-difluoroéthoxy)-3-(3,4-diméthoxybenzyl)-2,4-dioxo-3,4-dihydroquinazolin-1(2H)-yl]pyrrolidine-1-carbaldéhyde | ¹H NMR (500 MHz, DMSO-*d*₆) mélange de deux isomères δ ppm 2.18-2.27(m, 1H) 2.36-2.46(m, 1H) 3.41-3.48(m, 0.5H) 3.53-3.60(m, 0.5H) 3.63-3.73(m, 1.5H) 3.85-3.95(m, 1.5H) 4.43(td, 2H) 5.06(s, 2H) 5.38-5.45(m, 0.5H) 5.46-5.54(m, 0.5H) 6.41 (tt, 1H) 6.82-6.88(m, 2H) 7.00(s, 1H) 7.46 - 7.51(m, 1H) 7.61(d, 1H) 7.66(d, 0.5H) 7.72(d, 0.5H) 8.16(s, 0.5H) 8.20(s, 0.5H) |
| 31 | 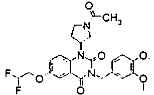 | 1-(1-acétylpyrrolidin-3-yl)-6-(2,2-difluoroéthoxy)-3-(3,4-diméthoxybenzyl)quinazoline-2,4(1H,3H)-dione | ¹H NMR (500 MHz, DMSO-*d*₆) mélange de deux isomères δ ppm1.98(s, 1.5H) 2.04(s, 1.5H) 2.17-2.25(m, 0.5H) 2.27-2.35(m, 0.5H ) 2.57-2.66(m, 1H) 3.40-3.45(m, 0.5H) 3 56-3.61(m, 0.5H) 3.70-3.78(m, 7.5H) 3.82-3.95(m, 1.5H) 4.48(td, 2H) 5.10(d, 2H) 5.45-5.58(m, 1H) 6.46(tt, 1H) 6.87-6.92(m, 2H) 7.05(dd, 1H) 7.53(td, 1H) 7.66(t, 1H) 7.72 (dd, 1H) |
| 32 | 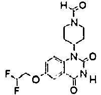 | 4-[6-(2,2-difluoroéthoxy)-2,4-dioxo-3,4-dihydroquinazolin-1(2H)-yl]pipéridine-1-carbaldéhyde | ¹H NMR (600 MHz, DMSO-*d*₆) δ ppm 1.72-1.82(m, 2H) 2.35-2.44(m, 1H) 2.47-2.55(m, 1H) 2.79(td, 1H) 3.25(td, 1H) 3.80(d, 1H) 4.32(d, 1H) 4.42(td, 2H) 4.71(brs, 1H) 6.40(tt, 1H) 7.43(dd, 1H) 7.55(d, 1H) 7.74(d, 1H) 8.03(s, 1H) |
| 33 | 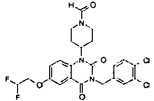 | 4-[3-(3,4-dichlorobenzyl)-6-(2,2-difluoroéthoxy)-2,4-dioxo-3,4-dihydroquinazolin-1(2H)-yl]pipéridine-1-carbaldéhyde | ¹H NMR (500 MHz, DMSO-*d*₆) δ ppm 1.80(dd, 2H) 2.32-2.43(m, 1H) 2.44-2.52(m, 1H) 2.80(td, 1H) 3.26(dt, 1H) 3.80(brd, 1H) 4.31(br d, 1H) 4.44(td, 2H) 4.78(brs, 1H) 5.09(s, 2H) 6.41(tt, 1H) 7.30(dd, 1H) 7.49(dd, 1H) 7.58(d, 1H) 7.60(d, 1H) 7.62(d, 1H) 7.81(d, 1H) 8.03(s, 1H) |
| 34 | 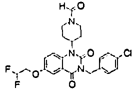 | 4-[3-(4-chlorobenzyl)-6-(2,2-difluoroéthoxy)-2,4-dioxo-3,4-dihydroquinazolin-1(2H)-yl]pipéridine-1-carbaldéhyde | ¹H NMR (500 MHz, DMSO-*d*₆) δ ppm 1.72-1.83(m, 2H) 2.32-2.44(m, 1H) 2.45-2.53(m, 1H) 2.80(dt, 1H) 3.25-3.30(dt, 1H) 3.80 (d, 1 H) 4.30 (d,1 H) 4.44 (td, 2H) 4.78(brs, 1H) 5.09(s, 2H) 6.42 (tt, 1H) 7.32-7.40(m, 4H) 7.48(dd, 1H) 7.61(d, 1H) 7.81(d, 1H) 8.03(s, 1H) |
| 35 | 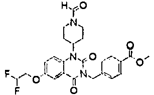 | 4-{[6-(2, 2-difluoroéthoxy)-1-(1-formylpipéridin-4-yl)-2,4-dioxo-1,4-dihydroquinazolin-3(2H)-yl]méthyl}benzoate de méthyle | ¹H NMR (500 MHz, DMSO-*d*₆) δ ppm 1.78(dd, 1H) 2.33-2.42(m, 1H) 2.44-2.52(m, 1H) 2.80(dt, 1H) 3.26(dt, 1H) 3.79(brd, 1H) 3.84(s, 3H) 4.30(brd, 1H) 4.45(td, 2H) 4.79(brs, 1H) 5.18(s, 2 H) 6.42(tt, 1H) 7.43(d, 2H) 7.49(dd, 1H) 7.62(d, 1H) 7.83(d, 1H) 7.91(d, 2H) 8.02(s, 1H) |
| 36 | 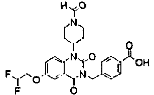 | acide 4-{[6-(2,2-difluoroéthoxy)-1-(1-formylpipéridin-4-yl)-2,4-dioxo-1,4-dihydroquinazolin-3(2H)-yl]méthyl}benzïque | ¹H NMR (500 MHz, DMSO-*d*₆) δ ppm 1.79(dd, 2H) 2.38(qd, 1H) 2.49(qd, 1H) 2.80(dt, 1H) 3.26(dt, 1H) 3.79(d, 1H) 4.31(d, 1H) 4.45(td, 2H) 4.79(brs, 1H) 5.17(s, 2H) 6.42(tt, 1H) 7.40(d, 2H) 7.49(dd, 1H) 7.62(d, 1H) 7.83(d, 1H) 7.88(d, 2H) 8.02(s, 1H) 12.94(brs, 1H) |
| 37 | 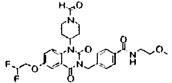 | 4-{[6-(2,2-difluoroéthoxy)-1-(1-formylpipéridin-4-yl)-2,4-dioxo-1,4-dihydroquinazolin-3(2H)-yl]méthyl}-N-(2-méthoxyéthyl)benzamide | ¹H NMR (500 MHz, DMSO-*d*₆) δppm 1.72-1.82(m, 2H) 2.32-2.42(m, 1H) 2.44-2.54(m, 1H) 2.75-2.83(m, 1H) 3.21-3.29(m, 4H) 3.37-3.45(m, 4H) 3.77(br.d., 1H) 4.29(br.d., 1H) 4.44(td, 2H) 4.78(br.s., 1H) 5.14(s, 2H) 6.41(tt, 1H) 7.35(m, 2H) 7.48(dd, 1H) 7.61(d, 1H) 7.77(m, 2H) 7.81 (d, 1H) 8.01 (s, 1H) 8.47(t, 1H) |
| 38 | 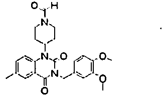 | 4-[3-(3,4-diméthoxybenzyl)-6-méthyl-2,4-dioxo-3,4-dihydroquinazolin-1(2H)-yl]pipéridine-1-carbaldéhyde | ¹H NMR (500 MHz, DMSO-*d*₆) δ ppm 1.76(dd, 2H) 2.37(s, 3H) 2.39-2.42(m, 1H) 2.50-2.54(m, 1H) 2.77-2.82(m, 1H) 3.19-3.30(m, 1H) 3.70(s, 6H) 3.79(d, 1H) 4.30(d, 1H) 4.76(brs, 1H) 5.02(s, 2H) 6.82(dd, 1H) 6.85(d, 1H) 6.97(d, 1H) 7.60(dd, 1H) 7.71(d, 1H) 7.89(s, 1H) 8.03(s, 1H) |
| 39 | 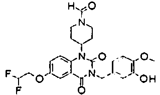 | 4-[6-(2,2-difluoroéthoxy)-3-(3-hydroxy-4-méthoxybenzyl)-2,4-dioxo-3,4-dihydroquinazolin-1(2H)-yl]pipéridine-1-carbaldéhyde | ¹H NMR (500 MHz, DMSO-*d*₆) δ ppm 1.70-1,80(m, 2H) 2.35-1,45(m, 1H) 2.47-2.56(m, 1H) 2.80(dt, 1H) 3.25(dt, 1H) 3.70(s, 3H) 3.79(brd, 1H) 4.30(brd, 1H) 4.43(td, 2H) 4.77(brs , 1H) 4.96(s, 2H) 6.41(tt, 1H) 6.73(d, 1H) 6.78-6.82(m, 2H) 7.46(dd, 1H) 7.60(d, 1H) 7.79(d, 1H) 8.03(s, 1H) 8.95 (s, 1H) |
| 40 | 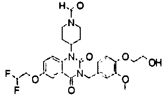 | 4-[6-(2,2-difluoroéthoxy)-3-[3-(2-hydroxyéthoxy)-4-méthoxybenzyl]-2,4-dioxo-3,4-dihydroquinazolin-1(2H)-yl]pipéridine-1-carbaldéhyde | ¹H NMR (600 MHz, DMSO-*d*₆) δ ppm 1.76(dd, 2H) 2.35-2.45(m, 1H) 2.50-2.54(m, 1H) 2.79(dt, 1H) 3.25(dt, 1H) 3.68(dt, 2H) 3.70(s, 3H) 3.79(brd, 1H) 3.90(t, 2H) 4.30(brd, 1H) 4.42(td, 2H) 4.75(brs, 1H) 4.77(t, 1H) 5.01(s, 2H) 6.39(tt, 1H) 6.85(m, 2H) 6.97(s, 1H) 7.45(dd, 1H) 7.60(d, 1H) 7.77(d, 1H) 8.02(s, 1H) |
| 41 | 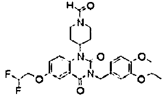 | 4-[6-(2,2-difluoroéthoxy)-3-(3-éthoxy-4-méthoxybenzyl)-2,4-dioxo-3,4-dihydroquinazolin-1(2H)-yl]pipéridine-1-carbaldéhyde | ¹H NMR (600 MHz, DMSO-*d*₆) δ ppm 1.29(t, 3H) 1.76(dd, 2H) 2.33-2.45(m, 1H) 2.50-2.56(m, 1H) 2.76-2.82(m, 1H) 3.21-3.31(m, 1H) 3.70(s, 3H) 3.79(brd, 1H) 3.94(q, 2H) 4.30(brd,1 H) 4.41(dt, 2H) 4.75(br5 , 1H) 5.01(s, 2H) 6.39(tt, 1H) 6.81-6.86(m, 2H) 6.96(s, 1H) 7.45(dd, 1H) 7.60(d, 1H) 7.77(d, 1H) 8.02(s, 1H) |
| 42 | 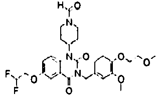 | 4-[6-(2,2-difluoroéthoxy)-3-[4-méthoxy-3-(2-méthoxyéthoxy)benzyl]-2,4-dioxo-3,4-dihydroquinazolin-1(2H)-yl]pipéridine-1-carbaldéhyde | ¹H NMR (600 MHz, DMSO-*d*₆) δ ppm 1.69-1.82(m, 2H) 2.35-2.45(m, 1H) 2.45-2.55(m, 1H) 2.78(t, 1H) 3.21-3.28(m, 1H) 3.61(brs, 2H) 3.70(s, 3H) 3.78(d, 1H) 4.00(brs, 2H) 4.30(d, 1H) 4.42(t, 2H) 4.75(brs, 1H) 5.01(s, 2H) 6.39(t, 1H) 6.82- 6.88(m, 2H) 6.97(s, 1H) 7.45(d, 1H) 7.59(s, 1H) 7.77(d, 1H) 8.02(s, 1H) |
| 43 | 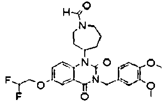 | 4-[6-(2,2-difluoroéthoxy)-3-(3,4-diméthoxybenzyl)-2,4-dioxo-3,4-dihydroquinazolin-1(2H)-yl]azépane-1-carbaldéhyde | ¹H NMR (500 MHz, DMSO-*d*₆) mélange de deux isomères δ ppm 1.72-1.81(m, 1H) 1.85(brd, 1H) 1.86-2.03(m, 2H) 2.47(brs, 2H) 3.30-3.40(m, 1H) 3.42-3.52(m, 1H) 3.58-3.64(m, 2H) 3.70(s, 3H) 3.71 (s, 3H) 4.42(td, 2H) 4.45(brs, 0.5H) 5.04(brs, 2H) 5.21(brs, 0.5H) 6.40(tt, 1H) 6.78-6.83(m, 1H) 6.86(d, 1H) 6.98(s, 1H) 7.38-7.48(m, 1H) 7.59(brs, 1H) 7.64-7.74(m, 1H) 8.08(brs, 0.5H) 8.12(s, 0.5H) |
| 44 | 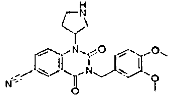 | 3-(3,4-diméthoxybenzyl)-2,4-dioxo-1-pyrrolidin-3-yl-1,2,3,4-tétrahydroquinazoline-6-carbonitrile | ¹H NMR (600 MHz, DMSO-*d*₆) δ ppm 1.96-2.09(m, 2H) 2.78-2.84(m, 1H) 3.05-3.12(m, 2H) 3.18-3.27(m, 1H) 3.70(s, 3H) 3.71(s, 3H) 5.04(s, 2H) 5.47-5.53(m, 1H) 6.83(brs, 2H) 6.99(s, 1H) 8.15(dd, 1H) 8.20(d, 1H) 8.47(d, 1H) |
| 45 | 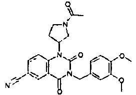 | 1-(1-acétylpyrrolidin-3-yl)-3-(3,4-diméthoxybenzyl)-2,4-dioxo-1,2,3,4-tétrahydroquinazoline-6-carbonitrile | ¹H NMR (600 MHz, DMSO-*d*₆) mélange de deux isomères δ ppm 1.91(s, 1.5H) 1.98(s, 1.5H) 2.14-2.22(m, 0.5H) 2.25-2.32(m, 0.5H) 2.43-2.50(m, 0.5H) 2.52-2.58(m, 0.5H) 3.31-3.39(m, 0.5H) 3.50-3.55(m, 0.5H) 3.63-3.73(m, 7.5H) 3.79-3.87(m, 1.5H) 5.00-5.06(m, 2H) 5.43-5.55(m, 1H) 6.82-6.90(m, 2H) 6.98(d, 1H) 7.84(dd, 1H) 8.17(dt, 1H) 8.43(dd, 1H) |
| 46 | 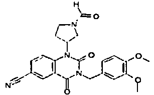 | 3-(3,4-diméthoxybenzyl)-1-(1-formylpyrrolidin-3-yl)-2,4-dioxo-1,2,3,4-tétrahydroquinazoline-6-carbonitrile | ¹H NMR (500 MHz, DMSO-*d*₆) mélange de deux isomères δ ppm 2.19-2.27(m, 1H) 2.36-2.43(m, 1H) 3.43-3.47(m, 0.4H) 3.53-3.59(m, 0.6) 3.62-3.73(m, 1.6H) 3.70(s, 3H) 3.71(s, 3H) 3.84-3.94(m, 1.4H) 5.04(s, 2H) 5.42-5.49(m, 0.4H) 5.50-5.56(m, 0.6H) 6.86(s, 2H) 6.99(s, 1H) 7.85(d, 0.6H) 7.90(d, 0.4H) 8.15-8.22(m, 2H) 8.45(s, 1H) |
| 47 | 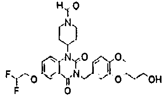 | 4-[6-(2,2-difluoroéthoxy)-3-[3-(3-hydroxypropoxy)-4-méthoxybenzyl]-2,4-dioxo-3,4-dihydroquinazolin-1(2H)-yl]pipéridine-1-carbaldéhyde | ¹H NMR (600 MHz, DMSO-*d*₆) δ ppm 1.72-1.79(m, 2H) 1.82(q, 2H) 2.36-2.44(m, 1H) 2.49-2.55(m, 1H) 2.78(td, 1H) 3.25(td, 1H) 3.51-3.54(m, 2H) 3.70(s, 3H) 3.76-3.80(m, 1H) 3.95(t, 2H) 4.28-4.32(m, 1H) 4.41 (td, 2H) 4.47(t, 1H) 4.75(s, 1H) 5.01(s, 2H) 6.39(tt, 1H) 6.81-6.86(m, 2H) 6.96-6.97(m, 1H) 7.45(dd, 1H) 7.59(d, 1H) 7.77(d, 1H) 8.02(s, 1H) |
| 48 | 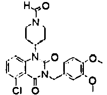 | 4-[5-chloro-3-(3,4-diméthoxybenz 2,4-dioxo-3,4-dihydroquinazolin-1(2H)-yl]pipéridine-1-carbaldéhyd | ¹H NMR (500 MHz, DMSO-*d*₆) δ ppm 1.78-1.88(m, 2H) 2.34-2.44(m, 1H) 2.46-2.56(m, 1H) 2.80(t, 1H) 3.25(t, 1H) 3.71(s, 6H) 3.80(d, 1H) 4.31(d, 1H) 4.71(brs, 1H) 5.00(s, 2H) 6.83(d, 1H) 6.87(d, 1H) 6.97(s, 1H) 7.37(d, 1H) 7.69(dd, 1H) 7.77(d, 1H) 8.03(s, 1H) |
| 49 | 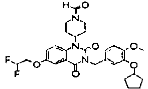 | 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde | 1H NMR (500 MHz, DMSO-*d*₆) δ ppm 1.5-1.6(m, 2H) 1.63-1.71 (m, 3H) 1.68-1.78(m, 1H) 1.78-1.98(m, 3H) 2.35-2.46(m, 1H) 2.47-2.57(m, 1H) 2.79(dt, 1H) 3.26(dt, 1H) 3.69(s, 3H) 3.79(br.d., 1H) 4.30(br.d., 1H) 4.43(td,2H) 4.66-4.70(m, 1H) 4.76(br.s., 1H) 5.01(s, 2H) 6.41 (tt, 1H) 6.82 - 6.86(m, 2H) 6.96(s, 1H) 7.46(dd, 1H) 7.6(d, 1H) 7.80(d, 1H) 8.03(s, 1H) |
| 50 | 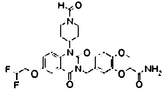 | 2-(5-{[6-(2,2-difluoroéthoxy)-1-(1-formylpipéridin-4-yl)-2,4-dioxo-1,4 dihydroquinazolin-3(2H)-yl]méthy 2-méthoxyphénoxy)acétamide | ¹H NMR (500 MHz, DMSO-*d*₆) δ ppm 1.72-1.82(m, 2H) 2.35-2.45(m, 1H) 2.46-2.56(m, 1H) 2.79(td, 1H) 3.25(td, 1H) 3.74(s, 3H) 3.79(d, 1H) 4.31(d, 1H) 4.35(s, 2H) 4.43(td, 2H) 4.76(brs, 1H) 5.00(s, 2H) 6.41(tt, 1H) 6.88-6.95(m, 3H) 7.31(brs, 1H) 7.37(brs, 1H) 7.46(dd, 1H) 7.59(d, 1H) 7.79(d, 1H) 8.02(s, 1H) |
| 51 | 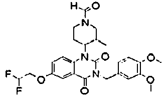 | 4-[6-(2,2-difluoroéthoxy)-3-(3,4-diméthoxybenzyl)-2,4-dioxo-3,4-dihydroquinazolin-1(2H)-yl]-3-méthylpipéridine-1-carbaldéhyde | ¹H NMR (500 MHz, DMSO-*d*₆) mélange de deux isomères δ ppm 0.92(t, 3H) 1.72-1.82(m, 1H) 2.23(brs, 1H) 2.70-2.77(m, 0.5H) 3.00-3.10(m, 0.5H) 3.12-3.20(m, 1.5H) 3.44(d, 0.5H) 3.63(dd, 0.5H) 3.69(s, 6H) 3.80(d, 0.5H) 4.04(d, 0.5H) 4.29(d, 0.5H) 4.43(td, 2H) 4.62-4.70(m, 1H) 5.03(s, 2H) 6.41(tt, 1H) 6.80(dd, 1H) 6.85(d, 1H) 6.97(d, 1H) 7.46(dd, 1H) 7.60(d, 1H) 7.64(dd, 1H) 7.97(s, 0.5H) 8.11(s, 0.5H) |
| 52 | 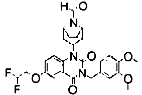 | 3-[6-(2,2-difluoroéthoxy)-3-(3,4-diméthoxybenzyl)-2,4-dioxo-3,4-dihydroquinazolin-1(2H)-yl]-8-azabicyclo[3.2.1]octane-8-carbaldéhyde | 1H NMR (600 MHz, DMSO-*d*₆) δ ppm 1.85-1.95(m, 4H) 2.18(t, 2H) 2.27-2.33(m, 1H) 2.34-2.41(m, 1H) 3.70(s, 1H) 3.71(s, 1H) 4.25-4.29(m, 1H) 4.40(td, 2H) 4.50-4.54(m , 1H) 4.55(br.s., 1H) 5.05(s, 2H) 6.38(tt, H) 6.80(d, 1H) 6.85(d, 1H) 6.98(s, 1H) 7.45(dd, 1H) 7.56(d, 1H) 7.58(d, 1H) 8.12(s, 1H) |
| 53 | 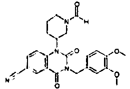 | 3-(3,4-diméthoxybenzyl)-1-(1-formylpipéridin-3-yl)-2,4-dioxo-1,2,3,4-tétrahydroquinazoline-6-carbonitrile | ¹H NMR (500 MHz, DMSO-*d*₆) mélange de deux isomères δ ppm 1.52-1.63(m, 0.5H) 1.65-1.73(m, 0.5H) 1.74-1.83(m, 1H) 1.87-1.93(m, 1H) 2.54-2.64(m, 1.5H) 3.06(dt, 0.5H) 3.57(t, 0.5H) 3.70(s, 3H) 3.71(s, 3H) 3.79(dd, 0.5H) 4.03(t, 0.5H) 4.20(d, 0.5H) 4.30(d, 0.5H) 4.38(brs, 0.5H) 4.53(brs, 0.5H) 4.99-5.07(m, 2H) 6.84-6.88(m, 2H) 6.98(s, 1H) 7.86(d, 0.5H) 8.01(d, 0.5H) 7.98(s, 0.5H) 8.08(s, 0.5H) 8.14-8.18(m, 1H) 8.44(s, 1H) |
| 54 | 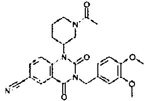 | 1-(1-acétylpipéridin-3-yl)-3-(3,4-diméthoxybenzyl)-2,4-dioxo-1,2,3,4-tétrahydroquinazoline-6-carbonitrile | ¹H NMR (500 MHz, DMSO-*d*₆) mélange de deux isomères δ ppm 1.56-1.66(m, 0.5H) 1.70-1.82(m, 1.5H) 1.87(brd, 1H) 1.97(s, 1.5H) 2.05(s, 1.5H) 2.54(dd, 1H) 3.05(brt, 0.5H) 3.48(t, 0.5H) 3.70-3.72(m, 6H) 3.83(d, 0.5H) 3.94(brd, 0.5H) 4.04(t, 0.5H) 4.33(brs, 0.5H) 4.45(dd, 1H) 4.53(brs, 0.5H) 4.99-5.07(m, 2H) 6.83-6.87(m, 2H) 6.98(d, 1H) 7.84(d, 0.5H) 8.02(d, 0.5H) 8.16(dt, 1H) 8.44(dd, 1H) |
| 55 | 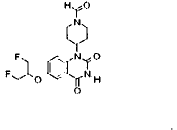 | 4-{6-[2-fluoro-1-(fluorométhyl)éthoxy]-2,4-dioxo-3,4-dihydroquinazolin-1(2H)-yl}pipéridine-1-carbaldéhyde | 1H NMR (600 MHz, DMSO-*d*₆) δ ppm 1.73-1.82(m, 2H) 2.36-2.44(m, 1H) 2.44-2.55(m, 1H) 2.79(td, 1H) 3.25(td, 1H) 3.80(brd, 1H) 4.32(brd, 1H) 4.62-4.67(m, 1H) 4.68-4.76(m, 3H) 4.76-4.82(m, 1H) 4.96-5.06(m, 1H) 7.44(dd, 1H) 7.61(d, 1H) 7.73(d, 1H) 8.03(s, 1H) 11.46(brs, 1H) |
| 56 | 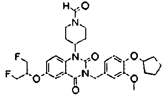 | 4-{3-[4-(cyclopentyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 572 |
| 57 | 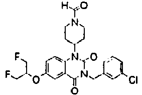 | 4-[3-(3-chlorobenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 492 |
| 58 | 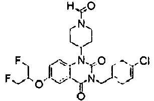 | 4-[3-(4-chlorobenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 492 |
| 59 | 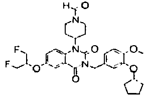 | 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 572 |
| 60 | 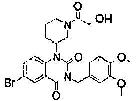 | 6-bromo-3-(3,4-diméthoxybenzyl)-1-[1-(hydroxyacétyl)pipéridin-3-yl]quinazoline-2,4(1H,3H)-dione | LCMS m/z [MH]+ 532 |
| 61 | 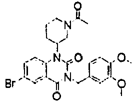 | 1-(1-acétylpipéridin-3-yl)-6-bromo-3-(34-diméthoxybenzyl)quinazoline-2,4(1H,3H)-dione | LCMS m/z [MH]+ 516 |
| 62 | 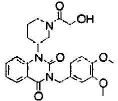 | 3-(3,4-diméthoxybenzyl)-1-[1-(hydroxyacétyl)pipéridin-3-yl]quinazoline-2,4(1H,3H)-dione | LCMS m/z [MH]+ 454 |
| 63 | 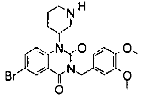 | 6-bromo-3-(3,4-diméthoxybenzyl)-1-[pipéridin-3-yl]quinazoline-2,4(1H,3H)-dione | LCMS m/z [MH]+ 474 |
| 64 | 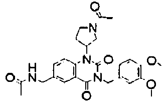 | N-{[1-(1-acétylpyrrolidin-3-yl)-3-(3,4-diméthoxybenzyl)-2,4-dioxo-1,2,3,4-tétrahydroquinazolin-6-yl]méthyl}acétamide | LCMS m/z [MH]+ 495 |
| 65 | 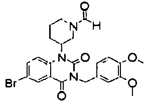 | 3-[6-bromo-3-(3,4-diméthoxybenzyl)-2,4-dioxo-3,4-dihydroquinazolin-1(2H)-yl]pipéridine-1-carbaldéhyde | LCMS m/z [MH]+ 502 |
| 66 | 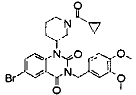 | 6-bromo-1-[1-(cyclopropylcarbonyl)pipéridi n-3-yl]-3-(3,4-diméthoxybenzyl)quinazoline -2,4(1H,3H)-dione | LCMS m/z [MH]+ 542 |
| 67 | 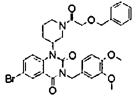 | 1-{1-[(benzyloxy)acétyl)pipéridin-3-yl}-6-bromo-3-(3,4-diméthoxybenzyl)quinazoline-2,4(1H,3H)-dione | LCMS m/z [MH]+ 622 |
| 68 | 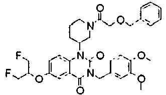 | 1-{1-[(benzyloxy)acétyl]pipéridin-3-yl}-3-(3,4-diméthoxybenzyl)-6-[2-fluoro-1-(fluorométhyl)éthoxy]quinazoline -2,4(1H,3H)-dione | LCMS m/z [MH]+ 638 |
| 69 | 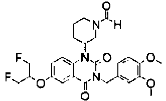 | 3-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 518 |
| 70 | 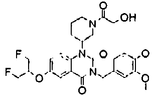 | 3-(3,4-diméthoxybenzyl)-6-[2-fluoro-1-(fluorométhyl)éthoxy]-1-[1-(hydroxyacétyl)pipéridin-3-yl]quinazoline-2,4(1H,3H)-dione | LCMS m/z [MH]+ 548 |
| 71 | 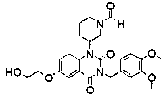 | 3-[3-(3,4-diméthoxybenryl)-6-(2-hydroxyéthoxy)-2,4-dioxo-3,4-dihydroquinazolin-1(2H)-yl]pipéridine-1-carbaldéhyde | LCMS m/z [MH]+ 484 |
| 72 | 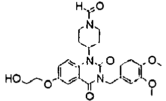 | 4-[3-(3,4-diméthoxybenzyl)-6-(2-hydroxyéthoxy)-2,4-dioxo-3,4-dihydroquinazolin-1(2H)-yl]pipéridine-1-carbaldéhyde | LCMS m/z [MH]+484 |
| 73 | 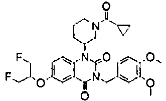 | 1-[1-(cyclopropylcarbonyl)pipéridin-3-yl]-3-(3,4-diméthoxybenzyl)-6-[2-fluoro-1-(fluorométhyl)éthoxy]quinazoli ne-2,4(1H,3H)-dione | LCMS m/z [MH]+ 558 |
| 74 | 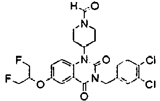 | 4-[3-(3,4-dichlorobenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 526 |
| 75 | 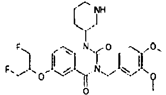 | 3-(3,4-diméthoxybenzyl)-6-[2-fluoro-1-(fluorométhyl)éthoxy]-1-pipéridin-3-ylquinazoline-2,4(1H,3H)-dione | LCMS m/z [MH]+ 490 |
| 76 | 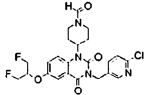 | 4-{3-[(6-chloropyridin-3-yl)méthyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 493 |
| 77 | 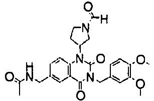 | N-{[3-(3,4-diméthoxybenzyl)-1-(1-formylpyrrolidin-3-yl)-2,4-dioxo-1,2,3,4-tétrahydroquinazolin-6-yl]méthyl}acétamide | LCMS m/z [MH]+ 481 |
| 78 | 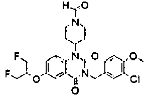 | 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 522 |
| 79 | 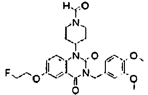 | 4-[3-(3,4-diméthoxybenzyl)-6-(2-fluoroéthoxy)-2,4-dioxo-3,4-dihydroquinazolin-1(2H)-yl]pipéridine-1-carbaldéhyde | LCMS m/z [MH]+ 486 |
| 80 | 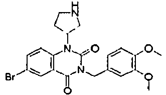 | 6-bromo-3-(3,4-diméthoxybenzyl)-1-[pyrrolidin-3-yl]quinazoline-2,4(1H,3H)-dione | LCMS m/z [MH]+ 460 |
| 81 | 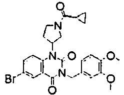 | 6-bromo-1-[1-(cyclopropylcarbonyl)pyrrolidin-3-yl]-3-(3,4-diméthoxybenzyl)quinazoline-2,4(1H,3H)-dione | LCMS m/z [MH]+ 528 |
| 82 | 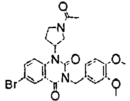 | 1-(1-acétylpyrrolidin-3-yl)-6-bromo-3-(3,4-diméthoxybenzyl)quinazoline-2,4(1H,3H)-dione | LCMS m/z [MH]+ 502 |
| 83 | 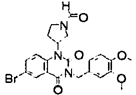 | 3-[6-bromo-3-(3,4-diméthoxybenzyl)-2,4-dioxo-3,4-dihydroquinazolin-1(2H)-yl]pyrrolidine-1-carbaldéhyde | LCMS m/z [MH]+ 488 |
| 84 | 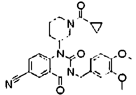 | 1-[1-(cyclopropylcarbonyl)pipéridin-3-yl]-3-(3,4-diméthoxybenzyl)-2,4-dioxo-1,2,3,4-tétrahydroquinazoline-6-carbonitrile | LCMS m/z [MH]+ 489 |
| 85 | 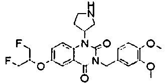 | 3-(3,4-diméthoxybenzyl)-6-[2-fluoro-1-(fluorométhyl)éthoxy]-1-pyrrolidin-3-ylquinazoline-2,4(1H,3H)-dione | LCMS m/z [MH]+ 476 |
| 86 | 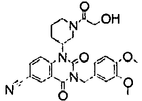 | 3-(3,4-diméthoxybenzyl)-1-[1-(hydroxyacétyl)pipéridin-3-yl]-2,4-dioxo-1,2,3,4-tétrahydroquinazoline-6-carbonitrile | LCMS m/z [MH]+ 479 |
| 87 | 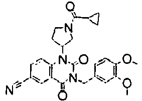 | 1-[1-(cyclopropylcarbonyl)pyrrolidin-3-yl]-3-(3,4-diméthoxybenzyl)-2,4-dioxo-1,2,3,4-tétrahydroquinazoline-6-carbonitrile | LCMS m/z [MH]+ 475 |
| 88 | 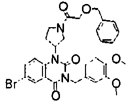 | 1-{1-[(benzyloxy)acétyl]pyrrolidin-3-yl}-6-bromo-3-(3,4-diméthoxybenzyl)quinazoline-2,4(1H,3H)-dione | LCMS m/z [MH]+ 608 |
| 89 | 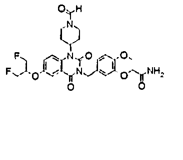 | 2-[5-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]acétamide | LCMS m/z [MH]+ 561 |
| 90 | 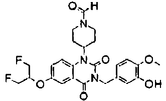 | 4-{6-[2-fluoro-1-(fluorométhyl)éthoxy]-3-(3-hydroxy-4-méthoxybenzyl)-2,4-dioxo-3,4-dihydroquinazolin-1(2H)-yl}pipéridine-1-carbaldéhyde | LCMS m/z [MH]+ 504 |
| 91 | 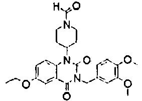 | 4-[3-(3,4-diméthoxybenzyl)-6-éthoxy-2,4-dioxo-3,4-dihydroquinazolin-1(2H)-yl]pipéridine-1-carbaldéhyde | LCMS m/z [MH]+ 468 |
| 92 | 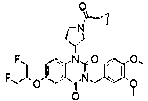 | 1-[1-(cyclopropylcarbonyl)pyrrolidin -3-yl]-3-(3,4-diméthoxybenzyl)-6-[2-fluoro-1-(fluorométhyl)éthoxy]quinazoli ne-2,4(1H,3H)-dione | LCMS m/z [MH]+ 544 |
| 93 | 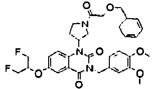 | 1-{1-[(benzyloxy)acétyl]pyrrolidin-3-yl}-3-(3,4-diméthoxybenzyl)-6-[2-fluoro-1-(fluorométhyl)éthoxy]quinazoli ne-2,4(1H,3H)-dione | LCMS m/z [MH]+ 624 |
| 94 | 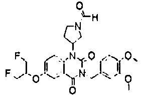 | 3-[3-(3,4-diméthoxybenzyl)-6-[2-fluoro-1-(fluorométhyl)éthoxy]-2,4-dioxo-3,4-dihydroquinazolin-1(2H)-yl]pyrrolidine-1-carbaldéhyde | LCMS m/z [MH]+ 504 |
| 95 | 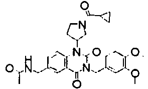 | N-({1-[1-(cyclopropylcarbonyl)pyrrolidin-3-yl]-3-(3,4-diméthoxybenzyl)-2,4-dioxo-1,2,3,4-tétrahydroquinazoli 6-yl}méthyl)acétamide | LCMS m/z [MH]+ 521 |
| 96 | 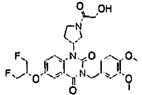 | 3-(3,4-diméthoxybenzyl)-6-[2-fluoro-1-(fluorométhyl)éthoxy]-1-[1-(hydroxyacétyl)pyrrolidin-3-yl]quinazoline-2,4(1H,3H)-dione | LCMS m/z [M+Na]+ 534 |
| 97 | 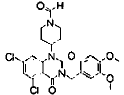 | 4-[5,7-dichloro-3-(3,4-diméthoxybenzyl)-2,4-dioxo-3,4-dihydroquinazolin-1(2H)-yl]pipéridine-1-carbaldéhyde | LCMS m/z [MH]+ 492 |
| 98 | 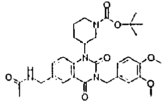 | 3-{6-[(acétylamino)méthyl]-3-(3,4-diméthoxybenzyl)-2,4-dioxo-3,4-dihydroquinazolin-1(2H)-yl}pipéridine-1-carboxylate de1,1-diméthyléthyle | LCMS m/z [M+Na]+ 589 |
| 99 | 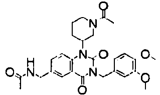 | N-{[3-(3,4-diméthoxybenzyl)-1-(1-formylpipéridin-3-yl)-2,4-dioxo-1,2,3,4-tétrahydroquinazolin-6-yl]méthyl}acétamide | LCMS m/z [MH]+ 495 |
| 100 | 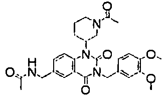 | N-{[1-(1-acétylpipéridin-3-yl)-3-(3,4-diméthoxybenzyl)-2,4-dioxo-1,2,3,4-tétrahydroquinazolin-6-yl]méthyl}acétamide | LCMS m/z [MH]+ 509 |
| 101 | 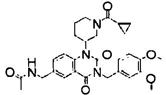 | N-({1-[1-(cyclopropylcarbonyl)pipéridin-3-yl]-3-(3,4-diméthoxybenzyl)-2,4-dioxo-1,2,3,4-tétrahydroquinazolin-6-yl}méthyl)acétamide | LCMS m/z [MH]+ 535 |
| 102 | 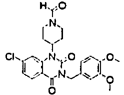 | 4-[7-chloro-3-(3,4-diméthoxybenzyl)-2,4-dioxo-3,4-dihydroquinazolin-1(2H)-yl]pipéridine-1-carbaldéhyde | LCMS m/z [MH]+ 458 |
| 103 | 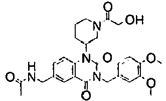 | N-({3-(3,4-diméthoxybenzyl)-1-[1-(hydroxyacétyl)pipéridin-3-yl]-2,4-dioxo-1,2,3,4-tétrahydroquinazolin-6-yl}méthyl)acétamide | LCMS m/z [MH]+ 525 |
| 104 | 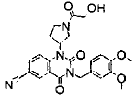 | 3-(3,4-diméthoxybenzyl)-1-[1-(hydroxyacétyl)pyrrolidin-3-yl]-2,4-dioxo-1,2,3,4-tétrahydroquinazoline-6-carbonitrile | LCMS m/z [MH]+ 465 |
| 105 | 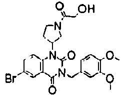 | 6-bromo-3-(3,4-diméthoxybenzyl)-1-[1-(hydroxyacétyl)pyrrolidin-3-yl]quinazoline-2,4(1H,3H)-dione | LCMS m/z [MH]+ 518 |
| 106 | 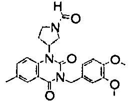 | 3-[3-(3,4-diméthoxybenzyl)-6-méthyl-2,4-dioxo-3,4-dihydroquinazolin-1(2H)-yl]pyrrolidine-1-carbaldéhyde | LCMS m/z [MH]+ 424 |
| 107 | 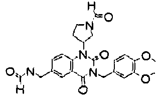 | N-{[3-(3,4-diméthoxybenzyl)-1-(1-formylpyrrolidin-3-yl)-2,4-dioxo-1,2,3,4-tétrahydroquinazolin-6-yl]méthyl}formamide | LCMS m/z [MH]+ 467 |
| 108 | 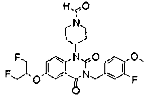 | 4-{6-[2-fluoro-1-(fluorométhyl)éthoxy]-3-(3-fluoro-4-méthoxybenzyl)-2,4-dioxo-3,4-dihydroquinazolin-1(2H)-yl}pipéridine-1-carbaldéhyde | LCMS m/z [MH]+ 506 |
| 109 | 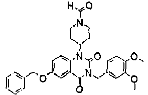 | 4-(6-(benzyloxy)-3-(3,4-diméthoxybenzyl)-2,4-dioxo-3,4-dihydroquinazolin-1(2H)-yl]pipéridine-1-carbaldéhyde | LCMS m/z [MH]+ 530 |
| 110 | 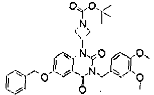 | 3-[6-(benzyloxy)-3-(3,4-diméthoxybenzyl)-2,4-dioxo-3,4-dihydroquinazolin-1(2H)-yl]azétidine-1-carboxylatede 1,1-diméthyléthyle | LCMS m/z [MH]+ 574 |
| 111 | 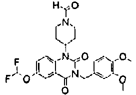 | 4-[6-(difluorométhoxy)-3-(3,4-diméthoxybenzyl)-2,4-dioxo-3,4-dihydroquinazolin-1(2H)-yl]pipéridine-1-carbaldéhyde | LCMS m/z [MH]+ 490 |
| 112 | 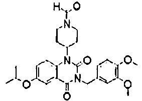 | 4-[3-(3,4-diméthoxybenzyl)-6-(1-méthyléthoxy)-2,4-dioxo-3,4-dihydroquinazolin-1(2H)-yl]pipéridine-1-carbaldéhyde | LCMS m/z [MH]+ 482 |
| 113 | 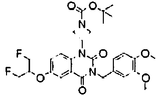 | 3-[3-(3,4-diméthoxybenzyl)-6-[2-fluoro-1-(fluorométhyl)éthoxy]-2,4-dioxo-3,4-dihydroquinazolin-1(2H)-yl]azétidine-1-carboxylate de 1,1-diméthyléthyle | LCMS m/z [MH]+ 562 |
| 114 | 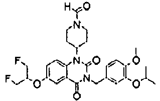 | 4-{6-[2-fIuoro-1-(fluorométhyl)éthoxy]-3-[4-méthoxy-3-(1-méthyléthoxy)benzyl]-2,4-dioxo-3,4-dihydroquinazolin-1(2H)-yl}pipéridine-1-carbaldéhyde | LCMS m/z [MH]+ 546 |
| 115 | 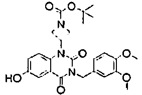 | 3-[3-(3,4-diméthoxybenzyl)-6-hydroxy-2,4-dioxo-3,4-dihydroquinazolin-1(2H)-yl]azétidine-1-carboxylate de1,1-diméthyléthyle | LCMS m/z [MH]+ 484 |
| 116 | 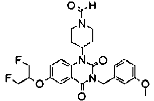 | 4-{6-[2-fluoro-1-(fluorométhyl)éthoxy]-3-(3-méthoxybenzyl)-2,4-dioxo-3,4-dihydroquinazolin-1(2H)-yl}pipéridine-1-carbaldéhyde | LCMS m/z [MH]+ 488 |
| 117 | 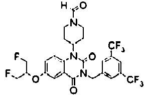 | 4-{3-[3,5-bis(trifluorométhyl)benzyl]-6-[2-fluoro-1-(fluorométhyl)éthoxy]-2,4-dioxo-3,4-dihydroquinazolin-1(2H)-yl}pipéridine-1-carbaldéhyde | LCMS m/z [M]+ 564 |
| 118 | 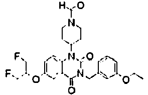 | 4-[3-(3-éthoxybenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 502 |

| ***Composé No*** | ***STRUCTURE*** | ***NOMENCLATURE*** | ***MASSE LCUVMS MH+*** |
|---|---|---|---|
| 119 | 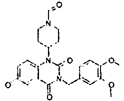 | 4-[3-(3,4-diméthoxybenzyl)-6-hydroxy-2,4-dioxo-3,4-dihydroquinazolin-1(2H)-yl]pipéridine-1-carbaldéhyde | 440 |
| 120 | 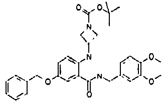 | 3-({4-(benzyloxy)-2-[(3,4-diméthoxybenzyl)carbamoyl]phényl}amino)azétidine-1-carboxylate de 1,1-diméthyléthyle | 548 |
| 121 | 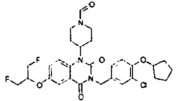 | 4-{3-[3-chloro-4-(cyclopentyloxy)benzyl]-6-[2-fluoro-1-(fluorométhyl)éthoxy]-2,4-dioxo-3,4-dihydroquinazolin-1(2H)-yl}pipéridine-1-carbaldéhyde | 576 |
| 122 | 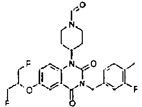 | 4-{6-[2-fluoro-1-(fluorométhyl)éthoxy]-3-(3-fluoro-4-méthylbenzyl)-2,4-dioxo-3,4-dihydroquinazolin-1(2H)-yl}pipéridine-1-carbaldéhyde | 490 |
| 123 | 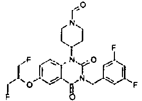 | 4-[3-(3,5-difluorobenzyl)-6-[2-fluoro-1-(fluorométhyl)éthoxy]-2,4-dioxo-3,4-dihydroquinazolin-1(2H)-yl]pipéridine-1-carbaldéhyde | 494 |
| 124 | 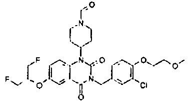 | 4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde | 566 |
| 125 | 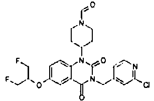 | 4-{3-[(2-chloropyridin-4-yl)méthyl]-6-[2-fluoro-1-(fluorométhyl)éthoxy]-2,4-dioxo-3,4-dihydroquinazolin-1(2H)-yl}pipéridine-1-carbaldéhyde | 493 |
| 126 | 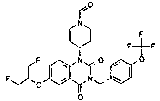 | 4-{6-[2-fluoro-1-(fluorométhyl)éthoxy]-2,4-dioxo-3-[4-(trifluorométhoxy)benzyl]-3,4-dihydroquinazolin-1(2H)-yl}pipéridine-1-carbaldéhyde | 542 |
| 127 | 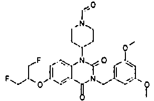 | 4-[3-(3,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 518 |
| 128 | 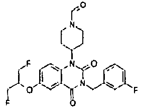 | 4-[3-(3-fluorobenzyl)-6-[2-fluoro-1-(fluorométhyl)éthoxy]-2,4-dioxo-3,4-dihydroquinazolin-1(2H)-yl]pipéridine-1-carbaldéhyde | 476 |
| 129 | 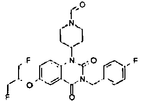 | 4-[3-(4-fluorobenzyl)-6-[2-fluoro-1-(fluorométhyl)éthoxy]-2,4-dioxo-3,4-dihydroquinazolin-1(2H)-yl]pipéridine-1-carbaldéhyde | 476 |
| 130 | 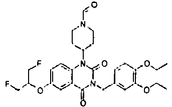 | 4-[3-(3,4-diéthoxybenzyl)-6-[2-fluoro-1-(fluorométhyl)éthoxy]-2,4-dioxo-3,4-dihydroquinazolin-1(2H)-yl]pipéridine-1-carbaldéhyde | 546 |
| 131 | 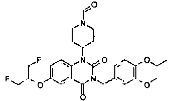 | 4-[3-(4-éthoxy-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde | 532 |
| 132 | 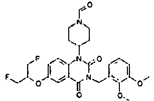 | 4-[3-(2,3-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 518 |
| 133 | 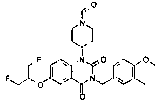 | 4-{6-[2-fluoro-1-(fluorométhyl)éthoxy]-3-(4-méthoxy-3-méthylbenzyl)-2,4-dioxo-3,4-dihydroquinazolin-1(2H)-yl}pipéridine-1-carbaldéhyde | 502 |
| 134 | 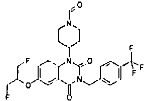 | 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde | 526 |
| 135 | 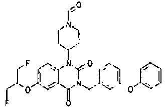 | 4-{6-[2-fluoro-1-(fluorométhyl)éthoxy]-2,4-dioxo-3-(3-phénoxybenzyl)-3,4-dihydroquinazolin-1(2H)-yl}pipéridine-1-carbaldéhyde | 550 |
| 136 | 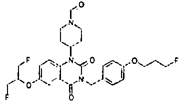 | 4-{6-[2-fluoro-1-(fluorométhyl)éthoxy]-3-[4-(3-fluoropropoxy)benzylj-2,4-dioxo-3,4-dihydroquinazolin-1(2H)-yl}pipéridine-1-carbaldéhyde | 534 |
| 137 | 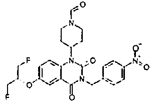 | 4-{6-[2-fluoro-1-(fluorométhyl)éthoxy]-3-(4-nitrobenzyl)-2,4-dioxo-3,4-dihydroquinazolin-1(2H)-yl}pipéridine-1-carbaldéhyde | 503 |
| 138 | 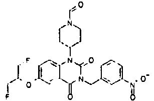 | 4-{6-[2-fluoro-1-(fluorométhyl)éthoxy]-3-(3-nitrobenzyl)-2,4-dioxo-3,4-dihydroquinazolin-1(2H)-yl}pipéridine-1-carbaldéhyde | 503 |
| 139 | 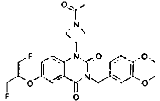 | 1-(1-acétylazétidin-3-yl)-3-(3,4-diméthoxybenzyl)-6-[2-fluoro-1-(fluorométhyl)éthoxy]quinazoline-2,4(1H,3H)-dione | 504 |
| 140 | 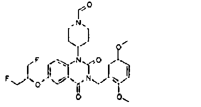 | 4-[3-(2,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 518 |
| 141 | 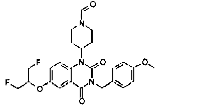 | 4-{6-[2-fluoro-1-(fluorométhyl)éthoxy]-3-(4-méthoxybenzyl)-2,4-dioxo-3,4-dihydroquinazolin-1(2H)-yl}pipéridine-1-carbaldéhyde | 488 |
| 142 | 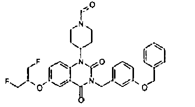 | 4-{3-[3-(benzyloxy)benzyl]-6-[2-fluoro-1-(fluorométhyl)éthoxy]-2,4-dioxo-3,4-dihydroquinazolin-1(2H)-yl}pipéridine-1-carbaldéhyde | 564 |
| 143 | 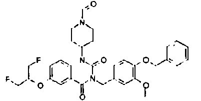 | 4-{3-[4-(benzyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 594 |
| 144 | 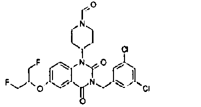 | 4-[3-(3,5-dichlorobenzyl)-6-[2-fluoro-1-(fluorométhyl)éthoxy]-2,4-dioxo-3,4-dihydroquinazolin-1(2H)-yl]pipéridine-1-carbaldéhyde | 526 |
| 145 | 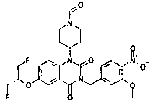 | 4-{6-[2-fluoro-1-(fluorométhyl)éthoxy]-3-(3-méthoxy-4-nitrobenzyl)-2,4-dioxo-3,4-dihydroquinazolin-1(2H)-yl}pipéridine-1-carbaldéhyde | 533 |
| 146 | 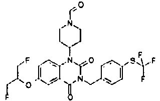 | 4-{6-[2-fluoro-1-(fluorométhyl)éthoxy]-2,4-dioxo-3-{4-[(trifluorométhyl)sulfanyl]benzyl}-3,4-dihydroquinazolin-1(2H)-yl}pipéridine-1-carbaldéhyde | 558 |
| 147 | 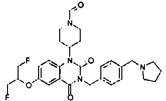 | 4-{6-[2-fluoro-1-(fluorométhyl)éthoxy]-2,4-dioxo-3-[4-(pyrrolidin-1-ylméthyl)benzyl]-3,4-dihydroquinazolin-1(2H)-yl}pipéridine-1-carbaldéhyde | 541 |
| 148 | 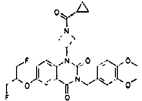 | 1-[1-(cyclopropylcarbonyl)azétidin-3-yl]-3-(3,4-diméthoxybenzyl)-6-[2-fluoro-1-(fluorométhyl)éthoxy]quinazoline-2,4(1H,3H)-dione | 530 |
| 149 | 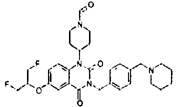 | 4-{6-[2-fluoro-1-(fluorométhyl)éthoxy]-2,4-dioxo-3-[4-(pipéridin-1-ylméthyl)benzyl]-3,4-dihydroquinazolin-1(2H)-yl}pipéridine-1-carbaldéhyde | 555 |
| 150 | 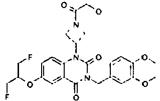 | 3-(3,4-diméthoxybenzyl)-6-[2-fluoro-1-(fluorométhyl)éthoxy]-1-[1-(hydroxyacétyl)azétidin-3-yl]quinazoline-2,4(1H,3H)-dione | 520 |
| 151 | 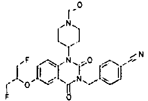 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile | 483 |
| 152 | 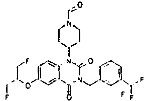 | 4-{6-[2-fluoro-1-(fluorométhyl)éthoxy]-2,4-dioxo-3-[3-(trifluorométhyl)benzyl]-3,4-dihydroquinazolin-1(2H)-yl}pipéridine-1-carbaldéhyde | 526 |
| 153 | 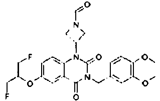 | 3-[3-(3,4-diméthoxybenzyl)-6-[2-fluoro-1-(fluorométhyl)éthoxy]-2,4-dioxo-3,4-dihydroquinazolin-1(2H)-yl]azétidine-1-carbaldéhyde | 490 |
| 154 | 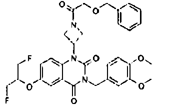 | 1-{1-[(benzyloxy)acétyl]azétidin-3-yl}-3-(3,4-diméthoxybenzyl)-6-[2-fluoro-1-(fluorométhyl)éthoxy]quinazoline-2,4(1H,3H)-dione | 610 |
| 155 | 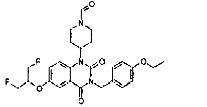 | 4-[3-(4-éthoxybenzyl)-6-[2-fluoro-1-(fluorométhyl)éthoxy]-2,4-dioxo-3,4-dihydroquinazolin-1(2H)-yl]pipéridine-1-carbaldéhyde | 502 |
| 156 | 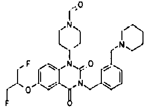 | 4-{6-[2-fluoro-1-(fluorométhyl)éthoxy]-2,4-dioxo-3-[3-(pipéridin-1-ylméthyl)benzyl]-3,4-dihydroquinazolin-1(2H)-yl}pipéridine-1-carbaldéhyde | 555 |
| 157 | 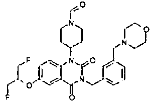 | 4-{6-[2-fluoro-1-(fluorométhyl)éthoxy]-3-[3-(morpholin-4-ylméthyl)benzyl]-2,4-dioxo-3,4-dihydroquinazolin-1(2H)-yl}pipéridine-1-carbaldéhyde | 557 |
| 158 | 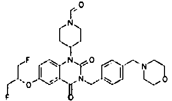 | 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde | 557 |
| 159 | 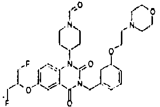 | 4-{6-[2-fluoro-1-(fluorométhyl)éthoxy]-3-[3-(2-morpholin-4-yléthoxy)benzyl]-2,4-dioxo-3,4-dihydroquinazolin-1(2H)-yl}pipéridine-1-carbaldéhyde | 587 |
| 160 | 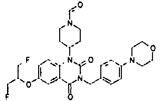 | 4-{6-[2-fluoro-1-(fluorométhyl)éthoxy]-3-(4-morpholin-4-ylbenzyl)-2,4-dioxo-3,4-dihydroquinazolin-1(2H)-yl)pipéridine-1-carbaldéhyde | 543 |
| 161 | 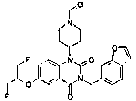 | 4-[3-(1-benzofur-6-ylméthyl)-6-[2-fluoro-1-(fluorométhyl)éthoxy]-2,4-dioxo-3,4-dihydroquinazolin-1(2H)-yl]pipéridine-1-carbaldéhyde | 498 |
| 162 | 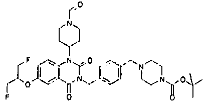 | 4-[4-({6-[2-fluoro-1-(fluorométhyl)éthoxy]-1-(1-formylpipéridin-4-yl)-2,4-dioxo-1,4-dihydroquinazolin-3(2H)-yl}méthyl)benzyl]pipérazine-1-carboxylate de 1,1-diméthyléthyle | 656 |
| 163 | 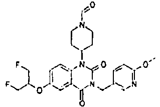 | 4-{6-[2-fluoro-1-(fluorométhyl)éthoxy]-3-[(6-méthoxypyridin-3-yl)méthyl]-2,4-dioxo-3,4-dihydroquinazolin-1(2H)-yl}pipéridine-1-carbaldéhyde | 489 |
| 164 | 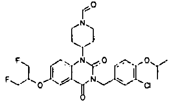 | 4-{3-[3-chloro-4-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 550 |
| 165 | 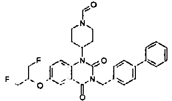 | 4-[3-(biphényl-4-ylméthyl)-6-[2-fluoro-1-(fluorométhyl)éthoxy]-2,4-dioxo-3,4-dihydroquinazolin-1(2H)-yl]pipéridine-1-carbaldéhyde | 534 |
| 166 | 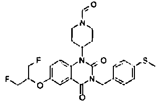 | 4-{6-[2-fluoro-1-(fluorométhyl)éthoxy]-3-[4-(méthylsulfanyl)benzyl]-2,4-dioxo-3,4-dihydroquinazolin-1(2H)-yl}pipéridine-1-carbaldéhyde | 504 |
| 167 | 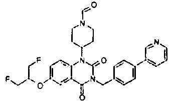 | 4-{6-[2-fluoro-1-(fluorométhyl)éthoxy]-2,4-dioxo-3-(4-pyridin-3-ylbenzyl)-3,4-dihydroquinazolin-1(2H)-yl}pipéridine-1-carbaldéhyde | 535 |
| 168 | 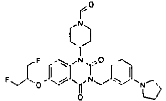 | 4-{6-[2-fluoro-1-(fluorométhyl)éthoxy]-2,4-dioxo-3-(3-pyrrolidin-1-ylbenzyl)-3,4-dihydroquinazolin-1(2H)-yl}pipéridine-1-carbaldéhyde | 527 |
| 169 | 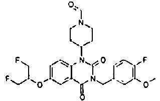 | 4-{6-[2-fluoro-1-(fluorométhyl)éthoxy]-3-(4-fluoro-3-méthoxybenzyl)-2,4-dioxo-3,4-dihydroquinazolin-1(2H)-yl}pipéridine-1-carbaldéhyde | 506 |
| 170 | 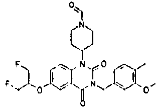 | 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde | 502 |
| 171 | 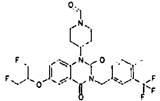 | 4-{6-[2-fluoro-1-(fluorométhyl)éthoxy]-3-[4-méthyl-3-(trifluorométhyl)benzyl]-2,4-dioxo-3,4-dihydroquinazolin-1(2H)-yl}pipéridine-1-carbaldéhyde | 540 |
| 172 | 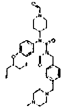 | 4-{6-[2-fluoro-1-(fluorométhyl)éthoxy]-3-{4-[(4-méthylpipérazin-1-yl)méthyl]benzyl}-2,4-dioxo-3,4-dihydroquinazolin-1(2H)-yl}pipéridine-1-carbaldéhyde | 570 |
| 173 | 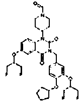 | 4-[3-{4-(cyclopentyloxy)-3-[2-fluoro-1-(fluorométhyl)éthoxy]benzyl}-6-[2-fluoro-1-(fluorométhyl)éthoxy]-2,4-dioxo-3,4-dihydroquinazolin-1(2H)-yl]pipéridine-1-carbaldéhyde | 636 |
| 174 | 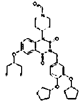 | 3-[3,4-bis(cyclopentyloxy)benzyl]-1-(1-éthénylpipéridin-4-yl)-6-[2-fluoro-1-(fluorométhyl)éthoxy]quinazoline-2,4(1H,3H)-dione | 626 |
| 175 | 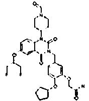 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide | 615 |
| 176 | 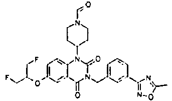 | 4-{6-[2-fluoro-1-(fluorométhyl)éthoxy]-3-[3-(5-méthyl-1,2,4-oxadiazol-3-yl)benzyl]-2,4-dioxo-3,4-dihydroquinazolin-1(2H)-yl}pipéridine-1-carbaldéhyde | 540 |
| 177 | 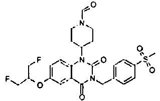 | 4-{6-[2-fluoro-1-(fluorométhyl)éthoxy]-3-[4-(méthylsulfonyl)benzyl]-2,4-dioxo-3,4-dihydroquinazolin-1(2H)-yl}pipéridine-1-carbaldéhyde | 536 |
| 178 | 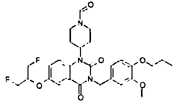 | 4-{6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde | 546 |
| 179 | 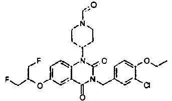 | 4-[3-(3-chloro-4-éthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 180 | 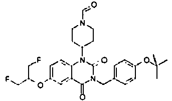 | 4-{3-[4-(1,1-diméthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 530 |
| 181 | 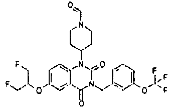 | 4-(6-[2-fluoro-1-(fluorométhyl)éthoxy]-2,4-dioxo-3-[3-(trifluorométhoxy)benzyl]-3,4-dihydroquinazolin-1(2H)-yl}pipéridine-1-carbaldéhyde | 542 |
| 182 | 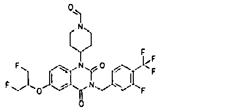 | 4-{6-[2-fluoro-1-(fluorométhyl)éthoxyJ-3-[3-fluoro-4-(trifluorométhyl)benzyl]-2,4-dioxo-3,4-dihydroquinazolin-1(2H)-yl}pipéridine-1-carbaldéhyde | 544 |
| 183 | 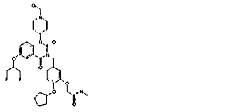 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylacétamide | 629 |
| 184 | 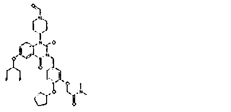 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N,N-diméthylacétamide | 643 |
| 185 | 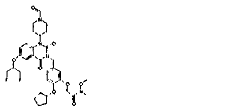 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide | 659 |
| 186 | 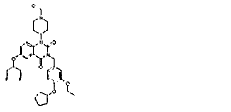 | 4-{3-[4-(cyclopentyloxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde | 586 |
| 187 | 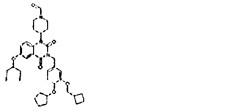 | 4-{3-[3-(cyclobutylméthoxy)-4-(cyclopentyloxy)benzyl]-6-[2-fluoro-1-(fluorométhyl)éthoxy]-2,4-dioxo-3,4-dihydroquinazolin-1(2H)-yl}pipéridine-1-carbaldéhyde | 626 |
| 188 | 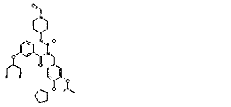 | 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 600 |
| 189 | 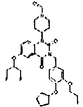 | 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde | 600 |
| 190 | 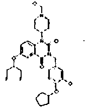 | 4-{3-[4-(cyclopentyloxy)-3-hydroxybenzyl]-6-[2-fluoro-1-(fluorométhyl)éthoxy]-2,4-dioxo-3,4-dihydroquinazolin-1(2H)-yl}pipéridine-1-carbaldéhyde | 558 |
| 191 | 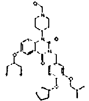 | 4-{3-[4-(cyclopentyloxy)-3-(2-méthylpropoxy)benzyl]-6-[2-fluoro-1-(fluorométhyl)éthoxy]-2,4-dioxo-3,4-dihydroquinazolin-1(2H)-yl)pipéridine-1-carbaldéhyde | 614 |
| 192 | 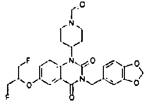 | 4-[3-(1,3-benzodioxol-5-ylméthyl)-6-[2-fluoro-1-(fluorométhyl)éthoxy]-2,4-dioxo-3,4-dihydroquinazolin-1(2H)-yl]pipéridine-1-carbaldéhyde | 502 |
| 193 | 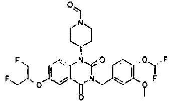 | 4-{3-[4-(difluorométhoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 554 |
| 194 | 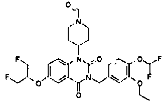 | 4-{3-[4-(difluorométhoxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde | 568 |
| 195 | 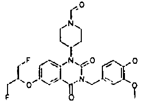 | 4-{6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde | 504 |
| 196 | 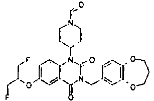 | 4-[3-(3,4-dihydro-2H-1,5-benzodioxépin-7-ylméthyl)-6-[2-fluoro-1-(fluorométhyl)éthoxy]-2,4-dioxo-3,4-dihydroquinazolin-1(2H)-yl]pipéridine-1-carbaldéhyde | 530 |
| 197 | 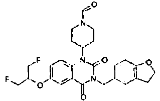 | 4-[3-(2,3-dihydro-1-benzofur-5-ylméthyl)-6-[2-fluoro-1-(fluorométhyl)éthoxy]-2,4-dioxo-3,4-dihydroquinazolin-1(2H)-yl]pipéridine-1-carbaldéhyde | 500 |
| 198 | 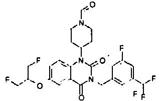 | 4-{6-[2-fluoro-1-(fluorométhyl)éthoxy]-3-[3-fluoro-5-(trifluorométhyl)benzyl]-2,4-dioxo-3,4-dihydroquinazolin-1(2H)-yl}pipéridine-1-carbaldéhyde | 544 |
| 199 | 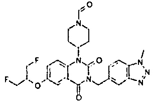 | 4-(6-[2-fluoro-1-(fluorométhyl)éthoxyl-3-[(1-méthyl-1H-benzotriazol-5-yl)méthyl]-2,4-dioxo-3,4-dihydroquinazolin-1(2H)-yl}pipéridine-1-carbaldéhyde | 513 |
| 200 | 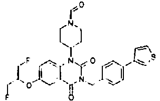 | 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde | 540 |
| 201 | 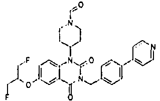 | 4-{6-[2-fluoro-1-(fluorométhyl)éthoxy]-2,4-dioxo-3-(4-pyridin-4-ylbenzyl)-3,4-dihydroquinazolin-1(2H)-yl}pipéridine-1-carbaldéhyde | 535 |
| 202 | 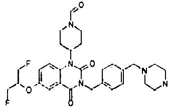 | 4-{6-[2-fluoro-1-(fluorométhyl)éthoxy]-2,4-dioxo-3-[4-(pipérazin-1-ylméthyl)benzyl]-3,4-dihydroquinazolin-1(2H)-yl}pipéridine-1-carbaldéhyde | 555 |
| 203 | 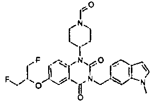 | 4-{6-[2-fluoro-1-(fluorométhyl)éthoxy]-3-[(1-méthyl-1H-indol-6-yl)méthyl]-2,4-dioxo-3,4-dihydroquinazolin-1(2H)-yl}pipéridine-1-carbaldéhyde | 511 |
| 204 | 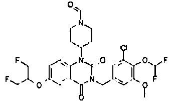 | 4-{3-[3-chloro-4-(difluorométhoxy)-5-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 588 |
| 205 | 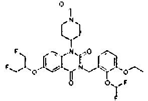 | 4-(3-[2-(difluorométhoxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde | 568 |
| 206 | 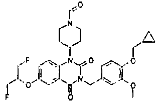 | 4-{3-[4-(cyclopropylméthoxy)-3-méthoxybenzyl]-6-[2-fluoro-1-(fluorométhyl)éthoxy]-2,4-dioxo-3,4-dihydroquinazolin-1(2H)-yl)pipéridine-1-carbaldéhyde | 558 |
| 207 | 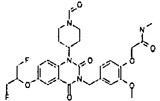 | 2-[4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]-N-méthylacétamide | 575 |
| 208 | 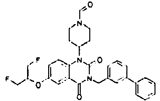 | 4-[3-(biphényl-3-ylméthyl)-6-[2-fluoro-1-(fluorométhyl)éthoxy]-2,4-dioxo-3,4-dihydroquinazolin-1(2H)-yl]pipéridine-1-carbaldéhyde | 534 |
| 209 | 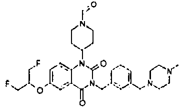 | 4-{6-[2-fluoro-1-(fluorométhyl)éthoxy]-3-{3-[(4-méthylpipérazin-1-yl)méthyl]benzyl}-2,4-dioxo-3,4-dihydroquinazolin-1(2H)-yl}pipéridine-1-carbaldéhyde | 570 |
| 210 | 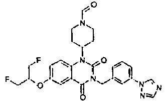 | 4-{6-[2-fluoro-1-(fluorométhyl)éthoxy]-2,4-dioxo-3-[3-(1H-1,2,4-triazol-1-yl)benzyl]-3,4-dihydroquinazolin-1(2H)-yl}pipéridine-1-carbaldéhyde | 525 |
| 211 | 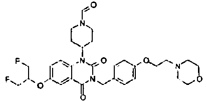 | 4-{6-[2-fluoro-1-(fluorométhyl)éthoxy]-3-[4-(2-morpholin-4-yléthoxy)benzyl]-2,4-dioxo-3,4-dihydroquinazolin-1(2H)-yl}pipéridine-1-carbaldéhyde | 587 |
| 212 | 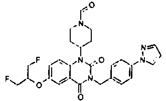 | 4-{6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde | 524 |
| 213 | 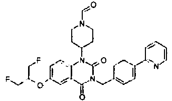 | 4-{6-[2-fluoro-1-(fluorométhyl)éthoxy]-2,4-dioxo-3-(4-pyridin-2-ylbenzyl)-3,4-dihydroquinazolin-1(2H)-yl}pipéridine-1-carbaldéhyde | 535 |
| 214 | 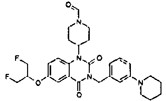 | 4-{6-[2-fluoro-1-(fluorométhyl)éthoxy]-2,4-dioxo-3-(3-pipéridin-1-ylbenzyl)-3,4-dihydroquinazolin-1(2H)-yl)pipéridine-1-carbaldéhyde | 541 |
| 215 | 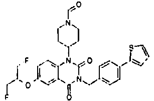 | 4-{6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde | 540 |
| 216 | 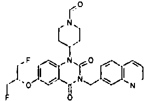 | 4-{6-[2-fluoro-1-(fluorométhyl)éthoxy]-2,4-dioxo-3-(quinoléin-7-ylméthyl)-3,4-dihydroquinazolin-1(2H)-yl}pipéridine-1-carbaldéhyde | 509 |
| 217 | 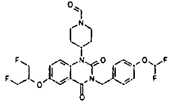 | 4-{3-[4-(difluorométhoxy)benzyl]-6-[2-fluoro-1-(fluorométhyl)éthoxy]-2,4-dioxo-3,4-dihydroquinazolin-1(2H)-yl}pipéridine-1-carbaldéhyde | 524 |
| 218 | 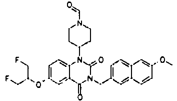 | 4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde | 538 |
| 219 | 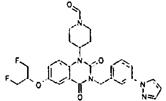 | 4-{6-[2-fluoro-1-(fluorométhyl)éthoxy]-2,4-dioxo-3-[3-(1H-pyrazol-1-yl)benzyl]-3,4-dihydroquinazolin-1(2H)-yl}pipéridine-1-carbaldéhyde | 524 |
| 220 | 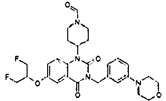 | 4-{6-[2-fluoro-1-(fluorométhyl)éthoxy]-3-(3-morpholin-4-ylbenzyl)-2,4-dioxo-3,4-dihydroquinazolin-1(2H)-yl}pipéridine-1-carbaldéhyde | 543 |
| 221 | 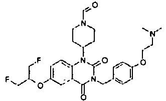 | 4-[3-{4-[2-(diméthylamino)éthoxy]benzyl}-6-[2-fluoro-1-(fluorométhyl)éthoxy]-2,4-dioxo-3,4-dihydroquinazolin-1(2H)-yl]pipéridine-1-carbaldéhyde | 545 |
| 222 | 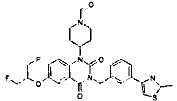 | 4-{6-[2-fluoro-1-(fluorométhyl)éthoxy]-3-[3-(2-méthyl-1,3-thiazol-4-yl)benzyl]-2,4-dioxo-3,4-dihydroquinazolin-1(2H)-yl}pipéridine-1-carbaldéhyde | 555 |
| 223 | 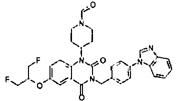 | 4-{3-[4-(1H-benzimidazol-1-yl)benzyl]-6-[2-fluoro-1-(fluorométhyl)éthoxy]-2,4-dioxo-3,4-dihydroquinazolin-1(2H)-yl}pipéridine-1-carbaldéhyde | 574 |
| 224 | 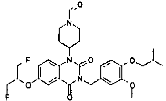 | 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde | 560 |
| 225 | 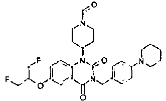 | 4-{6-[2-fluoro-1-(fluorométhyl)éthoxy]-2,4-dioxo-3-(4-pipéridin-1-ylbenzyl)-3,4-dihydroquinazolin-1(2H)-yl}pipéridine-1-carbaldéhyde | 541 |
| 226 | 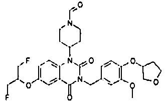 | 4-(6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl)pipéridine-1-carbaldéhyde | 574 |
| 227 | 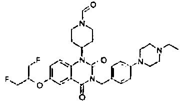 | 4-{3-[4-(4-éthylpipérazin-1-yl)benzyl]-6-[2-fluoro-1-(fluorométhyl)éthoxy]-2,4-dioxo-3,4-dihydroquinazolin-1(2H)-yl}pipéridine-1-carbaldéhyde | 570 |
| 228 | 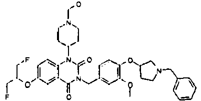 | 4-[3-{4-[(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 663 |
| 229 | 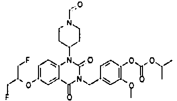 | carbonate de 4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle et de 1-méthyléthyle | 590 |
| 230 | 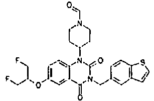 | 4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde | 514 |
| 231 | 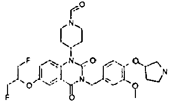 | 4-{6-[2-fluoro-1-(fluorométhyl)éthoxy]-3-[3-méthoxy-4-(pyrrolidin-3-yloxy)benzyl]-2,4-dioxo-3,4-dihydroquinazolin-1(2H)-yl}pipéridine-1-carbaldéhyde | 573 |
| 232 | 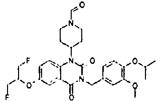 | 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde | 546 |
| 233 | 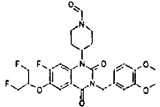 | 4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 536 |
| 234 | 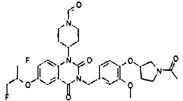 | 4-[3-{4-[(1-acétylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 615 |
| 235 | 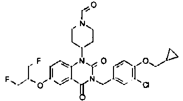 | 4-{3-[3-chloro-4-(cyclopropylméthoxy)benzyl]-6-[2-fluoro-1-(fluorométhyl)éthoxy]-2,4-dioxo-3,4-dihydroquinazolin-1(2H)-yl}pipéridine-1-carbaldéhyde | 562 |
| 236 | 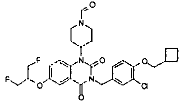 | 4-{3-[3-chloro-4-(cyclobutylméthoxy)benzyl]-6-[2-fluoro-1-(fluorométhyl)éthoxy]-2,4-dioxo-3,4-dihydroquinazolin-1(2H)-yl)pipéridine-1-carbaldéhyde | 576 |
| 237 | 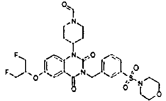 | 4-{6-[2-fluoro-1-(fluorométhyl)éthoxy]-3-[3-(morpholin-4-ylsulfonyl)benzyl]-2,4-dioxo-3,4-dihydroquinazolin-1(2H)-yl}pipéridine-1-carbaldéhyde | 607 |
| 238 | 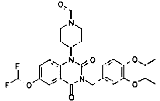 | 4-[3-(3,4-diéthoxybenzyl)-6-(difluorométhoxy)-2,4-dioxo-3,4-dihydroquinazolin-1(2H)-yl]pipéridine-1-carbaldéhyde | 518 |
| 239 | 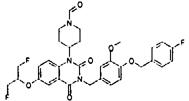 | 4-[3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 612 |
| 240 | 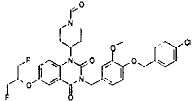 | 4-[3-{4-[(4-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 628 |
| 241 | 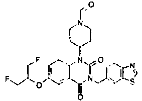 | 4-[3-(1,3-benzothiazol-6-ylméthyl)-6-[2-fluoro-1-(fluorométhyl)éthoxy]-2,4-dioxo-3,4-dihydroquinazolin-1(2H)-yl]pipéridine-1-carbaldéhyde | 515 |
| 242 | 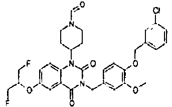 | 4-[3-{4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 628 |
| 243 | 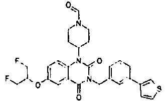 | 4-(6-[2-fluoro-1-(fluorométhyl)éthoxy]-2,4-dioxo-3-(3-thiophén-3-ylbenzyl)-3,4-dihydroquinazolin-1(2H)-yl}pipéridine-1-carbaldéhyde | 540 |
| 244 | 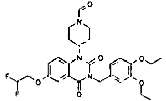 | 4-[3-(3,4-diéthoxybenzyl)-6-(2,2-difluoroéthoxy)-2,4-dioxo-3,4-dihydroquinazolin-1(2H)-yl]pipéridine-1-carbaldéhyde | 532 |
| 245 | 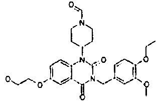 | 4-[3-(4-éthoxy-3-méthoxybenzyl)-6-(2-hydroxyéthoxy)-2,4-dioxo-3,4-dihydroquinazolin-1(2H)-yl]pipéridine-1-carbaldéhyde | 498 |
| 246 | 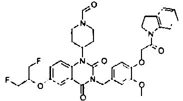 | 4-[3-{4-[2-(2,3-dihydro-1H-indol-1-yl)-2-oxoéthoxy]-3-méthoxybenzyl}-6-[2-fluoro-1(fluorométhyl)éthoxy]-2,4-dioxo-3,4-dihydroquinazolin-1(2H)-yl]pipéridine-1-carbaldéhyde | 663 |
| 247 | 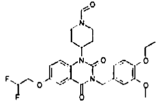 | 4-[6-(2,2-difluoroéthoxy)-3-(4-éthoxy-3-méthoxybenzyl)-2,4-dioxo-3,4-dihydroquinazolin-1(2H)-yl]pipéridine-1-carbaldéhyde | 518 |
| 248 | 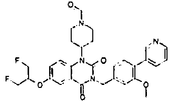 | 4-{6-[2-fluoro-1-(fluorométhyl)éthoxy]-3-(3-méthoxy-4-pyridin-3-ylbenzyl)-2,4-dioxo-3,4-dihydroquinazolin-1(2H)-yl}pipéridine-1-carbaldéhyde | 565 |
| 249 | 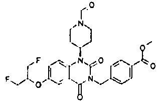 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)benzoate de méthyle | 516 |
| 250 | 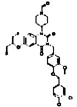 | 4-[3-{4-[(3,4-dichlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 662 |
| 251 | 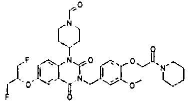 | 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde | 629 |
| 252 | 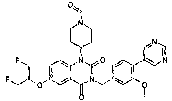 | 4-(6-[2-fluoro-1-(fluorométhyl)éthoxy]-3-(3-méthoxy-4-pyrimidin-5-ylbenzyl)-2,4-dioxo-3,4-dihydroquinazolin-1(2H)-yl)pipéridine-1-carbaldéhyde | 566 |
| 253 | 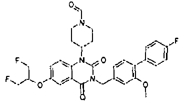 | 4-{6-[2-fluoro-1-(fluorométhyl)éthoxy]-3-[(4'-fluoro-2-méthoxybiphényl-4-yl)méthyl]-2,4-dioxo-3,4-dihydroquinazolin-1(2H)-yl}pipéridine-1-carbaldéhyde | 582 |
| 254 | 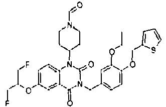 | 4-{3-[3-éthoxy-4-(thiophén-2-ylméthoxy)benzyl]-6-[2-fluoro-1-(fluorométhyl)éthoxy]-2,4-dioxo-3,4-dihydroquinazolin-1(2H)-yl}pipéridine-1-carbaldéhyde | 614 |
| 255 | 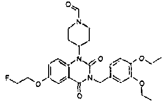 | 4-[3-(3,4-diéthoxybenzyl)-6-(2-fluoroéthoxy)-2,4-dioxo-3,4-dihydroquinazolin-1(2H)-yl]pipéridine-1-carbaldéhyde | 514 |
| 256 | 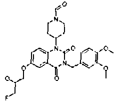 | 4-[3-(3,4-diméthoxybenzyl)-6-(3-fluoro-2-hydroxypropoxy)-2,4-dioxo-3,4-dihydroquinazolin-1(2H)-yl]pipéridine-1-carbaldéhyde | 516 |
| 257 | 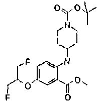 | 4-({4-[2-fluoro-1-(fluorométhyl)éthoxy]-2-(méthoxycarbonyl)phényl}amino)pipéridine-1-carboxylate de 1,1-diméthyléthyle | 429 |
| 258 | 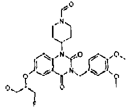 | 4-[3-(3,4-diméthoxybenzyl)-6-[2-fluoro-1-(hydroxyméthyl)éthoxy]-2,4-dioxo-3,4-dihydroquinazolin-1(2H)-yl]pipéridine-1-carbaldéhyde | 516 |
| 259 | 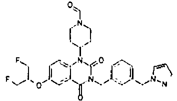 | 4-{6-[2-fluoro-1-(fluorométhyl)éthoxy]-2,4-dioxo-3-[3-(1H-pyrazol-1-ylméthyl)benzyl]-3,4-dihydroquinazolin-1(2H)-yl}pipéridine-1-carbaldéhyde | 538 |
| 260 | 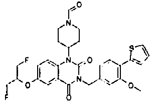 | 4-{6-[2-fluoro-1-(fluorométhyl)éthoxy]-3-(3-méthoxy-4-thiophén-2-ylbenzyl)-2,4-dioxo-3,4-dihydroquinazolin-1(2H)-yl}pipéridine-1-carbaldéhyde | 570 |
| 261 | 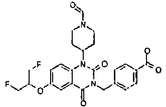 | acide 4-({6-[2-fluoro-1-(fluorométhyl)éthoxy]-1-(1-formylpipéridin-4-yl)-2,4-dioxo-1,4-dihydroquinazolin-3(2H)-yl}méthyl)benzôique | 502 |
| 262 | 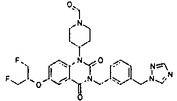 | 4-{6-[2-fluoro-1-(fluorométhyl)éthoxy]-2,4-dioxo-3-[3-(1H-1,2,4-triazol-1-ylméthyl)benzyl]-3,4-dihydroquinazolin-1(2H)-yl}pipéridine-1-carbaldéhyde | 539 |
| 263 | 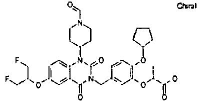 | acide (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque | 630 |
| 264 | 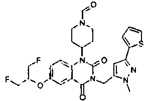 | 4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde | 544 |
| 265 | 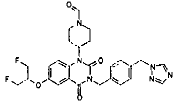 | 4-{6-[2-fluoro-1-(fluorométhyl)éthoxy]-2,4-dioxo-3-[4-(1H-1,2,4-triazol-1-ylméthyl)benzyl]-3,4-dihydroquinazolin-1(2H)-yl}pipéridine-1-carbaldéhyde | 539 |
| 266 | 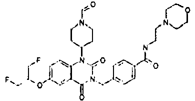 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)-N-(2-morpholin-4-yléthyl)benzamide | 614 |
| 267 | 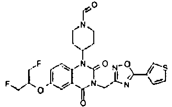 | 4-{6-[2-fluoro-1-(fluorométhyl)éthoxy]-2,4-dioxo-3-[(5-thiophén-3-yl-1,2,4-oxadiazol-3-yl)méthyl]-3,4-dihydroquinazolin-1(2H)-yl}pipéridine-1-carbaldéhyde | 532 |
| 268 | 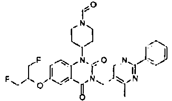 | 4-{6-[2-fluoro-1-(fluorométhyl)éthoxy]-3-[(4-méthyl-2-phénylpyrimidin-5-yl)méthyl]-2,4-dioxo-3,4-dihydroquinazolin-1(2H)-yl}pipéridine-1-carbaldéhyde | 550 |
| 269 | 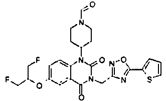 | 4-{6-[2-fluoro-1-(fluorométhyl)éthoxy]-2,4-dioxo-3-[(5-thiophén-2-yl-1,2,4-oxadiazol-3-yl)méthyl]-3,4-dihydroquinazolin-1(2H)-yl}pipéridine-1-carbaldéhyde | 532 |
| 270 | 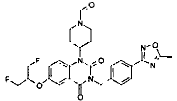 | 4-{6-[2-fluoro-1-(fluorométhyl)éthoxy]-3-[4-(5-méthyl-1,2,4-oxadiazol-3-yl)benzyl]-2,4-dioxo-3A-dihydroquinazolin-1(2H)-yl}pipéridine-1-carbaldéhyde | 540 |
| 271 | 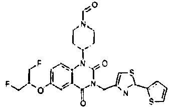 | 4-{6-[2-fluoro-1-(fluorométhyl)éthoxy]-2,4-dioxo-3-[(2-thiophén-2-yl-1,3-thiazol-4-yl)méthyl]-3,4-dihydroquinazolin-1(2H)-yl}pipéridine-1-carbaldéhyde | 547 |
| 272 | 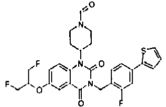 | 4-{6-[2-fluoro-1-(fluorométhyl)éthoxy]-3-(2-fluoro-4-thiophén-2-ylbenzyl)-2,4-dioxo-3,4-dihydroquinazolin-1(2H)-yl}pipéridine-1-carbaldéhyde | 558 |
| 273 | 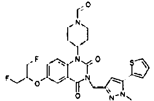 | 4-{6-[2-fluoro-1-(fluorométhyl)éthoxy]-3-[(1-méthyl-5-thiophén-2-yl-1H-pyrazol-3-yl)méthyl]-2,4-dioxo-3,4-dihydroquinazolin-1(2H)-yl}pipéridine-1-carbaldéhyde | 544 |
| 274 | 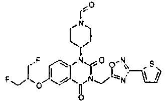 | 4-{6-[2-fluoro-1-(fluorométhyl)éthoxy]-2,4-dioxo-3-[(3-thiophén-2-yl-1,2,4-oxadiazol-5-yl)méthyl]-3,4-dihydroquinazolin-1(2H)-yl}pipéridine-1-carbaldéhyde | 532 |
| 275 | 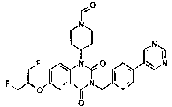 | 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde | 536 |
| 276 | 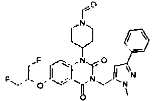 | 4-{6-[2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde | 538 |
| 277 | 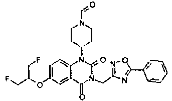 | 4-{6-[2-fluoro-1-(fluorométhyl)éthoxy]-2,4-dioxo-3-[(5-phényl-1,2,4-oxadiazol-3-yl)méthyl]-3,4-dihydroquinazolin-1(2H)-yl}pipéridine-1-carbaldéhyde | 526 |
| 278 | 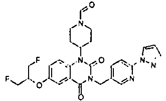 | 4-{6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)=yl}pipéridine-1-carbaldéhyde | 525 |
| 279 | 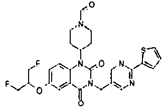 | 4-{6-[2-fluoro-1-(fluorométhyl)éthoxy]-2,4-dioxo-3-[(2-thiophén-2-ylpyrimidin-5-yl)méthyl]-3,4-dihydroquinazolin-1(2H)-yl}pipéridine-1-carbaldéhyde | 542 |
| 280 | 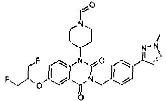 | 4-(6-[2-fluoro-1-(fluorométhyl)éthoxy]-3-[4-(1-méthyl-lH-pyrazol-3-yl)benzyl]-2,4-dioxo-3,4-dihydroquinazolin-1(2H)-yl}pipéridine-1-carbaldéhyde | 538 |
| 281 | 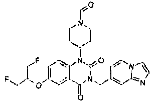 | 4-{6-[2-fluoro-1-(fluorométhyl)éthoxy]-3-(imidazo[1,2-a]pyridin-7-ylméthyl)-2,4-dioxo-3,4-dihydroquinazolin-1(2H)-yl}pipéridine-1-carbaldéhyde | 598 |
| 282 | 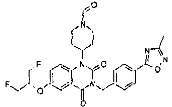 | 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde | 540 |
| 283 | 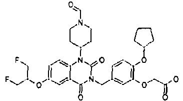 | acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl)méthyl)phénoxy]acétique | 616 |
| 284 | 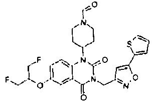 | 4-(6-[2-fluoro-1-(fluorométhyl)éthoxy]-2,4-dioxo-3-[(5-thiophén-2-ylisoxazol-3-yl)méthyl]-3,4-dihydroquinazolin-1(2H)-yl}pipéridine-1-carbaldéhyde | 531 |
| 285 | 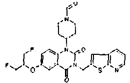 | 4-(6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde | 514 |
| 286 | 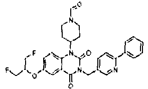 | 4-{6-[2-fluoro-1-(fluorométhyl)éthoxy]-2,4-dioxo-3-[(6-phénylpyridin-3-yl)méthyl]-3,4-dihydroquinazolin-1(2H)-yl}pipéridine-1-carbaldéhyde | 535 |
| 287 | 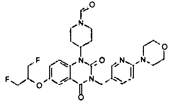 | 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)-yl}pipéridine-1-carbaldéhyde | 544 |
| 288 | 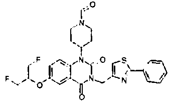 | 4-{6-[2-fluoro-1-(fluorométhyl)éthoxy]-2,4-dioxo-3-[(2-phényl-1,3-thiazol-4-yl)méthyl]-3,4-dihydroquinazolin-1(2H)-yl}pipéridine-1-carbaldéhyde | 541 |
| 289 | 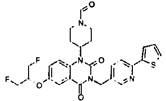 | 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde | 541 |
| 290 | 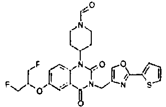 | 4-{6-[2-fluoro-1-(fluorométhyl)éthoxy]-2,4-dioxo-3-[(2-thiophén-2-yl-1,3-oxazol-4-yl)méthyl]-3,4-dihydroquinazolin-1(2H)-yl}pipéridine-1-carbaldéhyde | 531 |
| 291 | 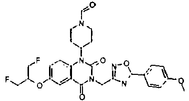 | 4-{6-[2-fluoro-1-(fluorométhyl)éthoxy]-3-{[5-(4-méthoxyphényl)-1,2,4-oxadiazol-3-yl]méthyl}-2,4-dioxo-3,4-dihydroquinazolin-1(2H)-yl}pipéridine-1-carbaldéhyde | 556 |
| 292 | 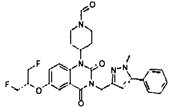 | 4-{6-[2-fluoro-1-(fluorométhyl)éthoxy]-3-[(1-méthyl-5-phényl-1H-pyrazol-3-yl)méthyl]-2,4-dioxo-3,4-dihydroquinazolin-1(2H)-yl}pipéridine-1-carbaldéhyde | 538 |
| 293 | 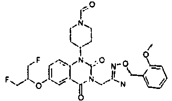 | 4-{6-[2-fluoro-1-(fluorométhyl)éthoxyJ-3-{[5-(2-méthoxyphényl)-1,2,4-oxadiazol-3-yl]méthyl}-2,4-dioxo-3,4-dihydroquinazolin-1(2H)-yl}pipéridine-1-carbaldéhyde | 556 |
| 294 | 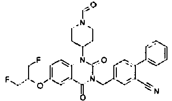 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile | 559 |
| 295 | 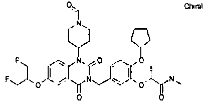 | (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide | 643 |
| 296 | 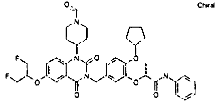 | (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-phénylpropanamide | 705 |
| 297 | 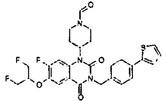 | 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde | 558 |
| 298 | 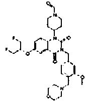 | 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde | 587 |
| 299 | 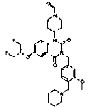 | 4-{6-[2-fluoro-1-(fluorométhyl)éthoxy]-3-[3-méthoxy-4-(pipéridin-1-ylméthyl)benzyl]-2,4-dioxo-3,4-dihydroquinazolin-1(2H)-yl}pipéridine-1-carbaldéhyde | 585 |
| 300 | 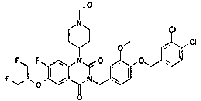 | 4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 301 | 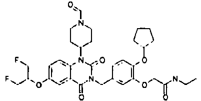 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide | 643 |
| 302 | 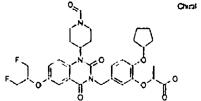 | acide (2S)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque | 630 |
| 303 | 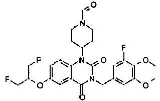 | 4-[3-(3-fluoro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 304 | 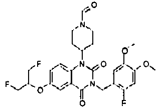 | 4-[3-(2-fluoro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 305 | 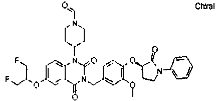 | 4-{6-[2-fluoro-1-(fluorométhyl)éthoxy]-3-(3-méthoxy-4-{[(3R)-2-oxo-1-phénylpyrrolidin-3-yl]oxy}benzyl)-2,4-dioxo-3,4-dihydroquinazolin-1(2H)-yl}pipéridine-1-carbaldéhyde | 663 |
| 306 | 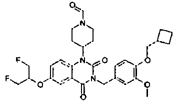 | 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 572 |
| 307 | 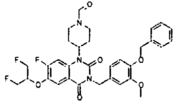 | 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 612 |
| 308 | 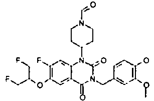 | 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde | 522 |
| 309 | 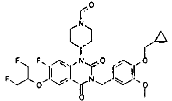 | 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 576 |
| 310 | 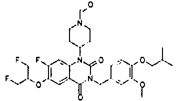 | 4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde | 578 |
| 311 | 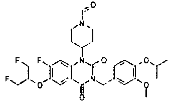 | 4-{7-fluoro-6-[2-fluoro-1-(fluorométhyl)éthoxy]-3-[3-méthoxy-4-(l-méthyléthoxy)benzyl]-2,4-dioxo-3,4-dihydroquinazolin-1(2H)-yl)pipéridine-1-carbaldéhyde | 564 |
| 312 | 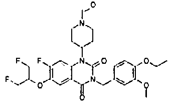 | 4-[3-(4-éthoxy-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 550 |
| 313 | 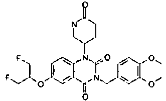 | 3-(3,4-diméthoxybenzyl)-6-[2-fluoro-1-(fluorométhyl)éthoxy]-1-(6-oxopipéridin-3-yl)quinazoline-2,4(1H,3H)-dione | 504 |
| 314 | 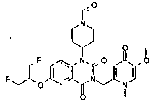 | 4-{6-[2-fluoro-1-(fluorométhyl)éthoxy]-3-[(5-méthoxy-1-méthyl-4-oxo-1,4-dihydropyridin-2-yl)méthyl]-2,4-dioxo-3,4-dihydroquinazolin-1(2H)-yl}pipéridine-1-carbaldéhyde | 519 |
| 315 | 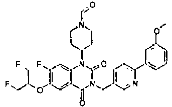 | 4-{7-fluoro-6-[2-fluoro-1-(fluorométhyl)éthoxy]-3-{[6-(3-méthoxyphényl)pyridin-3-yl]méthyl}-2,4-dioxo-3,4-dihydroquinazolin-1(2H)-yl}pipéridine-1-carbaldéhyde | 583 |
| 316 | 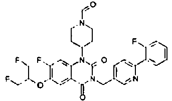 | 4-{7-fluoro-6-[2-fluoro-1-(fluorométhyl)éthoxy]-3-{[6-(2-fluorophényl)pyridin-3-yl]méthyl}-2,4-dioxo-3,4-dihydroquinazolin-1(2H)-yl}pipéridine-1-carbaldéhyde | 571 |
| 317 | 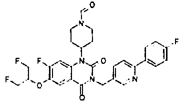 | 4-{7-fluoro-6-[2-fluoro-1-(fluorométhyl)éthoxy]-3-{[6-(4-fluorophényl)pyridin-3-yl]méthyl}-2,4-dioxo-3,4-dihydroquinazolin-1(2H)-yl}pipéridine-1-carbaldéhyde | 571 |
| 318 | 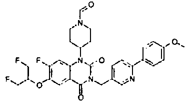 | 4-{7-fluoro-6-[2-fluoro-1-(fluorométhyl)éthoxy]-3-{[6-(4-méthoxyphényl)pyridin-3-yl]méthyl}-2,4-dioxo-3,4-dihydroquinazolin-1(2H)-yl}pipéridine-1-carbaldéhyde | 583 |
| 319 | 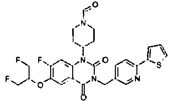 | 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde | 559 |
| 320 | 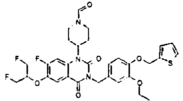 | 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 632 |
| 321 | 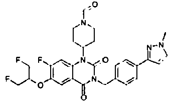 | 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde | 556 |
| 322 | 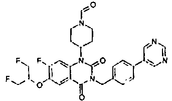 | 4-{7-fluoro-6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde | 554 |
| 323 | 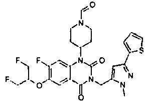 | 4-{7-fluoro-6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde | 562 |
| 324 | 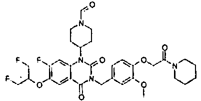 | 4-(7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde | 647 |
| 325 | 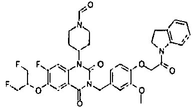 | 4-[3-{4-[2-(2,3-dihydro-1H-indol-1-yl)-2-oxoéthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 681 |
| 326 | 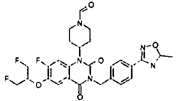 | 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde | 558 |
| 327 | 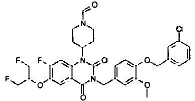 | 4-[3-{4-[(3-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 328 | 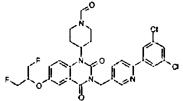 | 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde | 603 |
| 329 | 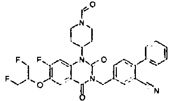 | 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile | 577 |
| 330 | 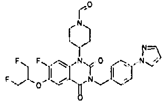 | 4-{7-fluoro-6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde | 542 |
| 331 | 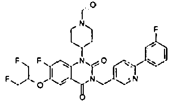 | 4-{7-fluoro-6-[2-fluoro-1-(fluorométhyl)éthoxy]-3-{[6-(3-fluorophényl)pyridin-3-yljméthyl}-2,4-dioxo-3,4-dihydroquinazolin-1(2H)-yl}pipéridine-1-carbaldéhyde | 571 |
| 332 | 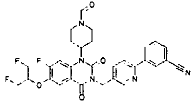 | 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile | 578 |
| 333 | 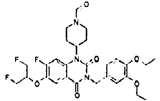 | 4-[3-(3,4-diéthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 564 |
| 334 | 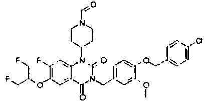 | 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 335 | 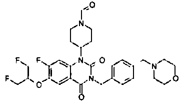 | 4-{7-fluoro-6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde | 575 |
| 336 | 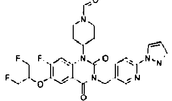 | 4-{7-fluoro-6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde | 543 |
| 337 | 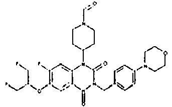 | 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde | 561 |
| 338 | 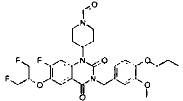 | 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde | 564 |
| 339 | 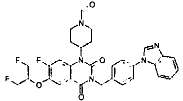 | 4-{3-[4-(1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 592 |
| 340 | 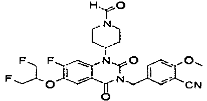 | 5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxybenzonitrile | 531 |
| 341 | 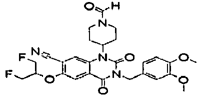 | 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile | 543 |
| 342 | 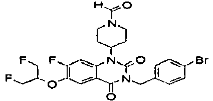 | 4-[3-(4-bromobenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 554 |
| 343 | 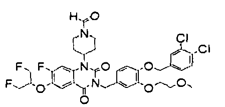 | 4-[3-(4-[(3,4-dichlorobenzyl)oxy]-3-(2-méthoxyéthoxy)benzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 724 |
| 344 | 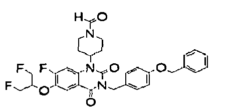 | 4-{3-[4-(benzyloxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 582 |
| 345 | 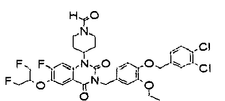 | 4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 346 | 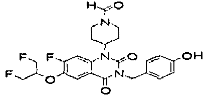 | 4-{7-fluoro-6-[2-fluoro-1-(fluorométhyl)éthoxy]-3-(4-hydroxybenzyl)-2,4-dioxo-3,4-dihydroquinazolin-1(2H)-yl}pipéridine-1-carbaldéhyde | 492 |
| 347 | 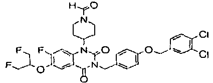 | 4-[3-{4-[(3,4-dichlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 348 | 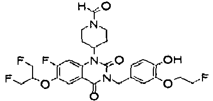 | 4-{7-fluoro-3-[3-(2-fluoroéthoxy)-4-hydroxybenzyl]-6-[2-fluoro-1-(fluorométhyl)éthoxy]-2,4-dioxo-3,4-dihydroquinazolin-1(2H)-yl)pipéridine-1-carbaldéhyde | 554 |
| 349 | 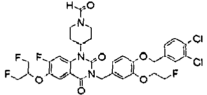 | 4-[3-{4-[(3,4-dichlorobenzyl)oxy]-3-(2-fluoroéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 712 |
| 350 | 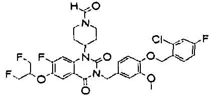 | 4-[3-(4-[(2-chloro-4-fluorobenzyl)oxy]-3-méthoxybenzyl)-7-fluoro-6-[2-fluoro-1-(fluorométhyl)éthoxyJ-2,4-dioxo-3,4-dihydroquinazolin-1(2H)-yl]pipéridine-1-carbaldéhyde | 664 |
| 351 | 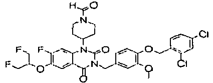 | 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 352 | 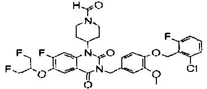 | 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 353 | 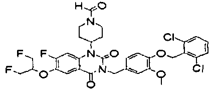 | 4-[3-{4-[(2,6-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 354 | 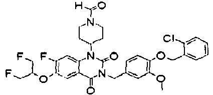 | 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 355 | 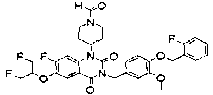 | 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 356 | 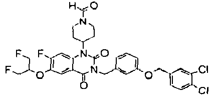 | 4-[3-{3-[(3,4-dichlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 357 | 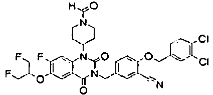 | 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile | 675 |
| 358 | 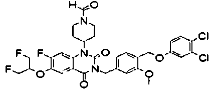 | 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 359 | 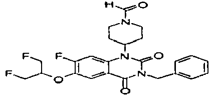 | 4-{3-benzyl-7-fluoro-6-[2-fluoro-1-(fluorométhyl)éthoxy]-2,4-dioxo-3,4-dihydroquinazolin-1(2H)-yl}pipéridine-1-carbaldéhyde | 476 |
| 360 | 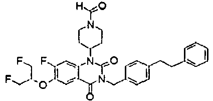 | 4-(7-fluoro-6-[2-fluoro-1-(fluorométhyl)éthoxy]-2,4-dioxo-3-[4-(2-phényléthyl)benzyl]-3,4-dihydroquinazolin-1(2H)-yl}pipéridine-1-carbaldéhyde | 580 |
| 361 | 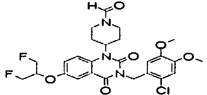 | 4-[3-(2-chloro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 552 |
| 362 | 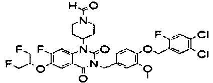 | 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 698 |
| 363 | 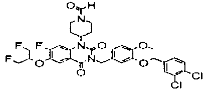 | 4-[3-{3-[(3,4-dichlorobenzyl)oxy]-4-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 364 | 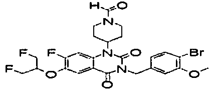 | 4-[3-(4-bromo-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 584 |
| 365 | 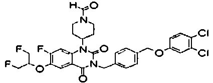 | 4-[3-{4-[(3,4-dichlorophenoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 366 | 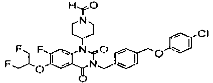 | 4-[3-{4-[(4-chlorophénoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 367 | 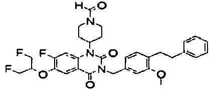 | 4-{7-fluoro-6-[2-fluoro-1-(fluorométhyl)éthoxy]-3-[3-méthoxy-4-(2-phényléthyl)benzyl]-2,4-dioxo-3,4-dihydroquinazolin-1(2H)-yl}pipéridine-1-carbaldéhyde | 610 |
| 368 | 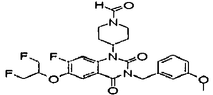 | 4-{7-fluoro-6-[2-fluoro-1-(fluorométhyl)éthoxy]-3-(3-méthoxybenzyl)-2,4-dioxo-3,4-dihydroquinazolin-1(2H)-yl}pipéridine-1-carbaldéhyde | 506 |
| 369 | 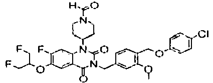 | 4-[3-{4-[(4-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 370 | 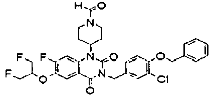 | 4-{3-[4-(benzyloxy)-3-chlorobenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 616 |
| 371 | 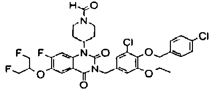 | 4-[3-{3-chloro-4-[(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 372 | 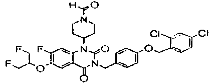 | 4-[3-{4-[(2,4-dichlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 373 | 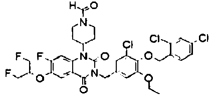 | 4-[3-(3-chloro-4-[(2,4-dichlorobenzyl)oxy]-5-éthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 728 |
| 374 | 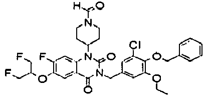 | 4-{3-[4-(benzyloxy)-3-chloro-5-éthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 660 |
| 375 | 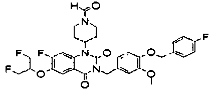 | 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 376 | 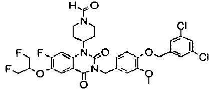 | 4-[3-(4-[(3,5-dichlorobenzyl)oxy]-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 377 | 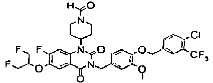 | 4-[3-(4-{[4-chloro-3-(trifluorométhyl)benzyl]oxy}-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 714 |
| 378 | 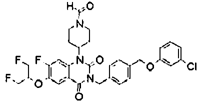 | 4-[3-{4-[(3-chlorophénoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 379 | 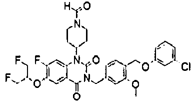 | 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 380 | 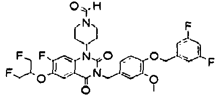 | 4-[3-{4-[(3,5-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 648 |
| 381 | 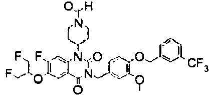 | 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 680 |
| 382 | 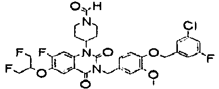 | 4-[3-{4-[(3-chloro-5-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 383 | 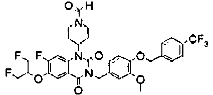 | 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde | 680 |
| 384 | 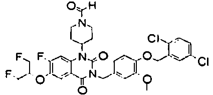 | 4-[3-{4-[(2,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 385 | 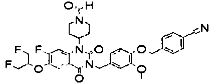 | 4-{[4-({7-fluoro-6-[2-fluoro-1-(fluorométhyl)éthoxyl-1-(l-formylpipéridin-4-yl)-2,4-dioxo-1,4-dihydroquinazolin-3(2H)-yl}méthyl)-2-méthoxyphénoxy]méthyl}benzonitrile | 637 |
| 386 | 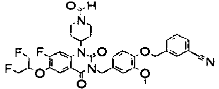 | 3-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile | 637 |
| 387 | 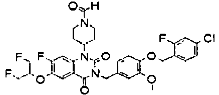 | 4-[3-{4-[(4-chloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 388 | 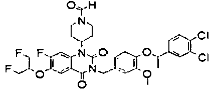 | 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 389 | 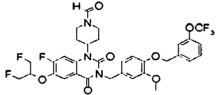 | 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1(2H)-yl}pipéridine-1-carbaldéhyde | 696 |
| 390 | 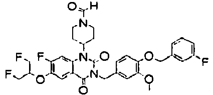 | 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 391 | 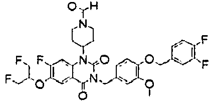 | 4-[3-(4-[(3,4-difluorobenzyl)oxy]-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 648 |
| 392 | 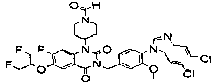 | 4-{3-[4-(5,6-dichloro-1H-benzimidazol-1-yl)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 660 |
| 393 | 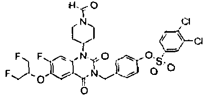 | 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle | 700 |
| 394 | 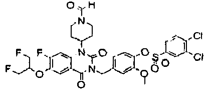 | 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle | 730 |
| 395 | 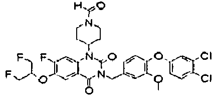 | 4-{3-[4-(3,4-dichlorophénoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 666 |
| 396 | 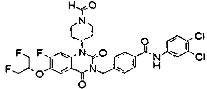 | N-(3,4-dichlorophényl)-4-({7-fluoro-6-[2-fluoro-1-(fluorométhyl)éthoxy]-1-(1-formylpipéridin-4-yl)-2,4-dioxo-1,4-dihydroquinazolin-3(2H)-yl)méthyl)benzamide | 645 |
| 397 | 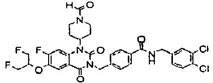 | N-(3,4-dichlorobenzyl)-4-({7-fluoro-6-[2-fluoro-1-(fluorométhyl)éthoxy]-1-(1-formylpipéridin-4-yl)-2,4-dioxo-1,4-dihydroquinazolin-3(2H)-yl)méthyl)benzamide | 659 |
| 398 | 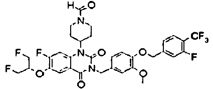 | 4-{7-fluoro-6-[2-fluoro-1-(fluorométhyl)éthoxy]-3-(4-{[3-fluoro-4-(trifluorométhyl)benzyl]oxy}-3-méthoxybenzyl)-2,4-dioxo-3,4-dihydroquinazolin-1(2H)-yl}pipéridine-1-carbaldéhyde | 698 |
| 399 | 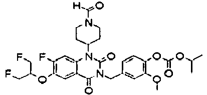 | carbonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle et de 1-méthyléthyle | 608 |
| 400 | 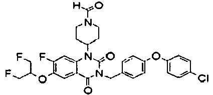 | 4-{3-[4-(4-chlorophénoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 602 |
| 401 | 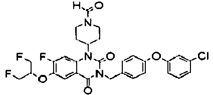 | 4-{3-[4-(3-chlorophénoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 602 |
| 402 | 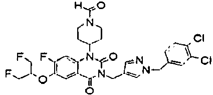 | 4-[3-{[1-(3,4-dichlorobenzyl)-1H-pyrazol-4-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 624 |
| 403 | 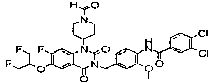 | 3,4-dichloro-N-[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényl]benzamide | 693 |
| 404 | 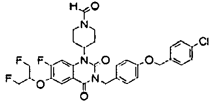 | 4-[3-{4-[(4-chlorobenzyl)oxyjbenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 405 | 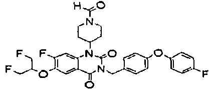 | 4-{7-fluoro-6-[2-fluoro-1-(fluorométhyl)éthoxy]-3-[4-(4-fluorophénoxy)benzyl]-2,4-dioxo-3,4-dihydroquinazolin-1(2H)-yl}pipéridine-1-carbaldéhyde | 586 |
| 406 | 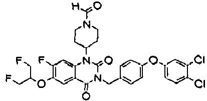 | 4-{3-[4-(3,4-dichlorophénoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 636 |
| 407 | 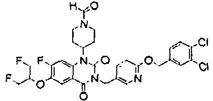 | 4-[3-({6-[(3,4-dichlorobenzyl)oxy]pyridin-3-yl}méthyl)-7-fluoro-6-[2-fluoro-1-(fluorométhyl)éthoxy]-2,4-dioxo-3,4-dihydroquinazolin-1(2H)-yl]pipéridine-1-carbaldéhyde | 651 |
| 408 | 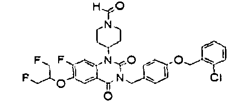 | 4-[3-(4-[(2-chlorobenzyl)oxylbenzyll-7-fluoro-6-[2-fluoro-1-(fluorométhyl)éthoxyl-2,4-dioxo-3,4-dihydroquinazolin-1(2H)-yl]pipéridine-1-carbaldéhyde | 616 |
| 409 | 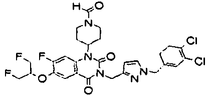 | 4-[3-{[1-(3,4-dichlorobenzyl)-1H-pyrazol-3-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 624 |
| 410 | 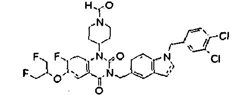 | 4-[3-{[1-(3,4-dichlorobenzyl)-1H-indol-5-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 673 |
| 411 | 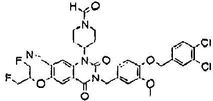 | 3-{4-[(3,4-dichlorobenzyl)oxyj-3-méthoxybenzyl}-6-[2-fluoro-1-(fluorométhyl)éthoxyj-1-(1-formylpipéridin-4-yl)-2,4-dioxo-1,2,3,4-tétrahydroquinazoline-7-carbonitrile | 687 |
| 412 | 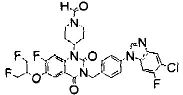 | 4-(3-[4-(5-chloro-6-fluoro-1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 644 |
| 413 | 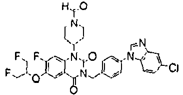 | 4-{3-[4-(5-chloro-1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 626 |
| 414 | 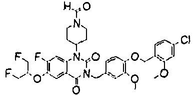 | 4-(3-{4-[(4-chloro-2-méthoxybenzyl)oxy]-3-méthoxybenzyl]-7-fluoro-6-[2-fluoro-1-(fluorométhyl)éthoxy]-2,4-dioxo-3,4-dihydroquinazolin-1(2H)-yl]pipéridine-1-carbaldéhyde | 676 |
| 415 | 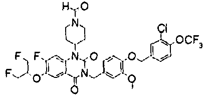 | 4-[3-{4-[(3-chloro-4-hydroxybenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 730 |

Les composés conformes à l'invention ont fait l'objet d'essais pharmacologiques qui ont montré leur intérêt comme substances thérapeutiquement actives.

### 1) Mesure de l'activité inhibitrice des composés conformes à l'invention vis-à-vis de la PDE7

La capacité des composés de formule (I) à inhiber la PDE7 est mesurée à l'aide d'un essai enzymatique basé sur la séparation de l'AMPc radioactif (substrat de la PDE7) du 5'-AMP radioactif (produit de la réaction enzymatique) par chromatographie en couche mince sur polyéthylèneimine (PEI) cellulose, après arrêt de la réaction enzymatique. Le 5'-AMP est extrait quantitativement de la PEI cellulose et sa radioactivité mesurée à l'aide d'un compteur à scintillation liquide.

L'activité inhibitrice des composés de formule (I) vis-à-vis de la PDE7 est représentée par la constante d'inhibition CI₅₀, définie comme la concentration du composé (inhibiteur) testé dans l'essai qui permet de réduire de 50% l'activité enzymatique de la PDE7. Plus les valeurs de CI₅₀ sont faibles, plus les composés sont de puissants inhibiteurs.

### Matériel

Le [³H]-AMPc (NET 275; 25 à 40 Ci/mmole) a été acheté chez Perkin Elmer (NEN Life Sciences, Boston, Etats-Unis), le rolipram chez Sigma (St Louis, MO, Etats-Unis), les feuilles de polyéthylèneimine cellulose F en plastique pour chromatographie sur couche mince chez Merck (Darmstadt, Allemagne). Tous les autres produits utilisés sont d'origine commerciale.

### Enzyme

La PDE7 humaine a été partiellement purifiée à partir de la lignée cellulaire HUT-78 en suivant une méthode analogue à celle décrite par Bloom et Beavo (Proc Natl Acad Sci USA, (1996) 93, 14188-14192). La préparation d'enzyme obtenue est conservée à -80°C dans un tampon contenant 20 mM de Tris-HCl (pH 7,0), 5 mM de MgCl₂, 4 mM d'EDTA, 1 mM de dithiothréitol et 20% de glycérol. La PDE7 partiellement purifiée étant contaminée par de la PDE4, il est nécessaire d'ajouter 10 µM de rolipram (inhibiteur sélectif de PDE4) dans l'essai enzymatique pour inhiber totalement l'activité PDE4. La constante de Michaelis (Km) de la PDE7 pour l'AMPc, mesurée à l'aide de l'essai radiochimique décrit ci-après, est de 21 nM.

### Solutions de composés conformes à l'invention

Les composés de formule (I) à tester comme inhibiteurs de PDE7 sont mis en solution dans le DMSO à une concentration de 10 mM. Ces solutions sont ensuite diluées en cascade dans le DMSO pour obtenir des solutions de concentrations désirées. Ces dernières sont ensuite diluées au vingtième dans le tampon d'essai pour donner des solutions à 5% de DMSO. Ces dernières sont finalement diluées au cinquième dans l'essai enzymatique.

La solution de rolipram (ajouté dans l'essai pour inhiber totalement l'activité PDE4 contaminante) est préparée de manière identique et apporte 1% de DMSO dans l'essai enzymatique.

### Essai enzymatique PDE7

L'essai est réalisé dans des tubes Eppendorf de 1,5 ml contenant 40 mM de Tris-HCl (pH 7,5), 15 mM de MgCl₂, 1 mM d'EGTA, 0,5 mg/ml d'albumine de sérum de boeuf, 0,063 µCi de [³H)-AMPc (correspondant à une concentration d'AMPc comprise entre 15 et 25 nM), 10 µM de rolipram et la PDE7 dans un volume final de 100 µl. L'essai est réalisé en absence (échantillon témoin) ou en présence (échantillon traité) des composés testés comme inhibiteurs de PDE7. La concentration finale de DMSO dans l'essai est de 2%. La réaction est initiée par addition d'enzyme et les échantillons sont maintenus à température ambiante pendant 30 minutes. La dilution enzymatique est ajustée de façon à obtenir un taux de conversion de 10 à 15%. La réaction enzymatique est arrêtée par immersion des tubes Eppendorf bouchés dans un bain-marie à 100°C pendant 3 minutes. Des blancs (réaction arrêtée immédiatement après addition de l'enzyme) sont inclus dans chaque expérience. Les échantillons sont ensuite centrifugés à 10,000xg pendant 1 minute et une part aliquote de 10 µl de surnageant est déposée à 2 cm du bord inférieur d'une feuille de PEI cellulose sur laquelle 10 µg d'AMPc et 10 µg de 5'-AMP ont été préalablement déposés. Pour faciliter la migration et le découpage ultérieur des bandes de PEI cellulose contenant le 5'-AMP, 18 voies de migration d'1 cm de largeur sont délimitées par plaque en grattant la cellulose avec une spatule sur 1 mm de largeur. Les plaques sont développées sur toute leur longueur avec une solution 0,30 M de LiCl dans l'eau par chromatographie ascendante. Le 5'-AMP (Rf = 0,20) et l'AMPc (Rf = 0,47) sont visualisés sous lumière U.V. à 254 nm. Les bandes de PEI cellulose contenant le 5'-AMP sont découpées et le nucléotide est extrait quantitativement dans des fioles de comptage avec 2 ml d'une solution contenant 16 M d'acide formique et 2 M de formate d'ammonium dans l'eau (agitateur rotatif pendant 15 min). Après ajout de 10 ml de liquide à scintillation (OptiPhase HiSafe 3 de Perkin Elmer / Wallac), la radioactivité est comptée à l'aide d'un compteur à scintillation liquide (modèle 1414, Perkin Elmer / Wallac). Chaque essai est réalisé en double. La radioactivité spécifiquement associée au 5'-AMP formé dans la réaction enzymatique est obtenue en soustrayant la valeur moyenne des blancs de la valeur moyenne des témoins (ou des traités).

Le pourcentage d'inhibition de la PDE7 à une concentration donnée du composé testé (inhibiteur) est calculé à l'aide de l'équation : 1% = [valeur moyenne des témoins - valeur moyenne des traités] x 100 / [valeur moyenne des témoins - valeur moyenne des blancs].

La CI₅₀ est la concentration du composé (inhibiteur) testé dans l'essai qui permet de réduire de 50% l'activité enzymatique de la PDE7.

### Résultats

A titre d'exemples illustratifs les quinazolinediones suivantes inhibent la PDE7 avec les valeurs de CI₅₀ indiquées ci-après:

| Composé | CI₅₀ (µM) |
|---|---|
| n°16 : 4-[6-(2,2-difluoroéthoxy}-3-(3,4-diméthoxybenzyl)-2,4-dioxo-3,4-dihydroquinazolin-1(2H)-yl]pipéridine-1-carbaldéhyde | 0.015 |
| n°11 : 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 0.039 |
| n °72 : 4-[3-(3,4-diméthoxybenzyl)-6-(2-hydroxyéthoxy)-2,4-dioxo-3,4-dihydroquinazolin-1(2H)-yl]pipéridine-1-carbaldéhyde | 0.089 |
| n°77 : N-{[3-(3,4-diméthoxybenzyl)-1-(1-formylpyrrolidin-3-yl)-2,4-dioxo-1,2,3,4-tétrahydroquinazolin-6-yl]méthyl}acétamide | 0.82 |
| n°97 : 4-[5,7-dichloro-3-(3,4-diméthoxybenzyl)-2,4-dioxo-3,4-dihydroquinazolin-1(2H)-yl]pipéridine-1-carbaldéhyde | 0.061 |
| n°111 : 4-[6-(difluorométhoxy)-3-(3,4-diméthoxybenzyl)-2,4-dioxo-3,4-dihydroquinazolin-1(2H)-yl]pipéridine-1-carbaldéhyde | 0.0067 |

### 2) Mesure de l'activité inhibitrice des composés conformes à l'invention vis-à-vis de la PDE8

En utilisant pour PDE8 un essai enzymatique équivalent à celui décrit pour PDE7, on obtient les valeurs suivantes de CI₅₀ :

| Composé | CI₅₀ PDE7 (µM) | CI₅₀ PDE8 (µM) |
|---|---|---|
| N° 11 | 0.039 | 0.14 |
| N° 251 | 0.046 | 0.39 |
| N° 294 | 0.037 | 0.015 |

### 3) Evaluation des composés conformes à l'invention dans un modèle in vitro d'activation microgliate

Les cellules gliales participent activement au mécanisme neurodégénératif par la production de facteurs neurotoxiques. La production de ces médiateurs fait partie du processus neuro-infiammatoire observé dans les maladies neurodégénératives et, en particulier, dans la maladie de Parkinson et la maladie d'Alzheimer.

De nombreux travaux ont démontré que l'inhibition de l'activation gliale et, par conséquent, la réduction de la production des facteurs pro-inflammatoires, telle l'interleukine-1, libérés par les cellules gliales activées réduit significativement la dégénérescence neuronale.

Les composés selon l'invention ont été évalués dans un modèle in vitro d'activation microgliale. Après activation par le lipopolysaccharide (LPS), les cellules microgliales sécrètent des quantités importantes d'interleukine-1 qui peuvent être quantifiées par un dosage spécifique dans les surnageants cellulaires.

Les cultures de cellules microgliales de la lignée MG7 dérivée de cortex de souris sont incubées en présence de concentrations variables de composés selon l'invention. Après 1 heure d'incubation à 37°C, les cellules sont mises en présence de LPS, inducteur de la production d'interleukine-1. Après 24h de traitement, les quantités d'interleukine-1 beta sont mesurées dans les surnageants de culture par dosage ELISA.

Le composé n° 297 diminue significativement et de manière concentration-dépendante la sécrétion d'interleukine-1 beta par les cellules microgliales MG7 activées par le LPS. L'effet inhibiteur maximal observé est de 47% à 3 µM.

Selon l'invention le composé de formule (I) définie plus haut, conforme à l'invention, peut être utilisé comme médicament ou pour la préparation d'un médicament destinés au traitement et/ou à la prévention de désordre(s) lié(s) au système nerveux central et/ou lié(s) au système nerveux périphérique.

Selon l'invention le composé de formule (I), conforme à l'invention, peut être utilisé comme médicament ou pour la préparation d'un médicament destinés au traitement et/ou à la prévention de désordre(s) psychiatrique(s) et/ou de désordre(s) neurologique(s).

Selon l'invention les composés de formule (I), conformes à l'invention, peuvent être utilisés comme médicaments ou pour la préparation d'un médicament destiné(s) au traitement ou à la prévention de désordres psychiatriques choisis parmi l'anxiété, la dépression, les troubles de l'humeur, l'insomnie, les troubles délirants, les troubles obsessionnels, les psychoses, les troubles liés à la schizophrénie, les troubles du déficit de l'attention et de l'hyperactivité (TDAH) chez les enfants hyperkinétiques, les troubles liés à l'utilisation de substances psychotropes, notamment dans le cas d'un abus d'une substance et/ou de dépendance à une substance, y compris la dépendance alcoolique et/ou la dépendance nicotinique, la migraine, le stress, les troubles liés à des maladies d'origine psychosomatique, les crises d'attaques de panique, l'épilepsie, les troubles mnésiques, les troubles cognitifs, en particulier les démences séniles, les troubles liés à la maladie d'Alzheimer, ainsi que les troubles de l'attention ou de la vigilance, l'ischémie, les troubles liés aux traumatismes crâniens, les troubles liés aux maladies neurodégénératives aiguës ou chroniques, incluant la chorée et la chorée de Huntington.

Selon l'invention les composés de formule (I), conformes à l'invention, peuvent être utilisés comme médicaments ou pour la préparation d'un médicament destiné(s) au traitement et/ou à la prévention de troubles neurologiques, pouvant se traduire par des anomalies de mouvement ou des désordres moteurs, associés à une pathologie choisie parmi les dyskinésies, la maladie de Parkinson, le syndrome parkinsonien post-encéphalitique, les dystonies dopa-sensible, le syndrome de Shy-Drager, le syndrome du désordre de mouvements périodiques des membres (PLMD), le syndrome des mouvements périodiques des membres dans le sommeil (PLMS), le syndrome de Tourette , le syndrome des jambes sans repos (RLS).

Selon un mode de réalisation, les composés de formule (I), conformes à l'invention, peuvent être utilisés comme médicaments ou pour fabriquer un médicament destiné(s) au traitement d'au moins une anomalie de mouvement ou un désordre moteur lié à la maladie de Parkinson en particulier ladite anomalie de mouvement ou ledit désordre moteur est choisi parmi un tremblement au repos, une rigidité, une bradykinesie et une déficience des réflexes posturaux.

Selon un mode de réalisation, les composés de formule (I), conformes à l'invention, peuvent être utilisés comme médicaments ou pour fabriquer un médicament destiné(s) à la prévention et/ou au traitement de troubles neurologiques se traduisant par des anomalies de mouvement ou des désordres moteurs, associés à des traumatismes de la moelle épinière, en particulier dans le traitement des traumatismes spinaux. Au sens de la présente invention, on entend par traumatisme de la moelle épinière les pathologies aiguës ou chroniques qui ont une origine externe et qui détruisent les tractus et/ou les neurones spinaux, et qui surviennent par exemple lors d'une chute, d'un choc, d'un écrasement ou d'un accident de la circulation.

Avantageusement, les composés de formule (I), conformes à l'invention, peuvent être utilisés comme médicaments ou pour fabriquer un médicament destiné(s) à la prévention et/ou au traitement des troubles :
- liés à la schizophrénie, en particulier dans (i) la prévention et/ou le traitement des symptomes positifs ou négatifs et/ou (ii) dans la prévention et/ou le traitement de déficits mnésiques ;
- associés à la maladie de Parkinson, en particulier (i) dans la prévention et/ou le traitement symptomatique des troubles moteurs, de la dépression et/ou des troubles cognitifs et/ou (ii) dans son traitement de fond (neuroprotecteur) ; et/ou
- liés à la maladie d'Alzheimer, en particulier (i) dans la prévention et/ou le traitement symptomatique des troubles cognitifs et/ou des troubles comportementaux (agressivité, dépression) et/ou (ii) dans son traitement de fond (neuroprotecteur).

L'utilisation des composés de formule générale (I), conformes à l'invention comme médicaments ou pour fabriquer un médicament destiné(s) à traiter et/ou prévenir les désordres ou les pathologies mentionné(e)s ci-dessus, fait partie intégrante de l'invention, et en particulier les composés de formule (I) choisis parmi les composés n° 1 à 6, 11 à 14, 16, 20, 22 à 25, 32 à 43, 47 à 52, 55 à 59, 72, 74, 76, 78, 79, 89 à 91, 97, 102, 108, 111, 112, 114, 116 à 118, 124, 130, 131, 133 à 135, 143, 145, 155, 158, 160, 165 à 167, 170, 175, 178, 183 à 186, 188, 189, 190, 193, 194, 200, 201, 203, 206, 207, 212, 213, 215, 216, 218, 223, 224, 226, 228, 230, 232 à 234, 239, 240, 242, 243, 245, 246, 250, 251, 254, 258, 263, 264, 270, 275, 276, 278 à 280, 282, 283, 285 à 287, 289, 292, 294, 295, 287 à 302, 305 à 312, 315 à 345, 349 à 355, 357, 358, 360, 362, 369, 371, 373, 375 à 377, 379 à 394, et 403.

L'invention a également pour objet un composé répondant à la formule générale (I) telle que définie plus haut pour le traitement et/ou à la prévention de désordre(s) lié(s) au système nerveux central et/ou lié(s) au système nerveux périphérique, le(s)dit(s) désordre(s) étant avantageusement choisi(s) parmi les désordres psychiatriques et les désordres neurologiques.

Des compositions pharmaceutiques comprenant, en tant que principe actif, au moins un composé conforme à l'invention sont utiles à la présente invention. Ces compositions pharmaceutiques contiennent une dose efficace d'au moins un composé de formule (I) conforme à l'invention, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques utiles à la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé conforme à l'invention sous forme de comprimé peut comprendre les composants suivants :

| | |
|---|---|
| Composé n°1 (2-{[3-(3,4-diméthoxybenzyl)-1-(1-formylpipéridin-4-yl)-2,4-dioxo-1,2,3,4-tétrahydroquinazolin-6-yl]oxy}propanenitrile) | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,5 mg à 800 mg de principe actif par individu, plus particulièrement de 0,5 mg à 200 mg, selon la forme galénique.

Il peut y avoir des cas où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Le traitement et/ou la prévention des pathologies indiquées ci-dessus comprend l'administration, à un patient, d'une dose efficace d'un composé conforme à l'invention, ou d'un de ses hydrates ou solvats.

## Revendications

1. Utilisation d'un composé répondant à la formule générale (I) 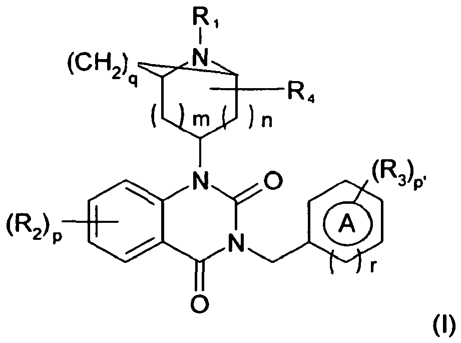 dans laquelle
- A représente un groupe aryle ou un groupe hétéroaryle;
- R₁ représente :
■ un atome d'hydrogène,
■ -C(O)R dans lequel R est un atome d'hydrogène, un groupe (C₁-C₆) alkoxy, un groupe aryle, un groupe (C₃-C₆) cycloalkyle, ou un groupe (C₁-C₆) alkyle, ledit alkyle étant éventuellement substitué par :
. un ou plusieurs groupe(s) hydroxy,
. un groupe benzyloxy,
. un groupe (C₁-C₆) alkoxy, éventuellement substitué par un aryle, ou
. un groupe (C₃-C₆) cycloalkyle,
■ un groupe (C₁-C₆) alkyle éventuellement substitué;
- R₂ représente :
■ un atome d'hydrogène,
■ un atome d'halogène,
■ un groupe cyano,
■ un groupe nitro,
■ un groupe (C₁-C₆) alkyle éventuellement substitué par un -NH₂, ou bien par un groupe -NHC(O)Rb, avec Rb tel que défini plus bas,
■ un groupe -ORa dans lequel Ra représente :
. un atome d'hydrogène,
. un groupe (C₁-C₆) alkyle éventuellement substitué par un ou plusieurs atome(s) d'halogène, par un ou plusieurs groupe(s) hydroxy, par un groupe aryle et/ou par un ou plusieurs groupe(s) cyano,
. un groupe (C₂-C₆) alcynyle,
. un groupe aryle ;
- R₃ représente :
■ un atome d'hydrogène,
■ un atome d'halogène,
■ un groupe hydroxy,
■ un groupe cyano,
■ un groupe -SCF₃,
■ un groupe nitro,
■ un groupe oxo,
■ un groupe -S(O)₀₋₂-alkyle, un groupe -S(O)₀₋₂-hétérocycloalkyle, un groupe - O- SO₂-aryle éventuellement substitué par un ou plusieurs atome(s) d'halogène ;
■ un groupe -alkyl-amino-alkyle ou -cycloalkyl-amino-alkyle, chacun éventuellement substitué sur l'alkyle terminal,
■ un groupe sulfonamide éventuellement substitué,
■ un groupe aryle ou un groupe hétéroaryle, ledit groupe étant monocyclique ou polycyclique et étant de plus éventuellement substitué par un groupe (C₁-C₆) alkyle, par un ou plusieurs atome(s) d'halogène, ou par un groupe (C₁-C₆) alkoxy,
■ un groupe hétérocycloalkyle éventuellement substitué par un groupe (C₁-C₆) alkyle,
■ un groupe (C₁-C₆) alkyle éventuellement substitué par :
- un ou plusieurs atome(s) d'halogène,
- un groupe aryle pouvant être substitué par un ou plusieurs atome(s) d'halogène, ou par un ou plusieurs groupe(s) hydroxy,
- un groupe hétéroaryle,
- un ou plusieurs groupe(s) hydroxy pouvant être substitué(s) par un groupe aryle lui-même éventuellement substitué par un ou plusieurs atome(s) d'halogène, ou
- un groupe hétérocycloalkyle éventuellement substitué par un groupe CO(O)Ra, ou par un groupe (C₁-C₆) alkyle, avec Ra tel que défini précédemment,
■ un groupe -C(O)NRbRc, avec Rb et Rc tels que définis plus bas,
■ un groupe -C(O)ORc, ou un groupe -O-C(O)ORc, avec Rc tel que défini plus bas,
■ un groupe (C₁-C₆) alkoxy, éventuellement substitué par
- un groupe amino-alkyle,
- un groupe amino-cycloalkyle,
- un groupe cycloalkyle,
- un groupe hétérocycloalkyle
- un groupe hétéroaryle monocyclique ou polycyclique,
- un ou plusieurs groupement(s) hydroxy,
- un ou plusieurs atome(s) d'halogène,
- un groupe (C₁-C₆) alkoxy,
- un groupe -C(O)ORc, avec Rc tel que défini plus bas,
- un groupe -C(O)NRbRc, avec Rb et Rc tels que définis plus bas,
- un groupe oxo, et/ou
- un groupe aryle, lui-même éventuellement substitué par un ou plusieurs atome(s) d'halogène, un groupe cyano, un groupe (C₁-C₆) alkoxy, un groupe -O- halogénoalkyle et/ou par un groupe halogénoalkyle,
■ un groupe -O-cycloalkyle, un groupe -O-aryle, ou un groupe -O-hétérocycloalkyle, chacun éventuellement substitué par
- un groupe aryle, lui-même éventuellement substitué par un ou plusieurs atome(s) d'halogène, ou par un groupe (C₁-C₆) alkyle,
- un groupe oxo,
- un ou plusieurs atome(s) d'halogène, et/ou
- un groupe (C₁-C₆) alkyle, lui-même pouvant être substitué par un groupe aryle et/ou un groupe oxo,
■ un groupe -NH-CO-NH-aryle, un groupe -NH-CO-NH-hétéroaryle, ou un groupe -NH-CO-NH-(C₁-C₆) alkyle, chacun étant éventuellement substitué par un ou plusieurs atome(s) d'halogène, par un groupe cyano, par un groupe nitro, par un ou plusieurs groupe(s) hydroxy, ou par un groupe (C₁-C₆) alkoxy,
■ un groupe -N-(C₁-C₆) alkyle, le groupe (C₁-C₆) alkyle pouvant être substitué par
- un ou plusieurs groupe(s) oxo, et/ou
- un ou plusieurs groupe(s) aryle éventuellement substitué(s) par un ou plusieurs atome(s) d'halogène et/ou par un groupe SO₂,
■ un groupe -NH-CO-aryle, un groupe -NH-CO-hétéroaryle, chacun étant éventuellement substitué par un ou plusieurs atome(s) d'halogène ;
ou bien R₃ forme avec A un groupe hétéroaryle polycyclique éventuellement substitué par un groupe (C₁-C₆) alkoxy, un groupe (C₁-C₆) alkyle éventuellement substitué par un groupe aryle pouvant lui-même être substitué par un ou plusieurs atome(s) d'halogène ;
- R₄ représente un atome d'hydrogène, un groupe oxo, ou un groupe (C₁-C₆) alkyle ;
- Rb représente :
. un atome d'hydrogène,
. un groupe (C₁-C₆) alkyle éventuellement substitué par un ou plusieurs atome(s) d'halogène, par un ou plusieurs groupe(s) hydroxy, cyano, amino, hétérocycloalkyle, (C₁-C₆) alkoxy, ou par un groupe aryle éventuellement substitué par un ou plusieurs atome(s) d'halogène,
. un groupe (C₃-C₆) cycloalkyle,
. un groupe (C₃-C₆₎ alcynyle,
. un groupe (C₂-C₆) alkoxy,
. un groupe aryle éventuellement substitué par un ou plusieurs atome(s) d'halogène;
- Rc représente un atome d'hydrogène, ou un groupe (C₁-C₆) alkyle éventuellement substitué par un ou plusieurs atome(s) d'halogène ;
ou alors Rb et Rc forment ensemble avec l'atome d'azote auquel ils sont attachés un groupe hétéroaryle polycyclique, ou un groupe hétérocycloalkyle ;
- m et n représentent indépendamment l'un de l'autre la valeur 0, 1 ou 2, étant entendu que m+n ≤ 3 ;
- p et p' représentent indépendamment l'un de l'autre la valeur 1, 2 ou 3, étant entendu que lorsque p est supérieur ou égal à 2, alors les groupes R₂ sont sur des atomes de carbone distincts et peuvent être différents les uns des autres, et lorsque p' est supérieur ou égal à 2 alors les groupes R₃ sont sur des atomes de carbone distincts et peuvent être différents les uns des autres;
- q représente la valeur 0 ou 2, étant entendu que lorsque q = 0, alors le groupe hétérocyclique azoté rattaché à l'azote situé en position 1 du noyau 2,4-dioxo-1,2,3,4 tétrahydroquinazoline n'est plus ponté et est du type : 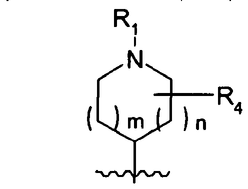
avec R₁, R₄, m et n tels que définis précédemment,
- r représente la valeur 0 ou 1 ;
à l'état de base ou de sel d'addition à un acide;
pour la préparation d'un médicament destinés au traitement et/ou à la prévention de désordres psychiatriques et de désordres neurologiques parmi lesquels les désordres psychiatriques sont choisis parmi l'anxiété, la dépression, les troubles de l'humeur, l'insomnie, les troubles délirants, les troubles obsessionnels, les psychoses, des troubles liés à la schizophrénie, les troubles du déficit de l'attention et de l'hyperactivité (TDAH) chez les enfants hyperkinétiques, les troubles liés à l'utilisation de substances psychotropes, notamment dans le cas d'un abus d'une substance et/ou de dépendance à une substance, y compris la dépendance alcoolique et la dépendance nicotinique, la migraine, le stress, les troubles liés à des maladies d'origine psychosomatique, les crises d'attaques de panique, l'épilepsie, les troubles mnésiques, les troubles cognitifs, en particulier les démences séniles, les troubles liés à la maladie d'Alzheimer, ainsi que les troubles de l'attention ou de la vigilance, l'ischémie, les troubles liés aux traumatismes crâniens, les troubles liés aux maladies neurodégénératives aiguës ou chroniques, incluant la chorée et la chorée de Huntington et les troubles neurologiques se traduisent par des anomalies de mouvement ou des désordres moteurs, associés à une pathologie choisie parmi les dyskinésies, la maladie de Parkinson, le syndrome parkinsonien post-encéphalitique, les dystonies dopa-sensible, le syndrome de Shy-Drager, le syndrome du désordre de mouvements périodiques des membres (PLMD), le syndrome des mouvements périodiques des membres dans le sommeil (PLMS), le syndrome de Tourette et le syndrome des jambes sans repos (RLS).

2. Utilisation selon la revendication 1, **caractérisée en ce que** les anomalies de mouvement ou les désordres moteurs sont associés à la maladie de Parkinson.

3. Utilisation selon la revendication 2, **caractérisée en ce que** les anomalies ou les désordres moteurs sont choisis parmi un tremblement au repos, une rigidité, une bradykinesie et une déficience des réflexes posturaux.

4. Utilisation selon l'une des revendications 1 à 3, (a) dans le traitement et/ou la prévention de troubles liés à la schizophrénie, en particulier dans (i) la prévention et/ou le traitement des symptomes positifs ou négatifs et/ou (ii) dans la prévention et/ou le traitement de déficits mnésiques, (b) dans le traitement et/ou la prévention de troubles associés à la maladie de Parkinson, en particulier (i) dans la prévention et/ou le traitement symptomatique des troubles moteurs, de la dépression et/ou des troubles cognitifs et/ou (ii) dans son traitement de fond, et/ou (c) dans le traitement et/ou la prévention de troubles liés à la maladie d'Alzheimer, en particulier (i) dans la prévention et/ou le traitement symptomatique des troubles cognitifs et/ou des troubles comportementaux et/ou (ii) dans son traitement de fond.

5. Utilisation selon la revendication 1, **caractérisée en ce que** les troubles neurologiques se traduisent par des anomalies de mouvement ou des désordres moteurs, associés à des traumatismes de la moelle épinière, en particulier des traumatismes spinaux.

6. Utilisation selon l'une quelconque des revendications précédentes, **caractérisée en ce que** A représente un groupe phényle ou un groupe pyridyle.

7. Utilisation selon l'une quelconque des revendications précédentes, **caractérisée en ce que** q = 0, m et n représente chacun 1.

8. Utilisation selon l'une quelconque des revendications précédentes, **caractérisée en ce que** R₂ représente un groupe (C₁-C₆) alkyle, en particulier un méthyle, substitué par un groupe -NH-C(O)-Rb, Rb étant tel que défini dans la revendication 1.

9. Utilisation selon l'une quelconque des revendications 1 à 7, **caractérisée en ce que** R₂ représente un groupe -ORa, Ra étant tel que défini dans la revendication 1.

10. Utilisation selon l'une quelconque des revendications 1 à 7, **caractérisée en ce que** R₂ est un atome d'halogène, ou un cyano, ou un hydrogène, ou un hydroxyle, ou un (C₁-C₆) alkyle éventuellement substitué par un -NH₂, ou bien par un groupe - NHC(O)Rb, Rb étant tel que défini dans la revendication 1.

11. Utilisation selon l'une quelconque des revendications 1 à 7, **caractérisée en ce que** A est un phényle, R₁ est un groupe -C(O)R dans lequel R représente un atome d'hydrogène, q est égal à 0, n et m valent chacun 1, et R₂ représente -ORa, Ra étant tel que défini dans la revendication 1.

12. Utilisation selon l'une quelconque des revendications 1 à 7, **caractérisée en ce que** A est un phényle, R₁ est un groupe -C(O)R dans lequel R représente un atome d'hydrogène, q est égal à 0, n et m valent chacun 1, p=1 et R₂ représente un méthyle substitué par un groupe -NH-CO-Rb, Rb étant tel que défini dans la revendication 1.

13. Utilisation selon l'une quelconque des revendications 1 à 7, **caractérisée en ce que** A est un phényle, R₁ est un groupe -C(O)R dans lequel R représente un atome d'hydrogène, q est égal à 0, n et m valent chacun 1, p est égal à 2, l'un des R₂ est - ORa, Ra étant tel que défini dans les revendications 1 ou 2, et l'autre des R₂ est un atome d'halogène.

14. Utilisation selon l'une quelconque des revendications précédentes, **caractérisée en ce que** le groupe R₂ est en position 6 du noyau 2,4-dioxo-1,2,3,4 tétrahydroquinazoline, et **en ce qu'**il peut de plus y avoir un groupe R2, identique ou différent dudit groupe R2 précédemment mentionné, en position 7 du noyau 2,4-dioxo-1,2,3,4 tétrahydroquinazoline, ledit composé de formule (I) étant à l'état de base, d'hydrate, de solvat, ou de leurs mélanges.

15. Utilisation selon l'une quelconque des revendications 1 à 5, **caractérisée en ce que** le composé de formule (I) est choisi parmi les composés
n°1 : 2-{[3-(3,4-diméthoxybenzyl)-1-(1-formylpipéridin-4-yl)-2,4-dioxo-1,2,3,4-tétrahydroquinazolin-6-yl]oxy}propanenitrile
n°2 : 1-(1-acétylpipéridin-4-yl)-3-(3,4-diméthoxybenzyl)-6-hydroxyquinazoline-2,4(1H,3H)-dione
n°3 : {[1-(1-acétylpipéridin-4-yl)-3-(3,4-diméthoxybenzyl)-2,4-dioxo-1,2,3,4-tétrahydroquinazolin-6-yl]oxy}acétonitrile
n°4 : 2-{[1-(1-acétylpipéridin-4-yl)-3-(3,4-diméthoxybenzyl)-2,4-dioxo-1,2,3,4-tétrahydroquinazolin-6-yl]oxy)propanenitrile
n°6: {[3-(3,4-diméthoxybenzyl)-1-(1-formylpipéridin-4-yl)-2,4-dioxo-1,2,3,4-tétrahydroquinazolin-6-yl]oxy}acétonitrile
n° 11 : 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde
n°12 :1-(1-acétylpipéridin-4-yl)-3-(3,4-diméthoxybenzyl)-6-[2-fluoro-1-(fluorométhyl)éthoxy]quinazoline-2,4(1H,3H)-dione
n° 13 : 4-[3-(3,4-diméthoxybenzyl)-2,4-dioxo-6-(2,2,2-trifluoroéthoxy)-3,4-dihydroquinazolin-1(2H)-yl]pipéridine-1-carbaldéhyde
n° 14 : 1-(1-acétylpipéridin-4-yl)-6-(2,2-difluoroéthoxy)-3-(3,4-diméthoxybenzyl)quinazoline-2,4(1H,3H)-dione
n° 16 :4-[6-(2,2-difluoroéthoxy)-3-(3,4-diméthoxybenzyl)-2,4-dioxo-3,4-dihydroquinazolin-1(2H)-yl]pipéridine-1-carbaldéhyde
n°20 : N-{[3-(3,4-diméthoxybenzyl)-1-(1-formylpipéridin-4-yl)-2,4-dioxo-1,2,3,4-tétrahydroquinazolin-6-yl]méthyl}acétamide
n°22 : chlorhydrate de 1-(1-acétylpipéridin-4-yl)-6-(aminométhyl)-3-(3,4-diméthoxybenzyl)quinazoline-2,4(1H,3H)-dione
n°23 : N-{[3-(3,4-diméthoxybenzyl)-1-(1-formylpipéridin-4-yl)-2,4-dioxo-1,2,3,4-tétrahydroquinazolin-6-yl]méthyl}formamide
n°24 : N-{[1-(1-acétylpipéridin-4-yl)-3-(3,4-diméthoxybenzyl)-2,4-dioxo-1,2,3,4-tétrahydroquinazolin-6-yl]méthyl}formamide
n°25: N-{[1-(1-acétylpipéridin-4-yl)-3-(3,4-diméthoxybenzyl)-2,4-dioxo-1,2,3,4-tétrahydroquinazolin-6-yl]méthyl}acétamide
n°32 : 4-[6-(2,2-difluoroéthoxy)-2,4-dioxo-3,4-dihydroquinazolin-1(2H)-ylJpipéridine-1-carbaldéhyde
n°33 : 4-[3-(3,4-dichlorobenzyl)-6-(2,2-difluoroéthoxy)-2,4-dioxo-3,4-dihydroquinazolin-1(2H)-yl]pipéridine-1-carbaldéhyde
n°34 : 4-[3-(4-chlorobenzyl)-6-(2,2-difluoroéthoxy)-2,4-dioxo-3,4-dihydroquinazolin-1(2H)-yl]pipéridine-1-carbaldéhyde
n°35 : 4-{[6-(2,2-difluoroéthoxy)-1-(1-formylpipéridin-4-yl)-2,4-dioxo-1,4-dihydroquinazolin-3(2H)-yl]méthyl}benzoate de méthyle
n°36 : acide 4-{[6-(2,2-difluoroéthoxy)-1-(1-formylpipéridin-4-yl)-2,4-dioxo-1,4-dihydroquinazolin-3(2H)-yl]méthyl}benzoïque
n°37 : 4-{[6-(2,2-difluoroéthoxy)-1-(1-formylpipéridin-4-yl)-2,4-dioxo-1,4-dihydroquinazolin-3(2H)-yl]méthyl}-N-(2-méthoxyéthyl)benzamide
n°38 : 4-[3-(3,4-diméthoxybenzyl)-6-méthyl-2,4-dioxo-3,4-dihydroquinazolin-1(2H)-yl]pipéridine-1-carbaldéhyde
n°39 : 4-[6-(2,2-difluoroéthoxy)-3-(3-hydroxy-4-méthoxybenzyl)-2,4-dioxo-3,4-dihydroquinazolin-1(2H)-yl]pipéridine-1-carbaldéhyde
n°40 : 4-[6-(2,2-difluoroéthoxy)-3-[3-(2-hydroxyéthoxy)-4-méthoxybenzyl]-2,4-dioxo-3,4-dihydroquinazolin-1(2H)-yl]pipéridine-1-carbaldéhyde
n°41 : 4-[6-(2,2-difluoroéthoxy)-3-(3-éthoxy-4-méthoxybenzyl)-2,4-dioxo-3,4-dihydroquinazolin-1(2H)-yl]pipéridine-1-carbaldéhyde
n°42 : 4-[6-(2,2-difluoroéthoxy)-3-[4-rnéthoxy-3-(2-méthoxyéthoxy)benzylj-2,4-dioxo-3,4-dihydroquinazolin-1(2H)-yl]pipéridine-1-carbaldéhyde
n°43 : 4-[6-(2,2-difluoroéthoxy)-3-(3,4-diméthoxybenzyl)-2,4-dioxo-3,4-dihydroquinazolin-1(2H)-yl]azépane-1-carbaldéhyde
n°47 : 4-[6-(2,2-difluoroéthoxy)-3-[3-(3-hydroxypropoxy)-4-méthoxybenzyl]-2,4-dioxo-3,4-dihydroquinazolin-1(2H)-yl]pipéridine-1-carbaldéhyde
n°48 : 4-[5-chloro-3-(3,4-diméthoxybenzyl)-2,4-dioxo-3,4-dihydroquinazolin-1 (2H)-yl]pipéridine-1-carbaldéhyde
n°49 : 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde
n°50 : 2-(5-{[6-(2,2-difluoroéthoxy)-1-(I-formylpipéridin-4-yl)-2,4-dioxo-1,4-dihydroquinazolin-3(2H)-yl]méthyl}-2-méthoxyphénoxy)acétamide
n°51 : 4-[6-(2,2-difluoroéthoxy)-3-(3,4-diméthoxybenzyl)-2,4-dioxo-3,4-dihydroquinazolin-1(2H)-ylj-3-méthylpipéridine-1-carbaldéhyde
n°52 : 3-[6-(2,2-difluoroéthoxy)-3-(3,4-diméthoxybenzyl)-2,4-dioxo-3,4-dihydroquinazolin-1(2H)-yl]-8-azabicyclo[3.2.1]octane-8-carbaldéhyde
n°56 : 4-{3-[4-(cyclopentyloxy)-3-méthoxybenzyl)-6-[2-fluoro-1-(fluorométhyl)éthoxy]-2,4-dioxo-3,4-dihydroquinazolin-1(2H)-yl}pipéridine-1-carbaldéhyde
n°57 : 4-[3-(3-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°58 : 4-[3-(4-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°59 : 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°72 : 4-[3-(3,4-diméthoxybenzyl)-6-(2-hydroxyéthoxy)-2,4-dioxo-3,4-dihydroquinazolin-1(2H)-yl]pipéridine-1-carbaldéhyde
n°74 : 4-[3-(3,4-dichlorobenzyl)-6-[2-fluoro-1-(fluorométhyl)éthoxy]-2,4-dioxo-3,4-dihydroquinazolin-1(2H)-yl]pipéridine-1-carbaldéhyde
n°76 : 4-{3-[(6-chloropyridin-3-yl)méthyll-6-[2-fluorô-l-(fluorométhyl)éthoxy]-2,4-dioxo-3,4-dihydroquinazolin-1(2H)-yl}pipéridine-1-carbaldéhyde
n°78 : 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°79 : 4-[3-(3,4-diméthoxybenzyl)-6-(2-fluoroéthoxy)-2,4-dioxo-3,4-dihydroquinazolin-1(2H)-yl]pipéridine-1-carbaldéhyde
n°89: 2-[5-({6-[2-fluoro-1-(fluorométhyl)éthoxy]-1-(1-formylpipéridin-4-yl)-2,4-dioxo-1,4-dihydroquinazolin-3(2H)-yl)méthyl)-2-méthoxyphénoxy]acétamide
n°90 : 4-{6-[2-fluoro-1-(fluorométhyl)éthoxy]-3-(3-hydroxy-4-méthoxybenzyl)-2,4-dioxo-3,4-dihydroquinazolin-1(2H)-yl}pipéridine-1-carbaldéhyde
n°91: 4-[3-(3,4-diméthoxybenzyl)-6-éthoxy-2,4-dioxo-3,4-dihydroquinazolin-1(2H)-yl]pipéridine-1-carbaldéhyde
n°97 : 4-[5,7-dichloro-3-(3,4-diméthoxybenzyl)-2,4-dioxo-3,4-dihydroquinazolin-1(2H)-yl]pipéridine-1-carbaldéhyde
n° 102: 4-[7-chloro-3-(3,4-diméthoxybenzyl)-2.,4-dioxo-3,4-dihydroquinazolin-1(2H)-yl]pipéridine-1-carbaldéhyde
n°108 : 4-(6-[2-fluoro-l-(fluorométhyl)éthoxy]-3-(3-fluoro-4-méthoxybenzyl)-2,4-dioxo-3,4-dihydroquinazolin-1(2H)-yl}pipéridine-1-carbaldéhyde
n°111 : 4-[6-(difluorométhoxy)-3-(3,4-diméthoxybenzyl)-2,4-dioxo-3,4-dihydroquinazolin-1(2H)-yl]pipéridine-1-carbaldéhyde
n° 112 : 4-[3-(3,4-diméthoxybenzyl)-6-(1-méthyléthoxy)-2,4-dioxo-3,4-dihydroquinazolin-1(2H)-yl]pipéridine-1-carbaldéhyde
n° 114 : 4-{6-[2-fluoro-1-(fluorométhyl)éthoxy]-3-[4-méthoxy-3-(1-méthyléthoxy)benzyl]-2,4-dioxo-3,4-dihydroquinazolin-1(2H)-yi)pipéridine-1-carbaldéhyde
n° 116 : 4-{6-[2-fluoro-1-(fluorométhyl)éthoxyj-3-(3-méthoxybenzyl)-2,4-dioxo-3,4-dihydroquinazolin-1(2H}-yl}pipéridine-1-carbaldéhyde
n° 117 : 4-{3-[3,5-bis(trifluorométhyl)benzyl]-6-[2-fluoro-1-(fluorométhyl)éthoxy]-2,4-dioxo-3,4-dihydroquinazolin-1(2H)-yl}pipéridine-1-carbaldéhyde
n° 118 : 4-[3-(3-éthoxybenzyl)-6-[2-fluoro-1-(fluorométhyl)éthoxy]-2,4-dioxo-3,4-dihydroquinazolin-1(2H)-yl]pipéridine-1-carbaldéhyde
n°124 : 4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°130:4-[3-(3,4-diéthoxybenzyl)-6-[2-fluoro-1-(fluorométhyl)éthoxy]-2,4-dioxo-3,4-dihydroquinazolin-1(2H)-yl]pipéridine-1-carbaldéhyde
n° 131 : 4-[3-(4-éthoxy-3-méthoxybenzyl)-6-[2-fluoro-1-(fluorométhyt)éthoxy]-2,4-dioxo-3,4-dihydroquinazolin-1(2H)-yl]pipéridine-1-carbaldéhyde
n° 133 : 4-{6-[2-fluoro-1-(fluorométhyl)éthoxy]-3-(4-méthoxy-3-méthylbenzyl)-2,4-dioxo-3,4-dihydroquinazolin-1(2H)-yl}pipéridine-1-carbaldéhyde
n° 134 : 4-{6-[2-fluoro-1-(fluorométhyl)éthoxy]-2,4-dioxo-3-[4-(trifluorométhyl)benzyl]-3,4-dihydroquinazolin-1(2H)-yl)pipéridine-1-carbaldéhyde
n°135 : 4-{6-[2-fluoro-1-(fluorométhyl)éthoxy]-2,4-dioxo-3-(3-phénoxybenzyl)-3,4-dihydroquinazolin-1(2H)-yl}pipéridine-1-carbaldéhyde
n° 143 : 4-{3-[4-(benzyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n° 145 : 4-{6-[2-fluoro-1-(fluorométhyl)éthoxy]-3-(3-méthoxy-4-nitrobenzyl)-2,4-dioxo-3,4-dihydroquinazolin-1(2H)-yl}pipéridine-1-carbaldéhyde
n° 155 : 4-[3-(4-éthoxybenzyl)-6-[2-fluoro-1-(fluorométhyl)éthoxy]-2,4-dioxo-3,4-dihydroquinazolin-1(2H)-yl]pipéridine-1-carbaldéhyde
n° 158 : 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°160 : 4-{6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°165 : 4-[3-(biphényl-4-ylméthyl)-6-[2-fluoro-1-(fluorométhyl)éthoxy]-2,4-dioxo-3,4-dihydroquinazolin-1(2H)-yl]pipéridine-1-carbaldéhyde
n°166 : 4-{6-[2-fluoro-1-(fluorométhyl)éthoxy]-3-[4-(méthylsulfanyl)benzyl]-2,4-dioxo-3,4-dihydroquinazolin-1(2H)-yl}pipéridine-1-carbaldéhyde
n°167 : 4-{6-[2-fluoro-1-(fluorométhyl)éthoxy]-2,4-dioxo-3-(4-pyridin-3-ylbenzyl)-3,4-dihydroquinazolin-1(2H)-yl}pipéridine-1-carbaldéhyde
n°170 : 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde
n°175 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide
n°178 : 4-{6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°183 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylacétamide
n°184 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N,N-diméthylacétamide
n°185 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide
n°186 : 4-{3-[4-(cyclopentyloxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
n°188 : 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°189 : 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde
n°190: 4-{3-[4-(cyclopentyloxy)-3-hydroxybenzyl]-6-[2-fluoro-1-(fluorométhyl)éthoxy]-2,4-dioxo-3,4-dihydroquinazolin-1(2H)-yl}pipéridine-1-carbaldéhyde
n°193 : 4-{3-[4-(difluorométhoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°194 : 4-{3-[4-(difluorométhoxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
n°200 : 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde
n°201 : 4-{6-[2-fluoro-1-(fluorométhyl)éthoxy]-2,4-dioxo-3-(4-pyridin-4-ylbenzyl)-3,4-dihydroquinazolin-1(2H)-yl}pipéridine-1-carbaldéhyde
n°203 : 4-{6-[2-fluoro-1-(fluorométhyl)éthoxy]-3-[(1-méthyl-1H-indol-6-yl)méthyl]-2,4-dioxo-3,4-dihydroquinazolin-1(2H)-yl}pipéridine-1-carbaldéhyde
n°206 : 4-{3-[4-(cyclopropylméthoxy)-3-méthoxybenzyl]-6-{2-fluoro-1-(fluorométhyl)éthoxy]-2,4-dioxo-3,4-dihydroquinazolin-1(2H)-yl}pipéridine-1-carbaldéhyde
n°207 : 2-[4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]-N-méthylacétamide
n°212 : 4-(6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°213 : 4-{6-[2-fluoro-1-(fluorométhyl)éthoxy]-2,4-dioxo-3-(4-pyridin-2-ylbenzyl)-3,4-dihydroquinazolin-1(2H)-yl}pipéridine-1-carbaldéhyde
n°215 : 4-{6-[2-fluoro-1-(fluorométhyl)éthoxy]-2,4-dioxo-3-(4-thiophén-2-ylbenzyl)-3,4-dihydroquinazolin-1 (2H)-yl}pipéridine-1-carbaldéhyde
n°216 : 4-{6-[2-fluoro-1-(fluorométhyl)éthoxy]-2,4-dioxo-3-(quinoléin-7-ylméthyl)-3,4-dihydroquinazolin-1 (2H)-yl}pipéridine-1-carbaldéhyde
n°218 : 4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde
n°223 : 4-{3-[4-(1H-benzimidazol-1-yl)benzyl]-6-[2-fluoro-1-(fluorométhyl)éthoxy]-2,4-dioxo-3,4-dihydroquinazolin-1(2H)-yl}pipéridine-1-carbaldéhyde
n°224 : 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n°226 : 4-{6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl}pipéridine-1-carbaldéhyde
n°228 : 4-[3-{4-[(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°230 : 4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde
n°232 : 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°233 : 4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°234 : 4-[3-{4-[(1-acétylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°239 : 4-[3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°240 : 4-[3-{4-[(4-chlorobenzyl)oxy]-3-méthoxybenzyl}-6-[2-fluoro-1-(fluorométhyl)éthoxy]-2,4-dioxo-3,4-dihydroquinazolin-1 (2H)-yl]pipéridine-1-carbaldéhyde
n°242 : 4-[3-(4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°243 : 4-{6-[2-fluoro-1-(fluorométhyl)éthoxy]-2,4-dioxo-3-(3-thiophén-3-ylbenzyl)-3,4-dihydroquinazolin-1(2H)-yl}pipéridine-1-carbaldéhyde
n°245 : 4-[3-(4-éthoxy-3-méthoxybenzyl)-6-(2-hydroxyéthoxy)-2,4-dioxo-3,4-dihydroquinazolin-1 (2H)-yl]pipéridine-1-carbaldéhyde
n°246 : 4-[3-{4-[2-(2,3-dihydro-1H-indol-1-yl)-2-oxoéthoxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°250 : 4-[3-{4-[(3,4-dichlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°251 : 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
n°254 : 4-{3-[3-éthoxy-4-(thiophén-2-ylméthoxy)benzyl]-6-[2-fluoro-1-(fluorométhyl)éthoxyl-2,4-dioxo-3,4-dihydroquinazolin-1(2H)-yl}pipéridine-1-carbaldéhyde
n°258 : 4-[3-(3,4-diméthoxybenzyl)-6-[2-fluoro-1-(hydroxyméthyl)éthoxy]-2,4-dioxo-3,4-dihydroquinazolin-1(2H)-yl]pipéridine-1-carbaldéhyde
n°263 : acide (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
n°264 : 4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
n°270 : 4-{6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
n°275 : 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°276 : 4-{6-[2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde
n°278 : 4-{6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°279 : 4-{6-[2-fluoro-1-(fluorométhyl)éthoxy]-2,4-dioxo-3-[(2-thiophén-2-ylpyrimidin-5-yl)méthyl]-3,4-dihydroquinazolin-1(2H)-yl}pipéridine-1-carbaldéhyde
n°280 : 4-{6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°282 : 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde
n°283 : acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétique
n°285 :4-{6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde
n°286 : 4-{6-[2-fluoro-1-(fluorométhyl)éthoxy]-2,4-dioxo-3-[(6-phénylpyridin-3-yl)méthyl]-3,4-dihydroquinazolin-1(2H)-yl}pipéridine-1-carbaldéhyde
n°287 : 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)-yl}pipéridine-1-carbaldéhyde
n°289 : 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°292 : 4-{6-[2-fluoro-1-(fluorométhyl)éthoxy]-3-[(1-méthyl-5-phényl-1H-pyrazol-3-yl)méthyl]-2,4-dioxo-3,4-dihydroquinazolin-1(2H)-yl}pipéridine-1-carbaldéhyde
n°294 : 4-({6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°295 : (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide
n°297 : 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°298 : 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde
n°299 : 4-{6-[2-fluoro-1-(fluorométhyl)éthoxy]-3-[3-méthoxy-4-(pipéridin-1-ylméthyl)benzyl]-2,4-dioxo-3,4-dihydroquinazolin-1(2H)-yl}pipéridine-1-carbaldéhyde
n°300 :4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°301 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide
n°302 : acide (2S)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
n°305 : 4-{6-[2-fluoro-1-(fluorométhyl)éthoxy]-3-(3-méthoxy-4-{[(3R)-2-oxo-1-phénylpyrrolidin-3-yl]oxy}benzyl)-2,4-dioxo-3,4-dihydroquinazolin-1(2H)-yl}pipéridine-1-carbaldéhyde
n°306 : 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°307 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°308 : 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde
n°309 : 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde n°310 : 4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n°311 : 4-{7-fluoro-6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°312 : 4-[3-(4-éthoxy-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°315 :4-{7-fluoro-6-[2-fluoro-1-(fluorométhyl)éthoxy]-3-{[6-(3-méthoxyphényl)pyridin-3-yl]méthyl}-2,4-dioxo-3,4-dihydroquinazolin-1(2H)-yl}pipéridine-1-carbaldéhyde
n°316 : 4-{7-fluoro-6-[2-fluoro-1-(fluorométhyl)éthoxy]-3-{[6-(2-fluorophényl)pyridin-3-yl]méthyl}-2,4-dioxo-3,4-dihydroquinazolin-1(2H)-yl}pipéridine-1-carbaldéhyde
n°317 : 4-{7-fluoro-6-[2-fluoro-1-(fluorométhyl)éthoxy]-3-{[6-(4-fluorophényl)pyridin-3-yl]méthyl}-2,4-dioxo-3,4-dihydroquinazolin-1(2H)-yl}pipéridine-1-carbaldéhyde
n°318 : 4-{7-fluoro-6-[2-fluoro-1-(fluorométhyl)éthoxy]-3-{[6-(4-méthoxyphényl)pyridin-3-yl]méthyl}-2,4-dioxo-3,4-dihydroquinazolin-1(2H)-yl}pipéridine-1-carbaldéhyde
n°319 : 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1 (2H)-yl}pipéridine-1-carbaldéhyde
n°320 : 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°321 : 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°322 : 4-{7-fluoro-6-[2-fluoro-1-(fluorométhyl)éthoxy]-2,4-dioxo-3-(4-pyrimidin-5-ylbenzyl)-3,4-dihydroquinazolin-1 (2H)-yl}pipéridine-1-carbaldéhyde
n°323 : 4-{7-fluoro-6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
n°324 : 4-{7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
n°325 : 4-[3-{4-[2-(2,3-dihydro-1H-indol-1-yl)-2-oxoéthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°326 : 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
n°327 : 4-[3-{4-[(3-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°328 : 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde
n°329 : 4-({7-fluoro-6-[2-fluoro-1-(fluorométhyl)éthoxy]-1-(1-formytpipéridin-4-yl)-2,4-dioxo-1,4-dihydroquinazolin-3(2H)-yl}méthyl)biphényl-2-carbonitrile
n°330 : 4-{7-fluoro-6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°331 :4-{7-fluoro-6-[2-fluoro-1-(fluorométhyl)éthoxy]-3-{[6-(3-fluorophényl)pyridin-3-yl]méthyl}-2,4-dioxo-3,4-dihydroquinazolin-1(2H)-yl}pipéridine-1-carbaldéhyde
n°332 : 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile
n°333 : 4-[3-(3,4-diéthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°334 : 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°335 : 4-{7-fluoro-6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°336 : 4-{7-fluoro-6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°337 : 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°338 : 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°339 : 4-{3-[4-(1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°340 : 5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxybenzonitrile
n°341 : 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile
n°342 : 4-[3-(4-bromobenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°343 : 4-[3-{4-[(3,4-dichlorobenzyl)oxy]-3-(2-méthoxyéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°344 : 4-{3-[4-(benzyloxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°345 : 4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°349 : 4-[3-{4-[(3,4-dichlorobenzyl)oxy]-3-(2-fluoroéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°350 : 4-[3-{4-[(2-chloro-4-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°351 : 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°352 : 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°353 : 4-[3-{4-[(2,6-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°354 : 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°355 : 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°357 : 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile
n°358 : 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°360 : 4-{7-fluoro-6-[2-fluoro-1-(fluorométhyl)éthoxy]-2,4-dioxo-3-[4-(2-phényléthyl)benzyl]-3,4-dihydroquinazolin-1(2H)-yl}pipéridine-1-carbaldéhyde
n°362 : 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde n°369 : 4-[3-{4-[(4-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°371 : 4-[3-{3-chloro-4-[(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°373 : 4-[3-{3-chloro-4-[(2,4-dichlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°375 : 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°376 : 4-[3-{4-[(3,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°377 : 4-[3-(4-{[4-chloro-3-(trifluorométhyl)benzyl]oxy}-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°379 : 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1 (2H)-yl]pipéridine-1-carbaldéhyde
n°380 : 4-[3-{4-[(3,5-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°381 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°382 : 4-[3-{4-[(3-chloro-5-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°383 : 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde
n°384 : 4-[3-{4-[(2,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°385 : 4-{[4-({7-fluoro-6-[2-fluoro-l-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
n°386 : 3-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
n°387 : 4-[3-{4-[(4-chloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°388 : 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°389 : 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1(2H)-yl}pipéridine-1-carbaldéhyde
n°390 : 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°391 : 4-[3-{4-[(3,4-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°392 : 4-{3-[4-(5,6-dichloro-1H-benzimidazol-1-yl)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°393 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle
n°394 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle
n°403 : 3,4-dichloro-N-[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényl]benzamide
à l'état de base ou de sel d'addition à un acide.

## Claims

1. Use of a compound corresponding to the general formula (I) 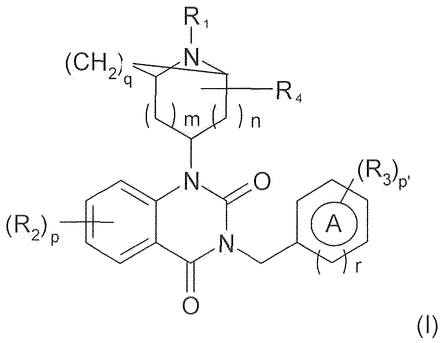 in which
- A represents an aryl group or a heteroaryl group;
- R₁ represents:
■ a hydrogen atom,
■ -C(O)R in which R is a hydrogen atom, a (C₁-C₆) alkoxy group, an aryl group, a (C₃-C₆) cycloalkyl group or a (C₁-C₆) alkyl group, the said alkyl optionally being substituted by:
. one or more hydroxyl group(s),
. a benzyloxy group,
. a (C₁-C₆) alkoxy group, optionally substituted by an aryl, or
. a (C₃-C₆) cycloalkyl group,
■ an optionally substituted (C₁-C₆) alkyl group;
- R₂ represents:
■ a hydrogen atom,
■ a halogen atom,
■ a cyano group,
■ a nitro group,
■ a (C₁-C₆) alkyl group optionally substituted by an -NH₂ or else by an -NHC(O)Rb group, with Rb as defined below,
■ an -ORa group in which Ra represents:
. a hydrogen atom,
. a (C₁-C₆) alkyl group optionally substituted by one or more halogen atom(s), by one or more hydroxyl group(s), by an aryl group and/or by one or more cyano group(s),
. a (C₂-C₆) alkynyl group,
. an aryl group;
- R₃ represents:
■ a hydrogen atom,
■ a halogen atom,
■ a hydroxyl group,
■ a cyano group,
■ an -SCF₃ group,
■ a nitro group,
■ an oxo group,
■ an -S(O)₀₋₂-alkyl group, an -S(O)₀₋₂-heterocycloalkyl group, an -O-SO₂-aryl group optionally substituted by one or more halogen atom(s);
■ an -alkylaminoalkyl or -cycloalkylaminoalkyl group, each optionally substituted on the end alkyl,
■ an optionally substituted sulphonamide group,
■ an aryl group or a heteroaryl group, the said group being monocyclic or polycyclic and in addition optionally being substituted by a (C₁-C₆) alkyl group, by one or more halogen atom(s) or by a (C₁-C₆) alkoxy group,
■ a heterocycloalkyl group optionally substituted by a (C₁-C₆) alkyl group,
■ a (C₁-C₆) alkyl group optionally substituted by:
- ane or more halogen atom(s),
- an aryl group which can be substituted by one or more halogen atom(s) or by one or more hydroxyl group(s),
- a heteroaryl group,
- one or more hydroxyl group(s) which can be substituted by an aryl group itself optionally substituted by one or more halogen atom(s), or
- a heterocycloalkyl group optionally substituted by a CO (O) Ra group or by a (C₁-C₆) alkyl group, with Ra as defined above,
■a -C(O)NRbRc group, with Rb and Rc as defined below,
■ a -C(O)ORc group or an -O-C(O)ORc group, with Rc as defined below,
■ a (C₁-C₆) alkoxy group, optionally substituted by
- an aminoalkyl group,
- an aminocycloalkyl group,
- a cycloalkyl group,
- a heteracycloalkyl group,
- a monocyclic or polycyclic heteroaryl group,
- one or more hydroxyl group(s),
- one or more halogen atom(s),
- a (C₁-C₆) alkoxy group,
- a -C(O)ORc group, with Rc as defined below,
- a -C (O) NRbRc group, with Rb and Rc as defined below,
- an oxo group, and/or
- an aryl group, itself optionally substituted by one or more halogen atom(s), a cyano group, a (C₁-C₆) alkoxy group, an -O-haloalkyl group and/or a haloalkyl group,
■ an -O-cycloalkyl group, an -O-aryl group or an
- O-heterocycloalkyl group, each optionally substituted by
- an aryl group, itself optionally substituted by one or more halogen atom(s) or by a (C₁-C₆) alkyl group,
- an oxo group,
- one or more halogen atom(s), and/or
- a (C₁-C₆) alkyl group, which can itself be substituted by an aryl group and/or an oxo group,
■ an -NH-CO-NH-aryl group, an -NH-CO-NH-heteroaryl group or an -NH-CO-NH-(C₁-C₆) alkyl group, each optionally being substituted by one or more halogen atom(s), by a cyano group, by a nitro group, by one or more hydroxyl group (s) or by a (C₁-C₆) alkoxy group,
■ an -N-(C₁-C₆) alkyl group, it being possible for the (C₁-C₆) alkyl group to be substituted by
- one or more oxo group(s), and/or
- one or more aryl group(s) optionally substituted by one or more halogen atom(s) and/or by an SO₂ group,
■ an -NH-CO-aryl group or an -NH-CO-heteroaryl group, each optionally being substitutes by one or more halogen atom(s);
or else R₃ forms, with A, a polycyclic heteroaryl group optionally substituted by a (C₁-C₆) alkoxy group or a (C₁-C₆) alkyl group optionally substituted by an aryl group which can itself be substituted by one or more halogen atom(s);
- R₄ represents a hydrogen atom, an oxo group or a (C₁-C₆) alkyl group;
- Rb represents:
. a hydrogen atom,
. a (C₁-C₆) alkyl group optionally substituted by one or more halogen atom(s), by one or more hydroxyl, cyano, amino, heterocycloalkyl, (C₁-C₆) alkoxy group(s) or by an aryl group optionally substituted by one or more halogen atom(s),
. a (C₃-C₆) cycloalkyl group,
. a (C₂-C₆) alkynyl group,
. a (C₁-C₆) alkoxy group,
. an aryl group optionally substituted by one or more halogen atom(s);
- Rc represents a hydrogen atom or a (C₁-C₆) alkyl group optionally substituted by one or more halogen atom(s);
or then Rb and Rc form, together with the nitrogen atom to which they are attached, a polycyclic heteroaryl group or a heterocycloalkyl group;
- m and n represent, independently of one another, the value 0, 1 or 2, it being understood that m+n≤3;
- p and p' represent, independently of one another, the value 1, 2 or 3, it being understood that, when p is greater than or equal to 2, then the R₂ groups are on separate carbon atoms and can be different from one another and, when p' is greater than or equal to 2, then the R₃ groups are on separate carbon atoms and can be different from one another;
- q represents the value 0 or 2, it being understood that, when q = 0, then the nitrogenous heterocyclic group attached to the nitrogen situated in the 1 position of the 2,4-dioxo-1,2,3,4-tetrahydroquinazoline ring system is no longer bridged and is of the type: 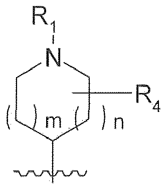 with R₁, R₄, m and n as defined above,
- r represents the value 0 or 1;
in the form of the base or of an addition salt with an acid;
in the preparation of a medicament intended for the treatment and/or prevention of psychiatric disorders and neurological disorders, among which the psychiatric disorders are chosen from anxiety, depression, mood disorders, insomnia, delusion disorders, obsessive disorders, psychoses, disorders related to schizophrenia, attention deficit hyperactivity disorders (ADHD) in hyperkinetic children, disorders related to the use of psychotropic substances, in particular in the case of abuse of a substance and/or of dependency on a substance, including alcohol dependence and/or nicotine dependence, migraine, stress, disorders related to illnesses of psychosomatic origin, panic attacks, epilepsy, memory disorders, cognitive disorders, in particular senile dementia or disorders related to Alzheimer's disease, and disorders of attention or of vigilance, ischaemia, disorders related to brain trauma or disorders related to acute or chronic neurodegenerative diseases, including chorea and Huntington's chorea, and the neurological disorders are reflected by movement anomalies or motor disorders associated with a pathology chosen from dyskinesia, Parkinson's disease, postencephalitic parkinsonism, dopa-sensitive dystonia, Shy-Drager syndrome, periodic limb movement disorder (PLMD) syndrome, periodic limb movements in sleep (PLMS) syndrome, Tourette's syndrome or restless legs syndrome (RLS).

2. Use according to Claim 1, **characterized in that** the movement anomalies or motor disorders are associated with Parkinson's disease.

3. Use according to Claim 2, **characterized in that** the movement anomalies or motor disorders are chosen from resting tremor, rigidity, bradykinesia and deficiency in postural reflexes.

4. Use according to one of Claims 1 to 3, (a) in the treatment and/or prevention of disorders related to schizophrenia, in particular (i) in the prevention and/or treatment of positive or negative symptoms and/or (ii) in the prevention and/or treatment of memory deficiency, (b) in the treatment and/or prevention of disorders associated with Parkinson's disease, in particular (i) in the symptomatic prevention and/or treatment of motor disorders, depression and/or cognitive disorders and/or (ii) in its basic treatment, and/or (c) in the treatment and/or prevention of disorders related to Alzheimer's disease, in particular (i) in the symptomatic prevention and/or treatment of cognitive disorders and/or behavioural disorders and/or (ii) in its basic treatment.

5. Use according to Claim 1, **characterized in that** the neurological disorders are reflected by movement anomalies or motor disorders associated with trauma of the spinal cord, in particular spinal trauma.

6. Use according to any one of the preceding claims, **characterized in that** A represents a phenyl group or a pyridyl group.

7. Use according to any one of the preceding claims, **characterized in that** q = 0, and m and n each represents 1.

8. Use according to any one of the preceding claims, **characterized in that** R₂ represents a (C₁-C₆) alkyl group, in particular a methyl, substituted by an - NH-C(O)-Rb group, Rb being as defined in Claim 1.

9. Use according to any one of Claims 1 to 7, **characterized in that** R₂ represents an -ORa group, Ra being as defined in Claim 1.

10. Use according to any one of Claims 1 to 7, **characterized in that** R₂ is a halogen atom or a cyano or a hydrogen or a hydroxyl or a (C₁-C₆) alkyl optionally substituted by an -NH₂ or else by an -NHC(O)Rb group, Rb being as defined in Claim 1.

11. Use according to any one of Claims 1 to 7, **characterized in that** A is a phenyl, R is a -C(O)R group in which R represents a hydrogen atom, q is equal to 0, n and m each have the value 1 and R₂ represents -ORa, Ra being as defined in Claim 1.

12. Use according to any one of Claims 1 to 7, **characterized in that** A is a phenyl, R₁ is a -C(O)R group in which R represents a hydrogen atom, q is equal to 0, n and m each have the value 1, p = 1 and R₂ represents a methyl substituted by an -NH-CO-Rb group, Rb being as defined in Claim 1.

13. Use according to any one of Claims 1 to 7, **characterized in that** A is a phenyl, R₁ is a -C(O)R group in which R represents a hydrogen atom, q is equal to 0, n and m each have the value 1, p is equal to 2, one of the R₂ groups is -ORa, Ra being as defined in Claim 1, and the other of the R₂ groups is a halogen atom.

14. Use according to any one of the preceding claims, **characterized in that** the R₂ group is in the 6 position of the 2,4-dioxo-1,2,3,4-tetrahydroquinazoline ring system and **in that** there may additionally be an R₂ group, identical to or different from the said abovementioned R₂ group, in the 7 position of the 2,4-dioxo-1,2,3,4-tetrahydroquinazoline ring system, the said compound of formula (I) being in the base, hydrate or solvate form, or in the form of their mixtures.

15. Use according to any one of Claims 1 to 5, **characterized in that** the compound of formula (I) is chosen from the following compounds:
No. 1: 2-{[3-(3,4-dimethoxybenzyl)-1-(1-formylpiperidin-4-yl)-2,4-dioxo-1,2,3,4-tetrahydroquinazolin-6-yl]oxy}propanenitrile
No. 2: 1-(1-acetylpiperidin-4-yl)-3-(3,4-dimethoxybenzyl)-6-hydroxyquinazoline-2,4(1H,3H)-dione
No. 3: {[1-(1-acetylpiperidin-4-yl)-3-(3,4-dimethoxybenzyl)-2,4-dioxo-1,2,3,4-tetrahydroquinazolin-6-yl]oxy}acetonitrile
No. 4: 2-{[1- (1-acetylpiperidin-4-yl)-3-(3,4-dimethoxybenzyl)-2,4-dioxo-1,2,3,4-tetrahydroquinazolin-6-yl]oxy}propanenitrile
No. 6: {[3-(3,4-dimethoxybenzyl)-1-(1-formylpiperidin-4-yl)-2,4-dioxo-1,2,3,4-tetrahydroquinazolin-6-yl]oxy}acetonitrile
No. 11 : 4-[3-(3,4-dimethoxybenzyl)-6-[2-fluoro-1-(fluaromethyl)ethoxy]-2,4-dioxo-3,4-dihydroquinazolin-1(2H)-yl]piperidine-1-carbaldehyde
No. 12: 1-(1-acetylpiperidin-4-yl)-3-(3,4-dimethoxybenzyl)-6-[2-fluoro-1-(fluoromethyl)ethoxy]quinazoline-2,4(1H,3H)-dione
No. 13: 4-[3-(3,4-dimethoxybenzyl)-2,4-dioxo-6-(2,2,2-trifluoroethoxy)-3,4-dihydroquinazolin-1(2H)-yl]piperidine-1-carbaldehyde
No. 14: 1-(1-acetylpiperidin-4-yl)-6-(2,2-difluoroethoxy)-3-(3,4-dimethoxybenzyl)quinazoline-2,4(1H,3H)-dione
No. 16: 4-[6-(2,2-difluoroethoxy)-3-(3,4-dimethoxybenzyl)-2,4-dioxo-3,4-dihydroquinazolin-1(2H)-yl]piperidine-1-carbaldehyde
No. 20: N-{[3-(3,4-dimethoxybenzyl)-1-(1-formylpiperidin-4-yl)-2,4-dioxo-1,2,3,4-tetrahydroquinazolin-6-yl]methyl}acetamide
No. 22: 1-(1-acetylpiperidin-4-yl)-6-(aminomethyl)-3-(3,4-dimethoxybenzyl)quinazoline-2,4(1H,3H)-dione hydrochloride
No. 23: N-{[3-(3,4-dimethoxybenzyl)-1-(1-formylpiperidin-4-yl)-2,4-dioxo-1,2,3,4-tetrahydroquinazolin-6-yl]methyl}formamide
No. 24: N-{[1-(1-acetylpiperidin-4-yl)-3-(3,4-dimethoxybenzyl)-2,4-dioxo-1,2,3,4-tetrahydroquinazolin-6-yl]methyl}formamide
No. 25: N-{[1-(1-acetylpiperidin-4-yl)-3-(3,4-dimethoxybenzyl)-2,4-dioxo-1,2,3,4-tetrahydroquinazolin-6-yl]methyl}acetamide
No. 32: 4-[6-(2,2-difluoroethoxy)-2,4-dioxo-3,4-dihydroquinazolin-1(2H)-yl]piperidine-1-carbaldehyde
No. 33: 4-[3-(3,4-dichlorobenzyl)-6-(2,2-difluoraethoxy)-2,4-dioxo-3,4-dihydraquinazalin-1(2H)-yl] piperidine-1-carbaldehyde
No. 34: 4-[3-(4-chlorobenzyl)-6-(2,2-difluoroethaxy)-2,4-dioxo-3,4-dihydroquinazolin-1(2H)-yl]piperidine-1-carbaldehyde
No. 35: methyl 4-{[6-(2,2-difluoroethoxy)-1-(1-formylpiperidin-4-yl)-2,4-dioxo-1,4-dihydroquirrazolin-3(2H)-yl]methyl}benzoate
No. 36: 4-{[6-(2,2-difluoroethoxy)-1-(1-formylpiperidin-4-yl)-2,4-dioxo-1,4-dihydroquinazolin-3(2H)-yl]methyl}benzoic acid
No. 37 : 4-{[6-(2,2-difluoroethoxy)-1-(1-formylpiperidin-4-yl)-2,4-dioxo-1,4-dihydroquinazolin-3(2H)-yl]methyl}-N-(2-methoxyethyl)benzamide
No. 38: 4-[3-(3,4-dimethoxybenzyl)-6-methyl-2,4-dioxo-3,4-dihydroquinazolin-1(2H)-yl] piperidine-1-carbaldehyde
No. 39: 4-[6-(2,2-difluoroethoxy)-3-(3-hydroxy-4-methoxybenzyl)-2,4-dioxo-3,4-dihydraquinazolin-1(2H)-yl]piperidine-1-carbaldehyde
No. 40: 4-[6-(2,2-difluoroethoxy)-3-[3-(2-hydroxyethoxy)-4-methoxybenzyl]-2,4-diaxo-3,4-dihydroquinazolin-1(2H)-yl]piperidine-1-carbaldehyde
No. 41: 4-[6-(2,2-difluoroethoxy)-3-(3-ethoxy-4-methoxybenzyl)-2,4-dioxo-3,4-dzhydroquinazolin-1(2H)-yl]piperidine-1-carbaldehyde
No. 42: 4-[6-(2,2-difluoroethoxy)-3-[4-methoxy-3-(2-methoxyethoxy)benzyl]-2,4-dioxo-3,4-dihydroquinazolin-1(2H)-yl]piperidine-1-carbaldehyde
No. 43: 4-[6-(2,2-difluoroethoxy)-3-(3,4-dimethoxybenzyl)-2,4-dioxo-3,4-dihydroquinazolin-1(2H)-yl]azepane-1-carbaldehyde
No. 47: 4-[6-(2,2-difluoroethoxy)-3-[3-(3-hydroxypropoxy)-4-methoxybenzyl]-2,4-dioxo-3,4-dihydroquinazolin-1(2H)-yl]piperidine-1-carbaldehyde
No. 48: 4-[5-chloro-3-(3,4-dimethaxybenzyl)-2,4-dioxo-3,4-dihydroquinazolin-1(2H)-yl]piperidine-1-carbaldehyde
No. 49: 4-{3-[3-(cyclopentyloxy)-4-methoxybenzyl]-6-(2,2-difluoroethoxy)-2,4-dioxo-3,4-dihydroquinazolin-1(2H)-yl}piperidine-1-carbaldehyde
No. 50: 2-(5-{[6-(2,2-difluoroethoxy)-1-(1-formylpiperidin-4-yl)-2,4-dioxo-1,4-dihydroquinazolin-3(2H)-yl]methyl}-2-methoxyphenaxy)acetamide
No. 51: 4-[6-(2,2-difluoroethoxy)-3-(3,4-dimethoxybenzyl)-2,4-dioxo-3,4-dihydroquinazolin-1(2H)-yl]-3-methylpiperidine-1-carbaldehyde
No. 52: 3-[6-(2,2-difluoroethoxy)-3-(3,4-dimethoxybenzyl)-2,4-dioxo-3,4-dihydroquinazolin-1(2H)-yl]-8-azabicyclo[3.2.1]octane-8-carbaldehyde No. 56: 4-{3-[4-(cyclopentyloxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 57: 4-[3-(3-chlorobenzyl)-6-[2-fluoro-1-(fluoromethyl)ethoxy]-2,4-dioxo-3,4-dihydroquinazolin-1(2H)-yl]piperidine-1-carbaldehyde
No. 58: 4-[3-(4-chlorobenzyl)-6-[2-fluoro-1-(fluoromethyl)ethoxy]-2,4-dioxo-3,4-dihydroquinazolin-1(2H)-yl]piperidine-1-carbaldehyde
No. 59: 4-{3-[3-(cyclopentyloxy)-4-methoxybenzyl]-6-[2-fluoro-1-(fluoromethyl)ethoxy]-2,4-dioxo-3,4-dihydroquinazolin-1(2H)-yl}piperidine-1-carbaldehyde
No. 72: 4-[3-(3,4-dimethoxybenzyl)-6-(2-hydroxyethoxy)-2,4-dioxo-3,4-dlhydroquinazolin-1(2H)-yl]piperidine-1-carbaldehyde No. 74: 4-[3-(3,4-dichlorobenzyl)-6-[2-fluoro-1-(fluoromethyl)ethoxy]-2,4-dioxo-3,4-dihydroquinazolin-1(2H)-yl]piperidine-1-carbaldehyde
No. 76: 4-{3-[(6-chloropyridin-3-yl)methyl]-6-[2-fluora-1-(fluoromethyl)ethoxy]-2,4-dioxo-3,4-dihydroquinazolin-1(2H)-yl}piperidin-1-carbaldehyde
No. 78: 4-[3-(3-chloro-4-methoxybenzyl)-6-[2-fluoro-1-(fluoromethyl)ethoxy]-2,4-dioxo-3,4-dihydroquinazolin-1(2H)-yl]piperidine-1-carbaldehyde
No. 79: 4-[3-(3,4-dimethoxybenzyl)-6-(2-fluoroethoxy)-2,4-dioxo-3,4-dihydraquinazolin-1(2H)-yl]piperidine-1-carbaldehyde
No. 89: 2-[5-({6-[2-fluoro-1-(fluoromethyl)ethoxy]-1-(1-formylpiperidin-4-yl)-2,4-dioxo-1,4-dihydroquinazolin-3(2H)-yl}methyl)-2-methaxyphenoxy] acetamide
No. 90: 4-{6-[2-fluoro-1-(fluoromethyl)ethoxy]-3-(3-hydroxy-4-methoxybenzyl)-2,4-dioxo-3,4-dihydroquinazolin-1(2H)-yl}piperidine-1-carbaldehyde
No. 91: 4-[3-(3,4-dimethoxybenzyl)-6-ethoxy-2,4-dioxo-3,4-dihydroquinazolin-1(2H)-yl]piperidine-1-carbaldehyde
No. 97: 4-[5,7-dichloro-3-(3,4-dimethoxybenzyl)-2,4-dioxo-3,4-dihydroquinazolin-1(2H)-yl]piperidine-1-carbaldehyde
No. 102: 4-[7-chloro-3-(3,4-dimethoxybenzyl)-2,4-dioxo-3,4-dihydroquinazolin-1(2H)-yl]piperidine-1-carbaldehyde
No. 108: 4-{6-[2 fluoro-1-(fluoromethyl)ethoxy]-3-(3-fluoro-4-methoxybenzyl)-2,4-dioxo-3,4-dihydroquinazolin-1(2H)-yl}piperidine-1-carbaldehyde
No. 111 : 4-[6-(difluoromethoxy)-3-(3,4-dimethoxybenzyl)-2,4-dioxo-3,4-dihydroquinazolin-1(2H)-yl]piperidine-1-carbaldehyde
No. 112: 4-[3-(3,4-dzmethoxybenzyl)-6-(1-methylethoxy)-2,4-dioxo-3,4-dihydroquinazolin-1(2H)-yl]piperidine-1-carbaldehyde
No. 114 : 4-{6-[2-fluoro-1-(fluoromethyl)ethoxy]-3-[4-methoxy-3-(1-methylethoxy)benzyl]-2,4-dioxo-3,4-dihydroquinazolin-1(2H)-yl}piperidine-1-carbaldehyde
No. 116: 4-{6-[2-fluoro-1-(fluoromethyl)ethoxy]-3-(3-methoxybenzyl)-2,4-dioxo-3,4-dihydroquinazolin-1(2H)-yl}piperidine-1-carbaldehyde
No. 117: 4-{3-[3,5-bis(trifluoromethyl)benzyl]-6-[2-fluoro-1-(fluoromethyl)ethoxy]-2,4-dioxo-3,4-dihydroquinazolin-1(2H)-yl}piperidine-1-carbaldehyde
No. 118 : 4-[3-(3-ethoxybenzyl)-6-[2-fluoro-1-(fluoromethyl)ethoxy]-2,4-dioxo-3,4-dihydroquznazolin-1(2H)-yl]piperidine-1-carbaldehyde
No. 124: 4-{3-[3-chloro-4-(2-methoxyethoxy)benzyl]-6-[2-fluoro-1-(fluoromethyl)ethoxy]-2,4-dioxo-3,4-dihydroquinazolin-1(2H)-yl}piperidine-1-carbaldehyde
No. 130: 4-[3-(3,4-diethoxybenzyl)-6-[2-fluoro-1-(fluoromethyl)ethoxy]-2,4-dioxo-3,4-dihydroquinazolin-1(2H)-yl]piperidine-1-carbaldehyde
No. 131: 4-[3-(4-ethoxy-3-methoxybenzyl)-6-[2-fluoro-1-(fluoromethyl)ethoxy]-2,4-dioxo-3,4-dihydroquinazolin-1(2H)-yl]piperidine-1-carbaldehyde
No. 133: 4-{6-[2-fluoro-1-(fluoromethyl)ethoxy]-3-(4-methoxy-3-methylbenzyl)-2,4-dioxo-3,4-dihydroquinazolin-1(2H)-yl}piperidine-1-carbaldehyde
No. 134: 4-{6-[2-fluoro-1-(fluoromethyl)ethoxy]-2,4-dioxo-3-[4-(trifluoromethyl)benzyl]-3,4-dihydroquinazolin-1(2H)-yl}piperidine-1-carbaldehyde
No. 135: 4-{6-[2-fluoro-1-(fluoromethyl)ethoxy]-2,4-dioxo-3-(3-phenoxybenzyl)-3,4_ dihydroquinazolin-1(2H)-yl}piperidine-1-carbaldehyde
No. 143: 4-{3-[4-(benzyloxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 145: 4-{6-[2-fluoro-1-(fluoromethyl)ethoxy]-3-(3-methoxy-4-nitrobenzyl)-2,4-dioxo-3,4-dihydroquinazolin-1(2H)-yl}piperidine-1-carbaldehyde
No. 155: 4-[3-(4-ethoxybenzyl)-6-[2-fluoro-1-(fluoromethyl)ethoxy]-2,4-dioxo-3,4-dihydroquinazolin-1(2H)-yl]piperidine-1-carbaldehyde
No. 158: 4-{6-[2-fluoro-1-(fluoromethyl)ethoxy]-3-[4-(morpholin-4-ylmethyl)benzyl]-2,4-dioxo-3,4-dihydroquinazolin-1(2H)-yl}piperidine-1-carbaldehyde
No. 160: 4-{6[2-fluoro-1-(fluoromethyl)ethoxy]-3-(4-morpholin-4-ylbenzyl)-2,4-dioxo-3,4-dihydroquinazolin-1(2H)-yl}piperidine-1-carbaldehyde
No. 165: 4- [3- (biphenyl-4-ylmethyl) -6-[2-fluoro-1-(fluoromethyl)ethoxy]-2,4-dioxo-3,4-dihydroquinazolin-1(2H)-yl]piperidine-1-carbaldehyde
No. 166: 4-{6-[2-fluoro-1-(fluoromethyl)ethoxy]-3-[4-(methylsulphanyl)benzyl]-2,4-dioxo-3,4-dihydroquinazolin-1(2H)-yl}piperidine-1-carbaldehyde
No. 167: 4-{6-[2-fluoro-1-(fluoromethyl)ethoxy]-2,4-dioxo-3-(4-(pyridin-3-yl)benzyl)-3,4-dihydroquinazolin-1(2H)-yl}piperidine-1-carbaldehyde
No. 170: 4-{6-[2-fluoro-1-(fluoromethyl)ethoxy]-3-(3-methoxy-4-methylbenzyl)-2,4-dioxo-3,4-dihydroquinazolin-1(2H)-yl}piperidine-1-carbaldehyde
No. 175: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]acetamide
No. 178: 4-{6-[2-fluoro-1-(fluoromethyl)ethoxy]-3-(3-methoxy-4-propoxybenzyl)-2,4-dioxo-3,4-dihydroquinazolin-1(2H)-yl}piperidine-1-carbaldehyde
No. 183: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-methylacetamide
No. 184: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N,N-dimethylacetamide
No. 185: 2-[2-(cyclopentyloxy)-5-({6-[2fluoro-1-(fluoromethyl)ethoxy]-1-(1-formylpiperidin-4-yl)-2,4-dioxo-1,4-dihydroquinazolin-3(2H)-yl}methyl)phenoxy]-N-methoxy-N-methylacetamide
No. 186: 4-{3-[4-(cyclopentyloxy)-3-ethoxybenzyl]-6-[2-fluoro-1-(fluoromethyl)ethoxy]-2,4-dioxo-3,4-dihydroquinazolin-1(2H)-yl}piperidine-1-carbaldehyde
No. 188: 4-{3-[4-(cyclopentyloxy)-3-(1-methylethoxy)benzyl]-6-[2-fluoro-1-(fluoromethyl)ethoxy]-2,4-dioxo-3,4-dihydroquinazolin-1(2H)-yl}piperidine-1-carbaldehyde
No. 189: 4-{3-4-(cyclopentyloxy)-3-propoxybenzyl]-6-[2-fluoro-1-(fluoromethyl)ethoxy]-2,4-dioxo-3,4-dihydroquinazolin-1(2H)-yl}piperidine-1-carbaldehyde
No. 190: 4-{3-[4-(cyclopentyloxy)-3-hydroxybenzyl]-6-[2-fluoro-1-(fluoromethyl)ethoxy]-2,4-dioxo-3,4-dihydroquinazolin-1(2H)-yl}piperidine-1-carbaldehyde
No. 193 : 4-{3-[4-(difluoromethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 194: 4-{3-[4-(difluoromethoxy)-3-ethoxybenzyl]-6-[2-fluoro-1-(fluoromethyl)ethoxy]-2,4-dioxo-3,4-dihydroquinazolin-1(2H)-yl})piperidine-1-carbaldehyde
No. 200: 4-{6-[2-fluoro-1-(fluoromethyl)ethoxy]-2,4-dioxo-3-(4-(thiophen-3-yl)benzyl)-3,4-dihydroquinazolin-1(2H)-yl}piperidine-1-carbaldehyde
No. 201: 4-{6-[2-fluoro-1-(fluoromethyl)ethoxy]-2,4-dioxo-3-(4-(pyridin-4-yl)benzyl)-3,4-dihydroquinazolin-1(2H)-yl}piperidine-1-carbaldehyde
No. 203: 4-{6-[2-fluoro-1-(fluoromethyl)ethoxy]-3-[(1-methyl-1H-indol-6-yl)methyl]-2,4-dioxo-3,4-dihydroquinazolin-1(2H)-yl}piperidine-1-carbaldehyde
No. 206: 4-{3-[4-(cyclopropylmethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 207: 2-[4-({6-[2-fluoro-1-(fluoromethyl)ethoxy]-1(1-formylpiperidin-4-yl)-2,4-dioxo-1,4-dihydroquinazolin-3(2H)-yl}methyl)-2-methoxyphenoxy]-N-methylacetamide
No. 212: 4-{6-[2-fluoro-1-(fluoromethyl)ethoxy]-2,4-dioxo-3-[4-(1H-pyrazol-1-yl)benzyl]-3,4-dihydroquinazolin-1(2H)-yl}piperidine-1-carbaldehyde
No. 213: 4-{6-[2-fluoro-1-(fluoromethyl)ethoxy]-2,4-dioxo-3-(4-(pyridin-2-yl)benzyl)-3,4-dihydroquinazolin-1(2H)-yl}piperidine-1-carbaldehyde
No. 215: 4-{6-[2-fluoro-1-(fluoromethyl)ethoxy]-2,4-dioxo-3-(4-(thiophen-2-yl)benzyl)-3,4-dihydroquinazolin-1(2H)-yl}piperidine-1-carbaldehyde
No. 216: 4-{6-[2-fluoro-1-(fluoromethyl)ethoxy]-2,4-dioxo-3-(quinolin-7-ylmethyl)-3,4-dihydroquinazolin-1(2H)-yl}piperidine-1-carbaldehyde
No. 218: 4-{6-[2-fluoro-1-(fluoromethyl)ethoxy]-3-[(6-methoxynaphthalen-2-yl)methyl]-2,4-dioxo-3,4-dihydroquinazolin-1(2H)-yl}piperidine-1-carbaldehyde
No. 223: 4-{3-[4-(1H-benzimidazol-1-yl)benzyl]-6-[2-fluoro-1-(fluoromethyl)ethoxy]-2,4-dioxo-3,4-dihydroquinazolin-1(2H)-yl}piperidine-1-carbaldehyde
No. 224: 4-{6-[2-fluoro-1-(fluoromethyl)ethoxy]-3-[3-methoxy-4-(2-methylpropoxy)benzyl]-2,4-dioxo-3,4-dihydroquinazolin-1(2H)-yl}piperidine-1-carbaldehyde
No. 226: 4-{6-[2-f1uoro-1(fluoromethyl)ethoxy]-3-[3-methoxy-4-(tetrahydrofuran-3-yloxy)benzyl]-2,4-dioxo-3,4-dihydroquinazolin-1(2H)-yl}piperidine-1-carbaldehyde
No. 228: 4-[3-{4-[(1-benzylpyrrolidin-3-yl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 230: 4-[3-(1-benzothiophen-5-ylmethyl)-6-[2-fluoro-1-(fluoromethyl)ethoxy]-2,4-dioxo-3,4-dihydroquinazolin-1(2H)-yl]piperidine-1-carbaldehyde
No. 232: 4-{6-[2-fluoro-1-(fluoromethyl)ethoxy]-3-[3-methoxy-4-(1-methylethoxy)benzyl]-2,4-dioxo-3,4-dihydroquinazolin-1(2H)-yl}piperidine-1-carbaldehyde
No. 233 : 4-[3-(3,4-dimethoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 234: 4-[3-{4-[(1-acetylpyrrolidin-3-yl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 239: 4-[3-{4-[(4-fluorobenzyl)oxy]-3-methoxybenzyl}-6-[2-fluoro-1-(fluoromethyl)ethaxy]-2,4-dioxo-3,4-dihydroquinazolin-1(2H)-yl]piperidine-1-carbaldehyde
No. 240: 4-[3-{4-[(4-chlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 242: 4-[3-{4-[(3-chlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 243: 4-{6-[2-fluoro-1-(fluoromethyl)ethoxy]-2,4-dioxo-3-(3-(thiophen-3-yl)benzyl)-3,4-dihydroquinazolin-1(2H)-yl}piperidine-1-carbaldehyde
No. 245: 4-[3-(4-ethoxy-3-methoxybenzyl)-6-(2-hydroxyethoxy)-2,4-dioxo-3,4-dihydroquinazolin-1(2H)-yl]piperidine-1-carbaldehyde
No. 246: 4-[3-{4-[2-(2,3-dihydro-1H-indol-1-yl)-2-oxoethoxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 250: 4-[3-{4-[(3,4-dichlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 251: 4-{6-[2-fluoro-1-(fluoromethyl)ethoxy]-3-[3-methoxy-4-(2-oxo-2-(piperidin-1-yl)ethoxy)benzyl]-2,4-dioxo-3,4-dihydroquinazolin-1(2H)-yl}piperidine-1-carbaldehyde
No. 254: 4-{3-[3-ethoxy-4-(thiophen-2-ylmethoxy)benzyl]-6-[2-fluoro-1-(fluoromethyl)ethoxy]-2,4-dioxo-3,4-dihydroquinazolin-1(2H)-yl}piperidine-1-carbaldehyde
No. 258: 4-[3-(3,4-dimethoxybenzyl)-6-[2-fluoro-1-(hydroxymethyl)ethoxy]-2,4-dioxo-3,4-dihydroquinazolin-1(2H)-yl]piperidine-1-carbaldehyde
No. 263: (2R)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]propanoic acid
No. 264: 4-{6-[2-fluoro-1-(fluoromethyl)ethoxy]-3-[(1-methyl-3-(thiophen-2-yl)-1H-pyrazol-5-yl)methyl]-2,4-dioxo-3,4-dihydroquinazolin-1(2H)-yl}piperidine-1-carbaldehyde
No. 270: 4-{6-[2-fluoro-1-(fluoromethyl)ethoxy]-3-[4-(5-methyl-1,2,4-oxadiazol-3-yl)benzyl]-2,4-dioxo-3,4-dihydroquinazolin-1(2H)-yl}piperidine-1-carbaldehyde
No. 275: 4-{6-[2-fluoro-1-(fluoromethyl)ethoxy]-2,4-dioxo-3-(4-(pyrimidin-5-yl)benzyl)-3,4-dihydroquinazolin-1(2H)-yl}piperidine-1-carbaldehyde
No. 276: 4-{6-[2-fluoro-1-(fluoromethyl)ethoxy]-3-[(1-methyl-3-phenyl-1H-pyrazol-5-yl)methyl]-2,4-dioxo-3,4-dihydroquinazolin-1(2H)-yl}piperidine-1-carbaldehyde
No. 278: 4-{6-[2fluoro-1(fluoromethyl)ethoxy]-2,4-dioxo-3-{[6-(1H-pyrazol-1-yl)pyridin-3-yl]methyl}-3,4-dihydroquinazolin-1(2H)-yl}piperidine-1-carbaldehyde
No. 279: 4-{6-[2-fluoro-1-(fluoromethyl)ethoxy]-2,4-dzoxo-3-[(2-(thiophen-2-yl)pyrimidin-5-yl)methyl]-3,4-dihydroquinazolin-1(2H)-yl}piperidine-1-carbaldehyde
No. 280: 4-{6[2-fluoro-1-(fluoromethyl)ethoxy]-3-[4-(1-methyl-1H-pyrazol-3-yl)benzyl]-2,4-dioxo-3,4-dihydroquinazolin-1(2H)-yl}piperidine-1-carbaldehyde
No. 282: 4{6-[2-fluoro-1-(fluoromethyl)ethoxy]-3-[4-(3-methyl-1,2,4-oxadiazol-5-yl)benzyl]-2,4-dioxo-3,4-dihydroquinazolin-1(2H)-yl}piperidine-1-carbaldehyde
No. 283: [2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]acetic acid
No. 285: 4-{6-[2-fluoro-1-(fluoromethyl)ethoxy]-2,4-dioxo-3-(thieno[2,3-b]pyridin-2-ylmethyl)-3,4-dihydroquinazolin-1(2H)-yl}piperidine-1-carbaldehyde
No. 286: 4-{6[2-fluoro-1-(fluoromethyl)ethoxy]-2,4-dioxo-3-[(6-phenylpyridin-3-yl)methyl]-3,4-dihydroquinazolin-1(2H)-yl}piperidine-1-carbaldehyde
No. 287: 4-{6-[2-fluoro-1-(fluoromethyl)ethoxy]-3-[(6-(morpholin-4-yl)pyridin-3-yl)methyl]-2,4-dioxo-3,4-dihydroquinazolin-1(2H)-yl}piperidine-1-carbaldehyde
No. 289: 4-{6-[2-fluoro-1-(fluoromethyl)ethoxy]-2,4-dioxo-3-[(6-(thiophen-2-yl)pyridin-3-yl)methyl]-3,4-dihydroquinazolin-1(2H)-yl}piperidine-1-carbaldehyde
No. 292: 4-{6-[2-fluoro-1-(fluoromethyl)ethoxy]-3-[(1-methyl-5-phenyl-1H-pyrazol-3-yl)methyl]-2,4-dioxo-3,4-dihydroquinazolin-1(2H)-yl}piperidine-1-carbaldehyde
No. 294: 4-({6-[2-fluoro-1-(fluoromethyl)ethoxy]-1-(1-formylpiperidin-4-yl)-2,4-dioxo-1,4-dihydroquinazolin-3(2H)-yl}methyl)biphenyl-2-carbonitrile
No. 295: (2R)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-methylpropanamide
No. 297: 4-{7-fluoro-6-[2-fluoro-1-(fluoromethyl)ethoxy]-2,4-dioxo-3-(4-(thiophen-2-yl)benzyl)-3,4-dihydroquinazolin-1(2H)-yl}piperidine-1-carbaldehyde
No. 298: 4-{6-[2-fluoro-1-(fluoromethyl)ethoxy]-3-[3-methoxy-4-(morpholin-4-ylmethyl)benzyl]-2,4-dioxo-3,4-dihydroquinazolin-1(2H)-yl}piperidine-1-carbaldehyde
No. 299: 4-{6-[2-fluoro-1-(fluoromethyl)ethoxy]-3-[3-methoxy-4-(piperidin-1-ylmethyl)benzyl]-2,4-dioxo-3,4-dihydroquinazolin-1(2H)-yl}piperidine-1-carbaldehyde
No. 300: 4-[3-{4-[(3,4-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 301: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-ethylacetamide
No. 302: (2S)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]propanoic acid
No. 305: 4-{6-[2-fluoro-1-(fluoromethyl)ethoxy]-3-(3-methoxy-4-{[(3R)-2-oxo-1-phenylpyrrolidin-3-yl]oxy}benzyl)-2,4-dioxo-3,4-dihydroquinazolin-1(2H)-yl}piperidine-1-carbaldehyde
No. 306: 4-{3-[4-(cyclobutylmethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 307: 4-{3-[4-(benzyloxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 308: 4-{7-fluoro-6-[2-fluoro-1-(fluoromethyl)ethoxy]-3-(4-hydroxy-3-methoxybenzyl)-2,4-dioxo-3,4-dihydroquinazolin-1(2H)-yl}piperidine-1-carbaldehyde
No. 309: 4-{3-[4-(cyclopropylmethoxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 310: 4-{7-fluoro-6-[2-fluoro-1-(fluoromethyl)ethoxy]-3-[3-methoxy-4-(2-methylpropoxy)benzyl]-2,4-dioxo-3,4-dihydroquinazolin-1(2H)-yl}piperidine-1-carbaldehyde
No. 311: 4-{7-fluoro-6-[2-fluoro-1-(fluoromethyl)ethoxyl-3-[3-methoxy-4-(1-methylethoxy)benzyl]-2,4-dioxo-3,4-dihydroquinazolin-1(2H)-yl}piperidine-1-carbaldehyde
No. 312: 4-[3-(4-ethoxy-3-methoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 315: 4-{7-fluoro-6-[2-fluoro-1-(fluoromethyl)ethoxy]-3-{[6-(3-methoxyphenyl)pyridin-3-yl]methyl}-2,4-dioxo-3,4-dihydroquinazolin-1(2H)-yl}piperidine-1-carbaldehyde
No. 316: 4-{7-fluoro-6-[2-fluoro-1-(fluoromethyl)ethoxy]-3-{[6-(2-fluorophenyl)pyridin-3-yl]methyl}-2,4-dioxo-3,4-dihydroquinazolin-1(2H)-yl}piperidine-1-carbaldehyde
No. 317: 4-{7-fluoro-6-[2-fluoro-1-(fluoromethyl)ethoxy]-3-{[6-(4-fluorophenyl)pyridin-3-yl]methyl}-2,4-dioxo-3,4-dihydroquinazolin-1(2H)-yl}piperidine-1-carbaldehyde
No. 318: 4-{7-fluoro-6-[2-fluoro-1-(fluoromethyl)ethoxy]-3-{[6-(4-methoxyphenyl)pyridin-3-yl]methyl}-2,4-dioxo-3,4-dihydroquinazolin-1(2H)-yl}piperidine-1-carbaldehyde
No. 319: 4-{7-fluoro-6-[2-fluoro-1-(fluoromethyl)ethoxy]-2,4-dioxo-3-[(6-(thiophen-2-yl)pyridin-3-yl)methyl]-3,4-dihydroquinazolin-1(2H)-yl}piperidine-1-carbaldehyde
No. 320: 4-{3-[3-ethoxy-4-(thiophen-2-ylmethoxy)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 321: 4-{7-fluoro-6-[2-fluoro-1-(fluoromethyl)ethoxy]-3-[4-(1-methyl-1H-pyrazol-3-yl)benzyl]-2,4-dioxo-3,4-dihydroquinazolin-1(2H)-yl}piperidine-1-carbaldehyde
No. 322: 4-{7-fluoro-6-[2-fluoro-1-(fluoromethyl)ethaxy]-2,4-dioxo-3-(4-(pyrimidin-5-yl)benzyl)-3,4-dihydroquinazolin-1(2H)-yl}piperidine-1-carbaldehyde
No. 323: 4-{7-fluoro-6-[2-fluoro-1-(fluoromethyl)ethoxy]-3-[(1-methyl-3-(thiophen-2-yl)-1H-pyrazol-5-yl)methyl]-2,4-dioxo-3,4-dihydroquinazolin-1(2H)-yl}piperidine-1-carbaldehyde
No. 324: 4-{7-fluoro-6-[2-fluoro-1-(fluoromethyl)ethoxy]-3-[3-methoxy-4-(2-oxo-2-(piperidin-1-yl)ethoxy)benzyl]-2,4-dioxo-3,4-dihydroquinazolin-1(2H)-yl}piperidine-1-carbaldehyde
No. 325: 4-[3-{4-[2-(2,3-dihydro-1H-indol-1-yl)-2-oxoethoxy]-3-methoxybenzyl}-7-fluoro-6-[2-fluoro-1-(fluoromethyl)ethoxyl-2,4-dioxo-3,4-dihydroquinazolin-1(2H)-yl]piperidine-1-carbaldehyde
No. 326: 4-{7-fluoro-6-[2-fluoro-1-(fluoromethyl)ethoxy]-3-[4-(5-methyl-1,2,4-oxadiazol-3-yl)benzyl]-2,4-dioxo-3,4-dihydroquinazolin-1(2H)-yl}piperidine-1-carbaldehyde
No. 327: 4-[3-{4-[(3-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 328: 4-[3-{[6-(3,5-dichlorophenyl)pyridin-3-yl]methyl}-6-[2-fluoro-1-(fluoromethyl)ethoxy]-2,4-dioxo-3,4-dihydroquinazolin-1(2H)-yl]piperidine-1-carbaldehyde
No. 329: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)biphenyl-2-carbonitrile
No. 330: 4-{7-fluoro-6-[2-fluoro-1-(fluoromethyl)ethoxy]-2,4-dioxo-3-[4-(1H-pyrazol-1-yl)benzyl]-3,4-dihydroquinazolin-1(2H)-yl}piperidine-1-carbaldehyde
No. 331: 4-{7-fluoro-6-[2-fluoro-1-(fluoromethyl)ethoxy]-3-{[6-(3-fluorophenyl)pyridin-3-yl]methyl}-2,4-dioxo-3,4-dihydroquinazolin-1(2H)-yl}piperidine-1-carbaldehyde
No. 332: 3-[5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)pyridin-2-yl]benzonitrile
No. 333: 4-[3-(3,4-diethoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 334: 4-[3-{4-[(4-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 335: 4-{7-fluoro-6-[2-fluoro-1-(fluoromethyl)ethoxy]-3-[4-(morpholin-4-ylmethyl)benzyl]-2,4-dioxo-3,4-dihydroquinazolin-1(2H)-yl}piperidine-1-carbaldehyde
No. 336: 4-{7-fluoro-6-[2-fluoro-1-(fluoromethyl)ethoxy]-2,4-dioxo-3-{[6-(1H-pyrazol-1-yl)pyridin-3-yl]methyl}-3,4-dihydroquinazolin-1(2H)-yl}piperidine-1-carbaldehyde
No. 337: 4-{7-fluoro-6-[2-fluoro-1-(fluoromethyl)ethoxy]-3-(4-(morpholin-4-yl)-benzyl)-2,4-dioxo-3,4-dihydroquinazolin-1(2H)-yl}piperidine-1-carbaldehyde
No. 338: 4-{7-fluoro-6-[2-fluoro-1-(fluoromethyl)ethoxy]-3-(3-methoxy-4-propoxybenzyl)-2,4-dioxo-3,4-dihydroquinazolin-1(2H)-yl}piperidine-1-carbaldehyde
No. 339: 4-{3-[4-(1H-benzimidazol-1-yl)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 340: 5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxybenzonitrile
No. 341: 3-(3,4-dimethoxybenzyl)-6-[2-fluoro-1-(fluoromethyl)ethoxy]-1-(1-formylpiperidin-4-yl)-2,4-dioxo-1,2,3,4-tetrahydroquinazoline-7-carbonitrile
No. 342: 4-[3-(4-bromobenzyl)-7-fluoro-6-[2-fluoro-1-(fluoromethyl)ethoxy]-2,4-dioxo-3,4-dihydroquinazalin-1(2H)-yl]piperidzne-1-carbaldehyde
No. 343: 4-[3-{4-[(3,4-dichlorobenzyl)oxy]-3-(2-methoxyethoxy)benzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 344: 4-{3-[4-(benzyloxy)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 345: 4-[3-{4-[(3,4-dichlorobenzyl)oxy]-3-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 349: 4-[3-{4-[(3,4-dichlorobenzyl)oxy]-3-(2-fluoroethoxy)benzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 350: 4-[3-{4-[(2-chloro-4-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 351: 4-[3-{4-[(2,4-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 352: 4-[3-{4-[(2-chloro-6-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 353: 4-[3-{4-[(2,6-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 354: 4-[3-{4-[(2-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 355: 4-[7-fluoro-3-{4-[(2-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 357: 2-[(3,4-dichlorobenzyl)oxy]-5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)benzonitrile
No. 358: 4-[3-{4-[(3,4-dichlorophenoxy)methyl]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 360: 4-{7-fluoro-6-[2-fluoro-1-(fluoromethyl)ethoxy]-2,4-dioxo-3-[4-(2-phenylethyl)benzyl]-3,4-dihydroquinazolin-1(2H)-yl}piperidine-1-carbaldehyde
No. 362: 4-[3-{4-[(4,5-dichloro-2-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 369: 4-[3-{4-[(4-chlorophenoxy)methyl]-3-methoxybenzyl}-7-fluoro-6-[2-Eluoro-1-(fluoromethyl)ethoxy]-2,4-dioxo-3,4-dihydroquinazolin-1(2H)-yl]piperidine-1-carbaldehyde
No. 371: 4-[3-{3-chloro-4-[(4-chlorobenzyl)oxy]-5-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 373: 4-[3-{3-chloro-4-[(2,4-dichlorobenzyl)oxy]-5-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 375: 4-[7-fluoro-3-{4-[(4-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 376: 4-[3-{4-[(3,5-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 377: 4-[3-(4-{[4-chloro-3-(trifluoromethyl)benzyl]oxy}-3-methoxybenzyl)-7-fluoro-6-[2-fluoro-1-(fluoromethyl)ethoxy]-2,4 dioxo-3,4-dihydroquinazolin-1(2H)-yl]piperidine-1-carbaldehyde
No. 379: 4-[3-{4-[(3-chlorophenoxy)methyl]-3-methoxybenzyl}-7-fluoro-6-[2-fluoro-1-(fluoromethyl)ethoxy]-2,4-dioxo-3,4-dihydroquinazolin-1(2H)-yl]piperidzne-1-carbaldehyde
No. 380: 4-[3-{4-[(3,5-difluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 381: 4-{3-[4-(benzyloxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 382: 4-[3-{4-[(3-chloro-5-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 383: 4-{7-fluoro-6-[2-fluoro-1-(fluoromethyl)ethoxy]-3-(3-methoxy-4-{[4-(trifluoromethyl)benzyl]oxy}benzyl)-2,4-dioxo-3,4-dihydroquinazolin-1(2H)-yl}piperidine-1-carbaldehyde
No. 384: 4-[3-{4-[(2,5-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 385: 4-{[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]methyl}benzonitrile
No. 386: 3-{[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]methyl}benzonitrile
No. 387: 4-[3-{4-[(4-chloro-2-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 388: 4-[3-{4-[1-(3,4-dichlorophenyl)ethoxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 389: 4-{7-fluoro-6-[2-fluoro-1-(fluoromethyl)ethoxy]-3-{4-[(3-hydroxybenzyl)oxy]-3-methoxybenzyl}-2,4-dioxo-3,4-dihydroquinazolin-1(2H)-yl}piperidine-1-carbaldehyde No. 390: 4-[7-fluoro-3-{4-[(3-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 391: 4-[3-{4-[(3,4-difluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 392: 4-{3-[4-(5,6-dichloro-1H-benzimidazol-1-yl)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 393: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)phenyl 3,4-dichlorobenzenesulphonate No. 394: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenyl 3,4-dichlorobenzenesulphonate
No. 403: 3,4-dichloro-N-[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenyl]benzamide in the form of the base or of an addition salt with an acid.

## Patentansprüche

1. Verwendung einer Verbindung der allgemeinen Formel (I) 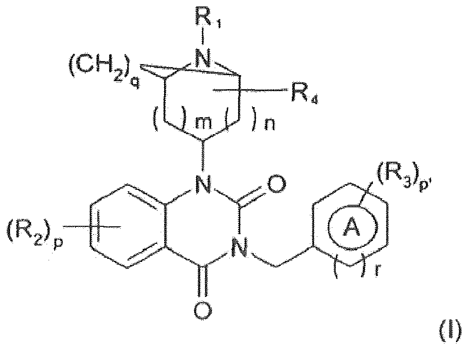 worin
- A für eine Arylgruppe oder eine Heteroarylgruppe steht;
- R₁ für:
■ ein Wasserstoffatom,
■ -C(O)R, worin R für ein Wasserstoffatom, eine (C₁-C₆)-Alkoxygruppe, eine Arylgruppe, eine (C₃-C₆)-Cycloalkylgruppe oder eine (C₁-C₆)-Alkylgruppe steht, wobei das Alkyl gegebenenfalls durch
. eine oder mehrere Hydroxygruppen,
. eine Benzyloxygruppe,
. eine (C₁-C₆)-Alkoxygruppe, die gegebenenfalls durch ein Aryl substituiert ist, oder
. eine (C₃-C₆)-Cycloalkylgruppe substituiert ist;
■ eine gegebenenfalls substituierte (C₁-C₆)-Alkylgruppe
steht;
- R₂ für:
■ ein Wasserstoffatom,
■ ein Halogenatom,
■ eine Cyanogruppe,
■ eine Nitrogruppe,
■ eine (C₁-C₆)-Alkylgruppe, die gegebenenfalls durch ein -NH₂ oder durch eine -NHC(O)Rb-Gruppe, worin Rb die nachstehend angegebene Bedeutung besitzt, substituiert ist,
■ eine -ORa-Gruppe, worin Ra für
. ein Wasserstoffatom,
. eine (C₁-C₆)-Alkylgruppe, die gegebenenfalls durch ein oder mehrere Halogenatome, durch eine oder mehrere Hydroxygruppen, durch eine Arylgruppe und/oder durch eine oder mehrere Cyanogruppen substituiert ist,
. eine (C₂-C₆)-Alkinylgruppe,
. eine Arylgruppe
steht, steht;
- R₃ für:
■ ein Wasserstoffatom,
■ ein Halogenatom,
■ eine Hydroxygruppe,
■ eine Cyanogruppe,
■ eine -SCF₃-Gruppe,
■ eine Nitrogruppe,
■ eine Oxogruppe,
■ eine -S(O)₀₋₂-Alkylgruppe, eine -S(O)₀₋₂-Heterocycloalkylgruppe, eine -O-SO₂-Arylgruppe, die gegebenenfalls durch ein oder mehrere Halogenatome substituiert ist,
■ eine -Alkylaminalkyl- oder -Cycloalkylaminoalkylgruppe, die am endständigen Alkyl gegebenenfalls substituiert ist,
■ eine gegebenenfalls substituierte Sulfonamidgruppe,
■ eine Arylgruppe oder eine Heteroarylgruppe, wobei die Gruppe monocyclisch oder polycyclisch ist und außerdem gegebenenfalls durch eine (C₁-C₆)-Alkylgruppe, durch ein oder mehrere Halogenatome oder durch eine (C₁-C₆)-Alkoxygruppe substituiert ist,
■ eine Heterocycloalkylgruppe, die gegebenenfalls durch eine (C₁-C₆)-Alkylgruppe substituiert ist,
■ eine (C₁-C₆)-Alkylgruppe, die gegebenenfalls durch
- ein oder mehrere Halogenatome,
- eine Arylgruppe, die durch ein oder mehrere Halogenatome oder eine oder mehrere Hydroxygruppen substituiert sein kann,
- eine Heteroarylgruppe,
- eine oder mehrere Hydroxygruppe, die durch eine Arylgruppe substituiert sein können, die selbst durch ein oder mehrere Halogenatome substituiert sein kann, oder
- eine Heterocycloalkylgruppe, die gegebenenfalls durch eine CO(O)Ra-Gruppe oder durch eine (C₁-C₆)-Alkylgruppe substituiert ist, wobei Ra die oben angegebene Bedeutung besitzt,
substituiert ist,
■ eine -C(O)NRbRc-Gruppe, wobei Rb und Rc die weiter unten angegebene Bedeutung besitzen,
■ eine -C(O)ORc-Gruppe oder eine -O-C(O)Orc-Gruppe, wobei Rc die weiter unten angegebene Bedeutung besitzt,
■ eine (C₁-C₆)-Alkoxygruppe, die gegebenenfalls durch
- eine Aminoalkylgruppe,
- eine Aminocycloalkylgruppe,
- eine Cycloalkylgruppe,
- eine Heterocycloalkylgruppe,
- eine monocyclische oder polycyclische Heteroarylgruppe,
- eine oder mehrere Hydroxygruppen,
- ein oder mehrere Halogenatome,
- eine (C₁-C₆)-Alkoxygruppe,
- eine -C(O)Rc-Gruppe, wobei Rc die weiter unten angegebene Bedeutung besitzt,
- eine -C(O)NRbRc-Gruppe, wobei Rb und Rc die weiter unten angegebene Bedeutung besitzen,
- eine Oxogruppe und/oder
- eine Arylgruppe, die selbst gegebenenfalls durch ein oder mehrere Halogenatome, eine Cyanogruppe, eine (C₁-C₆)-Alkoxygruppe, eine -O-Halogenalkylgruppe und/oder eine Halogenalkylgruppe substituiert ist, substituiert ist,
■ eine -O-Cycloalkylgruppe, eine -O-Arylgruppe oder eine -O-Heterocycloalkylgruppe, jeweils gegebenenfalls substituiert durch
- eine Arylgruppe, die selbst gegebenenfalls durch ein oder mehrere Halogenatome oder eine (C₁-C₆)-Alkylgruppe substituiert ist,
- eine Oxogruppe,
- ein oder mehrere Halogenatome und/oder
- eine (C₁-C₆)-Alkylgruppe, die selbst durch eine Arylgruppe und/oder eine Oxogruppe substituiert sein kann,
■ eine -NH-CO-NH-Arylgruppe, eine -NH-CO-NH-Heteroarylgruppe oder eine -NH-CO-NH-(C₁-C₆)-Alkylgruppe, jeweils gegebenenfalls substituiert durch ein oder mehrere Halogenatome, durch eine Cyanogruppe, durch eine Nitrogruppe, durch eine oder mehrere Hydroxygruppen oder durch eine (C₁-C₆)-Alkoxygruppe,
■ eine -N- (C₁-C₆)-Alkylgruppe, wobei die (C₁-C₆)-Alkylgruppe durch
- eine oder mehrere Oxogruppen und/oder
- eine oder mehrere Arylgruppen, gegebenenfalls substituiert durch ein oder mehrere Halogenatome und/oder durch eine SO₂-Gruppe, substituiert sein kann,
■ eine -NH-CO-Arylgruppe, eine -NH-CO-Heteroarylgruppe, jeweils gegebenenfalls substituiert durch ein oder mehrere Halogenatome steht;
oder R₃ mit A eine polycyclische Heteroarylgruppe bildet, die gegebenenfalls durch eine (C₁-C₆)-Alkoxygruppe, eine (C₁-C₆)-Alkylgruppe, die gegebenenfalls durch eine Arylgruppe, die selbst durch ein oder mehrere Halogenatome substituiert ist, substituiert ist;
- R₄ für ein Wasserstoffatom, eine Oxogruppe oder eine (C₁-C₆)-Alkylgruppe steht;
- Rb für:
. ein Wasserstoffatom;
. eine (C₁-C₆)-Alkylgruppe, die gegebenenfalls durch ein oder mehrere Halogenatome, durch eine oder mehrere Hydroxy-, Cyano-, Amino-Heterocycloalkyl- oder (C₁-C₆) -Alkoxygruppen oder durch eine Arylgruppe, die gegebenenfalls durch ein oder mehrere Halogenatome substituiert ist, substituiert ist,
. eine (C₃-C₆)-Cycloalkylgruppe,
. eine (C₂-C₆)-Alkinylgruppe,
. eine (C₁-C₆)-Alkoxygruppe,
. eine Arylgruppe, die gegebenenfalls durch ein
oder mehrere Halogenatome substituiert ist, steht;
- Rc für ein Wasserstoffatom oder eine (C₁-C₆)-Alkylgruppe, die gegebenenfalls durch ein oder mehrere Halogenatome substituiert ist, steht;
oder Rb und Rc zusammen mit dem Stickstoffatom, an das sie gebunden sind, eine polycyclische Heteroarylgruppe oder eine Heterocyeloalkylgruppe stehen;
- m und n unabhängig voneinander für den Wert 0, 1 oder 2 stehen, mit der Maßgabe, dass m+n ≤ 3;
- p und p' unabhängig voneinander für den Wert 1, 2 oder 3 stehen, mit der Maßgabe, dass dann, wenn p größer gleich 2 ist, die R₂-Gruppen an verschiedenen Kohlenstoffatomen stehen und gleich oder voneinander verschieden sein können, und dann, wenn p' größer gleich 2 ist, die R₃-Gruppen an verschiedenen Kohlenstoffatomen stehen und gleich oder voneinander verschieden sein können;
- q für den Wert 0 oder 2 steht, mit der Maßgabe, dass dann, wenn q = 0, die an den Stickstoff in 1-Position des 2,4-Dioxo-1,2,3,4-tetrahydrochinazolinkerns gebundene stickstoffhaltige heterocyclische Gruppe nicht mehr verbrückt ist und vom Typ: 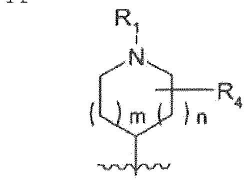 ist, wobei R₁, R₄, m und n die oben angegebene Bedeutung besitzen;
- r für den Wert 0 oder 1 steht;
in Basen- oder Säureadditionssalzform;
zur Herstellung eines Arzneimittels zur Behandlung und/oder Prävention von psychiatrischen Störungen und neurologischen Störungen einschließlich psychiatrischer Störungen, die unter Angst, Depression, Gemütsstörungen, Schlaflosigkeit, deliranten Störungen, obsessiven Störungen, Psychosen, Störungen in Verbindung mit Schizophrenie, Aufmerksamkeitsstörungen und Hyperaktivität (ADHD) bei hyperkinetischen Kindern, Störungen, die mit dem Missbrauch einer Substanz und/oder der Abhängigkeit von einer Substanz einschließlich Alkoholabhängigkeit und Nikotinabhängigkeit verbunden sind, Migräne, Stress, Störungen, die mit Krankheiten psychosomatischen Ursprungs verbunden sind, Panikattacken, Epilepsie, Gedächtnisstörungen, kognitiven Störungen, insbesondere seniler Demenz, Störungen in Verbindung mit Alzheimer-Krankheit sowie Aufmerksamkeits- oder Vigilanzstörungen, Ischämie, Sehädeltraumata, Strörungen in Verbindung mit akuten oder chronischen neurodegenerativen Erkrankungen einschließlich Chorea, Chorea Huntington ausgewählt sind, und neurologischer Störungen, die sich durch Bewegungsanomalien oder motorische Störungen in Assoziation mit einer unter Dyskinesien, Parkinson-Krankheit, Parkinson-Syndrom, Dopa-empfindlichen Dystonien, Shy-Drager-Syndrom, Syndrom der periodischen Gliedmaßenbewegungen (PLMD), Syndrom der periodischen Gliedmaßenbewegungen im Schlaf (PLMS), Tourette-Syndrom und Syndrom der unruhigen Beine ausgewählten Pathologie äußern.

2. Verwendung nach Anspruch 1, **dadurch gekennzeichnet, dass** die Bewegungsanomalien oder motorischen Störungen mit Parkinsan-Krankheit assoziiert sind.

3. Verwendung nach Anspruch 2, **dadurch gekennzeichnet, dass** die Anomalien oder motorischen Störungen aus Ruhetremor, Rigidität, Bradykinesie und Haltungsreflexdefizit ausgewählt sind.

4. Verwendung nach einem der Ansprüche 1 bis 3 (a) bei der Behandlung und/oder Prävention von Störungen in Verbindung mit Schizophrenie, insbesondere bei (i) der Prävention und/oder Behandlung von positiven oder negativen Symptomen und/oder (ii) bei der Prävention und/oder Behandlung von Gedächtnisdefiziten, (b) bei der Behandlung und/oder Prävention von Störungen, die mit Parkinson-Krankheit assoziiert sind, insbesondere (i) bei der symptomatischen Prävention und/oder Behandlung von motorischen Störungen, Depression und/oder kognitiven Störungen und/oder (ii) bei ihrer Basisbehandlung, und/oder (c) bei der Behandlung oder Prävention von Störungen, die mit Alzheimer-Krankheit assoziiert sind, insbesondere (i) bei der symptomatischen Prävention und/oder Behandlung von kognitiven Störungen und/oder Verhaltensstörungen und/oder (ii) bei ihrer Basisbehandlung.

5. Verwendung nach Anspruch 1, **dadurch gekennzeichnet, dass** die neurologischen Störungen sich durch Bewegungsanomalien oder motorische Störungen in Assoziation mit Traumata des Rückenmarks, insbesondere Spinaltraumata, äußern.

6. Verwendung nach einem der vorhergehenden Ansprüche, **dadurch gekennzeichnet, dass** A für eine Phenylgruppe oder Pyridylgruppe steht.

7. Verwendung nach einem der vorhergehenden Ansprüche, **dadurch gekennzeichnet, dass** q = 0 und m und n jeweils für 1 stehen.

8. Verwendung nach einem der vorhergehenden Ansprüche, **dadurch gekennzeichnet, dass** R₂ für eine (C₁-C₆)-Alkylgruppe, insbesondere Methylgruppe, die durch eine -NH-(CO)-Rb-Gruppe substituiert ist, wobei Rb die in Anspruch 1 angegebene Bedeutung besitzt, steht.

9. Verwendung nach einem der Ansprüche 1 bis 7, **dadurch gekennzeichnet, dass** R₂ für eine -ORa-Gruppe steht, wobei Ra die in Anspruch 1 angegebene Bedeutung besitzt.

10. Verwendung nach einem der Ansprüche 1 bis 7, **dadurch gekennzeichnet, dass** R₂ für ein Halogenatom oder ein Cyano oder einen Wasserstoff oder ein Hydroxyl oder ein (C₁-C₆)-Alkyl, das gegebenenfalls durch ein -NH₂ oder durch eine Gruppe-NH-(CO)-Rb-Gruppe substituiert ist, wobei Rb die in Anspruch 1 angegebene Bedeutung besitzt, steht.

11. Verwendung nach einem der Ansprüche 1 bis 7, **dadurch gekennzeichnet, dass** A für ein Phenyl steht, R₁ für eine -C(O)R-Gruppe, worin R für ein Wasserstoffatom steht, steht, q gleich 0 ist, n und m jeweils einen Wert von 1 haben und R₂ für -ORa steht, wobei Ra die in Anspruch 1 angegebene Bedeutung besitzt.

12. Verwendung nach einem der Ansprüche 1 bis 7, **dadurch gekennzeichnet, dass** A für ein Phenyl steht, R₁ für eine -C(O)R-Gruppe, worin R für ein Wasserstoffatom steht, steht, q gleich 0 ist, n und m jeweils einen Wert von 1 haben, p=1 und R₂ für ein Methyl, das durch eine Gruppe-NH-(CO)-Rb-Gruppe substituiert ist, wobei Rb die in Anspruch 1 angegebene Bedeutung besitzt, steht.

13. Verwendung nach einem der Ansprüche 1 bis 7, **dadurch gekennzeichnet, dass** A für ein Phenyl steht, R₁ für eine -C(O)R-Gruppe, worin R für ein Wasserstoffatom steht, steht, q gleich 0 ist, n und m jeweils einen Wert von 1 haben, p gleich 2 ist, einer der Reste R₂ für -ORa steht, wobei Ra die in Anspruch 1 oder 2 angegebene Bedeutung besitzt, und der andere Rest R₂ für ein Wasserstoffatom steht.

14. Verwendung nach einem der vorhergehenden Ansprüche, **dadurch gekennzeichnet, dass** die Gruppe R₂ in 6-Position des 2,4-Dioxo-1,2,3,4-tetrahydrochinazolinkerns steht und außerdem eine R₂-Gruppe, die mit der oben aufgeführten Gruppe R₂ identisch oder davon verschieben sein kann, in 7-Position des 2,4-Dioxo-1,2,3,4-tetrahydrochinazolinkerns vorliegen kann, wobei die Verbindung der Formel (I) in Basen-, Hydrat- oder Solvatform oder Mischungen davon vorliegt.

15. Verwendung nach einem der Ansprüche 1 bis 5, **dadurch gekennzeichnet, dass** die Verbindung der Formel (I) unter den folgenden Verbindungen ausgewählt ist:
Nr. 1: 2-{[3-(3,4-Dimethoxybenzyl)-1-(1-formylpiperidin-4-yl)-2,4-dioxo-1,2,3,4-tetrahydrochinazolin-6-yl]oxy}propanenitril
Nr. 2: 1-(1-Acetylpiperidin-4-yl)-3-(3,4-dimethoxybenzyl)-6-hydroxychinazolin-2,4(1H,3H)-dion
Nr. 3: {[1-(1-Acetylpiperidin-4-yl)-3-(3,4-dimethoxybenzyl)-2,4-dioxo-1,2,3,4-tetrahydrochinazolin-6-yl]oxy}acetonitril
Nr. 4: 2-{[1-(1-Acetylpiperidin-4-yl)-3-(3,4-dimethoxybenzyl)-2,4-dioxo-1,2,3,4-tetrahydrochinazolin-6-yl]oxy}propannitril
Nr. 6: {[3-(3,4-Dimethoxybenzyl)-1-(1-formylpiperidin-4-yl)-2,4-dioxo-1,2,3,4-tetrahydrochinazolin-6-yl]oxy}acetonitril
Nr. 11: 4-[3-(3,4-Dimethoxybenzyl)-6-[2-fluor-1-(fluormethyl)ethoxy]-2,4-dioxo-3,4-dihydrochlnazolin-1(2H)-yl]plperidin-1-carbaldehyd
Nr. 12: 1-(1-Acetylpiperidin-4-yl)-3-(3,4-dimethoxybenzyl)-6-[2-fluor-1 (fluormethyl)ethoxylchinazolin-2,4(1H,3H)-dion
Nr. 13: 4-[3-(3,4-Dimethoxybenzyl)-2,4-dioxo-6-(2,2,2-trifluorethoxy)-3,4-dihydrochinazolin-1(2H)-yl]piperidin-1-carbaldehyd
Nr. 14: 1-(1-Acetylpiperidin-4-yl)-6-(2,2-difluorethoxy)-3-(3,4-dimethoxybenzyl)chinazolin-2,4(1H,3H)-dion
Nr. 16: 4-[6-(2,2-Difluorethoxy)-3-(3,4-dimethoxybenzyl)-2,4-dioxo-3,4-dihydrochinazolin-1(2H)-yl]piperidin-1-carbaldehyd
Nr. 20: N-{[3-(3,4-Dimethoxybenzyl)-1-(1-formylpiperidin-4-yl)-2,4-dioxo-1,2,3,4-tetrahydrochinazolin-6-yl]methyl}acetamid
Nr. 22: 1-(1-Acetylpiperidin-4-yl)-6-(aminomethyl)-3-(3,4-dimethoxybenzyl)chinazolin-2,4(1H,3H)-dion-hydrochlorid
Nr. 23: N-{[3-(3,4-Dimethoxybenzyl)-1-(1-formylpiperidin-4-yl)-2,4-dioxo-1,2,3,4-tetrahydrochinazolin-6-yl]methyl}formamid
Nr. 24: N-{[1-(1-Acetylpiperidin-4-yl)-3-(3,4-dimethoxybenzyl)-2,4-dioxo-1,2,3,4-tetrahydrochinazolin-6-yl]methyl}formamid
Nr. 25: N-{[1-(1-Acetylpiperidin-4-yl)-3-(3,4-dimethoxybenzyl)-2,4-dioxo-1,2,3,4-tetrahydrochinazolin-6-yl]methyl}acetamid
Nr. 32: 4-[6-(2,2-Difluorethoxy)-2,4-dioxo-3,4-dihydrochinazolin-1(2H)-yl]piperidin-1-carbaldehyd
Nr. 33: 4-[3-(3,4-Dichlorbenzyl)-6-(2,2-difluorethoxy)-2,4-dsoxo-3,4-dihydrochinazolin-1(2H)-yl]piperidin-1-carbaldehyd
Nr. 34: 4-[3-(4-Chlorbenzyl)-6-(2,2-difluorethoxy)-2,4-dioxo-3,4-dihydrochinazolin-1(2H)-yl]piperidin-1-carbaldehyd
Nr. 35: 4-{[6-(2,2-Difluorethoxy)-1-(1-formylpiperidin-4-yl)-2,4-dioxo-1,4-dihydrochinazolin-3(2H)-yl]methyl}benzoesäuremethylester
Nr. 36: 4-{[6-(2,2-Difluorethoxy)-1-(1-formylpiperidin-4-yl)-2,4-dioxo-1,4-dihydrochinazolin-3(2H)-yl]methyl}benzoesäure
Nr. 37: 4-{[6-(2,2-Difluorethoxy)-1-(1-formylpiperidin-4-yl)-2,4-dioxo-1,4-dihydrochinazolin-3(2H)-yl]methyl}-N-(2-methoxyethyl)benzamid
Nr. 38: 4-[3-(3,4-Dimethoxybenzyl)-6-methyl-2,4-dioxo-3,4-dihydrochinazolin-1(2H)-yl]piperidin-1-carbaldehyd
Nr. 39: 4-[6-(2,2-Difluorethoxy)-3-(3-hydroxy-4-methoxybenzyl)-2,4-dioxo-3,4-dihydrochinazolin-1(2H)-yl]piperidin-1-carbaldehyd
Nr. 40: 4-[6-(2,2-Difluorethoxy)-3-[3-(2-hydroxyethoxy)-4-methoxybenzyl]-2,4-dioxo-3,4-dihydrochinazolin-1(2H)-yl]piperidin-1-carbaldehyd
Nr. 41: 4-[6-(2,2-Difluorethoxy)-3-(3-ethoxy-4-methoxybenzyl)-2,4-dioxo-3,4-dihydrochinazolin-1(2H)-yl]piperidin-1-carbaldehyd
Nr. 42: 4-[6-(2,2-Difluorethoxy)-3-[4-methoxy-3-(2-methoxyethoxy)benzyl]-2,4-dioxo-3,4-dihydrochinazolin-1(2H)-yl]piperidin-1-carbaldehyd
Nr. 43: 4-[6-(2,2-Difluorethoxy)-3-(3,4-dimethoxybenzyl)-2,4-dioxo-3,4-dihydrochinazolin-1(2H)-yl]azepane-1-carbaldehyd
Nr. 47: 4-[6-(2,2-Difluorethoxy)-3-[3-(3-hydroxypropoxy)-4-methoxybenzyl]-2,4-dioxo-3,4-dihydrochinazolin-1(2H)-yl]piperidin-1-carbaldehyd
Nr. 48: 4-[5-Chlor-3-(3,4-dimethoxybenzyl)-2,4-dioxo-3,4-dihydrochinazolin-1(2H)-yl]piperidin-1-carbaldehyd
Nr. 49: 4-{3-[3-(Cyclopentyloxy)-4-methoxybenzyl]-6-(2,2-difluorethoxy)-2,4-dioxo-3,4-dihydrochinazolin-1(2H)-yl}-piperidin-1-carbaldehyd
Nr. 50: 2-(5-{[6-(2,2-Difluorethoxy)-1-(1-formylpiperidin-4-yl)-2,4-dioxo-1,4-dihydrochinazolin-3(2H)-yl]-methyl}-2-methoxyphenoxy)-acetamid
Nr. 51: 4-[6-(2,2-Difluorethoxy)-3-(3,4-dimethoxybenzyl)-2,4-dioxo-3,4-dihydrochinazolin-1(2H)-yl]-3-methylpiperidin-1-carbaldehyd
Nr. 52: 3-[6-(2,2-Difluorethoxy)3-(3,4-dimethoxybenzyl)-2,4-dioxo-3,4-dihydrochinazolin-1(2H)-yl]-8-azabicyalo[3.2.1]octan-8-carbaldehyd
Nr. 56: 4-{3-[4-(Cyclopentyloxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 57: 4-[3-(3-Chlorbenzyl)-6-[2-fluor-1-(fluormethyl)ethoxy]-2,4-dioxo-3,4-dihydrochinazolin-1(2H)-yl]piperidin-1-carbaldehyd
Nr. 58: 4-[3-(4-Chlorbenzyl)-6-[2-fluor-1-(fluormethyl)ethoxy]-2,4-dioxo-3,4-dihydrochinazolin-1(2H)-yl]piperidin-1-carbaldehyd
Nr. 59: 4-{3-[3-(Cyclopentyloxy)4-methoxybenzyl]-6-[2-fluor-1-(fluormethyl)ethoxyl-2,4-dioxo-3,4-dihydrochinazolin-1(2H)-yl}piperidin-1-carbaldehyd
Nr. 72: 4-[3-(3,4-Dimethoxybenzyl)-6-(2-hydroxyethoxy)-2,4-dioxo-3,4-dihydrochinazolin-1(2H)-yl]piperidin-1-carbaldehyd
Nr. 74: 4-[3-(3,4-Dichlorbenzyl)-6-[2-fluor-1-(fluormethyl)ethoxy]-2,4-dioxo-3,4-dihydrochinazolin-1(2H)-yl]piperidin-1-carbaldehyd
Nr. 76: 4-{3-[(6-Chlorpyridin-3-yl)methyl]-6-[2-fluor-1-(fluormethyl)ethoxy]-2,4-dioxo-3,4-dihydrochinazolin-1(2H)-yl}piperidin-1-carbaldehyd
Nr. 78: 4-[3-(3-Chlor-4-methoxybenzyl)-6-[2-fluor-1-(fluormethyl)-ethoxy]-2,4-dioxo-3,4-dihydrochinazolin-1(2H)-yl]piperidin-1-carbaldehyd
Nr. 79: 4-[3-(3,4-Dimethoxybenzyl)-6-(2-fluorethoxy)-2,4-dioxo-3,4-dihydrochinazolin-1(2H)-yl]piperidin-1-carbaldehyd
Nr. 89: 2-[5-({6-[2-Fluor-1-(fluormethyl)ethoxy]-1-(1-formylpiperidin-4-yl)-2,4-dioxo-1,4-dihydrochinazolin-3(2H)-yl}methyl)-2-methoxyphenoxy]acetamid
Nr. 90: 4-{6-[2-Fluor-1-(fluormethyl)ethoxy]-3-(3-hydroxy-4-methoxybenzyl)-2,4-dioxo-3,4-dihydrochinazolin-1(2H)-yl}piperidin-1-carbaldehyd
Nr. 91: 4-[3-(3,4-Dimethoxybenzyl)-6-ethoxy-2,4-dioxo-3,4-dihydrochinazolin-1(2H)-yl]piperidin-1-carbaldehyd
Nr. 97: 4-[5,7-Dichlor-3-(3,4-dimethoxybenzyl)-2,4-dioxo-3,4-dihydrochinazolin-1(2H)-yl]piperidin-1-carbaldehyd
Nr. 102: 4-[7-Chlor-3-(3,4-dimethoxybenzyl)-2,4-dioxo-3,4-dihydrochinazolin-1(2H)-yl]piperidin-1-carbaldehyd
Nr. 108: 4-{6-[2-Fluor-1-(fluormethyl)-ethoxy]-3-(3-fluor-4-methoxybenzyl)-2,4-dioxo-3,4-dihydrochinazolin-1(2H)-yl}piperidin-1-carbaldehyd
Nr. 111: 4-[6-(Difluormethoxy)-3-(3,4-dimethoxybenzyl)-2,4-dioxo-3,4-dihydrochinazolin-1(2H)-yl]piperidin-1-carbaldehyd
Nr. 112: 4-[3-(3,4-Dimethoxybenzyl)-6-(1-methylethoxy)-2,4-dioxo-3,4-dihydrochinazolin-1(2H)-yl]piperidin-1-carbaldehyd
Nr. 114: 4-{6-[2-Fluor-1-(fluormethyl)ethoxy]-3-[4-methoxy-3-(1-methylethoxy)benzyl]-2,4-dioxo-3,4-dihydrochinazolin-1(2H)-yl}piperidin-1-carbaldehyd
Nr. 116: 4-{6-[2-Fluor-1-(fluormethyl)-ethoxy]-3-(3-methoxybenzyl)-2,4-dioxo-3,4-dihydrochinazolin-1(2H)-yl}piperidin-1-carbaldehyd
Nr. 117: 4-{3-[3,5-Bis(trifluormethyl)benzyl]-6-[2-fluor-1-(fluormethyl)ethoxy]-2,4-dioxo-3,4-dihydrochinazolin-1(2H)-yl}piperidin-1-carbaldehyd
Nr. 118: 4-[3-(3-Ethoxybenzyl)-6-[2-fluor-1-(fluormethyl)ethoxy]-2,4-dioxo-3,4-dihydrochinazolin-1(2H)-yl]plperidin-1-carbaldehyd
Nr. 124: 4-{3-[3-Chlor-4-(2-methoxyethoxy)benzyl]-6-[2-fluor-1-(fluormethyl)ethoxy]-2,4-dioxo-3,4-dihydrochinazolin-1(2H)-yl}piperidin-1-carbaldehyd
Nr. 130: 4-[3-(3,4-Diethoxybenzyl)-6-[2-fluor-1-(fluormethyl)ethoxy]-2,4-dioxo-3,4-dihydrochinazolin-1(2H)-yl]piperidin-1-carbaldehyd
Nr. 131: 4-[3-(4-Ethoxy-3-methoxybenzyl)-6-[2-fluor-1-(fluormethyl)ethoxy]-2,4-dioxo-3,4-dihydrochinazolin-1(2H)-yl]piperidin-1-carbaldehyd
Nr. 133: 4-{6-[2-Fluor-1-(fluormethyl)ethoxy]-3-(4-methoxy-3-methylbenzyl)-2,4-dioxo-3,4 dihydrochinazolin-1(2H)-yl}piperidin-1-carbaldehyd Nr. 134: 4-{6-[2-Fluor-1-(fluormethyl)ethoxy]-2,4-dioxo-3-[4-(trifluormethyl)benzyl]-3,4-dihydrochinazolin-1(2H)-yl}piperidin-1-carbaldehyd
Nr. 135: 4-{6-[2-Fluor-1-(fluormethyl)ethoxy]-2,4-dioxo-3-(3-phenoxybenzyl)-3,4-dihydrochinazolin-1(2H)-yl}piperidin-1-carbaldehyd
Nr. 143: 4-{3-[4-(Benzyloxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 145: 4-{6-[2-Fluor-1-(fluormethyl)ethoxy]-3-(3-methoxy-4-nitrobenzyl)-2,4-dioxo-3,4-dihydrochinazolin-1(2H)-yl}piperidin-1-carbaldehyd
Nr. 155: 4-[3-(4-Ethoxybenzyl)-6-[2-fluor-1-(fluormethyl)ethoxy]-2,4-dioxo-3,4-dihydrochinazolin-1(2H)-yl]piperidin-1-carbaldehyd
Nr. 158: 4-{6-[2-Fluor-1-(fluormethyl)ethoxy]-3-[4-(morpholin-4-ylmethyl)benzyl]-2,4-dioxo-3,4-dihydrochinazolin-1(2H)-yl}piperidin-1-carbaldehyd
Nr. 160: 4-{6-[2-Fluor-1-(fluormethyl)ethoxy]-3-(4-(morpholin-4-yl)benzyl)-2,4-dioxo-3,4-dihydrochinazolin-1(2H)-yl}piperidin-1-carbaldehyd
Nr. 165: 4-[3-(Biphenyl-4-ylmethyl)-6-[2-fluor-1-(fluormethyl-)ethoxy]-2,4-dioxo-3,4-dihydrochinazolin-1(2H)-yl]piperidin-1-carbaldehyd
Nr. 166: 4-{6-[2-1-(fluormethyl)ethoxy]-3-[4-(methylsulfanyl)benzyl]-2,4-dioxo-3,4-dihydrochinazolin-1(2H)-yl}piperidin-1-carbaldehyd
Nr. 167: 4-{6-[2-Fluor-1-(fluormethyl)ethoxy]-2,4-dioxo-3-(4-(pyridin-3-yl)benzyl)-3,4-dihydrochinazolin-1(2H)-yl}piperidin-1-carbaldehyd
Nr. 170: 4-{6-[2-Fluor-1-(fluormethyl)ethoxy]-3-(3-methoxy-4-methylbenzyl)-2,4-dioxo-3,4-dihydrochinazolin-1(2H)-yl}piperidin-1-carbaldehyd
Nr. 175: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]acetamide
Nr. 178: 4-{6-[2-Fluor-1-(fluormethyl)ethoxy]-3-(3-methoxy-4-propoxybenzyl)-2,4-dioxo-3,4-dihydrochinazolin-1(2H)-yl}piperidin-1-carbaldehyd
Nr. 183: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methylacetamid
Nr. 184: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N,N-dimethylacetamid
Nr. 185: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methoxy-N-methylacetamid
Nr. 186: 4-{3-[4-(Cyclopentyloxy)-3-ethoxybenzyl]-6-[2-fluor-1-(fluormethyl)ethoxy]-2,4-dioxo-3,4-dihydrochinazolin-1(2H)-yl}piperidin-1-carbaldehyd
Nr. 188: 4-{3-[4-(Cyclopentyloxy)-3-(1-methylethoxy)benzyl]-6-[2-fluor-1-(fluormethyl)ethoxy]-2,4-dioxo-3,4-dihydrochinazolin-1(2H)-yl}piperidin-1-carbaldehyd
Nr. 189: 4-{3-[4-(Cyclopentyloxy)-3-propoxybenzyl]-6-[2-fluor-1-(fluormethyl)ethoxy]-2,4-dioxo-3,4-dihydrochinazolin-1(2H)-yl}piperidin-1-carbaldehyd
Nr. 190: 4-{3-[4-(Cyclopentyloxy)-3-hydroxybenzyl]-6-[2-fluor-1-(fluormethyl)ethoxy]-2,4-dioxo-3,4-dihydrochinazolin-1(2H)-yl}piperidin-1-carbaldehyd
Nr. 193: 4-{3-[4-(Difluormethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 194: 4-{3-[4-(Difluormethoxy)-3-ethoxybenzyl]-6-[2-fluor-1-(fluormethyl)ethoxy]-2,4-dioxo-3,4-dihydrochinazolin-1(2H)-yl}piperidin-1-carbaldehyd
Nr. 200: 4-{6-[2-Fluor-1-(fluormethyl)ethoxy]-2,4-dioxo-3-(4-(thiophen-3-ylbenzyl)-3,4 dihydrochinazolin-1(2H)-yl}piperidin-1-carbaldehyd
Nr. 201: 4-{6-[2-Fluor-1-(fluormethyl)ethoxy]-2,4-dioxo-3-(4-(pyridin-4-ylbenzyl)-3,4-dihydrochinazolin-1(2H)-yl}piperidin-1-carbaldehyd
Nr. 203: 4-{6-[2-Fluor-1-(fluormethyl)ethoxy]-3-[(1-methyl-1H-indol-6-yl)methyl]-2,4-dioxo-3,4-dihydrochinazolin-1(2H)-yl}piperidin-1-carbaldehyd
Nr. 206: 4-{3-[4-(Cyclopropylmethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 207: 2-[4-({6-[2-Fluor-1-(fluormethyl)ethoxy]-1-(1-formylpiperidin-4-yl)-2,4-dioxo-1,4-dihydrochinazolin-3(2H)-yl}methyl)-2-methoxyphenoxy]-N-methylacetamid
Nr. 212: 4-{6-[2-Fluor-1-(fluormethyl)ethoxy]-2,4-dioxo-3-[4-(1H-pyrazol-1-yl)benzyl]-3,4-dihydrochinazolin-1(2H)-yl}piperidin-1-carbaldehyd
Nr. 213: 4-{6-[2-Fluor-1-(fluormethyl)ethoxy]-2,4-dioxo-3-(4-(pyridin-2-ylbenzyl)-3,4-dihydrochinazolin-1(2H)-yl)piperidin-1-carbaldehyd
Nr. 215: 4-{6-[2-Fluor-1-(fluormethyl)ethoxy]-2,4-dioxo-3-(4-(thiophen-2-ylbenzyl)-3,4-dihydrochinazolin-1(2H)-yl}piperidin-1-carbaldehyd
Nr. 216: 4-{6-[2-Fluor-1-(fluormethyl)ethoxy]-2,4-dioxo-3-(chinolin-7-ylmethyl)-3,4-dihydrochinazolin-1(2H)-yl}piperidin-1-carbaldehyd
Nr. 218: 4-{6-[2-Fluor-1-(fluormethyl)ethoxy]-3-[(6-methoxynaphth-2-yl)methyl]-2,4-dioxo-3,4-dihydrochinazolin-1(2H)-yl}piperidin-1-carbaldehyd
Nr. 223: 4-{3-[4-(1H-Benzimidazol-1-yl)benzyl]-6-[2-fluor-1-(fluormethyl)ethoxy]-2,4-dioxo-3,4-dihydrochinazolin-1(2H)-yl}piperidin-1-carbaldehyd
Nr. 224: 4-{6-[2-Fluor-1-(fluormethyl)ethoxy]-3-[3-methoxy-4-(2-methylpropoxy)benzyl]-2,4-dioxo-3,4-dihydrochinazolin-1(2H)-yl}piperidin-1-carbaldehyd
Nr. 226: 4-{6-[2-Fluor-1-(fluormethyl)ethoxy]-3-[3-methoxy-4-(tetrahydrofuran-3-yloxy)benzyl]-2,4-dioxo-3,4-dihydrochinazolin-1(2H)-yl}piperidin-1-carbaldehyd
Nr. 228: 4-[3-{4-[(1-Benzylpyrrolidin-3-yl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 230: 4-[3-(1-Benzothiophen-5-ylmethyl)-6-[2-fluor-1-(fluormethyl)ethoxy]-2,4-dioxo-3,4-dihydrochinazolin-1(2H)-yl]piperidin-1-carbaldehyd
Nr. 232: 4-{6-[2-Fluor-1-(fluormethyl)ethoxy]-3-[3-methoxy-4-(1-methylethoxy)benzyl]-2,4-dioxo-3,4-dihydrochinazolin-1(2H)-yl}piperidin-1-carbaldehyd
Nr. 233: 4-[3-(3,4-Dimethoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 234: 4-[3-{4-[(1-Acetylpyrrolidin-3-yl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 239: 4-[3-{4-[(4-Fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 240: 4-[3-{4-[(4-Chlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 242: 4-[3-{4-[(3-Chlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 243: 4-{6-[2-Fluor-1-(fluormethyl)ethoxy]-2,4-dioxo-3-(3-(thiophen-3-yl)benzyl)-3,4-dihydrochinazolin-1(2H)-yl}piperidin-1-carbaldehyd Nr. 245: 4-[3-(4-Ethoxy-3-methoxybenzyl)-6-(2-hydroxyethoxy)-2,4-dioxo-3,4-dihydrochinazolin-1(2H)-yl]piperidin-1-carbaldehyd
Nr. 246: 4-[3-{4-[2-(2,3-Dihydro-1H-indol-1-yl)-2-oxoethoxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 250: 4-[3-{4-[(3,4-Dichlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 251: 4-{6-[2-Fluor-1-(fluormethyl)ethoxy]-3-[3-methoxy-4-(2-oxo-2-(piperidin-1-yl)ethoxy)benzyl]-2,4-dioxo-3,4-dihydrochinazolin-1(2H)-yl}piperidin-1-carbaldehyd
Nr. 254: 4-{3-[3-Ethoxy-4-(thiophen-2-ylmethoxy)benzyl]-6-[2-fluor-1-(fluormethyl)ethoxy]-2,4-dioxo-3,4-dihydrochinazolin-1(2H)-yl}piperidin-1-carbaldehyd
Nr. 258: 4-[3-(3,4-Dimethoxybenzyl)-6-[2-fluor-1-(hydroxymethyl)ethoxy]-2,4-dioxo-3,4-dihydrochinazolin-1(2H)-yl]piperidin-1-carbaldehyd
Nr. 263: (2R)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]propansäure
Nr. 264: 4-{6-[2-Fluor-1-(fluormethyl)ethoxy]-3-[(1-methyl-3-(thiophen-2-yl)-1H-pyrazol-5-yl)methyl]-2,4-dioxo-3,4-dihydrochinazolin-1(2H)-yl}piperidin-1carbaldehyd
Nr. 270: 4-{6-[2-Fluor-1-(fluormethyl)ethoxy]-3-[4-(5-methyl-1,2,4-oxadiazol-3-yl)benzyl]-2,4-dioxo-3,4-dihydrochinazolin-1(2H)-yl}piperidin-1-carbaldehyd
Nr. 275: 4-{6-[2-Fluor-1-(fluormethyl)ethoxy]-2,4-dioxo-3-(4-(pyrimidin-5-yl)benzyl)-3,4-dihydrochinazolin-1(2H)-yl}piperidin-1-carbaldehyd
Nr. 276: 4-{6-[2-Fluor-1-(fluormethyl)ethoxy]-3-[(1-methyl-3-phenyl-1H-pyrazol-5-yl)methyl]-2,4-dioxo-3,4-dihydrochinazolin-1(2H)-yl}piperidin-1-carbaldehyd
Nr. 278: 4-{6-[2-Fluor-1-(fluormethyl)ethoxy]-2,4-dioxo-3-{[6-(1H-pyrazol-l-yl)pyridin-3-yl]methyl}-3,4-dihydrochinazolin-1(2H)-yl}piperidin-1-carbaldehyd
Nr. 279: 4-{6-[2-Fluor-1-(fluormethyl)ethoxy]-2,4-dioxo-3-[(2-(thiophen-2-yl)pyrimidin-5-yl)methyl]-3,4-dihydrochinazolin-1(2H)-yl}piperidin-1-carbaldehyd
Nr. 280: 4-{6-[2-Fluor-1-(fluormethyl)ethoxy]-3-[4-(1-methyl-1H-pyrazol-3-yl)benzyl]-2,4-dioxo-3,4-dihydrochinazolin-1(2H)-yl}piperidin-1-carbaldehyd
Nr. 282: 4-{6-[2-Fluor-1-(fluormethyl)ethoxy]-3-[4-(3-methyl-1,2,4-oxadiazol-5-yl)benzyl]-2,4-dioxo-3,4-dihydrochinazolin-1(2H)-yl}piperidin-1-carbaldehyd
Nr. 283: [2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]essigsäure
Nr. 285: 4-{6-[2-Fluor-1-(fluormethyl)ethoxy]-2,4-dioxo-3-(thieno[2,3-b]pyridin-2-ylmethyl)-3,4-dihydrochinazolin-1(2H)-yl}piperidin-1-carbaldehyd
Nr. 286: 4-{6-[2-Fluor-1-(fluormethyl)ethoxy]-2,4-dioxo-3-[(6-phenylpyridin-3-yl)methyl]-3,4-dihydrochinazolin-1(2H)-yl}piperidin-1-carbaldehyd
Nr. 287: 4-{6-[2-Fluor-1-(fluormethyl)ethoxy]-3-[(6-(morpholin-4-yl)pyridin-3-yl)methyl]-2,4-dioxo-3,4-dihydrochinazolin-1(2H)-yl}piperidin-1-carbaldehyd
Nr. 289: 4-{6-[2-Fluor-1-(fluormethyl)ethoxy]-2,4-dioxo-3-[(6-(thiophen-2-yl)pyridin-3-yl)methyl]-3,4-dihydrochinazolin-1(2H)-yl}piperidin-1-carbaldehyd
Nr. 292: 4-{6-[2-Fluor-1-(fluormethyl)ethoxy]-3-[(1-methyl-5-phenyl-1H-pyrazol-3-yl)methyl]-2,4-dioxo-3,4-dihydrochinazolin-1(2H)-yl}piperidin-1-carbaldehyd
Nr. 294: 4-({6-[2-Fluor-1-(fluormethyl)ethoxy]-1-(1-formylpiperidin-4-yl)-2,4-dioxo-1,4-dihydrochinazolin-3(2H)-yl}methyl)biphenyl-2-carbonitril
Nr. 295: (2R)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methylpropanamid Nr. 297: 4-{7-Fluor-6-[2-fluor-1-(fluormethyl)ethoxy]-2,4-dioxo-3-(4-(thiophen-2-yl)benzyl)-3,4-dihydrochinazolin-1(2H)-yl}piperidin-1-carbaldehyd
Nr. 298: 4-{6-[2-Fluor-1-(fluormethyl)ethoxy]-3-[3-methoxy-4-(morpholin-4-ylmethyl)benzyl]-2,4-dioxo-3,4-dihydrochinazolin-1(2H)-yl}piperidin-1-carbaldehyd
Nr. 299: 4-{6-[2-Fluor-1-(fluormethyl)ethoxy]-3-[3-methoxy-4-(piperidin-1-ylmethyl)benzyl]-2,4-dioxo-3,4-dihydrochinazolin-1(2H)-yl}piperidin-1-carbaldehyd
Nr. 300: 4-[3-{4-[(3,4-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 301: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-ethylacetamide
Nr. 302: (2S)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]propansäure
Nr. 305: 4-{6-[2-Fluor-1-(fluormethyl)ethoxy]-3-(3-methoxy-4-{[(3R)-2-oxo-1-phenylpyrrolidin-3-yl]oxy}benzyl)-2,4-dioxo-3,4-dihydrochinazolin-1(2H)-yl}piperidin-1-carbaldehyd
Nr. 306: 4-{3-[4-(Cyclobutylmethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 307: 4-{3-[4-(Benzyloxy)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 308: 4-{7-Fluor-6-[2-fluor-1-(fluormethyl)ethoxy]-3-(4-hydroxy-3-methoxybenzyl)-2,4-dioxo-3,4-dihydrochinazolin-1(2H)-yl}piperidin-1-carbaldehyd
Nr. 309: 4-{3-[4-(Cyclopropylmethoxy)-3-methoxybenzyl]-7-fluor-6-[2-fluor-1 (fluormethyl)ethoxy]-2,4-dioxo-3,4-dihydrochinazolin-1(2H)-yl}piperidin-1-carbaldehyd
Nr. 310: 4-{7-Fluor-6-[2-fluor-1-(fluormethyl)ethoxy]-3-[3-methoxy-4-(2-methylpropoxy)benzyl]-2,4-dioxo-3,4-dihydrochinazolin-1(2H)-yl}piperidin-1-carbaldehyd
Nr. 311: 4-{7-Fluor-6-[2-fluor-1-(fluormethyl)ethoxy]-3-[3-methoxy-4-(1-methylethoxy)benzyl]-2,4-dioxo-3,4-dihydrochinazolin-1(2H)-yl}piperidin-1-carbaldehyd
Nr. 312: 4-[3-(4-Ethoxy-3-methoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 315: 4-{7-Fluor-6-[2-fluor-1-(fluormethyl)ethoxy]-3-{[6-(3-methoxyphenyl)pyridin-3-yl]methyl}-2,4-dioxo-3,4-dihydrochinazolin-1(2H)-yl}piperidin-1-carbaldehyd
Nr. 316: 4-{7-Fluor-6-[2-fluor-1 (fluormethyl)ethoxy]-3-{[6-(2-fluorphenyl)pyridin-3-yl]methyl}-2,4-dioxo-3,4-dihydrochinazolin-1(2H)-yl}piperidin-1-carbaldehyd
Nr. 317: 4-{7-Fluor-6-[2-fluor-1-(fluormethyl)ethoxy]-3-{[6-(4-fluorphenyl)pyridin-3-yl]methyl}-2,4-dioxo-3,4-dihydrochinazolin-1(2H)-yl}piperidin-1-carbaldehyd
Nr. 318: 4-{7-Fluor-6-[2-fluor-1-(fluormethyl)ethoxy]-3-{[6-(4-methoxyphenyl)pyridin-3-yl]methyl}-2,4-dioxo-3,4-dihydrochinazolin-1(2H)-yl}piperidin-1-carbaldehyd
Nr. 319: 4-{7-Fluor-6-[2-fluor-1 (fluormethyl)ethoxy]-2,4-dioxo-3-[(6-(thiophen-2-yl)pyridin-3-yl)methyl]-3,4-dihydrochinazolin-1(2H)-yl}piperidin-1-carbaldehyd
Nr. 320: 4-{3-[3-Ethoxy-4-(thiophen-2-ylmethoxy)benzyl]-7-fluor-6-[2-fluor-1-(fluormethyl)ethoxy]-2,4-dioxo-3,4-dihydrochinazolin-1(2H)-yl}piperidin-1-carbaldehyd
Nr. 321: 4-{7-Fluor-6-[2-fluor-1-(fluormethyl)ethoxy]-3-[4-(1-methyl-1H-pyrazol-3-yl)benzyl]-2,4-dioxo-3,4-dihydrochinazolin-1(2H)-yl}piperidin-1-carbaldehyd
Nr. 322: 4-{7-Fluor-6-[2-fluor-1 (fluormethyl)ethoxy]-2,4-dioxo-3-(4-(pyrimidin-5-yl)benzyl)-3,4-dihydrochinazolin-1(2H)-yl}piperidin-1-carbaldehyd
Nr. 323: 4-{7-Fluor-6-[2-fluor-1-(fluormethyl)ethoxy]-3-[(1-methyl-3-(thiophen-2-yl)-1H-pyrazol-5-yl)methyl]-2,4-dioxo-3,4-dihydrochinazolin-1(2H)-yl}piperidin-1-carbaldehyd
Nr. 324: 4-{7-Fluor-6-[2-fluor-1-(fluormethyl)ethoxy]-3-[3-methoxy-4-(2-oxo-2-(piperidin-1-yl)ethoxy)benzyl]-2,4-dioxo-3,4-dihydrochinazolin-1(2H)-yl}piperidin-1-carbaldehyd
Nr. 325: 4-[3-{4-[2-(2,3-Dihydro-1H-indol-1-yl)-2-oxoethoxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 326: 4-{7-Fluor-6-[2-fluor-1 (fluormethyl)ethoxy]-3-[4-(5-methyl-1,2,4-oxadiazol-3-yl)benzyl]-2,4-dioxo-3,4-dihydrochinazolin-1(2H)-yl}piperidin-1-carbaldehyd
Nr. 327: 4-[3-{4-[(3-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidzn-1-carbaldehyd
Nr. 328: 4-[3-{[6-(3,5-Dichlorphenyl)pyridin-3-yl]methyl}-6-[2-fluor-1-(fluormethyl)ethoxy]-2,4-dioxo-3,4-dihydrochinazolin-1(2H)-yl]piperidin-1-carbaldehyd
Nr. 329: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)biphenyl-2-carbonitril
Nr. 330: 4-{7-Fluor-6-[2-fluor-1-(fluormethyl)ethoxy]-2,4-dioxo-3-[4-(1H-pyrazol-1-yl)benzyl]-3,4-dihydrochinazolin-1(2H)-yl}piperidin-1-carbaldehyd
Nr. 331: 4-{7-Fluor-6-[2-fluor-1-(fluormethyl)ethoxy]-3-{[6-(3-fluorphenyl)pyridin-3-yl]methyl}-2,4-dioxo-3,4-dihydrochinazolin-1(2H)-yl}piperidin-1-carbaldehyd
Nr. 332: 3-[5-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)pyridin-2-yl]benzonitril
Nr. 333: 4-[3-(3,4-Diethoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 334: 4-[3-{4-[(4-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 335: 4-{7-Fluor-6-[2-fluor-1-(fluormethyl)ethoxy]-3-[4-(morpholin-4-ylmethyl)benzyl]-2,4-dioxo-3,4-dihydrochinazolin-1(2H)-yl}piperidin-1-carbaldehyd
Nr. 336: 4-{7-Fluor-6-[2-fluor-1-(fluormethyl)ethoxy]-2,4-dioxo-3-{[6-(1H-pyrazol-1-yl)pyridin-3-yl]methyl}-3,4-dihydrochinazolin-1(2H)-yl}piperidin-1-carbaldehyd
Nr. 337: 4-{7-Fluor-6-[2-fluor-1-(fluormethyl)ethoxy]-3-(4-(morpholin-4-yl)benzyl)-2,4-dioxo-3,4-dihydrochinazolin-1(2H)-yl}piperidin-1-carbaldehyd
Nr. 338: 4-{7-Fluor-6-[2-fluor-1-(fluormethyl)ethoxy]-3-(3-methoxy-4-propoxybenzyl)-2,4-dioxo-3,4-dihydrochinazolin-1(2H)-yl}piperidin-1-carbaldehyd
Nr. 339: 4-{3-[4-(1H-Benzimidazol-1-yl)benzyl]-7-fluor-6-[2-fluor-1-(fluormethyl)ethoxy]-2,4-dioxo-3,4-dihydrochinazolin-1(2H)-yl}piperidin-1-carbaldehyd
Nr. 340: 5-({7-Fluor-6-[2-fluor-1-(fluormethyl)ethoxy]-2-(1-formylpiperidin-4-yl)-2,4-dioxo-1,4-dihydrochinazolin-3(2H)-yl}methyl)-2-methoxybenzonitril
Nr. 341: 3-(3,4-Dimethoxybenzyl)-6-[2-fluor-1-(fluormethyl)ethoxy]-1-(1-formylpiperidin-4-yl)-2,4-dioxo-1,2,3,4-tetrahydrochinazolin-7-carbonitril
Nr. 342: 4-[3-(4-Brombenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 343: 4-[3-{4-[(3,4-Dichlorbenzyl)oxy]-3-(2-methoxyethoxy)benzyl}-7-fluor-6-[2-fluor-1-(fluormethyl)ethoxy]-2,4-dioxo-3,4-dihydrochinazolin-1(2H)-yl]piperidin-1-carbaldehyd
Nr. 344: 4-{3-[4-(Benzyloxy)benzyl]-7-fluor-6-[2-fluor-1-(fluormethyl)ethoxy]-2,4-dioxo-3,4-dihydrochinazolin-1(2H)-yl}piperidin--carbaldehyd
Nr. 345: 4-[3-{4-[(3,4-Dichlorbenzyl)oxy]-3-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 349: 4-[3-{4-[(3,4-Dichlorbenzyl)oxy]-3-(2-fluorethoxy)benzyl}-7-fluor-6-[2-fluor-1-(fluormethyl)ethoxy]-2,4-dioxo-3,4-dihydrochinazolin-1(2H)-yl]piperidin-1-carbaldehyd
Nr. 350: 4-[3-{4-[(2-Chlor-4-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 351: 4-[3-{4-[(2,4-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 352: 4-[3-{4-[(2-Chlor-6-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 353: 4-[3-{4-[(2,6-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 354: 4-[3-{4-[(2-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 355: 4-[7-Fluor-3-{4-[(2-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 357: 2-[(3,4-Dichlorbenzyl)oxy]-5-({7-fluor-6-[2-fluor-1-(fluormethyl)ethoxy]-1-(1-formylpiperidin-4-yl)-2,4-dioxo-1,4-dihydrochinazolin-3(2H)-yl}methyl)benzonitril
Nr. 358: 4-[3-{4-[(3,4-Dichlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 360: 4-{7-Fluor-6-[2-fluor-1-(fluormethyl)ethoxy]-2,4-dioxo-3-[4-(2-phenylethyl)benzyl]-3,4-dihydrochinazolin-1(2H)-yl}piperidin-1-carbaldehyd
Nr. 362: 4-[3-{4-[(4,5-Dichlor-2-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 369: 4-[3-{4-[(4-Chlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 371: 4-[3-{3-Chlor-4-[(4-chlorbenzyl)oxy]-5-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 373: 4-[3-{3-Chlor-4-[(2,4-dichlorbenzyl)oxy]-5-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 375: 4-[7-Fluor-3-{4-[(4-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 376: 4-[3-{4-[(3,5-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 377: 4-[3-(4-{[4-Chlor-3-(trifluormethyl)benzyl]oxy}-3-methoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 379: 4-[3-{4-[(3-Chlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 380: 4-[3-{4-[(3,5-Difluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 381: 4-{3-[4-(Benzyloxy)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 382: 4-[3-{4-[(3-Chlor-5-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 383: 4-{7-Fluor-6-[2-fluor-1-(fluormethyl)ethoxy]-3-(3-methoxy-4-{[4-(trifluormethyl)benzyl]oxy}benzyl)-2,4-dioxo-3,4-dihydrochinazolin-1(2H)-yl}piperidin-1-carbaldehyd
Nr. 384: 4-[3-{4-[(2,5-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 385: 4-{[4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenoxy]methyl}benzonitril
Nr. 386: 3-{[4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenoxy]methyl}benzonitril
Nr. 387: 4-[3-{4-[(4-Chlor-2-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 388: 4-[3-{4-[1-(3,4-Dichlorphenyl)ethoxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 389: 4-{7-Fluor-6-[2-fluor-1-(fluormethyl)ethoxy]-3-{4-[(3-hydroxybenzyl)oxy]-3-methoxybenzyl}-2,4-dioxo-3,4-dihydrochinazolin-1(2H)-yl}piperidin-1-carbaldehyd
Nr. 390: 4-[7-Fluor-3-{4-[(3-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 391: 4-[3-{4-[(3,4-Difluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 392: 4-{3-[4-(5,6-Dichlor-1H-benzimidazol-1-yl)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 393: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)phenyl-3,4-dichlorbenzolsulfonat
Nr. 394: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenyl-3,4-dichlorbenzolsulfonat
Nr. 403: 3,4-Dichlor-N-[4-({7-fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenyl]benzamid
in Basen- oder Säureadditionssalzform.
